# EUROPEAN PATENT SPECIFICATION

(11) **EP 1 495 009 B1**
(45) Date of publication and mention of the grant of the patent: **12.03.2008**
(21) Application number: 03723865.6
(22) Date of filing: 01.04.2003
(51) Int. Cl.: C07D 295/18, C07C 261/04, C07D 217/06, A61K 31/275, A61K 31/535, A61P 19/10, A61P 29/00

(54) **CYANAMIDES USEFUL AS REVERSIBLE INHIBITORS OF CYSTEINE PROTEASES**
CYANAMIDE ALS REVERSIBLE HEMMER VON CYSTEIN PROTEASEN
CYANAMIDES UTILES EN TANT QU'INHIBITEURS REVERSIBLES DES CYSTEINE PROTEASES

(30) Priority: 05.04.2002 US 370368 P
(43) Date of publication of application: 12.01.2005
(73) Proprietor: Boehringer Ingelheim Pharmaceuticals Inc., Ridgefield, Connecticut 06877-0368 (US)
(72) Inventor: LIU, Weimin, Boehringer Ingelheim Pharmaceut. Inc., Ridgefield, CT 06877-0368 (US); GILMORE, Thomas A., Millennium Pharmaceuticals, Cambridge, MA 02139 (US); HICKEY, E.R., Boehringer Ingelheim Pharmaceut. Inc, Ridgefield, CT 06877-0368 (US); NEMOTO, P.A., Boehringer Ingelheim Pharmaceut. Inc, Ridgefield, CT 06877-0368 (US); SPERO, D.M., Boehringer Ingelheim Pharmaceut. Inc., Ridgefield, CT 06877-0368 (US)
(74) Representative: Hammann, Heinz
(86) International application number: PCT/US2003/009852
(87) International publication number: WO 2003/086325

(56) References cited:
- WO-A-00/49007
- WO-A-01/77073

## Description

### FIELD OF THE INVENTION

This invention relates to cyanamide compounds useful as reversible inhibitors of the cysteine proteases cathepsins S, K, F, L and B. Certain embodiments described are preferable for inhibition of cathepsin K. The compounds are therefore useful in the treatment of cysteine protease mediated diseases including osteoporosis, autoimmune diseases and other related diseases. The invention also relates to processes for preparing such compounds and pharmaceutical compositions comprising them.

### BACKGROUND OF THE INVENTION

Cathepsin K and cathepsin S are members of the papain family, within the papain superfamily of cysteine proteases. The papain family is the largest group of cysteine proteases and includes proteases such as cathepsins B, H, K, L, O and S. (A.J. Barrett et al., 1996, Perspectives in Drug Discovery and Design, 6, 1). The cysteine proteases have important roles in human biology and diseases including osteoporosis, chronic inflammation and immune disorders, atherosclerosis, and emphysema (H.A. Chapman et al., 1997, Ann. Rev. Physiol., 59, 63). Cysteine proteases are also involved in the pathogenesis of some infectious diseases, including malaria (A. Semenov et al., Antimicrobial Agents and Chemotherapy, 1998, 42, 2254) and Chagas' disease, (J.C. Engel et al., J. Exp. Med., 1998, 188, 725). Bacterial cysteine proteases contribute to the pathogenesis of gingivitis (J. Potempa et al., Perspectives in Drug Discovery and Design, 1994, 2, 445).

Cathepsin K has been found to be highly expressed in osteoclasts, cells involved in bone resorption (F.H. Drake et al., J. Biol. Chem., 1996, 271, 12511). Collagen and osteonectin, two protein components of bone matrix have been found to be substrates of activated cathepsin K (M.J. Bossard et al., J. Biol. Chem., 1996, 271, 12517). Inhibitors of cathepsin K have been shown to have anti-resorptive activity *in vitro* and *in vivo* (S.K. Thompson et al., Proc. Natl. Acad. Sci. USA, 1997, 94, 14249). The essential role of cathepsin K in bone resorption has also been confirmed in cells and organisms lacking this protease. At the cellular level, cathepsin K deficient osteoclasts, when tested for functional activity on dentine, produced fewer resorption pits as compared to wild-type osteoclasts (P. Saftig et al., Proc Natl Acad Sci U S A 1998, 95,13453). Cathepsin K knockout mice develop osteopetrosis, a disease characterized by an increase in bone mass, due to a deficit in matrix degradation but not demineralization of hydroxyapatite. These knockout mice displayed osteopetrosis of the long bones and vertebrae as well as abnormal joint morphology (M. Gowen et al., J. Bone Miner Res. 1999, 14, 1654). The phenotype of the cathepsin K knockout mice resembles the human genetic disorder pycnodysostosis which is due to a mutation in the cathepsin K gene (W-S Hu et al., Journal of Clinical Investigation, 1999, 103, 731; B. D. Gelb et al, Science, 1996, 273, 1236). Patients with this disease have short, dense bones. These and other findings suggest that cathepsin K may play an important role in diseases involving bone resorption, excessive bone loss or cartilage or bone matrix degradation including osteoporosis (D. S Yamashita et al, Current Pharmaceutical Design, 2000, 6, 1), Gaucher disease (M. T. Moran et al, Blood, 2000, 96, 1969), Paget's disease, gingivitis, and periodontitis (G. A. Rodan et al, Science, 2000, 289, 1508), and rheumatoid arthritis (K. M. Hummel et al, J. Rheumatol., 1998, 25, 1887) (also see for example H. A. Chapman et al, Annu. Rev. Physiol., 1997, 59, 63; M. Gowen, Exp. Opin. Invest. Drugs, 1997, 6, 1199; W.W. Smith et al., Exp. Opin. Ther. Patents, 1999, 9, 683).

The inhibition of cathepsin K has been described by B. D. Gelb et al (U.S. 5,830,850) as a method to ameliorate symptoms caused by bone resorption disorders, including osteoporosis, arthritides and periodontal disease, and damage caused by macrophage-mediated inflammatory processes. Studies in breast cancer research have shown that invading breast cancer cells have expressed low levels of Cathepsin K suggesting that these tumor cells may be able to directly resorb bone by the release of Cathepsin K. Inhibition of Cathepsin K may play a role in the metastatic potential and course of the disease (A. J. Littlewood-Evans et al, Cancer Research, 1997, 57, 5386). Increases in bone resorption and demineralization of bone are skeletal complications associated with many cancers and with bone metastases of breast and prostate tumors (G. A. Rodan et al, Science, 2000, 289, 1508). Cathepsin K has also been observed in giant cell aortitis suggesting that disorders associated with excessive elastin degradation such as lymphangiomyomatosis, vascular inflammation, and cardiovascular disease such as atherosclerosis may be attenuated with Cathepsin K inhibitors (H. A. Chapman et al, Annu. Rev. Physiol., 1997, 59, 63; D. S Yamashita et al, Current Pharmaceutical Design, 2000, 6, 1; G. K. Sukhova et al, J. Clin. Invest., 1998, 102, 576).

Cathepsin S plays a key role in regulating antigen presentation and immunity (H.A. Chapman, 1998, Current Opinion in Immunology, 10, 93; R. J. Riese et al., 1998, J. Clin. Invest., 101, 2351; R.J. Riese et al., 1996, Immunity, 4, 357). Cathepsin S deficient mice have impaired invariant chain degradation resulting in decreased antigen presentation and germinal center formation, and diminished susceptibility to collagen-induced arthritis indicating the therapeutic potential for a cathepsin S inhibitor (G. Shi et al., 1999, Immunity, 10, 197; T.Y. Nakagawa et al, 1999, Immunity, 10, 207).

Control of antigen-specific immune responses has long been desirable as a useful therapy for autoimmune diseases. Such diseases include Crohn's disease, rheumatoid arthritis, and Multiple Sclerosis, as well as other T-cell-mediated immune responses (C. Janeway and P. Travers, 1996, Immunobiology, The Immune System in Health and Disease, Chapter 12). Furthermore, cathepsin S, which has broad pH specificity, has been implicated in a variety of other diseases involving extracellular proteolysis, such as Alzheimer's disease (U. Muller-Ladner et al., 1996, Perspectives in Drug Discovery and Design, 6, 87), atherosclerosis (G.K. Sukhova et al., 1998, J. Clin. Invest., 102, 576) and endometriosis (WO 9963115, 1999). A cathepsin S inhibitor has been found to block the rise in IgE titers and eosinophil infiltration in the lung in a mouse model of pulmonary hypersensitivity, suggesting that cathepsin S may be involved in asthma (R.J. Riese et al., J. Clin. Investigation, 1998, 101, 2351).

Another cysteine protease, cathepsin F has been found in macrophages and is also involved in antigen processing. It has been postulated that cathepsin F in stimulated lung macrophages and possibly other antigen presenting cells could play a role in airway inflammation (G.-P. Shi et al., J. Exp. Med., 2000, 191, 1177).

Cysteine proteases are characterized by having a cysteine residue at the active site which serves as a nucleophile. The active site also contains a histidine residue. The imidazole ring on the histidine serves as a base to generate a thiolate anion on the active site cysteine, increasing its nucleophilicity. When a substrate is recognized by the protease, the amide bond to be cleaved is directed to the active site, where the thiolate attacks the carbonyl carbon forming an acyl-enzyme intermediate and cleaving the amide bond, liberating an amine. Subsequently, water cleaves the acyl-enzyme species regenerating the enzyme and liberating the other cleavage product of the substrate, a carboxylic acid.

Inhibitors of cysteine proteases contain a functionality that can react reversibly or irreversibly with the active site cysteine. Examples of reactive functionalities that have been described (D. Rasnick, 1996, Perspectives in Drug Discovery and Design, 6, 47) on cysteine protease inhibitors include peptidyl diazomethanes, epoxides, monofluoroalkanes and acyloxymethanes, which irreversibly alkylate the cysteine thiol. Other irreversible inhibitors include Michael acceptors such as peptidyl vinyl esters and other carboxylic acid derivatives (S. Liu et al., J. Med Chem., 1992, 35, 1067) and vinyl sulfones (J.T. Palmer et al., 1995, J. Med Chem., 38, 3193).

Reactive functionalities that form reversible complexes with the active site cysteine include peptidyl aldehydes (R.P. Hanzlik et al., 1991, Biochim. Biophys. Acta., 1073, 33), which are non-selective, inhibiting both cysteine and serine proteases as well as other nucleophiles. Peptidyl nitriles (R.P. Hanzlik et al., 1990, Biochim. Biophys. Acta., 1035, 62) are less reactive than aldehydes and therefore more selective for the more nucleophilic cysteine proteases. Various reactive ketones have also been reported to be reversible inhibitors of cysteine proteases (D. Rasnick, 1996, ibid). In addition to reacting with the nucleophilic cysteine of the active site, reactive ketones may react with water, forming a hemiketal which may act as a transition state inhibitor.

Inhibitors of cathepsin K have been reported in the literature. D.S. Yamashita et al., (J. Am. Chem. Soc., 1997, 119, 11351) described 1,3-diamino-2-propanone inhibitors. S.K. Thompson et al. (Proc. Natl. Acad. Sci. USA, 1997, 94, 14249) described bis-aza analogs of these propanones as well as an aza-thiazole derivative. Introduction of a conformational constraint to the 1,3-diamino-2-propanones has led to 3-amido-pyrrolidin-4-one derivatives, 4-amido-piperidin-3-one derivatives, and eventually to azapanone-based inhibitors of Cathepsin K, as reported by R. W. Marquis et al. (J. Med. Chem. 1998, 41, 3563; J. Med. Chem. 2001, 44, 725; J. Med. Chem. 2001, 44, 1380). R.W. Marquis et al. (Bioorg. and Med. Chem. Letters, 1999, 7, 581) described peptidic alkoxymethylketones and thiomethylketones as cathepsin K inhibitors. Peptidyl vinyl sulfone cathepsin K inhibitors were described by L. Xia et al. (Biological Chem., 1999, 380, 679). Peptide aldehyde inhibitors of cathepsin K were reported by B. J. Votta et al. (J. Bone & Mineral Res., 1997, 12, 1396). J.-P. Falgueyret et al. (J. Med. Chem. 2001, 44, 94) described non-peptidic cyanamides as potent Cathepsin K inhibitors. T. Gamble et al. (49th Annual American Society for Mass Spectrometry Conference, May 27-31, 2001, Chicago, IL, WO 01/77073) described *in vitro* metabolism studies on cyanamide-containing Cathepsin K inhibitors. D. F. Veber has discussed numerous inhibitors of Cathepsin K including the following: 1,5-diacylcarbohydrazides (Biochemistry, 1999, 38, 15893; J. Med. Chem. 1998, 41, 3923), conformationally constrained 1,3-diamino ketones (J. Med. Chem. 1998, 41, 3563), and 1,3-bis(acylamino)-2-butanones, (J. Combinatorial Chem. 1999, 1, 207; J. Am. Chem. Soc. 1998, 120, 9114; J. Am. Chem. Soc. 1997, 119, 11351).

Examples of cathepsin S inhibitors have been reported. J.L. Klaus et al. (WO 9640737) described reversible inhibitors of cysteine proteases including cathepsin S, containing an ethylene diamine. In US Patent No. 5,776,718, Palmer et al. disclosed in it's broadest generic aspect, a protease inhibitor comprising a targeting group linked through a two carbon atom chain to an electron withdrawing group (EWG). Other examples of cathepsin S inhibitors have been reported by E.T. Altmann et al, (WO 9924460, 1999) which describes dipeptide nitriles asserted to have activity as inhibitors of Cathepsins B, K, L and S. Certain acetamido acetonitrile derivatives have been disclosed by Tucker et al. (WO 00/49007) as inhibitors of cathepsin S and L.

Certain other cathepsin inhibitors have recently been disclosed, for example by Marquis et al. (WO 00/38687 and WO 00/39115), Altmann et al. (WO 00/48993), Buysse et al. (WO 00/55124), Singh et al. (WO 00/59881) and Cowen (WO 01/87828). Cathepsin inhibitors containing a cyclic cyanamide functionality were described by P. Prasit et al. (WO 01/77073). However, this WO publication does not disclose or suggest any compounds possessing the acyclic cyanamide moiety, as in the novel compounds according to the present invention.

Even though the novel compounds of the present invention contain functionalized N-substituted glycine groups, which also exist in classical peptoids, they should not be classified as classical peptoids because classical peptoids lack the acyclic cyanamide moiety which is present in the novel compounds of the present invention. Several reports of biologically active compounds containing the cyanamide moiety have appeared. For example, M.A. Patane et al. (U.S. 5,977,115) generically disclose cyanamide-containing compounds as alpha 1A adrenergic receptor antagonists. Opiate receptor ligands including some containing a cyanamide functionality were generically described by D.C. Spellmeyer et al. (U.S. 5,536,853). F. Clemence et al. (U.S. 5,190,974) describe analgesic, psychotropic and enkephalinase-inhibiting compounds, some containing the cyanamide moiety.

A reversible inhibitor presents a more attractive therapy than irreversible inhibitors. Covalent modification of an enzyme by an irreversible inhibitor could potentially generate an antibody response by acting as a hapten. Furthermore, any toxic effects resulting from inactivation of the target enzyme would be mitigated by reversible inhibitors, and could be easily remedied by modified or lower dosing.

In light of the above, there is a clear need for compounds which reversibly and selectively inhibit cysteine proteases such as cathepsin K and cathepsin S for indications in which these proteases exacerbate disease.

### SUMMARY OF THE INVENTION

It is therefore an object of this invention to provide novel compounds according to the following formula (I): 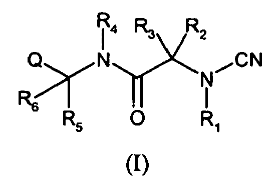 wherein the variables Q and R₁ to R₆ are as described herein which reversibly inhibit the cysteine proteases, such as cathepsins K, S, F, L and B. It is a further object of the invention to provide methods for treating diseases and pathological conditions exacerbated by these cysteine proteases such as, but not limited to rheumatoid arthritis, multiple sclerosis, and other autoimmune diseases, osteoporosis, asthma, Alzheimer's disease, atherosclerosis and endometriosis. It is yet a further object of the invention to provide novel processes for preparation of the above-mentioned novel compounds.

### DETAILED DESCRIPTION OF THE INVENTION

A proposed mechanism of action of the cysteine protease inhibitors of this invention is that the inhibitors contain a functionality that can react (reversibly or irreversibly) with the active site cysteine. The reactive functionality is attached to a peptide or peptide mimic that can be recognized and accommodated by the protease surrounding the active site. The nature of both the reactive functionality and the remaining portion of the inhibitor determine the degree of selectivity and potency toward a particular protease.

Given the similarity of the active sites in cysteine proteases, it may be anticipated that a given class of inhibitors might have activity against more that one cysteine protease. It may also be expected that due to structural differences between individual cysteine proteases, different compounds of the invention may have different inhibitory potencies against different cysteine proteases. Thus some of the compounds of the invention may also be expected to be most effective in treating diseases mediated by cysteine proteases that they inhibit most potently. The activity of particular compounds disclosed herein against cysteine proteases cathepsin K, S, F, L and B may be determined by the screens described in the section entitled "Assessment of Biological Properties."

### Definition of Terms and Conventions Used

Terms not specifically defined herein should be given the meanings that would be given to them by one of skill in the art in light of the disclosure and the context. As used in the specification and appended claims, however, unless specified to the contrary, the following terms have the meaning indicated and the following conventions are adhered to.

### A. Chemical Nomenclature, Terms, and Conventions

In the groups, radicals, or moieties defined below, the number of carbon atoms is often specified preceding the group, for example, C1-10 alkyl means an alkyl group or radical having 1 to 10 carbon atoms. The term "lower" applied to any carbon-containing group means a group containing from 1 to 8 carbon atoms, as appropriate to the group (i.e., a cyclic group must have at least 3 atoms to constitute a ring). In general, for groups comprising two or more subgroups, the last named group is the radical attachment point, for example, "alkylaryl" means a monovalent radical of the formula Alk-Ar-, while "arylalkyl" means a monovalent radical of the formula Ar-Alk- (where Alk is an alkyl group and Ar is an aryl group). Furthermore, the use of a term designating a monovalent radical where a divalent radical is appropriate shall be construed to designate the divalent radical and *vice versa.* Unless otherwise specified, conventional definitions of terms control and conventional stable atom valences are presumed and achieved in all formulas and groups.

The term "alkyl" refers to a saturated aliphatic hydrocarbon radical or a mono- or polyunsaturated aliphatic hydrocarbon radical. The mono- or polyunsaturated aliphatic hydrocarbon radical contains at least one double or triple bond, respectively. "Alkyl" refers to both branched and unbranched alkyl groups, each optionally partially or fully halogenated. Examples of "alkyl" include alkyl groups which are straight chain alkyl groups containing from one to eight carbon atoms and branched alkyl groups containing from three to eight carbon atoms. Other examples include alkyl groups which are straight chain alkyl groups containing from one to six carbon atoms and branched alkyl groups containing from three to six carbon atoms. This term is exemplified by groups such as methyl, ethyl, *n*-propyl, 1-methylethyl (isopropyl), *n*-butyl, *n*-pentyl, 1,1-dimethylethyl (*tert*-butyl), and the like. It may be abbreviated "Alk". It should be understood that any combination term using an "alk" or "alkyl" prefix refers to analogs according to the above definition of "alkyl". For example, terms such as "alkoxy", "alkythio" refer to alkyl groups linked to a second group via an oxygen or sulfur atom, respectively. "Alkanoyl" refers to an alkyl group linked to a carbonyl group (C=O).

The term "alkenyl" means a branched or straight-chain aliphatic hydrocarbon monovalent radical containing at least one carbon-carbon double bond. This term is exemplified by groups such as ethenyl, propenyl, *n*-butenyl, isobutenyl, 3-methylbut-2-enyl, *n*-pentenyl, heptenyl, octenyl, cyclohexylbutenyl, decenyl, and the like.

The terms "alkylcarbonyl" or "alkanoyl" mean a monovalent radical of the formula AlkC(O)-, where Alk is alkyl.

The terms "arylcarbonyl" or "aroyl" mean a monovalent radical of the formula ArC(O)-, where Ar is aryl.

The terms "alkoxycarbonyl" means a monovalent radical of the formula AlkO-C(O)-, where Alk is alkyl. Exemplary alkoxycarbonyl groups include methoxycarbonyl, ethoxycarbonyl, *tert*-butyloxycarbonyl, and the like.

The terms "aryloxycarbonyl" means a monovalent radical of the formula ArO-C(O)-, where Ar is aryl.

The terms "alkylcarbonyloxy" or "alkanoyloxy" mean a monovalent radical of the formula AlkC(O)O-, where Alk is alkyl.

The terms "arylcarbonyloxy" or "aroyloxy" mean a monovalent radical of the formula ArC(O)O-, where Ar is aryl.

The terms "alkylcarbonylamino" or "alkanoylamino" mean a monovalent radical of the formula AlkC(O)NH-, where Alk is alkyl. Exemplary alkylcarbonylamino groups include acetamido (CH₃C(O)NH-).

The terms "arylcarbonylamino" or "aroylamino" mean a monovalent radical of the formula ArC(O)NH-, wherein Ar is aryl. Exemplary arylcarbonylamino groups include benzoylamino (phenylC(O)NH-).

The terms "alkoxycarbonylamino" and "aryloxycarbonylamino" refer, respectively, to monovalent radicals of the formula AlkOC(O)NH- and ArOC(O)NH-, wherein Alk is alkyl and Ar is aryl.

The terms "alkylcarbamoyloxy" and "arylcarbamoyloxy" refer, respectively, to monovalent radicals of the formula AlkNHC(O)O- and ArNHC(O)O- wherein Alk is alkyl and Ar is aryl.

The terms "alkylsulfonylamino" and "arylsulfonylamino" refer, respectively, to monovalant radicals of the formula AlkS(O)₂NH- and ArS(O)₂NH-, wherein Alk is alkyl and Ar is aryl.

The terms "alkylaminosulfonyl" and "arylaminosulfonyl" refer, respectively, to monovalent radicals of the formula AlkNHS(O)₂- and ArNHS(O)₂- wherein Alk is alkyl and Ar is aryl.
The term "carbamoyl" means a monovalent radical of the formula NH₂C(O)-.

The term "cycloalkyl" refers to the mono- or polycyclic analogs of an alkyl group, as defined above, including fully saturated and mono- or polyunsaturated groups. Unless otherwise specified, the cycloalkyl ring may be attached at any carbon atom which results in a stable structure and, if substituted, may be substituted at any suitable carbon atom which results in a stable structure. Examples of cycloalkyl groups are saturated cycloalkyl groups containing from three to eight carbon atoms, for example, three to six carbon atoms. Exemplary cycloalkyl groups include cyclopropyl, cyclobutyl, cyclopentyl, cyclohexyl, cycloheptyl, cyclooctyl, cyclononyl, cyclodecyl, norbornane, adamantyl, tetrahydronaphthyl (tetralin), indanyl, 1-decalinyl, bicyclo[2.2.2]octanyl, 1-methylcyclopropyl, 2-methylcyclopentyl, 2-methylcyclooctyl, and the like.

The term "cycloalkyloxy" refers to a monovalent radical of the formula cycloalkyl-O-, i.e., a cycloalkyl group linked to a second group via an oxygen atom.

The term "halo" refers to a halogen radical selected from fluoro, chloro, bromo or iodo. Representative halo groups of the invention are fluoro, chloro and bromo.

The term "aryl" refers to 6-10 membered mono- or polycyclic aromatic carbocycles, for example, phenyl and naphthyl. Unless otherwise specified, the aryl ring may be attached at any carbon atom which results in a stable structure and, if substituted, may be substituted at any suitable carbon atom which results in a stable structure. The term "aryl" includes aryl groups that are optionally partially or fully halogenated.

The term "heterocycle" or "heterocyclyl" refers to a stable 4-8 membered (but preferably, 5 or 6 membered) monocyclic or 8-11 membered bicyclic heterocycle radical which may be either saturated or unsaturated, and is non-aromatic, and may be optionally partially or fully halogenated. Each heterocycle contains carbon atoms and from 1 to 4 heteroatoms independently chosen from nitrogen, oxygen and sulfur, wherein any sulfur heteroatom may optionally be oxidized and any nitrogen heteroatom may optionally be oxidized or quaternized. Unless otherwise specified, the heterocyclyl ring may be attached at any suitable heteroatom or carbon atom which results in a stable structure and, if substituted, may be substituted at any suitable heteroatom or carbon atom which results in a stable structure. Examples of "heterocycle" include radicals such as pyrrolinyl, pyrrolidinyl, pyrazolinyl, pyrazolidinyl, piperidinyl, morpholinyl, thiomorpholinyl, pyranyl, thiopyranyl, piperazinyl, indolinyl, tetrahydroquinoline, tetrahydroisoquinoline, azetidinyl, oxetanyl, dihydrobenzofuranyl, dihydrobenzothienyl, tetrahydropyranyl, tetrahydrothiopyranyl, tetrahydrofuranyl, hexahydropyrimidinyl, hexahydropyridazinyl, 1,4,5,6-tetrahydropyrimidin-2-ylamine, dihydro-oxazolyl, 1,2-thiazinanyl-1,1-dioxide, 1,2,6-thiadiazinanyl-1,1-dioxide, isothiazolidinyl-1,1-dioxide and imidazolidinyl-2,4-dione.

The term "heteroaryl" refers to a stable 5-8 membered (but preferably, 5 or 6 membered) monocyclic or 8-11 membered bicyclic aromatic heterocycle radical, each optionally partially or fully halogenated. Each heteroaryl contains carbon atoms and from 1 to 4 heteroatoms independently chosen from nitrogen, oxygen and sulfur, wherein any sulfur heteroatom may optionally be oxidized and any nitrogen heteroatom may optionally be oxidized or quaternized. Unless otherwise specified, the heteroaryl ring may be attached at any suitable heteroatom or carbon atom which results in a stable structure and, if substituted, may be substituted at any suitable heteroatom or carbon atom which results in a stable structure. Examples of "heteroaryl" include radicals such as furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, indolizinyl, indolyl, isoindolyl, benzofuranyl, benzothienyl, indazolyl, benzimidazolyl, benzothiazolyl, benzoxazolyl, benzisoxazolyl, benzisothiazolyl, purinyl, quinolizinyl, quinolinyl, isoquinolinyl, cinnolinyl, phthalazinyl, quinazolinyl, quinoxalinyl, naphthyridinyl, pteridinyl, carbazolyl, acridinyl, phenazinyl, phenothiazinyl and phenoxazinyl,

Each aryl or heteroaryl unless otherwise specified includes its partially or fully hydrogenated derivative. For example, quinolinyl may include decahydroquinolinyl and tetrahydroquinolinyl, naphthyl may include its hydrogenated derivatives such as tetrahydronaphthyl. Other partially or fully hydrogenated derivatives of the aryl and heteroaryl compounds described herein will be apparent to one of ordinary skill in the art.

The terms "heterocyclyl", "heteroaryl" or "aryl", when associated with another moiety, unless otherwise specified shall have the same meaning as given above.

In all alkyl groups or carbon chains where one or more carbon atoms or methylene groups are optionally replaced by heteroatoms: O, S or N, it shall be understood that if N is not indicated as substituted then it is NH. It shall also be understood that the heteroatoms may replace either terminal carbon atoms or internal carbon atoms, or both, within a branched or unbranched carbon chain. Such alkyl groups can also be substituted as described herein by various groups such as oxo to result in definitions such as but not limited to: alkyl, alkylene, alkoxyalkyl, alkoxycarbonylalkyl, alkylthioalkyl, alkylthiosulfonealkyl, alkylthiosulfonylalkyl, aminoalkyl, alkylamino, mono or di-alkylaminoalkyl, mono or di-alkylamidoC1-5 alkyl. For example, a C1-5alkyl group in which one carbon atom is replace by a nitrogen and which is also substituted by an oxo (=O) can result in radicals of the formula: CH₃-NH-C(O)-CH₂CH₂-, or CH₃-C(O)-NH-CH₂CH₂-, etc. If such alkyl groups are further substituted, the substituent can replace any hydrogen atom present on any of the carbon or nitrogen atoms in the group.

As used herein above and throughout this application, "nitrogen" and "sulfur" include any oxidized form of nitrogen and sulfur and the quaternized form of any basic nitrogen, including protonated species and quaternary ammonium salt forms.

The term "compounds of the invention", and equivalent expressions, are meant to embrace compounds of Formula (I) as herein described, including the tautomers, the pharmaceutically acceptable salts, and the solvates and hydrates thereof, where the context so permits. In general and preferably, the compounds of the invention and the formulas designating the compounds of the invention are understood to only include the stable compounds thereof and exclude unstable compounds, even if an unstable compound might be considered to be literally embraced by the compound formula. Similarly, reference to intermediates, whether or not they themselves are claimed, is meant to embrace their salts and solvates, where the context so permits. For the sake of clarity, particular instances when the context so permits are sometimes indicated in the text, but these instances are purely illustrative and it is not intended to exclude other instances when the context so permits.

The terms "optional" or "optionally" mean that the subsequently described event or circumstances may or may not occur, and that the description includes instances where the event or circumstance occurs and instances in which it does not. For example, "optionally substituted aryl" means that the aryl radical may or may not be substituted and that the description includes both substituted aryl radicals and aryl radicals having no substitution.

The terms "stable compound" or "stable structure" mean a compound that is sufficiently robust to survive isolation to a useful degree of purity from a reaction mixture, and formulation into an efficacious therapeutic or diagnostic agent. For example, a compound which would have a "dangling valency" or is a "carbanion" is not a compound contemplated by the invention.

The term "substituted" means that any one or more hydrogens on an atom of a group or moiety, whether specifically designated or not, is replaced with a selection from the indicated group of substituents, provided that the atom's normal valency is not exceeded and that the substitution results in a stable compound. If a bond to a substituent is shown to cross the bond connecting two atoms in a ring, then such substituent may be bonded to any atom on the ring. When a substituent is listed without indicating the atom via which such substituent is bonded to the rest of the compound, then such substituent may be bonded via any atom in such substituent. For example, when the substituent is piperazinyl, piperidinyl, or tetrazolyl, unless specified otherwise, such piperazinyl, piperidinyl, or tetrazolyl group may be bonded to the rest of the compound of the invention via any atom in such piperazinyl, piperidinyl, or tetrazolyl group. Generally, when any substituent or group occurs more than one time in any constituent or compound, its definition on each occurrence is independent of its definition at every other occurrence. Thus, for example, if a group is shown to be substituted with 0 to 2 R, then such group is optionally substituted with up to two R groups and R at each occurrence is selected independently from the defined list of possible R. Such combinations of substituents and/or variables, however, are permissible only if such combinations result in stable compounds.

The yield of each of the chemical reactions described herein is expressed as a percentage of the theoretical yield.

The term "salt" means an ionic form of the parent compound or the product of the reaction between the parent compound with a suitable acid or base to make the acid salt or base salt of the parent compound. Salts of the compounds of the present invention can be synthesized from the parent compounds which contain a basic or acidic moiety by conventional chemical methods. Generally, the salts are prepared by reacting the free base or acid parent compound with stoichiometric amounts or with an excess of the desired salt-forming inorganic or organic acid or base in a suitable solvent or various combinations of solvents.

The term "pharmaceutically acceptable salt" means a salt of a compound of the invention which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use. The term includes pharmaceutically-acceptable acid addition salts and pharmaceutically-acceptable base addition salts. As the compounds of the present invention are useful in both free base and salt form, in practice, the use of the salt form amounts to use of the base form. Lists of suitable salts are found in, e.g., S.M. Birge et al., J. Pharm. Sci., 1977, 66, pp. 1-19.

The term "pharmaceutically-acceptable acid addition salt" means those salts which retain the biological effectiveness and properties of the free bases and which are not biologically or otherwise undesirable, formed with inorganic acids such as hydrochloric acid, hydrobromic acid, hydroiodic acid, sulfuric acid, sulfamic acid, nitric acid, phosphoric acid, and the like, and organic acids such as acetic acid, trichloroacetic acid, trifluoroacetic acid, adipic acid, alginic acid, ascorbic acid, aspartic acid, benzenesulfonic acid, benzoic acid, 2-acetoxybenzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, heptanoic acid, hexanoic acid, formic acid, fumaric acid, 2-hydroxyethanesulfonic acid (isethionic acid), lactic acid, maleic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, picric acid, pivalic acid, propionic acid, pyruvic acid, pyruvic acid, salicylic acid, stearic acid, succinic acid, sulfanilic acid, tartaric acid, *p*-toluenesulfonic acid, undecanoic acid, and the like.

The term "pharmaceutically-acceptable base addition salt" means those salts which retain the biological effectiveness and properties of the free acids and which are not biologically or otherwise undesirable, formed with inorganic bases such as ammonia or hydroxide, carbonate, or bicarbonate of ammonium or a metal cation such as sodium, potassium, lithium, calcium, magnesium, iron, zinc, copper, manganese, aluminum, and the like. Particularly preferred are the ammonium, potassium, sodium, calcium, and magnesium salts. Salts derived from pharmaceutically-acceptable organic nontoxic bases include salts of primary, secondary, and tertiary amines, quaternary amine compounds, substituted amines including naturally occurring substituted amines, cyclic amines and basic ionexchange resins, such as methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, methylglucamine, theobromine, purines, piperazine, piperidine, N-ethylpiperidine, tetramethylammonium compounds, tetraethylammonium compounds, pyridine, *N,N*-dimethylaniline, *N*-methylpiperidine, *N*-methylmorpholine, dicyclohexylamine, dibenzylamine, *N,N*-dibenzylphenethylamine, 1-ephenamine, *N,N-*dibenzylethylenediamine, polyamine resins and the like. Particularly preferred organic nontoxic bases are isopropylamine, diethylamine, ethanolamine, trimethylamine, dicyclohexylamine, choline, and caffeine.

The term "solvate" means a physical association of a compound with one or more solvent molecules or a complex of variable stoichiometry formed by a solute (for example, a compound of Formula (I)) and a solvent, for example, water, ethanol, or acetic acid. This physical association involves varying degrees of ionic and covalent bonding, including hydrogen bonding. In certain instances, the solvate will be capable of isolation, for example, when one or more solvent molecules are incorporated in the crystal lattice of the crystalline solid. In general, the solvents selected do not interfere with the biological activity of the solute. Solvates encompasses both solution-phase and isolatable solvates. Representative solvates include hydrates, ethanolates, methanolates, and the like.

The term "hydrate" means a solvate wherein the solvent molecule(s) is/are H₂O.

The compounds of the present invention as discussed below include the free base or acid thereof, their salts, and solvates and may include oxidized sulfur atoms or quaternized nitrogen atoms in their structure, although not explicitly stated or shown, particularly the pharmaceutically acceptable forms thereof. Such forms, particularly the pharmaceutically acceptable forms, are intended to be embraced by the appended claims.

### C. Isomer Terms and Conventions

The term "isomers" mean compounds having the same number and kind of atoms, and hence the same molecular weight, but differing in respect to the arrangement or configuration of the atoms in space. The term includes stereoisomers, optical isomers and geometric isomers.

The term "optical isomer" means a stable isomer that has at least one chiral atom or restricted rotation giving rise to perpendicular dissymmetric planes (e.g., certain biphenyls, allenes, and spiro compounds) and can rotate plane-polarized light. Because asymmetric centers and other chemical structure exist in the compounds of the invention which may give rise to optical isomerism, the invention contemplates optical isomers and mixtures thereof. The compounds of the invention and their salts include asymmetric carbon atoms and may therefore exist as single stereoisomers, racemates, and as mixtures of enantiomers and diastereomers. Typically, such compounds will be prepared as a racemic mixture. If desired, however, such compounds can be prepared or isolated as pure optical isomers, i.e., as individual enantiomers or diastereomers, or as stereoisomer-enriched mixtures. Individual stereoisomers of compounds are prepared by synthesis from optically active starting materials containing the desired chiral centers or by preparation of mixtures of enantiomeric products followed by separation, such as conversion to a mixture of diastereomers followed by separation or recrystallization, chromatographic techniques, use of chiral resolving agents, or direct separation of the enantiomers on chiral chromatographic columns. Starting compounds of particular stereochemistry are either commercially available or are made by the methods described below and resolved by techniques well-known in the art.

The term "enantiomers" means a pair of optical isomers that are non-superimposable mirror images of each other.

The term "diastereoisomers" means stereoisomers which are not mirror images of each other.
The term "stereoisomers" means compounds having the same molecular formula and functional groups, but differ in the arrangment of the groups in space. The term includes enantiomers and diastereomers.

The term "racemic mixture" or "racemate" means a mixture containing equal parts of individual enantiomers.

The term "non-racemic mixture" means a mixture containing unequal parts of individual enantiomers.

The term "geometrical isomer" means a stable isomer which results from restricted freedom of rotation about double bonds (e.g., *cis*-2-butene and *trans*-2-butene) or in a cyclic structure (e.g., *cis*-1,3-dichlorocyclobutane and *trans*-1,3-dichlorocyclobutane). Because carbon-carbon double (olefinic) bonds, C=N double bonds, cyclic structures, and the like may be present in the compounds of the invention, the invention contemplates each of the various stable geometric isomers and mixtures thereof resulting from the arrangement of substituents around these double bonds and in these cyclic structures. The substituents and the isomers are designated using the *cisltrans* convention or using the *E* or *Z* system, wherein the term "*E*" means higher order substituents on opposite sides of the double bond, and the term "*Z*" means higher order substituents on the same side of the double bond. A thorough discussion of *E* and *Z* isomerism is provided in J. March, Advanced Organic Chemistry: Reactions, Mechanisms, and Structure, 4th ed., John Wiley & Sons, 1992. Several of the following examples represent single *E* isomers, single *Z* isomers and mixtures of *E*/*Z* isomers. Determination of the *E* and *Z* isomers can be done by analytical methods such as x-ray crystallography, ¹H NMR, and ¹³C NMR.

Some of the compounds of the invention can exist in more than one tautomeric form. As mentioned above, the compounds of the invention include all such tautomers.

In general, all tautomeric forms and isomeric forms and mixtures, whether individual geometric isomers or optical isomers or racemic or non-racemic mixtures, of a chemical structure or compound is intended, unless the specific stereochemistry or isomeric form is specifically indicated in the compound name or structure.

### D. Pharmaceutical Administration and Treatment Terms and Conventions

The term "patient" includes both human and non-human mammals.

The term "effective amount" means an amount of a compound according to the invention which, in the context of which it is administered or used, is sufficient to achieve the desired effect or result.

The terms "pharmaceutically effective amount" or "therapeutically effective amount" means an amount of a compound according to the invention which, when administered to a patient in need thereof, is sufficient to effect treatment for disease-states, conditions, or disorders for which the compounds have utility. Such an amount would be sufficient to elicit the biological or medical response of a tissue, system, or patient that is sought by a researcher or clinician. The amount of a compound of according to the invention which constitutes a therapeutically effective amount will vary depending on such factors as the compound and its biological activity, the composition used for administration, the time of administration, the route of administration, the rate of excretion of the compound, the duration of treatment, the type of disease-state or disorder being treated and its severity, drugs used in combination with or coincidentally with the compounds of the invention, and the age, body weight, general health, sex, and diet of the patient. Such a therapeutically effective amount can be determined routinely by one of ordinary skill in the art having regard to their own knowledge, the prior art, and this disclosure.

The term "cysteine protease mediated disease" means a disease in which cysteine protease activity contributes to the pathology and/or symptomatology of the disease. Likewise, "cathepsin S [or K] mediated disease" means a disease in which cathepsin S [or K] activity contributes to the pathology and/or symptomatology of the disease.

The terms "treating" or "treatment" mean the treatment of a disease-state in a patient, and include:
(i) preventing the disease-state from occurring in a patient, in particular, when such patient is predisposed to the disease-state but has not yet been diagnosed as having it;
(ii) inhibiting or ameliorating the disease-state, i.e., arresting or slowing its development, such as by inhibiting or ameloirating pathalogic symptoms of the disease state; or
(iii) relieving the disease-state, i.e., causing regression or cure of the disease-state.

### Embodiments of the Invention

In the broadest generic aspect, the invention provides novel compounds of the formula (I): 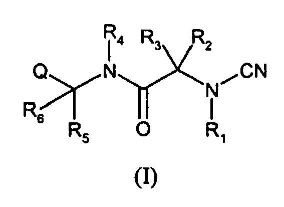 wherein:
R₁ is hydrogen, a C1-10 saturated or unsaturated alkyl group wherein one or more C atoms are optionally replaced by O, NH, S(O), S(O)₂ or S and wherein said alkyl group is optionally independently substituted with one or more: oxo groups, -NH₂, C1-10 alkyl, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, tetrahydropyranyl, thiopyranyl, tetrahydrothiopyranyl, furanyl, tetrahydrofuranyl, thienyl, pyrrolyl, pyrazolyl, oxazolyl, isoxazolyl, thiazolyl, imidazolyl pyridinyl, pyrimidinyl, pyrazinyl, indolyl, benzofuranyl, benzothienyl, benzimidazolyl, benzothiazolyl, benzoxazolyl, quinolyl, isoquinolyl, C1-10 alkoxy, aryloxy, C1-10 alkanoyl, aroyl, C1-10 alkoxycarbonyl, aryloxycarbonyl, C1-10 alkanoyloxy, or aroyloxy,
or R₁ is C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, arylsulfonyl, heteroarylsulfonyl, tetrahydronaphthyl, indenyl, indanyl, tetrahydropyranyl, tetrahydrothiopyranyl, tetrahydrofuranyl, pyrrolidinyl, piperidinyl, morpholinyl, thiomorpholinyl, indolinyl, C3-8cycloalkylsulfonylC1-5lkyl, arylsulfonylC1-5alkyl, aryloxyC1-5alkyl, C1-10alkanoyl, aroyl, C1-10alkoxycarbonyl, arylC1-5alkoxycarbonyl or aryloxycarbonyl,
or R₁ is carbamoyl wherein the nitrogen atom may be independently mono or di-substituted by C1-10 alkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
each R₁ may be further optionally independently substituted by one or more Rₐ;
Rₐ is a C1-10 saturated or unsaturated alkyl group wherein one or more C atoms are optionally replaced by O NH, S(O), S(O)₂ or S and wherein said alkyl group is optionally independently substituted with one or two oxo groups, or one or more: -NH₂, C1-10 alkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₐ is a C1-10alkoxy, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, C3-8 cycloalkyloxy, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl; C1-10alkanoyl, aroyl, C1-10alkanoyloxy, aryloxy, benzyloxy, C1-10 alkoxycarbonyl, arylC1-3alkoxycarbonyl, aryloxycarbonyl or aroyloxy,
or Rₐ is carbamoyl wherein the nitrogen atom may be independently mono or di-substituted by C1-10 alkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₐ is ureido wherein either nitrogen atom may be independently substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₐ is amino wherein the nitrogen atom may be independently mono or di-substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
wherein Rₐ may be further optionally substituted by one or more R_{b};
R_{b} is C3-8 cycloalkyl, tolylsulfonyl, C1-5 alkoxy, aryl, aryloxy, benzyloxy, halogen, hydroxy, oxo, carboxy, cyano, nitro, carboxamide, amidino or guanidino;
or R_{b} is aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or R_{b} is amino wherein the nitrogen atom may be independently mono or di-substituted by C1-10 alkyl,C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or R_{b} is a C1-10 saturated or unsaturated alkyl group wherein one or more C atoms are optionally replaced by O, NH, S(O), S(O)₂ or S and wherein said alkyl group is optionally independently substituted with one or two oxo groups, or one or more: -NH₂, C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
R₂, R₃, R₅, and R₆ independently are hydrogen, or a C1-5 alkyl group;
R₂ and R₃ together with the carbon to which they are attached, and/or R₅ and R₆ together with the carbon to which they are attached, each may independently optionally form a nonaromatic 3-6 membered cycloalkyl;
R₄ is hydrogen, C2-10alkenyl, C3-8 cycloalkyl, arylC1-10alkyl, aryl or a C1-10 alkyl group wherein one or more of the C atoms are optionally replaced by O, NH, C(=O)-, S, S(O) or S(O)₂; wherein R₄ is optionally substituted by one or more Rₑ; or R₄ is Rₑ;
Rₑ is C1-10 alkyl, C3-8 cycloalkyl, aryl, indanyl, indenyl, bicyclo[2.2.1]heptanyl, bicyclo[2.2.2]octanyl, bicyclo[4.1.0]heptanyl, bicyclo[3.1.0]hexanyl, bicyclo[1.1.1]pentanyl, cubanyl, 1,2,3,4-tetrahydronaphthyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl; C1-10 alkoxy, aryloxy, C1-10 alkanoyl, aroyl, C1-10 alkoxycarbonyl, aryloxycarbonyl, C1-10 alkanoyloxy, or aroyloxy;
or Rₑ is carbamoyl wherein the nitrogen atom may be independently mono or di-substituted by C1-10 alkyl, aryl, indanyl, indenyl, tetrahydronaphthyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₑ is C1-10 alkanoylamino, aroylamino, C1-10 alkylthio wherein the sulfur atom may be oxidized to a sulfoxide or sulfone, arylthio wherein the sulfur atom may be oxidized to a sulfoxide or sulfone, ureido wherein either nitrogen atom may be independently substituted by C1-10 alkyl, C1-C10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₑ is C1-10 alkoxycarbonylamino, aryloxycarbonylamino, C1-10 alkylcarbamoyloxy, arylcarbamoyloxy, C1-10 alkylsulfonylamino, arylsulfonylamino, C1-10 alkylaminosulfonyl, arylaminosulfonyl, amino wherein the nitrogen atom may be independently mono or di-substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₑ is amino, halogen, hydroxy, oxo, carboxy, cyano, amidino or guanidino; Each Rₑ may be further optionally substituted by one or more R_{f;}
R_{f} is C1-5 alkyl, C3-6 cycloalkyl, aryl, arylC1-4-alkyl, C1-5 alkoxy, aryloxy, arylC1-5alkoxy, aroyl, halogen, hydroxy, oxo or cyano;
Q is R_{g}, C(O)R_{g}, S(O)R_{g} or S(O)₂R_{g};
wherein R_{g} is C2-l0alkenyl, C1-10 alkoxy, aryloxy, C3-8 cycloalkyl, aryl, arylC1-10alkyl, C1-10 alkyl wherein one or more of the C atoms are optionally replaced by O, NH, -C(=O)-, S, S(O) or S(O)₂; indenyl, indanyl, bicyclo[2.2.1]heptanyl, bicyclo[2.2.2]octanyl, bicyclo[4.1.0]heptanyl, bicyclo[3.1.0]hexanyl, bicyclo[1.1.1]pentanyl, cubanyl, tetrahydronaphthyl, C1-10alkylsulfonylC1-10alkyl, C3-8cycloalkylsulfonylC1-10alkyl, arylsulfonylC1-10alkyl, azetidinyl, pyrrolidinyl, piperidinyl, morpholinyl, thiomorpholinyl, piperazinyl, perhydroazepinyl, perhydrodiazepinyl, indolinyl, isoindolinyl, pyranyl, tetrahydropyranyl, thiopyranyl, tetrahydrothiopyranyl, furanyl, tetrahydrofuranyl, thienyl, pyrrolyl, oxazolyl, isoxazolyl, thiazolyl, imidazolyl, pyrazolyl, pyridinyl, pyrimidinyl, pyrazinyl, pyridazinyl, tetrazolyl, triazolyl, indolyl, benzofuranyl, benzothienyl, dihydrobenzofuranyl, dihydrobenzothienyl, indazolyl, isoindazolyl, benzimidazolyl, benzoxazolyl, benzothiazolyl, benzisoxazolyl, benzisothiazolyl, benzotriazolyl, quinolinyl, tetrahydroquinolinyl, isoquinolinyl, tetrahydroisoquinolinyl, quinazolinyl, tetrahydroquinazolinyl, quinoxalinyl, or amino; wherein R_{g} is optionally substituted by one or more Rₕ;
Rₕ is C1-10 alkyl, C3-8 cycloalkyl, aryl, tetrahydronaphthyl, indenyl, indanyl, azetidinyl, pyrrolidinyl, piperidinyl, morpholinyl, thiomorpholinyl, piperazinyl, perhydroazepinyl, perhydrodiazepinyl, indolinyl, isoindolinyl, pyranyl, tetrahydropyranyl, thiopyranyl, tetrahydrothiopyranyl, furanyl, tetrahydrofuranyl, thienyl, pyrrolyl, oxazolyl, isoxazolyl, thiazolyl, imidazolyl, pyrazolyl, pyridinyl, pyrimidinyl, pyrazinyl, pyridazinyl, tetrazolyl, triazolyl, indolyl, benzofuranyl, benzothienyl, dihydrobenzofuranyl, dihydrobenzothienyl, indazolyl, isoindazolyl, benzimidazolyl, benzoxazolyl, benzothiazolyl, benzisoxazolyl, benzisothiazolyl, benzotriazolyl, benzodioxolyl, quinolinyl, tetrahydroquinolinyl, isoquinolinyl, tetrahydroisoquinolinyl, quinazolinyl, tetrahydroquinazolinyl, quinoxalinyl, C1-10 alkoxy, C1-10alkanoyl, C1-10alkanoyloxy, aryloxy, benzyloxy, C1-10 alkoxycarbonyl, aryloxycarbonyl, aroyloxy, bicyclo[2.2.1]heptanyl, bicyclo[2.2.2]octanyl, bicyclo[4.1.0]heptanyl, bicyclo[3.1.0]hexanyl, bicyclo[1.1.1]pentanyl, or cubanyl,
or Rₕ is carbamoyl wherein the nitrogen atom may be independently mono or di-substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, azetidinyl, pyrrolidinyl, piperidinyl, morpholinyl, thiomorpholinyl, piperazinyl, perhydroazepinyl, perhydrodiazepinyl, indolinyl, isoindolinyl, pyranyl, tetrahydropyranyl, thiopyranyl, tetrahydrothiopyranyl, furanyl, tetrahydrofuranyl, thienyl, pyrrolyl, oxazolyl, isoxazolyl, thiazolyl, imidazolyl, pyrazolyl, pyridinyl, pyrimidinyl, pyrazinyl, pyridazinyl, tetrazolyl, triazolyl, indolyl, benzofuranyl, benzothienyl, dihydrobenzofuranyl, dihydrobenzothienyl, indazolyl, isoindazolyl, benzimidazolyl, benzoxazolyl, benzothiazolyl, benzisoxazolyl, benzisothiazolyl, benzotriazolyl, quinolinyl, tetrahydroquinolinyl, isoquinolinyl, tetrahydroisoquinolinyl, quinazolinyl, tetrahydroquinazolinyl, or quinoxalinyl,
or Rₕ is C1-10 alkanoylamino, aroylamino, C1-10 alkylthio, arylthio wherein the sulfur atom may be oxidized to a sulfoxide or sulfone, or Rₕ is ureido wherein either nitrogen atom may be independently substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, isoindolinyl, azetidinyl, pyrrolidinyl, piperidinyl, morpholinyl, thiomorpholinyl, piperazinyl, perhydroazepinyl, perhydrodiazepinyl, indolinyl, pyranyl, tetrahydropyranyl, thiopyranyl, tetrahydrothiopyranyl, furanyl, tetrahydrofuranyl, thienyl, pyrrolyl, oxazolyl, isoxazolyl, thiazolyl, imidazolyl, pyrazolyl, pyridinyl, pyrimidinyl, pyrazinyl, pyridazinyl, tetrazolyl, triazolyl, indolyl, benzofuranyl, benzothienyl, dihydrobenzofuranyl, dihydrobenzothienyl, indazolyl, isoindazolyl, benzimidazolyl, benzoxazolyl, benzothiazolyl, benzisoxazolyl, benzisothiazolyl, benzotriazolyl, quinolinyl, tetrahydroquinolinyl, isoquinolinyl, tetrahydroisoquinolinyl, quinazolinyl, tetrahydroquinazolinyl, or quinoxalinyl;
or Rₕ is C1-10 alkoxycarbonylamino, aryloxycarbonylamino, C1-10 alkylcarbamoyloxy, arylcarbamoyloxy, C1-10 alkylsulfonylamino, arylsulfonylamino, C1-10 alkylaminosulfonyl, arylaminosulfonyl, amino wherein the nitrogen atom may be independently mono or di-substituted by C1-10 alkyl, C1-10alkoxyC1-10alkyl, C1-10alkylaminoC1-10alkyl, C1-10alkylhioC1-10alkyl wherein the sulfur atom may be oxidized to a sulfoxide or sulfone, C3-8 cycloalkyl, aryl, tetrahydronaphthyl, indenyl, indanyl, isoindolinyl, azetidinyl, pyrrolidinyl, piperidinyl, morpholinyl, thiomorpholinyl, piperazinyl, piperazinylcarbonyl, perhydroazepinyl, perhydrodiazepinyl, indolinyl, pyranyl, tetrahydropyranyl, thiopyranyl, tetrahydrothiopyranyl, furanyl, tetrahydrofuranyl, thienyl, pyrrolyl, oxazolyl, isoxazolyl, thiazolyl, imidazolyl, pyrazolyl, pyridinyl, pyrimidinyl, pyrazinyl, pyridazinyl, tetrazolyl, triazolyl, indolyl, benzofuranyl, benzothienyl, dihydrobenzofuranyl, dihydrobenzothienyl, indazolyl, isoindazolyl, benzimidazolyl, benzoxazolyl, benzothiazolyl, benzisoxazolyl, benzisothiazolyl, benzotriazolyl, quinolinyl, tetrahydroquinolinyl, isoquinolinyl, tetrahydroisoquinolinyl, quinazolinyl, tetrahydroquinazolinyl, quinoxalinyl, halogen, hydroxy, oxo, carboxy, cyano, nitro, amidino or guanidino, Rₕ may be further optionally substituted by one or more R;;
Rᵢ is C1-10 alkyl, C3-8 cycloalkyl, aryl, arylC1-10alkyl, heterocyclyl, heterocyclylC1-10alkyl, C1-10 alkoxy, C1-10 alkoxycarbonyl, aryloxy, benzyloxy, halogen, hydroxy, oxo, carboxy, cyano, nitro, amidino or guanidino;
or the pharmaceutically acceptable salts, solvates, or tautomers thereof;
with the proviso that when R₁ is hydrogen or C1-10alkyl, and R₂, R₃, R₅ and R₆ are each independently hydrogen or C1-5alkyl, and R₄ is hydrogen or C1-10alkyl, then Q is not C1-10alkyl or C1-10alkoxycarbonyl.

Another embodiment of the invention is directed to the compounds of formula (I) described immediately above, and wherein:
R₁ is hydrogen, a C1-7 saturated or unsaturated alkyl group wherein one or more C atoms are optionally replaced by O, NH, S(O), S(O)₂ or S and wherein said alkyl group is optionally independently substituted with one or more: oxo groups, C1-4 alkyl, C3-8 cycloalkyl, aryl, pyrrolidinyl, piperidinyl, morpholinyl, thiomorpholinyl, piperazinyl, indolinyl, pyranyl, thiopyranyl, tetrahydropyranyl, tetrahydrothiopyranyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or R₁ is C1-7alkoxyC1-7alkyl, C1-7alkylthioC1-7alkyl wherein the sulfur atom may be oxidized to a sulfoxide or sulfone, C3-8 cycloalkyl, aryl, arylsulfonyl, heteroarylsulfonyl, tetrahydronaphthyl, indenyl, indanyl, arylsulfonylC1-5alkyl, aryloxyC1-5alkyl, C1-7alkanoyl, aroyl, tetrahydropyranyl, pyrrolidinyl, piperidinyl, morpholinyl, indolinyl, carbamoyl wherein the nitrogen atom may be independently mono or di-substituted by C1-7 alkyl, aryl, pyrrolidinyl, piperidinyl, morpholinyl, thiomorpholinyl, piperazinyl, indolinyl, furanyl, thienyl, pyrrolyl, oxazolyl, thiazolyl, imidazolyl, pyridinyl, pyrimidinyl, pyrazinyl, indolyl, benzofuranyl, benzothienyl, benzimidazolyl, benzothiazolyl, quinolyl or isoquinolyl;
each R₁ may be further optionally independently substituted by one or more Rₐ;
Rₐ is a C1-7 saturated or unsaturated alkyl group wherein one or more C atoms are optionally replaced by O, S(O), S(O)₂ or S and wherein said alkyl group is optionally independently substituted with one or two oxo groups, or one or more: -NH₂, C₁₋₄alkyl, aryl, pyrrolidinyl, piperidinyl, morpholinyl, thiomorpholinyl, piperazinyl, indolinyl, pyranyl, thiopyranyl, tetrahydropyranyl, tetrahydrothiopyranyl, furanyl, thienyl, pyrrolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₐ is a C1-7alkoxy, C1-7alkoxyC1-7alkyl, C1-7alkylthioC1-7alkyl wherein the sulfur atom may be oxidized to a sulfoxide or sulfone, C3-8 cycloalkyl, C3-8 cycloalkyloxy, aryl, tetrahydronaphthyl, indenyl, indanyl, pyrrolidinyl, piperidinyl, morpholinyl, thiomorpholinyl, piperazinyl, tetrahydropyranyl, tetrahydrothiopyranyl, furanyl, thienyl, pyrrolyl, pyridinyl, pyrimidinyl, pyrazinyl, indolyl, benzofuranyl, benzothienyl, benzimidazolyl, benzothiazolyl, benzoxazolyl, quinolyl, isoquinolyl, C1-7alkanoyl, aroyl, C1-7alkanoyloxy, aryloxy, benzyloxy, C1-7 alkoxycarbonyl, arylC1-3alkoxycarbonyl, aryloxycarbonyl, aroyloxy, carbamoyl wherein the nitrogen atom may be independently mono or di-substituted by C1-7 alkyl, aryl, pyrrolidinyl, piperidinyl, morpholinyl, thiomorpholinyl, piperazinyl, indolinyl, furanyl, thienyl, pyrrolyl, pyridinyl, pyrimidinyl, pyrazinyl, indolyl, benzofuranyl, benzothienyl, benzimidazolyl, benzothiazolyl, quinolyl or isoquinolyl;
or Rₐ is ureido wherein either nitrogen atom may be independently substituted by C1-7 alkyl, aryl, pyrrolidinyl, piperidinyl, morpholinyl, thiomorpholinyl, piperazinyl, tetrahydropyranyl, indolinyl, furanyl, thienyl, pyrrolyl, pyridinyl, pyrimidinyl, pyrazinyl, indolyl, benzofuranyl, benzothienyl, benzimidazolyl, benzothiazolyl, quinolyl or isoquinolyl,
or Rₐ is amino wherein the nitrogen atom may be independently mono or di-substituted by C1-7 alkyl, aryl, pyrrolidinyl, piperidinyl, morpholinyl, thiomorpholinyl, piperazinyl, tetrahydropyranyl, indolinyl, furanyl, thienyl, pyrrolyl, pyridinyl, pyrimidinyl, pyrazinyl, indolyl, benzofuranyl, benzothienyl, benzimidazolyl, benzothiazolyl, benzoxazolyl, quinolyl or isoquinolyl,
wherein Rₐ may be further optionally substituted by one or more R_{b};
R_{b} is C3-6 cycloalkyl, C1-5 alkoxy, aryl, aryloxy, benzyloxy, halogen, hydroxy, oxo, carboxy, cyano, nitro, carboxamide, amidino or guanidino;
or R_{b} is aryl, pyrrolidinyl, piperidinyl, morpholinyl, thiomorpholinyl, piperazinyl, tetrahydropyranyl, indolinyl, furanyl, thienyl, pyrrolyl, pyridinyl, pyrimidinyl, pyrazinyl, indolyl, benzofuranyl, benzothienyl, benzimidazolyl, benzothiazolyl, quinolyl or isoquinolyl;
or R_{b} is amino wherein the nitrogen atom may be independently mono or di-substituted by C1-7 alkyl, aryl, pyrrolidinyl, piperidinyl, morpholinyl, thiomorpholinyl, piperazinyl, tetrahydropyranyl, indolinyl, furanyl, thienyl, pyrrolyl, pyridinyl, pyrimidinyl, pyrazinyl, indolyl, benzofuranyl, benzothienyl, benzimidazolyl, benzothiazolyl, quinolyl or isoquinolyl;
or R_{b} is a C1-7 saturated or unsaturated alkyl group wherein one or more C atoms are optionally replaced by O, S(O), S(O)₂ or S and wherein said alkyl group is optionally independently substituted with one or two oxo groups, or one or more: -NH₂, C₁₋₄ alkyl, aryl, pyrrolidinyl, piperidinyl, morpholinyl, thiomorpholinyl, piperazinyl, tetrahydropyranyl, indolinyl, furanyl, thienyl, pyrrolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
R₂, R₃, R₅, and R₆ independently are hydrogen or a C1-5 alkyl group;
R₂ and R₃ together with the carbon to which they are attached, and/or R₅ and R₆ together with the carbon to which they are attached, each may independently optionally form a nonaromatic 3-6 membered cycloalkyl;
R₄ is hydrogen, C2-5alkenyl, C3-7 cycloalkyl, arylC1-3alkyl, aryl or a C1-6 alkyl group wherein one or two of the C atoms are optionally replaced by O, -C(=O)-, S, S(O) or S(O)₂; wherein R₄ is optionally substituted by one or more Rₑ; or R₄ is Rₑ;
Rₑ is C1-5 alkyl, C3-7 cycloalkyl, aryl, indanyl, indenyl, bicyclo[2.2.1]heptanyl, bicyclo[2.2.2]octanyl, bicyclo[4. 1.0]heptanyl, bicyclo[3.1.0]hexanyl, bicyclo[1.1.1]pentanyl, cubanyl, 1,2,3,4-tetrahydronaphthyl, pyrrolidinyl, piperidinyl, morpholinyl, thiomorpholinyl, piperazinyl, indolinyl, furanyl, tetrahydropyranyl, thienyl, pyrrolyl, oxazolyl, thiazolyl, pyrazolyl, pyridinyl, pyrimidinyl, pyrazinyl, indolyl, benzofuranyl, benzothienyl, benzimidazolyl, benzothiazolyl, quinolinyl, or isoquinolinyl, C1-5 alkoxy, aryloxy, C1-5 alkanoyl, aroyl, C1-5 alkoxycarbonyl, aryloxycarbonyl, C1-5 alkanoyloxy, aroyloxy, carbamoyl wherein the nitrogen atom may be independently mono or di-substituted by C1-5 alkyl, aryl, pyrrolidinyl, piperidinyl, morpholinyl, thiomorpholinyl, piperazinyl, indolinyl, furanyl, thienyl, pyrrolyl, oxazolyl, thiazolyl, imidazolyl, pyridinyl, pyrimidinyl, pyrazinyl, indolyl, benzofuranyl, benzothienyl, benzimidazolyl, benzothiazolyl, quinolinyl or isoquinolinyl,
or Rₑ is C1-5 alkanoylamino, aroylamino, C1-5 alkylthio wherein the sulfur atom may be oxidized to a sulfoxide or sulfone, arylthio wherein the sulfur atom may be oxidized to a sulfoxide or sulfone, ureido wherein either nitrogen atom may be independently substituted by C1-5 alkyl, aryl, pyrrolidinyl, piperidinyl, morpholinyl, thiomorpholinyl, piperazinyl, indolinyl, furanyl, thienyl, pyrrolyl, oxazolyl, thiazolyl, imidazolyl, pyridinyl, pyrimidinyl, pyrazinyl, indolyl, benzofuranyl, benzothienyl, benzimidazolyl, benzothiazolyl, quinolinyl, or isoquinolinyl,
or Rₑ is C1-5 alkoxycarbonylamino, aryloxycarbonylamino, C1-5 alkylcarbamoyloxy, arylcarbamoyloxy, C1-5 alkylsulfonylamino, arylsulfonylamino, C1-5 alkylaminosulfonyl, arylaminosulfonyl, amino wherein the nitrogen atom may be independently mono or di-substituted by C1-5 alkyl, aryl, pyrrolidinyl, piperidinyl, morpholinyl, thiomorpholinyl, piperazinyl, indolinyl, furanyl, thienyl, pyrrolyl, oxazolyl, thiazolyl, imidazolyl, pyridinyl, pyrimidinyl, pyrazinyl, indolyl, benzofuranyl, benzothienyl, benzimidazolyl, benzothiazolyl, quinolinyl or isoquinolinyl,
or Rₑ is halogen, hydroxy, oxo, carboxy, cyano, amidino or guanidino; Each Rₑ may be further optionally substituted by one or more R_{f};
R_{f} is C1-5 alkyl, C3-6 cycloalkyl, aryl, arylC1-4-alkyl, C1-5 alkoxy, aryloxy, arylC1-5alkoxy, aroyl, halogen, hydroxy, oxo or cyano;
Q is R_{g}, C(O)R_{g}, S(O)R, or S(O)₂R_{g};
wherein R_{g} is C1-5 alkyl wherein one or more C atoms are optionally replaced by O or NH, C1-5 alkoxy, aryloxy, C3-7 cycloalkyl, phenyl, benzyl, naphthyl, tetrahydronaphthyl, C1-5alkylsulfonylC1-5alkyl, C3-7cycloalkylsulfonylC1-5alkyl, arylsulfonylC1-5alkyl, pyrrolidinyl, piperidinyl, morpholinyl, thiomorpholinyl, piperazinyl, indolinyl, isoindolinyl, furanyl, thienyl, pyrrolyl, oxazolyl, isoxazolyl, thiazolyl, imidazolyl, pyridinyl, pyrimidinyl, pyrazinyl, indolyl, benzofuranyl, benzothienyl, benzimidazolyl, benzthiazolyl, quinolinyl, isoquinolinyl, tetrahydroisoquinolyl, quinoxalinyl or benzoxazolyl, or amino; wherein R_{g} is optionally substituted by one or more Rₕ;
Rₕ is C1-5 alkyl, C3-7 cycloalkyl, phenyl, naphthyl, indenyl, indanyl, pyrrolidinyl, piperidinyl, morpholinyl, thiomorpholinyl, piperazinyl, indolinyl, isoindolinyl, furanyl, thienyl, pyrrolyl, oxazolyl, thiazolyl, imidazolyl, pyridinyl, pyrimidinyl, pyrazinyl, indolyl, benzofuranyl, benzothienyl, benzimidazolyl, benzthiazolyl, benzoxazolyl, benzisoxazolyl, benzodioxolyl, quinolinyl, isoquinolinyl or tetrahydroisoquinolyl, C1-5 alkoxy, C1-5alkanoyl, C1-5alkanoyloxy, aryloxy, benzyloxy; C1-5 alkoxycarbonyl, aryloxycarbonyl, aroyloxy, carbamoyl wherein the nitrogen atom may be independently mono or di-substituted by C1-6 alkyl, aryl, pyrrolidinyl, piperidinyl, morpholinyl, thiomorpholinyl, piperazinyl, indolinyl, furanyl, thienyl, pyrrolyl, oxazolyl, thiazolyl, imidazolyl, pyridinyl, pyrimidinyl, pyrazinyl, indolyl, benzofuranyl, benzothienyl, benzimidazolyl, benzthiazolyl, quinolinyl or isoquinolinyl,
or Rₕ is C1-5 alkanoylamino, aroylamino, C1-5 alkylthio, arylthio wherein the sulfur atom may be oxidized to a sulfoxide or sulfone, or Rₕ is ureido wherein either nitrogen atom may be independently substituted by alkyl, aryl, pyrrolidinyl, piperidinyl, morpholinyl, thiomorpholinyl, piperazinyl, indolinyl, isoindolinyl, furanyl, thienyl, pyrrolyl, oxazolyl, thiazolyl, imidazolyl, pyridinyl, pyrimidinyl, pyrazinyl, indolyl, benzofuranyl, benzothienyl, benzimidazolyl, benzthiazolyl, quinolinyl, or isoquinolinyl,
or Rₕ is C1-5 alkoxycarbonylamino, aryloxycarbonylamino, C1-5 alkylcarbamoyloxy, arylcarbamoyloxy, C1-5 alkylsulfonylamino, arylsulfonylamino, C1-5 alkylaminosulfonyl, arylaminosulfonyl, amino wherein the nitrogen atom may be independently mono or di-substituted by alkyl, aryl, pyrrolidinyl, piperidinyl, morpholinyl, thiomorpholinyl, piperazinyl, piperazinylcarbonyl, indolinyl, isoindolinyl, furanyl, thienyl, pyrrolyl, oxazolyl, thiazolyl, imidazolyl, pyridinyl, pyrimidinyl, pyrazinyl, indolyl, benzofuranyl, benzothienyl, benzimidazolyl, benzthiazolyl, quinolinyl or isoquinolinyl, halogen, hydroxy, oxo, carboxy, cyano, nitro, amidino or guanidino, Rₕ may be further optionally substituted by one or more Rᵢ;
Rᵢ is C1-5 alkyl, C3-6 cycloalkyl, aryl, arylC1-Salkyl, pyrrolidinyl, piperidinyl, piperazinyl, morpholinyl, thiomorpholinyl, C1-5 alkoxy, C1-5 alkoxycarbonyl, aryloxy, benzyloxy, halogen, hydroxy, oxo, carboxy, cyano, nitro, amidino or guanidino;
wherein one or more of the amino nitrogens in the amidino or guanidino groups in the compound of formula I may be optionally substituted with C₁₋₃alkyl, phenylC₀₋₃alkyl or C₁₋₃ alkoxy;
or the pharmaceutically acceptable salts, solvates, or tautomers thereof.

A further embodiment of the invention is directed to the compounds of formula (I) described immediately above, wherein:
R₁ is hydrogen, a C1-6 saturated or unsaturated alkyl group wherein one or more C atoms are optionally replaced by O, NH, S, S(O) or S(O)₂ and wherein said alkyl group is optionally independently substituted with one or more: oxo groups, C1-4 alkyl, C3-6 cycloalkyl, aryl, pyrrolidinyl, piperidinyl, morpholinyl, thiomorpholinyl, piperazinyl, indolinyl, pyranyl, thiopyranyl, tetrahydropyranyl, tetrahydrothiopyranyl, pyridinyl, pyrimidinyl, pyrazinyl or indolyl;
or R₁ is C1-6alkoxyC1-6alkyl, C1-6alkylthioC1-6alkyl wherein the sulfur atom may be oxidized to a sulfoxide or sulfone, C3-6 cycloalkyl, aryl, arylsulfonyl, arylsulfonylC1-6alkyl, heteroarylsulfonyl, aryloxyC1-6alkyl, C1-6alkanoyl, aroyl, pyrrolidinyl, piperidinyl, morpholinyl, carbamoyl wherein the nitrogen atom may be independently mono or di-substituted by C1-6 alkyl, or aryl;
each R₁ may be further optionally independently substituted by one or more Rₐ;
Rₐ is a C1-6 saturated or unsaturated alkyl group wherein one or more C atoms are optionally replaced by O, S, S(O) or S(O)₂ and wherein said alkyl group is optionally independently substituted with one or two oxo groups, or one or more: -NH₂, C₁₋₄alkyl, or aryl;
or Rₐ is a C1-6alkoxy, C1-6alkoxyC1-6alkyl, C1-6alkylthioC1-6alkyl, C3-6 cycloalkyl, C3-6 cycloalkyloxy, aryl, pyrrolidinyl, piperidinyl, morpholinyl, thiomorpholinyl, piperazinyl, tetrahydropyranyl, pyridinyl, indolyl, C1-6alkanoyl, aroyl, C1-6alkanoyloxy, aryloxy, benzyloxy, C1-6alkoxycarbonyl, arylC1-3alkoxycarbonyl, aryloxycarbonyl, aroyloxy, carbamoyl wherein the nitrogen atom may be independently mono or di-substituted by C1-6 alkyl, or aryl;
or Rₐ is ureido wherein either nitrogen atom may be independently substituted by C1-6 alkyl or aryl,
or Rₐ is amino wherein the nitrogen atom may be independently mono or di-substituted by C1-6 alkyl, or aryl,
wherein Rₐ may be further optionally substituted by one or more R_{b};
R_{b} is C3-6 cycloalkyl, C1-5 alkoxy, aryl, aryloxy, benzyloxy, halogen, hydroxy, oxo, carboxy, cyano, nitro or carboxamide;
or R_{b} is amino wherein the nitrogen atom may be independently mono or di-substituted by C1-6 alkyl or aryl;
or R_{b} is a C1-6 saturated or unsaturated alkyl group wherein one or two C atoms are optionally replaced by O or S and wherein said alkyl group is optionally independently substituted with one or two oxo groups, or one or more: -NH₂, C₁₋₄ alkyl, or aryl;
R₂, R₃, R₅, and R₆ independently are hydrogen or a C1-4 alkyl group;
R₂ and R₃ together with the carbon to which they are attached, and/or R₅ and R₆ together with the carbon to which they are attached, each may independently optionally form a nonaromatic 3-6 membered cycloalkyl;
R₄ is hydrogen, C2-5alkenyl, C3-6 cycloalkyl, arylC1-3alkyl, aryl or a C1-6 alkyl group wherein one or two of the C atoms are optionally replaced by O, S or S(O)₂; wherein R₄ is optionally substituted by one or more Rₑ; or R₄ is Rₑ;
Rₑ is C1-5 alkyl, C3-6 cycloalkyl, aryl, indanyl, indenyl, pyridinyl, indolyl, bicyclo[2.2.1]heptanyl, bicyclo[2.2.2]octanyl, bicyclo[4.1.0]heptanyl, bicyclo[3.1.0]hexanyl, bicyclo[1.1.1]pentanyl, cubanyl, 1,2,3,4-tetrahydronaphthyl, C1-5 alkoxy, aryloxy, C1-5 alkanoyl, aroyl, C1-5 alkoxycarbonyl, aryloxycarbonyl, C1-5 alkanoyloxy, aroyloxy, carbamoyl wherein the nitrogen atom may be independently mono or di-substituted by C1-5 alkyl or aryl,
or Rₑ is C1-5 alkanoylamino, aroylamino, C1-5 alkylthio, arylthio wherein the sulfur atom may be oxidized to a sulfoxide or sulfone, ureido wherein either nitrogen atom may be independently substituted by C1-5 alkyl or aryl,
or Rₑ is C1-5 alkoxycarbonylamino, aryloxycarbonylamino, C1-5 alkylcarbamoyloxy, arylcarbamoyloxy, C1-5 alkylsulfonylamino, arylsulfonylamino, C1-5 alkylaminosulfonyl, arylaminosulfonyl, amino wherein the nitrogen atom may be independently mono or di-substituted by C1-5 alkyl or aryl,
or Rₑ is halogen, hydroxy, oxo, carboxy, cyano, amidino or guanidino; Each Rₑ may be further optionally substituted by one or more R_{f};
R_{f} is C1-5 alkyl, C3-6 cycloalkyl, aryl, arylC1-4-alkyl, C1-5 alkoxy, aryloxy, arylC1-5alkoxy, aroyl, halogen, hydroxy, oxo or cyano;
Q is R_{g}, C(O)R_{g}, S(O)R_{g} or S(O)₂R_{g};
wherein R_{g} is C1-5 alkyl wherein one or more C atoms are optionally replaced by O or NH, C1-5 alkoxy, aryloxy, C3-6 cycloalkyl, phenyl, benzyl, naphthyl, tetrahydronaphthyl, C1-5alkylsulfonylC1-5alkyl, C3-6cycloalkylsulfonylC1-5alkyl, aryIsulfonylC1-5alkyl, pyrrolidinyl, piperidinyl, morpholinyl, thiomorpholinyl, piperazinyl, isoindolinyl, furanyl, thienyl, oxazolyl, isoxazolyl, thiazolyl, pyridinyl, indolyl, benzofuranyl, benzothienyl, quinolinyl, isoquinolinyl, tetrahydroisoquinolyl, pyrazinyl or quinoxalinyl, or amino;
wherein R_{g} is optionally substituted by one or more Rₕ;
Rₕ is C1-5 alkyl, C3-6 cycloalkyl, phenyl, naphthyl, indenyl, indanyl, pyrrolidinyl, piperidinyl, morpholinyl, thiomorpholinyl, piperazinyl, pyridinyl, isoindolinyl, quinolinyl, isoquinolinyl, tetrahydroisoquinolyl or benzodioxolyl, C1-5 alkoxy, C1-5alkanoyl, C1-5alkanoyloxy, aryloxy, benzyloxy, C1-5 alkoxycarbonyl, aryloxycarbonyl, aroyloxy, carbamoyl wherein the nitrogen atom may be independently mono or di-substituted by C1-6 alkyl or aryl,
or Rₕ is C1-5 alkanoylamino, aroylamino, C1-5 alkylthio, arylthio wherein the sulfur atom may be oxidized to a sulfoxide or sulfone, or Rₕ is ureido wherein either nitrogen atom may be independently substituted by alkyl or aryl,
or Rₕ is C1-5 alkoxycarbonylamino, aryloxycarbonylamino, C1-5 alkylcarbamoyloxy, arylcarbamoyloxy, C1-5 alkylsulfonylamino, arylsulfonylamino, C1-5 alkylaminosulfonyl, arylaminosulfonyl, amino wherein the nitrogen atom may be independently mono or di-substituted by alkyl, aryl, piperidinyl, morpholinyl, thiomorpholinyl, piperazinyl or piperazinylcarbonyl, halogen, hydroxy, oxo, carboxy, cyano, nitro, amidino or guanidino,
Rₕ may be further optionally substituted by one or more Rᵢ;
Rᵢ is C1-5 alkyl, C3-6 cycloalkyl, aryl, arylC1-5 alkyl, piperidinyl, piperazinyl, morpholinyl, thiomorpholinyl, C1-5 alkoxy, C1-5 alkoxycarbonyl, aryloxy, benzyloxy, halogen, hydroxy, oxo, carboxy, cyano, nitro, amidino or guanidino;
wherein one or more of the amino nitrogens in the amidino or guanidino groups in the compound of formula I may be optionally substituted with C₁₋₃alkyl, phenylC₀₋₃alkyl or C₁₋₃ alkoxy;
or the pharmaceutically acceptable salts, solvates, or tautomers thereof.

A further embodiment of the invention is directed to the compound of formula (I) described immediately above, wherein:
R₁ is hydrogen, a C1-6 saturated or unsaturated alkyl group wherein one or two C atoms are optionally replaced by O, NH, S, S(O) or S(O)₂, and wherein said alkyl group is optionally independently substituted with one to three: oxo groups, C1-4 alkyl, C3-6 cycloalkyl, aryl, pyrrolidinyl, piperidinyl, piperazinyl morpholinyl, indolinyl, pyranyl, thiopyranyl, tetrahydropyranyl, tetrahydrothiopyranyl, pyridinyl or indolyl; pyranyl,
or R₁ is C1-6alkylthioC1-6alkyl wherein the sulfur atom may be oxidized to a sulfoxide or sulfone, C3-6 cycloalkyl, phenyl, naphthyl, phenylsulfonyl, pyridinylsulfonyl, phenyloxyC1-6alkyl, C1-6alkanoyl, or piperidinyl;
each R₁ may be further optionally independently substituted by one or more Rₐ;
Rₐ is a C1-6 saturated or unsaturated alkyl group wherein one or two C atoms are optionally replaced by O or S;
or Rₐ is a C1-6alkoxy, C3-6 cycloalkyl, phenyl, naphthyl, pyrrolidinyl, piperidinyl, morpholinyl, thiomorpholinyl, tetrahydropyranyl, piperazinyl, indolyl, or pyridinyl;
wherein Rₐ may be further optionally substituted by one or more R_{b};
R_{b} is C3-6 cycloalkyl, C1-5 alkoxy, halogen, hydroxy, oxo, carboxy, cyano, nitro or carboxamide;
or R_{b} is phenyl, benzyl or naphthyl;
or R_{b} is amino wherein the nitrogen atom may be independently mono or di-substituted by C1-6 alkyl, phenyl or naphthyl;
or R_{b} is a C1-6 saturated or unsaturated alkyl group wherein one or two C atoms are optionally replaced by O or S, and wherein said alkyl group is optionally independently substituted with one or two oxo group;
R₂, R₃, R₅, and R₆ independently are hydrogen or a C1-5 alkyl group;
R₂ and R₃ together with the carbon to which they are attached, and/or R₅ and R₆ together with the carbon to which they are attached, each may independently optionally form a nonaromatic 3-6 membered cycloalkyl;
R₄ is hydrogen, C3-6 cycloalkyl, phenylC1-3alkyl, naphthylC1-3alkyl, phenyl, naphthyl, pyridyl or a C1-6 alkyl group wherein one or two of the C atoms are optionally replaced by O, S or S(O)₂; wherein R₄ is optionally substituted by one or more Rₑ;
Rₑ is C1-5 alkyl, C3-6 cycloalkyl, phenyl, naphthyl, indanyl or indolyl,
or Rₑ is halogen, hydroxy, oxo, carboxy, cyano, amidino or guanidino;
Each Rₑ may be further optionally substituted by one or more R_{f},
R_{f} is C1-5 alkyl, C3-6 cycloalkyl, C1 -5 alkoxy, halogen, hydroxy, oxo or cyano;
Q is R_{g}, C(O)R_{g} or S(O)₂R_{g};
wherein R_{g} is C1-5 alkyl wherein one or more C atoms are optionally replaced by O or NH, C1-5 alkoxy, phenyl, benzyl, naphthyl, pyrrolidinyl, piperidinyl, morpholinyl, thiomorpholinyl, piperazinyl, furanyl, thienyl, oxazolyl, thiazolyl, pyridinyl, benzofuranyl, benzothienyl, quinolinyl, isoquinolinyl, isoindolinyl, tetrahydroisoquinolyl, pyrazinyl, quinoxalinyl or amino; wherein R_{g} is optionally substituted by one or more Rₕ;
Rₕ is C1-5 alkyl, C3-6 cycloalkyl, phenyl, naphthyl, indenyl, indanyl, morpholinyl, thiomorpholinyl, pyridinyl, isoindolinyl, isoquinolinyl, tetrahydroisoquinolinyl or benzodioxolyl, or C1-5alkoxy,
or Rₕ is C₁₋₅ alkylthio,
or Rₕ is amino wherein the nitrogen atom may be independently mono or di-substituted by alkyl, phenyl, naphthyl, piperidinyl, morpholinyl, thiomorpholinyl, piperazinyl or piperazinylcarbonyl, halogen, hydroxy, oxo, carboxy, cyano, nitro, amidino or guanidino,
Rₕ may be further optionally substituted by one or more Rᵢ;
Rᵢ is C1-5 alkyl, C3-6 cycloalkyl, aryl, arylC1-5alkyl, morpholinyl, thiomorpholinyl, C1-5 alkoxy, C1-5 alkoxycarbonyl, halogen, hydroxy, oxo, carboxy, cyano, nitro, amidino or guanidino;
wherein one or more of the amino nitrogens in the amidino or guanidino groups in the compound of formula I may be optionally substituted with C₁₋₃alkyl, phenylC₀₋₃alkyl or C₁₋₃ alkoxy;
or the pharmaceutically acceptable salts, solvates, or tautomers thereof.

An even further embodiment of the invention is directed to the compounds of formula (I) described immediately above, wherein:
R₁ is hydrogen, a C1-6 saturated alkyl group wherein one C atom is optionally replaced by O, S, S(O), S(O)₂ or NH and wherein said alkyl group is optionally independently substituted with one or two: oxo groups, phenyl, naphthyl, cyclohexyl, pyrrolidinyl, piperidinyl, piperazinyl, morpholinyl, indolinyl, tetrahyropyranyl, pyridinyl or indolyl;
or R₁ is C1-3alkylthioC1-3alkyl wherein the sulfur atom may be oxidized to a sulfoxide, cyclohexyl, phenyl, phenylsulfonyl, pyridinylsulfonyl, phenyloxyC1-4alkyl, C1-6alkanoyl or piperidinyl;
each R₁ may be further optionally independently substituted by one or two Rₐ;
Rₐ is a C1-6 saturated alkyl group;
or Rₐ is a C1-6alkoxy, cyclohexyl, phenyl, naphthyl, pyrrolidinyl, piperidinyl, indolyl, piperazinyl, pyridinyl or tetrahydropyranyl;
wherein Rₐ may be further optionally substituted by one or two R_{b};
R_{b} is C3-6 cycloalkyl, phenyl, benzyl, C1-5 alkoxy, halogen, hydroxy, oxo, carboxy, cyano, nitro or carboxamide;
or R_{b} is a C1-6 saturated or unsaturated alkyl group, or is a C1-4alkoxycarbonyl group;
R₂, R₃, R₅, and R₆ independently are hydrogen or a C1-5 alkyl group;
R₂ and R₃ together with the carbon to which they are attached, and/or R₅ and R₆ together with the carbon to which they are attached, each may independently optionally form a cyclopropyl, cyclobutyl, cyclopentyl or cyclohexyl group;
R₄ is hydrogen, cyclopropyl, cyclobutyl, cyclopentyl, cyclohexyl, phenylC1-3alkyl, naphthylC1-3alkyl, phenyl, naphthyl, pyridinyl or a C1-6 alkyl group where a C atom is optionally replaced by S(O)₂; wherein R₄ is optionally substituted by one or more Rₑ;
Rₑ is C1-3 alkyl, cyclopropyl, cyclobutyl, cyclopentyl, cyclohexyl, phenyl, naphthyl, indanyl or indolyl,
or Rₑ is hydroxy, halogen, oxo, carboxy or cyano;
Q is R_{g}, C(O)R_{g} or S(O)₂ R_{g};
wherein R_{g} is C1-5alkyl wherein one or more C atoms are optionally replaced by O or NH, phenyl, naphthyl, pyrrolidinyl, piperidinyl, morpholinyl, thiomorpholinyl, piperazinyl, furanyl, thienyl, thiazolyl, benzofuranyl, benzothienyl, quinolyl, isoquinolyl, isoindolinyl, tetrahydroisoquinolyl, pyrazinyl or quinoxalinyl, or amino; wherein R_{g} is optionally substituted by one or more Rₕ;
Rₕ is C1-5 alkyl, C1-5alkoxy, halogen, phenyl, naphthyl, indenyl, indanyl, morpholinyl, thiomorpholinyl, pyridinyl, isoquinolinyl, isoindolinyl, tetrahydroisoquinolyl benzodioxolyl, piperidinylamino or piperazinylcarbonylamino;
Rₕ may be further optionally substituted by one or more Rᵢ;
Rᵢ is C1-3 alkyl, C3-6 cycloalkyl, phenyl, naphthyl, benzyl, morpholinyl, C1-5 alkoxy, C1-5 alkoxycarbonyl, halogen, hydroxy, oxo, carboxy, cyano or nitro;
or the pharmaceutically acceptable salts, solvates, or tautomers thereof.

In yet a further embodiment, the present invention is directed to the compounds of formula (I) described immediately above, wherein:
R₁ is hydrogen, C1-6alkyl, C1-6alkanoyl, phenylsulfonyl or pyridylsulfonyl each optionally substituted by cyclohexyl, phenyl, pyrrolidinyl, piperidinyl, indolyl, piperazinyl, pyridinyl, tetrahydropyranyl or naphthyl, or R₁ is benzyloxylC1-3alkyl or benzyloxyC1-3alkanoyl;
R₂, R₃, R₅, and R₆ independently are hydrogen or C1-3 alkyl;
R₂ and R₃ together with the carbon to which they are attached, and/or R₅ and R₆ together with the carbon to which they are attached, each may independently optionally form a cyclopropyl, cyclobutyl, cyclopentyl or cyclohexyl group;
R₄ is hydrogen, a C1-6 alkyl group, phenylC1-6alkyl, cyclopropylC1-6alkyl, cyclohexylC1-6alkyl or pyridinyl;
Q is C(O)R_{g};
wherein R_{g} is piperidinyl, morpholinyl, thiomorpholinyl, piperazinyl, isoindolinyl, benzofuranyl, tetrahydroisoquinolyl, phenylC1-5alkylamino or naphthylC1-5alkylamino, each optionally substituted by C1-5alkyl, morpholinyl or morpholinylC1-5alkoxy;
or the pharmaceutically acceptable salts, solvates, or tautomers thereof.

In yet a further embodiment, the present invention is directed to the compounds of formula (I) described immediately above, wherein:
R₁ is C1-6alkanoyl optionally substituted by cyclohexyl or phenyl;
R₂, R₃, R₅, and R₆ are hydrogen;
R₄ is a C1-6 saturated alkyl group;
Q is C(O)R_{g};
wherein R_{g} is morpholinyl, tetrahydroisoquinolyl or phenylC1-3alkylamino;
or the pharmaceutically acceptable salts, solvates, or tautomers thereof Additional embodiments of the present invention are described below with respect to the individual variables in the compounds of formula (I):

### Variable R₁

The compounds of formula (I) wherein:
R₁ is hydrogen, a C1-6 saturated alkyl group wherein one C atom is optionally replaced by O S, S(O), S(O)₂ or NH and wherein said alkyl group is optionally independently substituted with one or two: oxo groups, phenyl, naphthyl, cyclohexyl, pyrrolidinyl, piperidinyl, piperazinyl, morpholinyl, indolinyl, tetrahydropyranyl, pyridinyl or indolyl;
or R₁ is C1-3alkylthioC1-3alkyl wherein the sulfur atom may be oxidized to a sulfoxide, cyclohexyl, phenyl, phenylsulfonyl, pyridinylsulfonyl, phenyloxyC1-4alkyl, C1-6alkanoyl, or piperidinyl;
each R₁ may be further optionally independently substituted by one or two Rₐ;
Rₐ is a C1-6 saturated alkyl group;
or Rₐ is a C1-6alkoxy, cyclohexyl, phenyl, naphthyl, pyrrolidinyl, piperidinyl, piperazinyl or pyridinyl;
wherein Rₐ may be further optionally substituted by one or two R_{b};
R_{b} is C3-6 cycloalkyl, phenyl, benzyl, C1-5 alkoxy, halogen, hydroxy, oxo, carboxy, cyano, nitro or carboxamide;
or R_{b} is a C1-6 saturated or unsaturated alkyl group, or is a C1-4alkoxycarbonyl group;
or the pharmaceutically acceptable salts, solvates, or tautomers thereof.

In yet another embodiment, the compounds of formula (I) wherein:
R₁ is hydrogen, C1-6alkyl or C1-6alkanoyl, each optionally substituted by cyclohexyl, phenyl, or naphthyl, or R₁ is benzyloxylC1-3alkyl or benzyloxyC1-3alkanoyl;
or the pharmaceutically acceptable salts, solvates, or tautomers thereof.

### Variables R₂, R₃, R₅ and R₆

The compounds of formula (I) wherein:
R₂, R₃, R₅, and R₆ independently are hydrogen or a C1-5 alkyl group;
R₂ and R₃ together with the carbon to which they are attached, and/or R₅ and R₆ together with the carbon to which they are attached, each may independently optionally form a cyclopropyl, cyclobutyl, cyclopentyl or cyclohexyl group;
or the pharmaceutically acceptable salts, solvates, or tautomers thereof.

In another embodiment, the compounds of formula (I) wherein:
R₂, R₃, R₅, and R₆ independently are hydrogen or C1-3 alkyl;
R₂ and R₃ together with the carbon to which they are attached, and/or R₅ and R₆ together with the carbon to which they are attached, each may independently optionally form a cyclopropyl, cyclopentyl or cyclohexyl group;
or the pharmaceutically acceptable salts, solvates, or tautomers thereof.

### Variable R₄

The compounds of formula (I) wherein:
R₄ is hydrogen, cyclopropyl, cyclopentyl, cyclohexyl, phenylC1-3alkyl, naphthylC1-3alkyl, phenyl, naphthyl, pyridinyl or a C1-6 alkyl group where a C atom is optionally replaced by S(O)₂; wherein R₄ is optionally substituted by one or more Rₑ;
   Rₑ is C1-3 alkyl, cyclopropyl, cyclobutyl, cyclopentyl, cyclohexyl, phenyl, naphthyl, indanyl or indolyl,
   or Rₑ is hydroxy, halogen, oxo, carboxy or cyano;
or the pharmaceutically acceptable salts, solvates, or tautomers thereof.

In another embodiment, the compounds of formula (I) wherein:
R₄ is hydrogen, a C1-6 alkyl group, phenylC1-6alkyl, cyclopropylC1-6alkyl, cyclohexylC1-6alkyl or pyridinyl;
or the pharmaceutically acceptable salts, solvates, or tautomers thereof.

### Variable Q

The compounds of formula (I) wherein:
Q is R_{g}, C(O)R_{g} or S(O)₂ R_{g};
wherein R_{g} is C1-5alkyl wherein one or more C atoms are optionally replaced by O or NH, phenyl, naphthyl, pyrrolidinyl, piperidinyl, morpholinyl, thiomorpholinyl, piperazinyl, furanyl, thienyl, thiazolyl, benzofuranyl, benzothienyl, quinolyl, isoquinolyl, isoindolinyl, tetrahydroisoquinolyl, pyrazinyl or quinoxalinyl, or amino; wherein R_{g} is optionally substituted by one or more Rₕ;
Rₕ is C1-5 alkyl, C1-5alkoxy, halogen, phenyl, naphthyl, indenyl, indanyl, morpholinyl, thiomorpholinyl, pyridinyl, isoquinolinyl, isoindolinyl, tetrahydroisoquinolyl, benzodioxolyl, piperidinylamino or piperazinylcarbonylamino;
Rₕ may be further optionally substituted by one or more Rᵢ;
Rᵢ is C1-3 alkyl, C3-6 cycloalkyl, phenyl, naphthyl, benzyl, morpholinyl, C1-5 alkoxy, C1-5 alkoxycarbonyl, halogen, hydroxy, oxo, carboxy, cyano or nitro;
or the pharmaceutically acceptable salts, solvates, or tautomers thereof.

In another embodiment, the compounds of formula (I) wherein:
Q is C(O)R_{g};
   wherein R_{g} is piperidinyl, morpholinyl, thiomorpholinyl, piperazinyl, isoindolinyl, benzofuranyl, tetrahydroisoquinolyl, phenylC-1-Salkylamino or naphthylC1-5alkylamino, each optionally substituted by C1-5alkyl, morpholinyl or morpholinylC1-5alkoxy;
   or the pharmaceutically acceptable salts, solvates, or tautomers thereof

In another embodiment, the present invention is directed to compounds of subgeneric Formula (Ia) as set forth below, wherein Q, R₄ and R₁ are defined as the A, B and C groups, respectively, in Table I below. Any and all combinations of the A, B, and C groups in Table I within the structural limitations of Formula (Ia), comprise compounds of the invention. That is, Q is independently selected from groups A1 to A34; R₄ is independently selected from groups B1 to B26; and R₁ is independently selected from groups C1 to C41, from within Table I. For example, the compound: 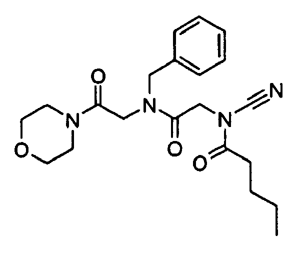 would represent the combination of A6, B17 and C2.

The compounds of formula (Ia): 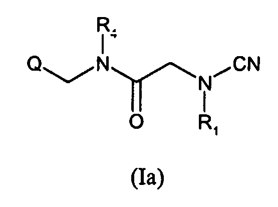 wherein Q is independently selected from groups A1 to A34; R₄ is independently selected from groups B1 to B26; and R₁ is independently selected from groups C1 to C41; wherein groups A1 to A34, B1 to B26 and C1 to C41 are as defined in the following Table I:

**Table I**

| **A** | **Q** | **B** | **R₄** | **C** | **R₁** |
|---|---|---|---|---|---|
| A1 | 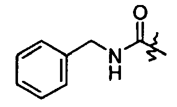 | B1 | -H | C1 | -H |
| A2 | 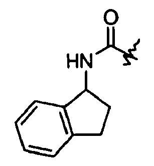 | B2 | -CH₃ | C2 | 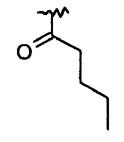 |
| A3 | 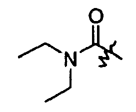 | B3 | 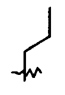 | C3 | 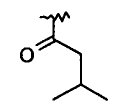 |
| A4 | 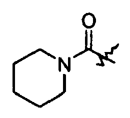 | B4 | 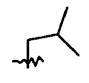 | C4 | 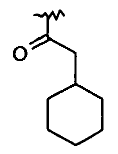 |
| A5 | 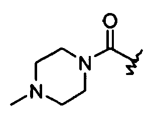 | B5 | 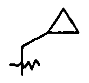 | C5 | 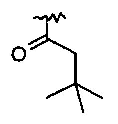 |
| A6 | 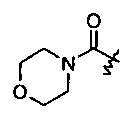 | B6 | 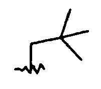 | C6 | 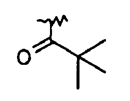 |
| A7 | 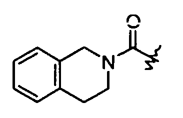 | B7 | 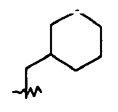 | C7 | 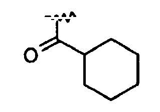 |
| A8 | 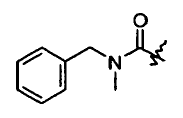 | B8 | 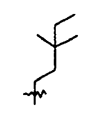 | C8 | 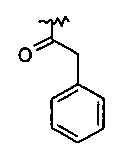 |
| A9 | 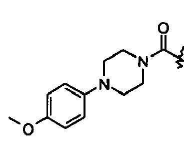 | B9 | 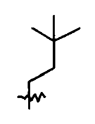 | C9 | 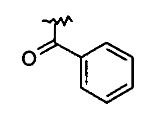 |
| A10 | 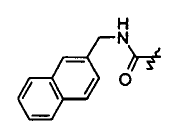 | B10 | 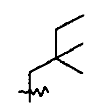 | C10 | 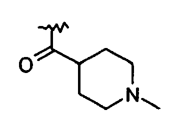 |
| A11 | 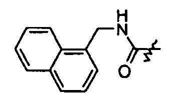 | B11 | 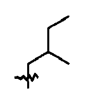 | C11 | 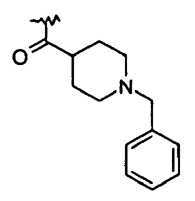 |
| A12 | 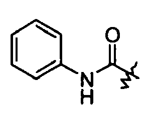 | B12 | 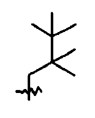 | C12 | 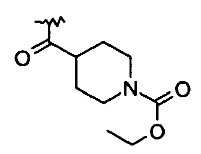 |
| A13 | 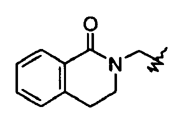 | B13 | 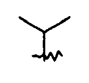 | C13 | 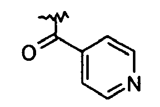 |
| A14 | 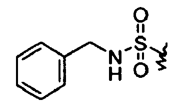 | B14 | 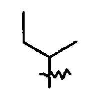 | C14 | 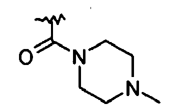 |
| A15 | 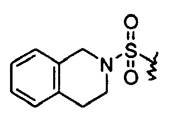 | B15 | 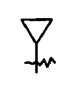 | C15 | 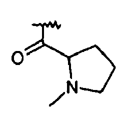 |
| A16 | 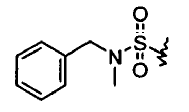 | B16 | 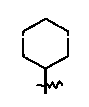 | C16 | 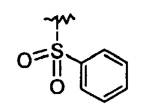 |
| A17 | 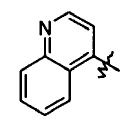 | B17 | 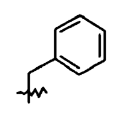 | C17 | 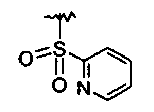 |
| A18 | 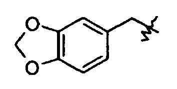 | B18 | 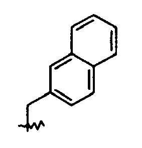 | C18 | -CH₃ |
| A19 | 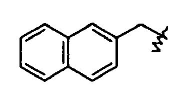 | B19 | 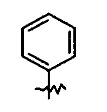 | C19 | 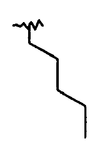 |
| A20 | 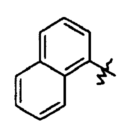 | B20 | 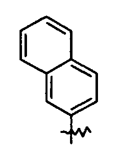 | C20 | 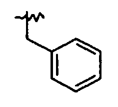 |
| A21 | 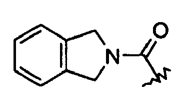 | B21 | 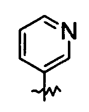 | C21 | 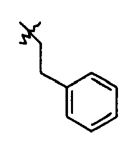 |
| A22 | 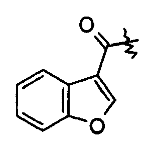 | B22 | 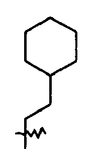 | C22 | 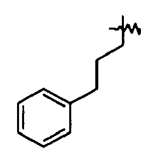 |
| A23 | 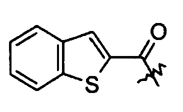 | B23 | 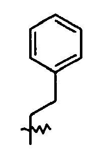 | C23 | 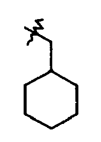 |
| A24 | 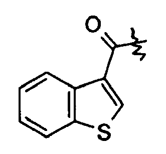 | B24 | 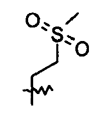 | C24 | 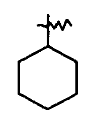 |
| A25 | 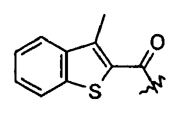 | B25 | 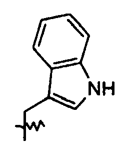 | C25 | 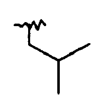 |
| A26 | 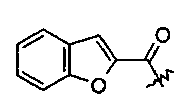 | B26 | 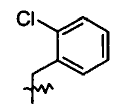 | C26 | 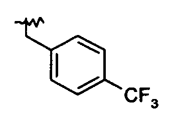 |
| A27 | 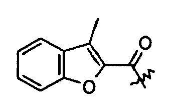 | | | C27 | 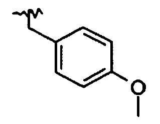 |
| A28 | 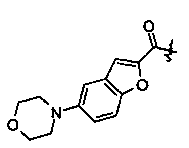 | | | C28 | 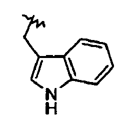 |
| A29 | 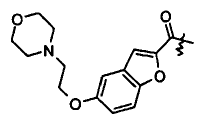 | | | C29 | 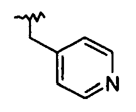 |
| A30 | 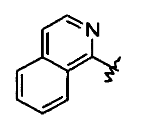 | | | C30 | 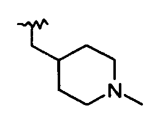 |
| A31 | 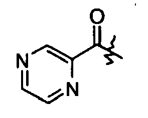 | | | C31 | 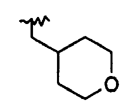 |
| A32 | 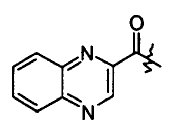 | | | C32 | 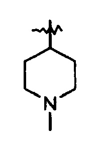 |
| A33 | 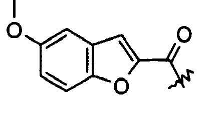 | | | C33 | 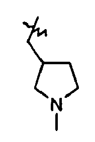 |
| A34 | 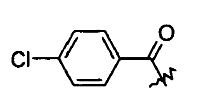 | | | C34 | 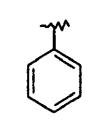 |
| | | | | C35 | 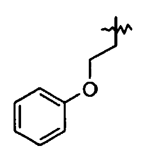 |
| | | | | C36 | 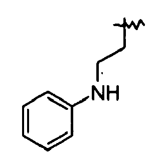 |
| | | | | C37 | 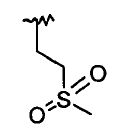 |
| | | | | C38 | 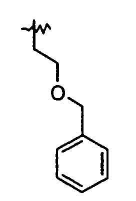 |
| | | | | C39 | 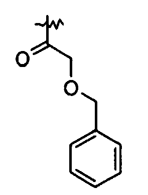 |
| | | | | C40 | 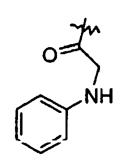 |
| | | | | C41 | 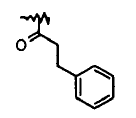 |

or the pharmaceutically acceptable salts, solvates, or tautomers thereof Listed below are specific compounds of the invention falling within the scope of Table I above and postulated to preferably possess Cathepsin K or Cathepsin S activity as indicated. The compounds are listed as specific combinations of the A, B and C groups found in Table I, in which the A, B and C groups listed identify the Q, R₄ and R₁ groups, respectively, in the compound of formula (Ia) above. For example, the following specific combination: "A6 B4 C8" would represent the following specific compound:

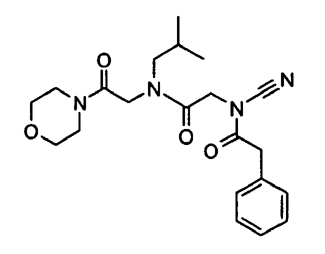

Specific compounds that are preferred for Cathepsin K inhibitory activity:

| | | | | | | | | | |
|---|---|---|---|---|---|---|---|---|---|
| A1 B4 C1 | A1 B5 C1 | A1 B6 C1 | A1 B7 C1 | A1 B11 C1 | A1 B13 C1 | A1 B14 C1 | A1 B15 C1 | A1 B16 C1 | A1 B17 C1 |
| A1 B4 C2 | A1 B5 C2 | A1 B6 C2 | A1 B7 C2 | A1 B11 C2 | A1 B13 C2 | A1 B14 C2 | A1 B15 C2 | A1 B16 C2 | A1 B17 C2 |
| A1 B4 C3 | A1 B5 C3 | A1 B6 C3 | A1 B7 C3 | A1 B11 C3 | A1 B13 C3 | A1 B14 C3 | A1 B15 C3 | A1 B16 C3 | A1 B17 C3 |
| A1 B4 C4 | A1 B5 C4 | A1 B6 C4 | A1 B7 C4 | A1 B11 C4 | A1 B13 C4 | A1 B14 C4 | A1 B15 C4 | A1 B16 C4 | A1 B17 C4 |
| A1 B4 C5 | A1 B5 C5 | A1 B6 C5 | A1 B7 C5 | A1 B11 C5 | A1 B13 C5 | A1 B14 C5 | A1 B15 C5 | A1 B16 C5 | A1 B17 C5 |
| A1 B4 C6 | A1 B5 C6 | A1 B6 C6 | A1 B7 C6 | A1 B11 C6 | A1 B13 C6 | A1 B14 C6 | A1 B15 C6 | A1 B16 C6 | A1 B17 C6 |
| A1 B4 C7 | A1 B5 C7 | A1 B6 C7 | A1 B7 C7 | A1 B11 C7 | A1 B13 C7 | A1 B14 C7 | A1 B15 C7 | A1 B16 C7 | A1 B17 C7 |
| A1 B4 C8 | A1 B5 C8 | A1 B6 C8 | A1 B7 C8 | A1 B11 C8 | A1 B13 C8 | A1 B14 C8 | A1 B15 C8 | A1 B16 C8 | A1 B17 C8 |
| A1 B4 C9 | A1 B5 C9 | A1 B6 C9 | A1 B7 C9 | A1 B11 C9 | A1 B13 C9 | A1 B14 C9 | A1 B15 C9 | A1 B16 C9 | A1 B17 C9 |
| A1 B4 C10 | A1 B5 C10 | A1 B6 C10 | A1 B7 C10 | A1 B11 C10 | A1 B13 C10 | A1 B14 C10 | A1 B15 C10 | A1 B16 C10 | A1 B17 C10 |
| A1 B4 C11 | A1 B5 C11 | A1 B6 C11 | A1 B7 C11 | A1 B11 C11 | A1 B13 C11 | A1 B14 C11 | A1 B15 C11 | A1 B16 C11 | A1 B17 C11 |
| A1 B4 C12 | A1 B5 C12 | A1 B6 C12 | A1 B7 C12 | A1 B11 C12 | A1 B13 C12 | A1 B14 C12 | A1 B15 C12 | A1 B16 C12 | A1 B17 C12 |
| A1 B4 C13 | A1 B5 C13 | A1 B6 C13 | A1 B7 C13 | A1 B11 C13 | A1 B13 C13 | A1 B14 C13 | A1 B15 C13 | A1 B16 C13 | A1 B17 C13 |
| A1 B4 C14 | A1 B5 C14 | A1 B6 C14 | A1 B7 C14 | A1 B11 C14 | A1 B13 C14 | A1 B14 C14 | A1 B15 C14 | A1 B16 C14 | A1 B17 C14 |
| A1 B4 C15 | A1 B5 C15 | A1 B6 C15 | A1 B7 C15 | A1 B11 C15 | A1 B13 C15 | A1 B14 C15 | A1 B15 C15 | A1 B16 C15 | A1 B17 C15 |
| A1 B4 C16 | A1 B5 C16 | A1 B6 C16 | A1 B7 C16 | A1 B11 C16 | A1 B13 C16 | A1 B14 C16 | A1 B15 C16 | A1 B16 C16 | A1 B17 C16 |
| A1 B4 C17 | A1 B5 C17 | A1 B6 C17 | A1 B7 C17 | A1 B11 C17 | A1 B13 C17 | A1 B14 C17 | A1 B15 C17 | A1 B16 C17 | A1 B17 C17 |
| A1 B4 C18 | A1 B5 C18 | A1 B6 C18 | A1 B7 C18 | A1 B11 C18 | A1 B13 C18 | A1 B14 C18 | A1 B15 C18 | A1 B16 C18 | A1 B17 C18 |
| A1 B4 C19 | A1 B5 C19 | A1 B6 C19 | A1 B7 C19 | A1 B11 C19 | A1 B13 C19 | A1 B14 C19 | A1 B15 C19 | A1 B16 C19 | A1 B17 C19 |
| A1 B4 C20 | A1 B5 C20 | A1 B6 C20 | A1 B7 C20 | A1 B11 C20 | A1 B13 C20 | A1 B14 C20 | A1 B15 C20 | A1 B16 C20 | A1 B17 C20 |
| A1 B4 C21 | A1 B5 C21 | A1 B6 C21 | A1 B7 C21 | A1 B11 C21 | A1 B13 C21 | A1 B14 C21 | A1 B15 C21 | A1 B16 C21 | A1 B17 C21 |
| A1 B4 C22 | A1 B5 C22 | A1 B6 C22 | A1 B7 C22 | A1 B11 C22 | A1 B13 C22 | A1 B14 C22 | A1 B15 C22 | A1 B16 C22 | A1 B17 C22 |
| A1 B4 C23 | A1 B5 C23 | A1 B6 C23 | A1 B7 C23 | A1 B11 C23 | A1 B13 C23 | A1 B14 C23 | A1 B15 C23 | A1 B16 C23 | A1 B17 C23 |
| A1 B4 C24 | A1 B5 C24 | A1 B6 C24 | A1 B7 C24 | A1 B11 C24 | A1 B13 C24 | A1 B14 C24 | A1 B15 C24 | A1 B16 C24 | A1 B17 C24 |
| A1 B4 C25 | A1 B5 C25 | A1 B6 C25 | A1 B7 C25 | A1 B11 C25 | A1 B13 C25 | A1 B14 C25 | A1 B15 C25 | A1 B16 C25 | A1 B17 C25 |
| A1 B4 C26 | A1 B5 C26 | A1 B6 C26 | A1 B7 C26 | A1 B11 C26 | A1 B13 C26 | A1 B14 C26 | A1 B15 C26 | A1 B16 C26 | A1 B17 C26 |
| A1 B4 C27 | A1 B5 C27 | A1 B6 C27 | A1 B7 C27 | A1 B11 C27 | A1 B13 C27 | A1 B14 C27 | A1 B15 C27 | A1 B16 C27 | A1 B17 C27 |
| A1 B4 C28 | A1 B5 C28 | A1 B6 C28 | A1 B7 C28 | A1 B11 C28 | A1 B13 C28 | A1 B14 C28 | A1 B15 C28 | A1 B16 C29 | A1 B17 C28 |
| A1 B4 C29 | A1 B5 C29 | A1 B6 C29 | A1 B7 C29 | A1 B11 C29 | A1 B13 C29 | A1 B14 C29 | A1 B15 C29 | A1 B16 C29 | A1 B17 C29 |
| A1 B4 C30 | A1 B5 C30 | A1 B6 C30 | A1 B7 C30 | A1 B11 C30 | A1 B13 C30 | A1 B14 C30 | A1 B15 C30 | A1 B16 C30 | A1 B17 C30 |
| A1 B4 C31 | A1 B5 C31 | A1 B6 C31 | A1 B7 C31 | A1 B11 C31 | A1 B13 C31 | A1 B14 C31 | A1 B15 C31 | A1 B16 C31 | A1 B17 C31 |
| A1 B4 C32 | A1 B5 C32 | A1 B6 C32 | A1 B7 C32 | A1 B11 C32 | A1 B13 C32 | A1 B14 C32 | A1 B15 C32 | A1 B16 C32 | A1 B17 C32 |
| A1 B4 C33 | A1 B5 C33 | A1 B6 C33 | A1 B7 C33 | A1 B11 C33 | A1 B13 C33 | A1 B14 C33 | A1 B15 C33 | A1 B16 C33 | A1 B17 C33 |
| A1 B4 C34 | A1 B5 C34 | A1 B6 C34 | A1 B7 C34 | A1 B11 C34 | A1 B13 C34 | A1 B14 C34 | A1 B15 C34 | A1 B16 C34 | A1 B17 C34 |
| A1 B4 C35 | A1 B5 C35 | A1 B6 C35 | A1 B7 C35 | A1 B11 C35 | A1 B13 C35 | A1 B14 C35 | A1 B15 C35 | A1 B16 C35 | A1 B17 C35 |
| A1 B4 C36 | A1 B5 C36 | A1 B6 C36 | A1 B7 C36 | A1 B11 C36 | A1 B13 C36 | A1 B14 C36 | A1 B15 C36 | A1 B16 C36 | A1 B17 C36 |
| A1 B4 C37 | A1 B5 C37 | A1 B6 C37 | A1 B7 C37 | A1 B11 C37 | A1 B13 C37 | A1 B14 C37 | A1 B15 C37 | A1 B16 C37 | A1 B17 C37 |
| A1 B4 C38 | A1 B5 C38 | A1 B6 C38 | A1 B7 C38 | A1 B11 C38 | A1 B13 C38 | A1 B14 C38 | A1 B15 C38 | A1 B16 C38 | A1 B17 C38 |
| A1 B4 C39 | A1 B5 C39 | A1 B6 C39 | A1 B7 C39 | A1 Bit C39 | A1 B13 C39 | A1 B14 C39 | A1 B15 C39 | A1 B16 C39 | A1 B17 C39 |
| A1 B4 C40 | A1 B5 C40 | A1 B6 C40 | A1 B7 C40 | A1 B11 C40 | A1 B13 C40 | A1 B14 C40 | A1 B15 C40 | A1 B16 C40 | A1 B17 C40 |
| A1 B4 C41 | A1 B5 C41 | A1 B6 C41 | A1 B7 C41 | A1 B11 C41 | A1 B13 C41 | A1 B14 C41 | A1 B15 C41 | A1 B16 C41 | A1 B17 C41 |
| | | | | | | | | | |
| A5 B4 C1 | A5 B5 C1 | A5 B6 C1 | A5 B7 C1 | A5 B11 C1 | A5 B13 C1 | A5 B14 C1 | A5 B15 C1 | A5 B16 C1 | A5 B17 C1 |
| A5 B4 C2 | A5 B5 C2 | A5 B6 C2 | A5 B7 C2 | A5 B11 C2 | A5 B13 C2 | A5 B14 C2 | A5 B15 C2 | A5 B16 C2 | A5 B17 C2 |
| A5 B4 C3 | A5 B5 C3 | A5 B6 C3 | A5 B7 C3 | A5 B11 C3 | A5 B13 C3 | A5 B14 C3 | A5 B15 C3 | A5 B16 C3 | A5 B17 C3 |
| A5 B4 C4 | A5 B5 C4 | A5 B6 C4 | A5 B7 C4 | A5 B11 C4 | A5 B13 C4 | A5 B14 C4 | A5 B15 C4 | A5 B16 C4 | A5 B17 C4 |
| A5 B4 C5 | A5 B5 C5 | A5 B6 C5 | A5 B7 C5 | A5 B11 C5 | A5 B13 C5 | A5 B14 C5 | A5 B15 C5 | A5 B16 C5 | A5 B17 C5 |
| A5 B4 C6 | A5 B5 C6 | A5 B6 C6 | A5 B7 C6 | A5 B11 C6 | A5 B13 C6 | A5 B14 C6 | A5 B15 C6 | A5 B16 C6 | A5 B17 C6 |
| A5 B4 C7 | A5 B5 C7 | A5 B6 C7 | A5 B7 C7 | A5 B11 C7 | A5 B13 C7 | A5 B14 C7 | A5 B15 C7 | A5 B16 C7 | A5 B17 C7 |
| A5 B4 C8 | A5 B5 C8 | A5 B6 C8 | A5 B7 C8 | A5 B11 C8 | A5 B13 C8 | A5 B14 C8 | A5 B15 C8 | A5 B16 C8 | A5 B17 C8 |
| A5 B4 C9 | A5 B5 C9 | A5 B6 C9 | A5 B7 C9 | A5 B11 C9 | A5 B13 C9 | A5 B14 C9 | A5 B15 C9 | A5 B16 C9 | A5 B17 C9 |
| A5 B4 C10 | A5 B5 C10 | A5 B6 C10 | A5 B7 C10 | A5 B11 C10 | A5 B13 C10 | A5 B14 C10 | A5 B15 C10 | A5 B16 C10 | A5 B17 C10 |
| A5 B4 C11 | A5 B5 C11 | A5 B6 C11 | A5 B7 C11 | A5 B11 C11 | A5 B13 C11 | A5 B14 C11 | A5 B15 C11 | A5 B16 C11 | A5 B17 C11 |
| A5 B4 C12 | A5 B5 C12 | A5 B6 C12 | A5 B7 C12 | A5 B11 C12 | A5 B13 C12 | A5 B14 C12 | A5 B15 C12 | A5 B16 C12 | A5 B17 C12 |
| A5 B4 C13 | A5 B5 C13 | A5 B6 C13 | A5 B7 C13 | A5 B11 C13 | A5 B13 C13 | A5 B14 C13 | A5 B15 C13 | A5 B16 C13 | A5 B17 C13 |
| A5 B4 C14 | A5 B5 C14 | A5 B6 C14 | A5 B7 C14 | A5 B11 C14 | A5 B13 C14 | A5 B14 C14 | A5 B15 C14 | A5 B16 C14 | A5 B17 C14 |
| A5 B4 C15 | A5 B5 C15 | A5 B6 C15 | A5 B7 C15 | A5 B11 C15 | A5 B13 C15 | A5 B14 C15 | A5 B15 C15 | A5 B16 C15 | A5 B17 C15 |
| A5 B4 C16 | A5 B5 C16 | A5 B6 C16 | A5 B7 C16 | A5 B11 C16 | A5 B13 C16 | A5 B14 C16 | A5 B15 C16 | A5 B16 C16 | A5 B17 C16 |
| A5 B4 C17 | A5 B5 C17 | A5 B6 C17 | A5 B7C17 | A5 B11 C17 | A5 B13 C17 | A5 B14 C17 | A5 B15 C17 | A5 B16 C17 | A5 B17 C17 |
| A5 B4 C18 | A5 B5 C18 | A5 B6 C18 | A5 B7 C18 | A5 B11 C18 | A5 B13 C18 | A5 B14 C18 | A5 B15 C18 | A5 B16 C18 | A5 B17 C18 |
| A5 B4 C19 | A5 B5 C19 | A5 B6 C19 | A5 B7 C19 | A5 B11 C19 | A5 B13 C19 | A5 B14 C19 | A5 B15 C19 | A5 B16 C19 | A5 B17 C19 |
| A5 B4 C20 | A5 B5 C20 | A5 B6 C20 | A5 B7 C20 | A5 B11 C20 | A5 B13 C20 | A5 B14 C20 | A5 B15 C20 | A5 B16 C20 | A5 B17 C20 |
| A5 B4 C21 | A5 B5 C21 | A5 B6 C21 | A5 B7 C21 | A5 B11 C21 | A5 B13 C21 | A5 B14 C21 | A5 B15 C21 | A5 B16 C21 | A5 B17 C21 |
| A5 B4 C22 | A5 B5 C22 | A5 B6 C22 | A5 B7 C22 | A5 B11 C22 | A5 B13 C22 | A5 B14 C22 | A5 B15 C22 | A5 B16 C22 | A5 B17 C22 |
| A5 B4 C23 | A5 B5 C23 | A5 B6 C23 | A5 B7 C23 | A5 B11 C23 | A5 B13 C23 | A5 B14 C23 | A5 B15 C23 | A5 B16 C23 | A5 B17 C23 |
| A5 B4 C24 | A5 B5 C24 | A5 B6 C24 | A5 B7 C24 | A5 B11 C24 | A5 B13 C24 | A5 B14 C24 | A5 B15 C24 | A5 B16 C24 | A5 B17 C24 |
| A5 B4 C25 | A5 B5 C25 | A5 B6 C25 | A5 B7 C25 | A5 B11 C25 | A5 B13 C25 | A5 B14 C25 | A5 B15 C25 | A5 B16 C25 | A5 B17 C25 |
| A5 B4 C26 | A5 B5 C26 | A5 B6 C26 | A5 B7 C26 | A5 B11 C26 | A5 B13 C26 | A5 B14 C26 | A5 B15 C26 | A5 B16 C26 | A5 B17 C26 |
| A5 B4 C27 | A5 B5 C27 | A5 B6 C27 | A5 B7 C27 | A5 B11 C27 | A5 B13 C27 | A5 B14 C27 | A5 B15 C27 | A5 B16 C17 | A5 B17 C27 |
| A5 B4 C28 | A5 B5 C28 | A5 B6 C28 | A5 B7 C28 | A5 B11 C28 | A5 B13 C28 | A5 B14 C28 | A5 B15 C28 | A5 516 C28 | A5 B17 C28 |
| A5 B4 C29 | A5 B5 C29 | A5 B6 C29 | A5 B7 C29 | A5 B11 C29 | A5 B13 C29 | A5 B14 C29 | A5 B15 C29 | A5 B16 C29 | A5 B17 C29 |
| A5 B4 C30 | A5 B5 C30 | A5 B6 C30 | A5 B7 C30 | A5 B11 C30 | A5 B13 C30 | A5 B14 C30 | A5 B15 C30 | A5 B16 C30 | A5 B17 C30 |
| A5 B4 C31 | A5 B5 C31 | A5 B6 C31 | A5 B7 C31 | A5 B11 C31 | A5 B13 C31 | A5 B14 C31 | A5 B15 C31 | A5 B16 C31 | A5 B17 C31 |
| A5 B4 C32 | A5 B5 C32 | A5 B6 C32 | A5 B7 C32 | A5 B11 C32 | A5 B13 C32 | A5 B14 C32 | A5 B15 C32 | A5 B16 C32 | A5 B17 C32 |
| A5 B4 C33 | A5 B5 C33 | A5 B6 C33 | A5 B7 C33 | A5 B11 C33 | A5 B13 C33 | A5 B14 C33 | A5 B15 C33 | A5 B16 C33 | A5 B17 C33 |
| A5 B4 C34 | A5 B5 C34 | A5 B6 C34 | A5 B7 C34 | A5 B11 C34 | A5 B13 C34 | A5 B14 C34 | A5 B15 C34 | A5 B16 C34 | A5 B17 C34 |
| A5 B4 C35 | A5 B5 C35 | A5 B6 C35 | A5 B7 C35 | A5 B11 C35 | A5 B13 C35 | A5 B14 C35 | A5 B15 C35 | A5 B16 C35 | A5 B17 C35 |
| A5 B4 C36 | A5 B5 C36 | A5 B6 C36 | A5 B7 C36 | A5 B11 C36 | A5 B13 C36 | A5 B14 C36 | A5 B15 C36 | A5 B16 C36 | A5 B17 C36 |
| A5 B4 C37 | A5 B5 C37 | A5 B6 C37 | A5 B7 C37 | A5 B11 C37 | A5 B13 C37 | A5 B14 C37 | A5 B15 C37 | A5 B16 C37 | A5 B17 C37 |
| A5 B4 C38 | A5 B5 C38 | A5 B6 C38 | A5 B7 C38 | A5 B11 C38 | A5 B13 C38 | A5 B14 C38 | A5 B15 C38 | A5 B16 C38 | A5 B17 C38 |
| A5 B4 C39 | A5 B5 C39 | A5 B6 C39 | A5 B7 C39 | A5 B11 C39 | A5 B13 C39 | A5 B14 C39 | A5 B15 C39 | A5 B16 C39 | A5 B17 C39 |
| A5 B4 C40 | A5 B5 C40 | A5 B6 C40 | A5 B7 C40 | A5 B11 C40 | A5 B13 C40 | A5 B14 C40 | A5 B15 C40 | A5 B16 C40 | A5 B17 C40 |
| A5 B4 C41 | A5 B5 C41 | A5 B6 C41 | A5 B7 C41 | A5 B11 C41 | A5 B13 C41 | A5 B14 C41 | A5 B15 C41 | A5 B16 C41 | A5 B17 C41 |
| | | | | | | | | | |
| A6 B4 C1 | A6 B5 C1 | A6 B6 C1 | A6 B7 C1 | A6 B11 C1 | A6 B13 C1 | A6 B14 C1 | A6 B15 C1 | A6 B16 C1 | A6 B17 C1 |
| A6 B4 C2 | A6 B5 C2 | A6 B6 C2 | A6 B7 C2 | A6 B11 C2 | A6 B13 C2 | A6 B14 C2 | A6 B15 C2 | A6 B16 C2 | A6 B17 C2 |
| A6 B4 C3 | A6 B5 C3 | A6 B6 C3 | A6 B7 C3 | A6 B11 C3 | A6 B13 C3 | A6 B14 C3 | A6 B15 C3 | A6 B16 C3 | A6 B17 C3 |
| A6 B4 C4 | A6 B5 C4 | A6 B6 C4 | A6 B7 C4 | A6 B11 C4 | A6 B13 C4 | A6 B14 C4 | A6 B15 C4 | A6 B16 C4 | A6 B17 C4 |
| A6 B4 C5 | A6 B5 C5 | A6 B6 C5 | A6 B7 C5 | A6 B11 C5 | A6 B13 C5 | A6 B14 C5 | A6 B15 C5 | A6 B16 C5 | A6 B17 C5 |
| A6 B4 C6 | A6 B5 C6 | A6 B6 C6 | A6 B7 C6 | A6 B11 C6 | A6 B13 C6 | A6 B14 C6 | A6 B15 C6 | A6 B16 C6 | A6 B17 C6 |
| A6 B4 C7 | A6 B5 C7 | A6 B6 C7 | A6 B7 C7 | A6 B11 C7 | A6 B13 C7 | A6 B14 C7 | A6 B15 C7 | A6 B16 C7 | A6 B17 C7 |
| A6 B4 C8 | A6 B5 C8 | A6 B6 C8 | A6 B7 C8 | A6 B11 C8 | A6 B13 C8 | A6 B14 C8 | A6 B15 C8 | A6 B16 C8 | A6 B17 C8 |
| A6 B4 C9 | A6 B5 C9 | A6 B6 C9 | A6 B7 C9 | A6 B11 C9 | A6 B13 C9 | A6 B14 C9 | A6 B15 C9 | A6 B16 C9 | A6 B17 C9 |
| A6 B4 C10 | A6 B5 C10 | A6 B6 C10 | A6 B7 C10 | A6 B11 C10 | A6 B13 C10 | A6 B14 C10 | A6 B15 C10 | A6 B16 C10 | A6 B17 C10 |
| A6 B4 C11 | A6 B5 C11 | A6 B6 C11 | A6 B7 C11 | A6 B11 C11 | A6 B13 C11 | A6 B14 C11 | A6 B15 C11 | A6 B16 C11 | A6 B17 C11 |
| A6 B4 C12 | A6 B5 C12 | A6 B6 C12 | A6 B7 C12 | A6 B11 C12 | A6 B13 C12 | A6 B14 C12 | A6 B15 C12 | A6 B16 C12 | A6 B17 C12 |
| A6 B4 C13 | A6 B5 C13 | A6 B6 C13 | A6 B7 C13 | A6 B11 C13 | A6 B13 C13 | A6 B14 C13 | A6 B15 C13 | A6 B16 C13 | A6 B17 C13 |
| A6 B4 C14 | A6 B5 C14 | A6 B6 C14 | A6 B7 C14 | A6 B11 C14 | A6 B13 C14 | A6 B14 C14 | A6 B15 C14 | A6 B16 C14 | A6 B17 C14 |
| A6 B4 C15 | A6 B5 C15 | A6 B6 C15 | A6 B7 C15 | A6 B11 C15 | A6 B13 C15 | A6 B14 C15 | A6 B15 C15 | A6 B16 C15 | A6 B17 C15 |
| A6 B4 C16 | A6 B5 C16 | A6 B6 C16 | A6 B7 C16 | A6 B11 C16 | A6 B13 C16 | A6 B14 C16 | A6 B15 C16 | A6 B16 C16 | A6 B17 C16 |
| A6 B4 C17 | A6 B5 C17 | A6 B6 C17 | A6 B7 C17 | A6 B11 C17 | A6 B13 C17 | A6 B14 C17 | A6 B15 C17 | A6 B16 C17 | A6 B17 C17 |
| A6 B4 C18 | A6 B5 C18 | A6 B6 C18 | A6 B7 C18 | A6 B11 C18 | A6 B13 C18 | A6 B14 C18 | A6 B15 C18 | A6 B16 C18 | A6 B17 C18 |
| A6 B4 C19 | A6 B5 C19 | A6 B6 C19 | A6 B7 C19 | A6 B11 C19 | A6 B13 C19 | A6 B14 C19 | A6 B15 C19 | A6 B16 C19 | A6 B17 C19 |
| A6 B4 C20 | A6 B5 C20 | A6 B6 C20 | A6 B7 C20 | A6 B11 C20 | A6 B13 C20 | A6 B14 C20 | A6 B15 C20 | A6 B16 C20 | A6 B17 C20 |
| A6 B4 C21 | A6 B5 C21 | A6 B6 C21 | A6 B7 C21 | A6 B11 C21 | A6 B13 C21 | A6 B14 C21 | A6 B15 C21 | A6 B16 C21 | A6 B17 C21 |
| A6 B4 C22 | A6 B5 C22 | A6 B6 C22 | A6 B7 C22 | A6 B11 C22 | A6 B13 C22 | A6 B14 C22 | A6 B15 C22 | A6 B16 C22 | A6 B17 C22 |
| A6 B4 C23 | A6 B5 C23 | A6 B6 C23 | A6 B7 C23 | A6 B11 C23 | A6 B13 C23 | A6 B14 C23 | A6 B15 C23 | A6 B16 C23 | A6 B17 C23 |
| A6 B4 C24 | A6 B5 C24 | A6 B6 C24 | A6 B7 C24 | A6 B11 C24 | A6 B13 C24 | A6 B14 C24 | A6 B15 C24 | A6 B16 C24 | A6 B17 C24 |
| A6 B4 C25 | A6 B5 C25 | A6 B6 C25 | A6 B7 C25 | A6 B11 C25 | A6 B13 C25 | A6 B14 C25 | A6 B15 C25 | A6 B16 C25 | A6 B17 C25 |
| A6 B4 C26 | A6 B5 C26 | A6 B6 C26 | A6 B7 C26 | A6 B11 C26 | A6 B13 C26 | A6 B14 C26 | A6 B15 C26 | A6 B16 C26 | A6 B17 C26 |
| A6 B4 C27 | A6 B5 C27 | A6 B6 C27 | A6 B7 C27 | A6 B11 C27 | A6 B13 C27 | A6 B14 C27 | A6 B15 C27 | A6 B16 C27 | A6 B17 C27 |
| A6 B4 C28 | A6 B5 C28 | A6 B6 C28 | A6 B7 C28 | A6 B11 C28 | A6 B13 C28 | A6 B14 C28 | A6 B15 C28 | A6 B16 C28 | A6 B17 C28 |
| A6 B4 C29 | A6 B5 C29 | A6 B6 C29 | A6 B7 C29 | A6 B11 C29 | A6 B13 C29 | A6 B14 C29 | A6 B15 C29 | A6 B16 C29 | A6 B17 C29 |
| A6 B4 C30 | A6 B5 C30 | A6 B6 C30 | A6 B7 C30 | A6 B11 C30 | A6 B13 C30 | A6 B14 C30 | A6 B15 C30 | A6 B16 C30 | A6 B17 C30 |
| A6 B4 C31 | A6 B5 C31 | A6 B6 C31 | A6 B7 C31 | A6 B11 C31 | A6 B13 C31 | A6 B14 C31 | A6 B15 C31 | A6 B16 C31 | A6 B17 C31 |
| A6 B4 C32 | A6 B5 C32 | A6 B6 C32 | A6 B7 C32 | A6 B11 C32 | A6 B13 C32 | A6 B14 C32 | A6 B15 C32 | A6 B16 C32 | A6 B17 C32 |
| A6 B4 C33 | A6 B5 C33 | A6 B6 C33 | A6 B7 C33 | A6 B11 C33 | A6 B13 C33 | A6 B14 C33 | A6 B15 C33 | A6 B16 C33 | A6 B17 C33 |
| A6 B4 C34 | A6 B5 C34 | A6 B6 C34 | A6 B7 C34 | A6 B11 C34 | A6 B13 C34 | A6 B14 C34 | A6 B15 C34 | A6 B16 C34 | A6 B17 C34 |
| A6 B4 C35 | A6 B5 C35 | A6 B6 C35 | A6 B7 C35 | A6 B11 C35 | A6 B13 C35 | A6 B14 C35 | A6 B15 C35 | A6 B16 C35 | A6 B17 C35 |
| A6 B4 C36 | A6 B5 C36 | A6 B6 C36 | A6 B7 C36 | A6 B11 C36 | A6 B13 C36 | A6 B14 C36 | A6 B15 C36 | A6 B16 C36 | A6 B17 C36 |
| A6 B4 C37 | A6 B5 C37 | A6 B6 C37 | A6 B7 C37 | A6 B11 C37 | A6 B13 C37 | A6 B14 C37 | A6 B15 C37 | A6 B16 C37 | A6 B17 C37 |
| A6 B4 C38 | A6 B5 C38 | A6 B6 C38 | A6 B7 C38 | A6 B11 C38 | A6 B13 C38 | A6 B14 C38 | A6 B15 C38 | A6 B16 C38 | A6 B17 C38 |
| A6 B4 C39 | A6 B5 C39 | A6 B6 C39 | A6 B7 C39 | A6 B11 C39 | A6 B13 C39 | A6 B14 C39 | A6 B15 C39 | A6 B16 C39 | A6 B17 C39 |
| A6 B4 C40 | A6 B5 C40 | A6 B6 C40 | A6 B7 C40 | A6 B11 C40 | A6 B13 C40 | A6 B14 C40 | A6 B15 C40 | A6 B16 C40 | A6 B17 C40 |
| A6 B4 C41 | A6 85 C41 | A6 B6 C41 | A6 B7 C41 | A6 B11 C41 | A6 B13 C41 | A6 B14 C41 | A6 B15 C41 | A6 B16 C41 | A6 B17 C41 |
| | | | | | | | | | |
| A7 B4 C1 | A7 B5 C1 | A7 B6 C1 | A7 B7 C1 | A7 B11 C1 | A7 B13 C1 | A7 B14 C1 | A7 B15 C1 | A7 B16 C1 | A7 B17 C1 |
| A7 B4 C2 | A7 B5 C2 | A7 B6 C2 | A7 B7 C2 | A7 B11 C2 | A7 B13 C2 | A7 B14 C2 | A7 B15 C2 | A7 B16 C2 | A7 B17 C2 |
| A7 B4 C3 | A7 B5 C3 | A7 B6 C3 | A7 B7 C3 | A7 B11 C3 | A7 B13 C3 | A7 B14 C3 | A7 B15 C3 | A7 B16 C3 | A7 B17 C3 |
| A7 B4 C4 | A7 B5 C4 | A7 B6 C4 | A7 B7 C4 | A7 B11 C4 | A7 B13 C4 | A7 B14 C4 | A7 B15 C4 | A7 B16 C4 | A7 B17 C4 |
| A7 B4 C5 | A7 B5 C5 | A7 B6 C5 | A7 B7 C5 | A7 B11 C5 | A7 B13 C5 | A7 B14 C5 | A7 B15 C5 | A7 B16 C5 | A7 B17 C5 |
| A7 B4 C6 | A7 B5 C6 | A7 B6 C6 | A7 B7 C6 | A7 B11 C6 | A7 B13 C6 | A7 B14 C6 | A7 B15 C6 | A7 B16 C6 | A7 B17 C6 |
| A7 B4 C7 | A7 B5 C7 | A7 B6 C7 | A7 B7 C7 | A7 B11 C7 | A7 B13 C7 | A7 B14 C7 | A7 B15 C7 | A7 B16 C7 | A7 B17 C7 |
| A7 B4 C8 | A7 B5 C8 | A7 B6 C8 | A7 B7 C8 | A7 B11 C8 | A7 B13 C8 | A7 B14 C8 | A7 B15 C8 | A7 B16 C3 | A7 B17 C8 |
| A7 B4 C9 | A7 B5 C9 | A7 B6 C9 | A7 B7 C9 | A7 B11 C9 | A7 B13 C9 | A7 B14 C9 | A7 B15 C9 | A7 B16 C9 | A7 B17 C9 |
| A7 B4 C10 | A7 B5 C10 | A7 B6 C10 | A7 B7 C10 | A7 B11 C10 | A7 B13 C10 | A7 B14 C10 | A7 B15 C10 | A7 B16 C10 | A7 B17 C10 |
| A7 B4 C11 | A7 B5 C11 | A7 B6 C11 | A7 B7 C11 | A7 B11 C11 | A7 B13 C11 | A7 B14 C11 | A7 B15 C11 | A7 B16 C11 | A7 B17 C11 |
| A7 B4 C12 | A7 B5 C12 | A7 B6 C12 | A7 B7 C12 | A7 B11 C12 | A7 B13 C12 | A7 B14 C12 | A7 B15 C12 | A7 B16 C12 | A7 B17 C12 |
| A7 B4 C13 | A7 B5 C13 | A7 B6 C13 | A7 B7 C13 | A7 B11 C13 | A7 B13 C13 | A7 B14 C13 | A7 B15 C13 | A7 B16 C13 | A7 B17 C13 |
| A7 B4 C14 | A7 B5 C14 | A7 B6 C14 | A7 B7 C14 | A7 B11 C14 | A7 B13 C14 | A7 B14 C14 | A7 B15 C14 | A7 B16 C14 | A7 B17 C14 |
| A7 B4 C15 | A7 B5 C15 | A7 B6 C15 | A7 B7 C15 | A7 B11 C15 | A7 B13 C15 | A7 B14 C15 | A7 B15 C15 | A7 B16 C15 | A7 B17 C15 |
| A7 B4 C16 | A7 B5 C16 | A7 B6 C16 | A7 B7 C16 | A7 B11 C16 | A7 B13 C16 | A7 B14 C16 | A7 B15 C16 | A7 B16 C16 | A7 B17 C16 |
| A7 B4 C17 | A7 B5 C17 | A7 B6 C17 | A7 B7 C17 | A7 B11 C17 | A7 B13 C17 | A7 B14 C17 | A7 B15 C17 | A7 B16 C17 | A7 B17 C17 |
| A7 B4 C18 | A7 B5 C18 | A7 B6 C18 | A7 B7 C18 | A7 B11 C18 | A7 B13 C18 | A7 B14 C18 | A7 B15 C18 | A7 B16 C18 | A7 B17 C18 |
| A7 B4 C19 | A7 B5 C19 | A7 B6 C19 | A7 B7 C19 | A7 B11 C19 | A7 B13 C19 | A7 B14 C19 | A7 B15 C19 | A7 B16 C19 | A7 B17 C19 |
| A7 B4 C20 | A7 B5 C20 | A7 B6 C20 | A7 B7 C20 | A7 B11 C20 | A7 B13 C20 | A7 B14 C20 | A7 B15 C20 | A7 B16 C20 | A7 B17 C20 |
| A7 B4 C21 | A7 B5 C21 | A7 B6 C21 | A7 B7 C21 | A7 B11 C21 | A7 B13 C21 | A7 B14 C21 | A7 B15 C21 | A7 B16 C21 | A7 B17 C21 |
| A7 B4 C22 | A7 B5 C22 | A7 B6 C22 | A7 B7 C22 | A7 B11 C22 | A7 B13 C22 | A7 B14 C22 | A7 B15 C22 | A7 B16 C22 | A7 B17 C22 |
| A7 B4 C23 | A7 B5 C23 | A7 B6 C23 | A7 B7 C23 | A7 B11 C23 | A7 B13 C23 | A7 B14 C23 | A7 B15 C23 | A7 B16 C23 | A7 B17 C23 |
| A7 B4 C24 | A7 B5 C24 | A7 B6 C24 | A7 B7 C24 | A7 B11 C24 | A7 B13 C24 | A7 B14 C24 | A7 B15 C24 | A7 B16 C24 | A7 B17 C24 |
| A7 B4 C25 | A7 B5 C25 | A7 B6 C25 | A7 B7 C25 | A7 B11 C25 | A7 B13 C25 | A7 B14 C25 | A7 B15 C25 | A7 B16 C25 | A7 B17 C25 |
| A7 B4 C26 | A7 B5 C26 | A7 B6 C26 | A7 B7 C26 | A7 B11 C26 | A7 B13 C26 | A7 B14 C26 | A7 B15 C26 | A7 B16 C116 | A7 B17 C26 |
| A7 B4 C27 | A7 B5 C27 | A7 B6 C27 | A7 B7 C27 | A7 B11 C27 | A7 B13 C27 | A7 B14 C27 | A7 B15 C27 | A7 B16 C27 | A7 B17 C27 |
| A7 B4 C28 | A7 B5 C28 | A7 B6 C28 | A7 B7 C28 | A7 B11 C28 | A7 B13 C28 | A7 B14 C28 | A7 B15 C28 | A7 B16 C28 | A7 B17 C28 |
| A7 B4 C29 | A7 B5 C29 | A7 B6 C29 | A7 B7 C29 | A7 B11 C29 | A7 B13 C29 | A7 B14 C29 | A7 B15 C29 | A7 B16 C29 | A7 B17 C29 |
| A7 B4 C30 | A7 B5 C30 | A7 B6 C30 | A7 B7 C30 | A7 B11 C30 | A7 B13 C30 | A7 B14 C30 | A7 B15 C30 | A7 B16 C30 | A7 B17 C30 |
| A7 B4 C31 | A7 B5 C31 | A7 B6 C31 | A7 B7 C31 | A7 B11 C31 | A7 B13 C31 | A7 B14 C31 | A7 B15 C31 | A7 B16 C31 | A7 B17 C31 |
| A7 B4 C32 | A7 B5 C32 | A7 B6 C32 | A7 B7 C32 | A7 B11 C32 | A7 B13 C32 | A7 B14 C32 | A7 B15 C32 | A7 B16 C32 | A7 B17 C32 |
| A7 B4 C33 | A7 B5 C33 | A7 B6 C33 | A7 B7 C33 | A7 B11 C33 | A7 B13 C33 | A7 B14 C33 | A7 B15 C33 | A7 B16 C33 | A7 B17 C33 |
| A7 B4 C34 | A7 B5 C34 | A7 B6 C34 | A7 B7 C34 | A7 B11 C34 | A7 B13 C34 | A7 B14 C34 | A7 B15 C34 | A7 B16 C34 | A7 B17 C34 |
| A7 B4 C35 | A7 B5 C35 | A7 B6 C35 | A7 B7 C35 | A7 B11 C35 | A7 B13 C35 | A7 B14 C35 | A7 B15 C35 | A7 B16 C35 | A7 B17 C35 |
| A7 B4 C36 | A7 B5 C36 | A7 B6 C36 | A7 B7 C36 | A7 B11 C36 | A7 B13 C36 | A7 B14 C36 | A7 B15 C36 | A7 B16 C36 | A7 B17 C36 |
| A7 B4 C37 | A7 B5 C37 | A7 B6 C37 | A7 B7 C37 | A7 B11 C37 | A7 B13 C37 | A7 B14 C37 | A7 B15 C37 | A7 B16 C37 | A7 B17 C37 |
| A7 B4 C38 | A7 B5 C38 | A7 B6 C38 | A7 B7 C38 | A7 B11 C38 | A7 B13 C38 | A7 B14 C38 | A7 B15 C38 | A7 B16 C38 | A7 B17 C38 |
| A7 B4 C39 | A7 B5 C39 | A7 B6 C39 | A7 B7 C39 | A7 B11 C39 | A7 B13 C39 | A7 B14 C39 | A7 B15 C39 | A7 B16 C39 | A7 B17 C39 |
| A7 B4 C40 | A7 B5 C40 | A7 B6 C40 | A7 B7 C40 | A7 B11 C40 | A7 B13 C40 | A7 B14 C40 | A7 B15 C40 | A7 B16 C40 | A7 B17 C40 |
| A7 B4 C41 | A7 B5 C41 | A7 B6 C41 | A7 B7 C41 | A7 B11 C41 | A7 B13 C41 | A7 B14 C41 | A7 B15 C41 | A7 B16 C41 | A7 B17 C41 |
| | | | | | | | | | |
| A8 B4 C1 | A8 B5 C1 | A8 B6 C1 | A8 B7 C1 | A8 B11 C1 | A8 B13 C1 | A5 B14 C1 | A8 B15 C1 | A8 B16 C3 | A8 B17 C1 |
| A8 B4 C2 | A8 B5 C2 | A8 B6 C2 | A8 B7 C2 | A8 B11 C2 | A8 B13 C2 | A5 B14 C2 | A8 B15 C2 | A8 B16 C2 | A8 B17 C2 |
| A8 B4 C3 | A8 B5 C3 | A8 B6 C3 | A8 B7 C3 | A8 B11 C3 | A8 B13 C3 | A5 B14 C3 | A8 B15 C3 | A8 B16 C3 | A8 B17 C3 |
| A8 B4 C4 | A8 B5 C4 | A8 B6 C4 | A8 B7 C4 | A8 B11 C4 | A8 B13 C4 | A5 B14 C4 | A8 B15 C4 | A8 B16 C4 | A8 B17 C4 |
| A8 B4 C5 | A8 B5 C5 | A8 B6 C5 | A8 B7 C5 | A8 B11 C5 | A8 B13 C5 | A5 B14 C5 | A8 B15 C5 | A8 B16 C5 | A8 B17 C5 |
| A8 B4 C6 | A8 B5 C6 | A8 B6 C6 | A8 B7 C6 | A8 B11 C6 | A8 B13 C6 | A5 B14 C6 | A8 B15 C6 | A8 B16 C6 | A8 B17 C6 |
| A8 B4 C7 | A8 B5 C7 | A8 B6 C7 | A8 B7 C7 | A8 B11 C7 | A8 B13 C7 | A5 B14 C7 | A8 B15 C7 | A8 B16 C7 | A8 B17 C7 |
| A8 B4 C8 | A8 B5 C8 | A8 B6 C8 | A8 B7 C8 | A8 B11 C8 | A8 B13 C8 | A5 B14 C8 | A8 B15 C8 | A8 B16 C8 | A8 B17 C8 |
| A8 B4 C9 | A8 B5 C9 | A8 B6 C9 | A8 B7 C9 | A8 B11 C9 | A8 B13 C9 | A5 B14 C9 | A8 B15 C9 | A8 B16 C9 | A8 B17 C9 |
| A8 B4 C10 | A8 B5 C10 | A8 B6 C10 | A8 B7 C10 | A8 B11 C10 | A8 B13 C10 | A5 B14 C10 | A8 B15 C10 | A8 B16 C10 | A8 B17 C10 |
| A8 B4 C11 | A8 B5 C11 | A8 B6 C11 | A8 B7 C11 | A8 B11 C11 | A8 B13 C11 | A5 B14 C11 | A8 B15 C11 | A8 B16 C11 | A8 B17 C11 |
| A8 B4 C12 | A8 B5 C12 | A8 B6 C12 | A8 B7 C12 | A8 B11 C12 | A8 B13 C12 | A5 B14 C12 | A8 B15 C12 | A8 B16 C12 | A8 B17 C12 |
| A8 B4 C13 | A8 B5 C13 | A8 B6 C13 | A8 B7 C13 | A8 B11 C13 | A8 B13 C13 | A5 B14 C13 | A8 B15 C13 | A8 B16 C13 | A8 B17 C13 |
| A8 B4 C14 | A8 B5 C14 | A8 B6 C14 | A8 B7 C14 | A8 B11 C14 | A8 B13 C14 | A5 B14 C14 | A8 B15 C14 | A8 B16 C14 | A8 B17 C14 |
| A8 B4 C15 | A8 B5 C15 | A8 B6 C15 | A8 B7 C15 | A8 B11 C15 | A8 B13 C15 | A5 B14 C15 | A8 B15 C15 | A8 B16 C15 | A8 B17 C15 |
| A8 B4 C16 | A8 B5 C16 | A8 B6 C16 | A8 B7 C16 | A8 B11 C16 | A8 B13 C16 | A5 B14 C16 | A8 B15 C16 | A8 B16 C16 | A8 B17 C16 |
| A8 B4 C17 | A8 B5 C17 | A8 B6 C17 | A8 B7 C17 | A8 B11 C17 | A8 B13 C17 | A5 B14 C17 | A8 B15 C17 | A8 B16 C17 | A8 B17 C17 |
| A8 B4 C18 | A8 B5 C18 | A8 B6 C18 | A8 B7 C18 | A8 B11 C18 | A8 B13 C18 | A5 B14 C18 | A8 B15 C18 | A8 B16 C18 | A8 B17 C18 |
| A8 B4 C19 | A8 B5 C19 | A8 B6 C19 | A8 B7 C19 | A8 B11 C19 | A8 B13 C19 | A5 B14 C19 | A8 B15 C19 | A8 B16 C19 | A8 B17 C19 |
| A8 B4 C20 | A8 B5 C20 | A8 B6 C20 | A8 B7 C20 | A8 B11 C20 | A8 B13 C20 | A5 B14 C20 | A8 B15 C20 | A8 B16 C20 | A8 B17 C20 |
| A8 B4 C21 | A8 B5 C21 | A8 B6 C21 | A8 B7 C21 | A8 B11 C21 | A8 B13 C21 | A5 B14 C21 | A8 B15 C21 | A8 B16 C21 | A8 B17 C21 |
| A8 B4 C22 | A8 B5 C22 | A8 B6 C22 | A8 B7 C22 | A8 B11 C22 | A8 B13 C22 | A5 B14 C22 | A8 B15 C22 | A8 B16 C22 | A8 B17 C22 |
| A8 B4 C23 | A8 B5 C23 | A8 B6 C23 | A8 B7 C23 | A8 B11 C23 | A8 B13 C23 | A5 B14 C23 | A8 B15 C23 | A8 B16 C23 | A8 B17 C23 |
| A8 B4 C24 | A8 B5 C24 | A8 B6 C24 | A8 B7 C24 | A8 B11 C24 | A8 B13 C24 | A5 B14 C24 | A8 B15 C24 | A8 B16 C24 | A8 B17 C24 |
| A8 B4 C25 | A8 B5 C25 | A8 B6 C25 | A8 B7 C25 | A8 B11 C25 | A8 B13 C25 | A5 B14 C25 | A8 B15 C25 | A8 B16 C25 | A8 B17 C25 |
| A8 B4 C26 | A8 B5 C26 | A8 B6 C26 | A8 B7 C26 | A8 B11 C26 | A8 B13 C26 | A5 B14 C26 | A8 B15 C26 | A8 B16 C26 | A8 B17 C26 |
| A8 B4 C27 | A8 B5 C27 | A8 B6 C27 | A8 B7 C27 | A8 B11 C27 | A8 B13 C27 | A5 B14 C27 | A8 B15 C27 | A8 B16 C27 | A8 B17 C27 |
| A8 B4 C28 | A8 B5 C28 | A8 B6 C28 | A8 B7 C28 | A8 B11 C28 | A8 B13 C28 | A5 B14 C28 | A8 B15 C28 | A8 B16 C28 | A8 B17 C28 |
| A8 B4 C29 | A8 B5 C29 | A8 B6 C29 | A8 B7 C29 | A8 B11 C29 | A8 B13 C29 | A5 B14 C29 | A8 B15 C29 | A8 B16 C29 | A8 B17 C29 |
| A8 B4 C30 | A8 B5 C30 | A8 B6 C30 | A8 B7 C30 | A8 B11 C30 | A8 B13 C30 | A5 B14 C30 | A8 B15 C30 | A8 B16 C30 | A8 B17 C30 |
| A8 B4 C31 | A8 B5 C31 | A8 B6 C31 | A8 B7 C31 | A8 B11 C31 | A8 B13 C31 | A5 B14 C31 | A8 B15 C31 | A8 B16 C31 | A8 B17 C31 |
| A8 B4 C32 | A8 B5 C32 | A8 B6 C32 | A8 B7 C32 | A8 B11 C32 | A8 B13 C32 | A5 B14 C32 | A8 B15 C32 | A8 B16 C32 | A8 B17 C32 |
| A8 B4 C33 | A8 B5 C33 | A8 B6 C33 | A8 B7 C33 | A8 B11 C33 | A8 B13 C33 | A5 B14 C33 | A8 B15 C33 | A8 B16 C33 | A8 B17 C33 |
| A8 B4 C34 | A8 B5 C34 | A8 B6 C34 | A8 B7 C34 | A8 B11 C34 | A8 B13 C34 | A5 B14 C34 | A8 B15 C34 | A8 B16 C34 | A8 B17 C34 |
| A8 B4 C35 | A8 B5 C35 | A8 B6 C35 | A8 B7 C35 | A8 B11 C35 | A8 B13 C35 | A5 B14 C35 | A8 B15 C35 | A8 B16 C35 | A8 B17 C35 |
| A8 B4 C36 | A8 B5 C36 | A8 B6 C36 | A8 B7 C36 | A8 B11 C36 | A8 B13 C36 | A5 B14 C36 | A8 B15 C36 | A8 B16 C36 | A8 B17 C36 |
| A8 B4 C37 | A8 B5 C37 | A8 B6 C37 | A8 B7 C37 | A8 B11 C37 | A8 B13 C37 | A5 B14 C37 | A8 B15 C37 | A8 B16 C37 | A8 B17 C37 |
| A8 B4 C38 | A8 B5 C38 | A8 B6 C38 | A8 B7 C38 | A8 B11 C38 | A8 B13 C38 | A5 B14 C38 | A8 B15 C38 | A8 B16 C38 | A8 B17 C38 |
| A8 B4 C39 | A8 B5 C39 | A8 B6 C39 | A8 B7 C39 | A8 B11 C39 | A8 B13 C39 | A5 B14 C39 | A8 B15 C39 | A8 B16 C39 | A8 B17 C39 |
| A8 B4 C40 | A8 B5 C40 | A8 B6 C40 | A8 B7 C40 | A8 B11 C40 | A8 B13 C40 | A5 B14 C40 | A8 B15 C40 | A8 B16 C40 | A8 B17 C40 |
| A8 B4 C41 | A8 B5 C41 | A8 B6 C41 | A8 B7 C41 | A8 B11 C41 | A8 B13 C41 | A5 B14 C41 | A8 B15 C41 | A8 B16 C41 | A8 B17 C41 |
| | | | | | | | | | |
| A9 B4 C1 | A9 B5 C1 | A9 B6 C1 | A9 B7 C1 | A9 B11 C1 | A9 B13 C1 | A9 B14 C1 | A9 B15 C1 | A9 B16 C1 | A9 B17 C1 |
| A9 B4 C2 | A9 B5 C2 | A9 B6 C2 | A9 B7 C2 | A9 B11 C2 | A9 B13 C2 | A9 B14 C2 | A9 B15 C2 | A9 B16 C2 | A9 B17 C2 |
| A9 B4 C3 | A9 B5 C3 | A9 B6 C3 | A9 B7 C3 | A9 B11 C3 | A9 B13 C3 | A9 B14 C3 | A9 B15 C3 | A9 B16 C3 | A9 B17 C3 |
| A9 B4 C4 | A9 B5 C4 | A9 B6 C4 | A9 B7 C4 | A9 B11 C4 | A9 B13 C4 | A9 B14 C4 | A9 B15 C4 | A9 B16 C4 | A9 B17 C4 |
| A9 B4 C5 | A9 B5 C5 | A9 B6 C5 | A9 B7 C5 | A9 B11 C5 | A9 B13 C5 | A9 B14 C5 | A9 B15 C5 | A9 B16 C5 | A9 B17 C5 |
| A9 B4 C6 | A9 B5 C6 | A9 B6 C6 | A9 B7 C6 | A9 B11 C6 | A9 B13 C6 | A9 B14 C6 | A9 B15 C6 | A9 B16 C6 | A9 B17 C6 |
| A9 B4 C7 | A9 B5 C7 | A9 B6 C7 | A9 B7 C7 | A9 B11 C7 | A9 B13 C7 | A9 B14 C7 | A9 B15 C7 | A9 B16 C7 | A9 B17 C7 |
| A9 B4 C8 | A9 B5 C8 | A9 B6 C8 | A9 B7 C8 | A9 B11 C8 | A9 B13 C8 | A9 B14 C8 | A9 B15 C8 | A9 B16 C8 | A9 B17 C8 |
| A9 B4 C9 | A9 B5 C9 | A9 B6 C9 | A9 B7 C9 | A9 B11 C9 | A9 B13 C9 | A9 B14 C9 | A9 B15 C9 | A9 B16 C9 | A9 B17 C9 |
| A9 B4 C10 | A9 B5 C10 | A9 B6 C10 | A9 B7 C10 | A9 B11 C10 | A9 B13 C10 | A9 B14 C10 | A9 B15 C10 | A9 B16 C10 | A9 B17 C10 |
| A9 B4 C11 | A9 B5 C11 | A9 B6 C11 | A9 B7 C11 | A9 B11 C11 | A9 B13 C11 | A9 B14 C11 | A9 B15 C11 | A9 B16 C11 | A9 B17 C11 |
| A9 B4 C12 | A9 B5 C12 | A9 B6 C12 | A9 B7 C12 | A9 B11 C12 | A9 B13 C12 | A9 B14 C12 | A9 B15 C12 | A9 B16 C12 | A9 B17 C12 |
| A9 B4 C13 | A9 B5 C13 | A9 B6 C13 | A9 B7 C13 | A9 B11 C13 | A9 B13 C13 | A9 B14 C13 | A9 B15 C13 | A9 B16 C13 | A9 B17 C13 |
| A9 B4 C14 | A9 B5 C14 | A9 B6 C14 | A9 B7 C14 | A9 B11 C14 | A9 B13 C14 | A9 B14 C14 | A9 B15 C14 | A9 B16 C14 | A9 B17 C14 |
| A9 B4 C15 | A9 B5 C15 | A9 B6 C15 | A9 B7 C15 | A9 B11 C15 | A9 B13 C15 | A9 B14 C15 | A9 B15 C15 | A9 B16 C15 | A9 B17 C15 |
| A9 B4 C16 | A9 B5 C16 | A9 B6 C16 | A9 B7 C16 | A9 B11 C16 | A9 B13 C16 | A9 B14 C16 | A9 B15 C16 | A9 B16 C16 | A9 B17 C16 |
| A9 B4 C17 | A9 B5 C17 | A9 B6 C17 | A9 B7 C17 | A9 B11 C17 | A9 B13 C17 | A9 B14 C17 | A9 B15 C17 | A9 B16 C17 | A9 B17 C17 |
| A9 B4 C18 | A9 B5 C18 | A9 B6 C18 | A9 B7 C18 | A9 B11 C18 | A9 B13 C18 | A9 B14 C18 | A9 B15 C18 | A9 B16 C18 | A9 B17 C18 |
| A9 B4 C19 | A9 B5 C19 | A9 B6 C19 | A9 B7 C19 | A9 B11 C19 | A9 B13 C19 | A9 B14 C19 | A9 B15 C19 | A9 B16 C19 | A9 B17 C19 |
| A9 B4 C20 | A9 B5 C20 | A9 B6 C20 | A9 B7 C20 | A9 B11 C20 | A9 B13 C20 | A9 B14 C20 | A9 B15 C20 | A9 B16 C20 | A9 B17 C20 |
| A9 B4 C21 | A9 B5 C21 | A9 B6 C21 | A9 B7 C21 | A9 B11 C21 | A9 B13 C21 | A9 B14 C21 | A9 B15 C21 | A9 B16 C21 | A9 B17 C21 |
| A9 B4 C22 | A9 B5 C22 | A9 B6 C22 | A9 B7 C22 | A9 B11 C22 | A9 B13 C22 | A9 B14 C22 | A9 B15 C22 | A9 B16 C22 | A9 B17 C22 |
| A9 B4 C23 | A9 B5 C23 | A9 B6 C23 | A9 B7 C23 | A9 B11 C23 | A9 B13 C23 | A9 B14 C23 | A9 B15 C23 | A9 B16 C23 | A9 B17 C23 |
| A9 B4 C24 | A9 B5 C24 | A9 B6 C24 | A9 B7 C24 | A9 B11 C24 | A9 B13 C24 | A9 B14 C24 | A9 B15 C24 | A9 B16 C24 | A9 B17 C24 |
| A9 B4 C25 | A9 B5 C25 | A9 B6 C25 | A9 B7 C25 | A9 B11 C25 | A9 B13 C25 | A9 B14 C25 | A9 B15 C25 | A9 B16 C25 | A9 B17 C25 |
| A9 B4 C26 | A9 B5 C26 | A9 B6 C26 | A9 B7 C26 | A9 B11 C26 | A9 B13 C26 | A9 B14 C26 | A9 B15 C26 | A9 B16 C26 | A9 B17 C26 |
| A9 B4 C27 | A9 B5 C27 | A9 B6 C27 | A9 B7 C27 | A9 B11 C27 | A9 B13 C27 | A9 B14 C27 | A9 B15 C27 | A9 B16 C27 | A9 B17 C27 |
| A9 B4 C28 | A9 B5 C28 | A9 B6 C28 | A9 B7 C28 | A9 B11 C28 | A9 B13 C28 | A9 B14 C28 | A9 B15 C28 | A9 B16 C28 | A9 B17 C28 |
| A9 B4 C29 | A9 B5 C29 | A9 B6 C29 | A9 B7 C29 | A9 B11 C29 | A9 B13 C29 | A9 B14 C29 | A9 B15 C29 | A9 B16 C29 | A9 B17 C29 |
| A9 B4 C30 | A9 B5 C30 | A9 B6 C30 | A9 B7 C30 | A9 B11 C30 | A9 B13 C30 | A9 B14 C30 | A9 B15 C30 | A9 B16 C30 | A9 B17 C30 |
| A9 B4 C31 | A9 B5 C31 | A9 B6 C31 | A9 B7 C31 | A9 B11 C31 | A9 B13 C31 | A9 B14 C31 | A9 B15 C31 | A9 B16 C31 | A9 B17 C31 |
| A9 B4 C32 | A9 B5 C32 | A9 B6 C32 | A9 B7 C32 | A9 B11 C32 | A9 B13 C32 | A9 B14 C32 | A9 B15 C32 | A9 B16 C32 | A9 B17 C32 |
| A9 B4 C33 | A9 B5 C33 | A9 B6 C33 | A9 B7 C33 | A9 B11 C33 | A9 B13 C33 | A9 B14 C33 | A9 B15 C33 | A9 B16 C33 | A9 B17 C33 |
| A9 B4 C34 | A9 B5 C34 | A9 B6 C34 | A9 B7 C34 | A9 B11 C34 | A9 B13 C34 | A9 B14 C34 | A9 B15 C34 | A9 B16 C34 | A9 B17 C34 |
| A9 B4 C35 | A9 B5 C35 | A9 B6 C35 | A9 B7 C35 | A9 B11 C35 | A9 B13 C35 | A9 B14 C35 | A9 B15 C35 | A9 B16 C35 | A9 B17 C35 |
| A9 B4 C36 | A9 B5 C36 | A9 B6 C36 | A9 B7 C36 | A9 B11 C36 | A9 B13 C36 | A9 B14 C36 | A9 B15 C36 | A9 B16 C36 | A9 B17 C36 |
| A9 B4 C37 | A9 B5 C37 | A9 B6 C37 | A9 B7 C37 | A9 B11 C37 | A9 B13 C37 | A9 B14 C37 | A9 B15 C37 | A9 B16 C37 | A9 B17 C37 |
| A9 B4 C38 | A9 B5 C38 | A9 B6 C38 | A9 B7 C38 | A9 B11 C38 | A9 B13 C38 | A9 B14 C38 | A9 B15 C38 | A9 B16 C38 | A9 B17 C38 |
| A9 B4 C39 | A9 B5 C39 | A9 B6 C39 | A9 B7 C39 | A9 B11 C39 | A9 B13 C39 | A9 B14 C39 | A9 B15 C39 | A9 B16 C39 | A9 B17 C39 |
| A9 B4 C40 | A9 B5 C40 | A9 B6 C40 | A9 B7 C40 | A9 B11 C40 | A9 B13 C40 | A9 B14 C40 | A9 B15 C40 | A9 B16 C40 | A9 B17 C40 |
| A9 B4 C41 | A9 B5 C41 | A9 B6 C41 | A9 B7 C41 | A9 B11 C41 | A9 B13 C41 | A9 B14 C41 | A9 B15 C41 | A9 B16 C41 | A9 B17 C41 |
| | | | | | | | | | |
| A10 84 C1 | A10 B5 C1 | A10 86 C1 | A10 B7 C1 | A10 B11 C1 | A10 B13 C1 | A10 B14 C1 | A10 B15 C1 | A10 B16 C1 | A10 B17 C1 |
| A10 B4 C2 | A10 B5 C2 | A10 B6 C2 | A10 B7 C2 | A10 B11 C2 | A10 B13 C2 | A10 B14 C2 | A10 B15 C2 | A10 B16 C2 | A10 B17 C2 |
| A10 B4 C3 | A10 B5 C3 | A10 B6 C3 | A10 B7 C3 | A10 B11 C3 | A10 B13 C3 | A10 B14 C3 | A10 B15 C3 | A10 B16 C3 | A10 B17 C3 |
| A10 B4 C4 | A10 B5 C4 | A10 B6 C4 | A10 B7 C4 | A10 B11 C4 | A10 B13 C4 | A10 B14 C4 | A10 B15 C4 | A10 B16 C4. | A10 B17 C4 |
| A10 84 C5 | A10 B5 C5 | A10 B6 C5 | A10 B7 C5 | A10 B11 C5 | A10 B13 C5 | A10 B14 C5 | A10 B15 C5 | A10 B16 C5 | A10 B17 C5 |
| A10 B4 C6 | A10 B5 C6 | A10 B6 C6 | A10 B7 C6 | A10 B11 C6 | A10 B13 C6 | A10 B14 C6 | A10 B15 C6 | A10 B16 C6 | A10 B17 C6 |
| A10 B4 C7 | A10 B5 C7 | A10 B6 C7 | A10 B7 C7 | A10 B11 C7 | A10 B13 C7 | A10 B14 C7 | A10 B15 C7 | A10 B16 C7 | A10 B17 C7 |
| A10 B4 C8 | A10 85 C8 | A10 B6 C8 | A10 B7 C8 | A10 B11 C8 | A10 B13 C8 | A10 B14 C8 | A10 B15 C8 | A10 B16 C8 | A10 B17 C8 |
| A10 B4 C9 | A10 B5 C9 | A10 B6 C9 | A10 B7 C9 | A10 B11 C9 | A10 B13 C9 | A10 B14 C9 | A10 B15 C9 | A10 B16 C9 | A10 B17 C9 |
| A10 B4 C10 | A10 B5 C10 | A10 B6 C10 | A10 B7 C10 | A10 B11 C10 | A10 B13 C10 | A10 B14 C10 | A10 B15 C10 | A10 B16 C10 | A10 B17 C10 |
| A10 B4 C11 | A10 B5 C11 | A10 B6 C11 | A10 B7 C11 | A10 B11 C11 | A10 B13 C11 | A10 B14 C11 | A10 B15 C11 | A10 B16 C11 | A10 B17 C11 |
| A10 B4 C12 | A10 B5 C12 | A10 B6 C12 | A10 B7 C12 | A10 B11 C12 | A10 B13 C12 | A10 B14 C12 | A10 B15 C12 | A10 B16 C12 | A10 B17 C12 |
| A10 B4 C13 | A10 B5 C13 | A10 B6 C13 | A10 B7 C13 | A10 B11 C13 | A10 B13 C13 | A10 B14 C13 | A10 B15 C13 | A10 B16 C13 | A10 B17 C13 |
| A10 B4 C14 | A10 B5 C14 | A10 B6 C14 | A10 B7 C14 | A10 B11 C14 | A10 B13 C14 | A10 B14 C14 | A10 B15 C14 | A10 B16 C14 | A10 B17 C14 |
| A10 B4 C15 | A10 B5 C15 | A10 B6 C15 | A10 B7 C15 | A10 B11 C15 | A10 B13 C15 | A10 B14 C15 | A10 B15 C15 | A10 B16 C15 | A10 B17 C15 |
| A10 B4 C16 | A10 B5 C16 | A10 B6 C16 | A10 B7 C16 | A10 B11 C16 | A10 B13 C16 | A10 B14 C16 | A10 B15 C16 | A10 B16 C16 | A10 B17 C16 |
| A10 B4 C17 | A10 B5 C17 | A10 B6 C17 | A10 B7 C17 | A10 B11C17 | A10 B13 C17 | A10 B14 C17 | A10 B15 C17 | A10 B16 C17 | A10 B17 C17 |
| A10 B4 C18 | A10 B5 C18 | A10 B6 C18 | A10 B7 C18 | A10 B11 C18 | A10 B13 C18 | A10 B14 C18 | A10 B15 C18 | A10 B16 C18 | A10 B17 C18 |
| A10 B4 C19 | A10 B5 C19 | A10 B6 C19 | A10 B7 C19 | A10 B11 C19 | A10 B13 C19 | A10 B14 C19 | A10 B15 C19 | A10 B16 C19 | A10 B17 C19 |
| A10 B4 C20 | A10 B5 C20 | A10 B6 C20 | A10 B7 C20 | A10 B11 C20 | A10 B13 C20 | A10 B14 C20 | A10 B15 C20 | A10 B16 C10 | A10 B17 C20 |
| A10 B4 C21 | A10 B5 C21 | A10 B6 C21 | A10 B7 C21 | A10 B11 C21 | A10 B13 C21 | A10 B14 C21 | A10 B15 C21 | A10 B16 C21 | A10 B17 C21 |
| A10 B4 C22 | A10 B5 C22 | A10 B6 C22 | A10 B7 C22 | A10 B11 C22 | A10 B13 C22 | A10 B14 C22 | A10 B15 C22 | A10 B16 C22 | A10 B17 C22 |
| A10 B4 C23 | A10 B5 C23 | A10 B6 C23 | A10 B7 C23 | A10 B11 C23 | A10 B13 C23 | A10 B14 C23 | A10 B15 C23 | A10 B16 C23 | A10 B17 C23 |
| A10 B4 C24 | A10 B5 C24 | A10 B6 C24 | A10 B7 C24 | A10 B11 C24 | A10 B13 C24 | A10 B14 C24 | A10 B15 C24 | A10 B16 C24 | A10 B17 C24 |
| A10 B4 C25 | A10 B5 C25 | A10 B6 C25 | A10 B7 C25 | A10 B11 C25 | A10 B13 C25 | A10 B14 C25 | A10 B15 C25 | A10 B16 C25 | A10 B17 C25 |
| A10 B4 C26 | A10 B5 C26 | A10 B6 C26 | A10 B7 C26 | A10 B11 C26 | A10 B13 C26 | A10 B14 C26 | A10 B15 C26 | A10 B16 C26 | A10 B17 C26 |
| A10 B4 C27 | A10 B5 C27 | A10 B6 C27 | A10 B7 C27 | A10 B11 C27 | A10 B13 C27 | A10 B14 C27 | A10 B15 C27 | A10 B16 C17 | A10 B17 C27 |
| A10 B4 C28 | A10 B5 C28 | A10 B6 C28 | A10 B7 C28 | A10 B11 C28 | A10 B13 C28 | A10 B14 C28 | A10 B15 C28 | A10 B16 C:!8 | A10 B17 C28 |
| A10 B4 C29 | A10 B5 C29 | A10 B6 C29 | A10 B7 C29 | A10 B11 C29 | A10 B13 C29 | A10 B14 C29 | A10 B15 C29 | A10 B16 C29 | A10 B17 C29 |
| A10 B4 C30 | A10 B5 C30 | A10 B6 C30 | A10 B7 C30 | A10 B11 C30 | A10 B13 C30 | A10 B14 C30 | A10 B15 C30 | A10 B16 C30 | A10 B17 C30 |
| A10 B4 C31 | A10 B5 C31 | A10 B6 C31 | A10 B7 C31 | A10 B11 C31 | A10 B13 C31 | A10 B14 C31 | A10 B15 C31 | A10 B16 C31 | A10 B17 C31 |
| A10 B4 C32 | A10 B5 C32 | A10 B6 C32 | A10 B7 C32 | A10 B11 C32 | A10 B13 C32 | A10 B14 C32 | A10 B15 C32 | A10 B16 C12 | A10 B17 C32 |
| A10 B4 C33 | A10 B5 C33 | A10 B6 C33 | A10 B7 C33 | A10 B11 C33 | A10 B13 C33 | A10 B14 C33 | A10 B15 C33 | A10 B16 C33 | A10 B17 C33 |
| A10 B4 C34 | A10 B5 C34 | A10 B6 C34 | A10 B7 C34 | A10 B11 C34 | A10 B13 C34 | A10 B14 C34 | A10 B15 C34 | A10 B16 C14 | A10 B17 C34 |
| A10 B4 C35 | A10 B5 C35 | A10 B6 C35 | A10 B7 C35 | A10 B11 C35 | A10 B13 C35 | A10 B14 C35 | A10 B15 C35 | A10 B16 C35 | A10 B17 C35 |
| A10 B4 C36 | A10 B5 C36 | A10 B6 C36 | A10 B7 C36 | A10 B11 C36 | A10 B13 C36 | A10 B14 C36 | A10 B15 C36 | A10 B16 C36 | A10 B17 C36 |
| A10 B4 C37 | A10 B5 C37 | A10 B6 C37 | A10 B7 C37 | A10 B11 C37 | A10 B13 C37 | A10 B14 C37 | A10 B15 C37 | A10 B16 C37 | A10 B17 C37 |
| A10 B4 C38 | A10 B5 C38 | A10 B6 C38 | A10 B7 C38 | A10 B11 C38 | A10 B13 C38 | A10 B14 C38 | A10 B15 C38 | A10 B16 C38 | A10 B17 C38 |
| A10 B4 C39 | A10 B5 C39 | A10 B6 C39 | A10 B7 C39 | A10 B11 C39 | A10 B13 C39 | A10 B14 C39 | A10 B15 C39 | A10 B16 C39 | A10 B17 C39 |
| A10 B4 C40 | A10 B5 C40 | A10 B6 C40 | A10 B7 C40 | A10 B11 C40 | A10 B13 C40 | A10 B14 C40 | A10 B15 C40 | A10 B16 C40 | A10 B17 C40 |
| A10 B4 C41 | A10 B5 C41 | A10 B6 C41 | A10 B7 C41 | A10 B11 C41 | A10 B13 C41 | A10 B14 C41 | A10 B15 C41 | A10 B16 C41 | A10 B17 C41 |
| | | | | | | | | | |
| A11 B4 C1 | A11 B5 C1 | A11 B6 C1 | A11 B7 C1 | A11 B11 C1 | A11 B13 C1 | A11 B14 C1 | A11 B15 C1 | A11 B16 C1 | A11 B17 C1 |
| A11 B4 C2 | A11 B5 C2 | A11 B6 C2 | A11 B7 C2 | A11 B11 C2 | A11 B13 C2 | A1 B14 C2 | A11 B15 C2 | A11 B16 C2 | A11 B17 C2 |
| A11 B4 C3 | A11 B5 C3 | A11 B6 C3 | A11 B7 C3 | A11 B11 C3 | A11 B13 C3 | A11 B14 C3 | A11 B15 C3 | A1 B16 C3 | A11 B17 C3 |
| A11 B4 C4 | A11 B5 C4 | A11 B6 C4 | A11 B7 C4 | A11 B11 C4 | A11 B13 C4 | A11 B14 C4 | A11 B15 C4 | A11 B16 C4 | A11 B17 C4 |
| A11 B4 C5 | A11 B5 C5 | A11 B6 C5 | A11 B7 C5 | A1 B11 C5 | A11 B13 C5 | A11 B14 C5 | A11 B15 C5 | A11 B16 C5 | A11 B17 C5 |
| A11 B4 C6 | A11 B5 C6 | A11 B6 C6 | A11 B7 C6 | A1 B11 C6 | A11 B13 C6 | A11 B14 C6 | A11 B15 C6 | A11 B16 C6 | A11 B17 C6 |
| A11 B4 C7 | A11 B5 C7 | A11 B6 C7 | A11 B7 C7 | A1 B11 C7 | A1 B13 C7 | A11 B14 C7 | A11 B15 C7 | A11 B16 C7 | A11 B17 C7 |
| A11 B4 C8 | A11 B5 C8 | A11 B6 C8 | A11 B7 C8 | A1 B11 C8 | A11 B13 C8 | A1 B14 C8 | A11 B15 C8 | A11 B16 C8 | A11 B17 C8 |
| A1 B4 C9 | A11 B5 C9 | A11 B6 C9 | A11 B7 C9 | A11 B11 C9 | A11 B13 C9 | A11 B14 C9 | A11 B15 C9 | A11 B16 C9 | A11 B17 C9 |
| A11 B4 C10 | A11 B5 C10 | A11 B6 C10 | A1 B7 C10 | A11 B11 C10 | A11 B13 C10 | A11 B14 C10 | A11 B15 C10 | A11 B16 C10 | A11 B17 C10 |
| A11 B4 C11 | A11 B5 C11 | A11 B6 C11 | A11 B7 C11 | A11 B11 C11 | A11 B13 C11 | A11 B14 C11 | A11 B15 C11 | A11 B16 C11 | A11 B17 C11 |
| A11 B4 C12 | A11 B5 C12 | A11 B6 C12 | A11 B7 C12 | A11 B11 C12 | A11 B13 C12 | A11 B14 C12 | A11 B15 C12 | A11 B16 C12 | A11 B17 C12 |
| A11 B4 C13 | A11 B5 C13 | A11 B6 C13 | A11 B7 C13 | A11 B11 C13 | A11 B13 C13 | A11 B14 C13 | A11 B15 C13 | A11 B16 C13 | A11 B17 C13 |
| A11 B4 C14 | A11 B5 C14 | A11 B6 C14 | A11 B7 C14 | A11 B11 C14 | A11 B13 C14 | A11 B14 C14 | A11 B15 C14 | A11 B16 C14 | A11 B17 C14 |
| A11 B4 C15 | A11 B5 C15 | A11 B6 C15 | A11 B7 C15 | A11 B11 C15 | A11 B13 C15 | A11 B14 C15 | A11 B15 C15 | A11 B16 C15 | A11 B17 C15 |
| A11 B4 C16 | A11 B5 C16 | A11 B6 C16 | A11 B7 C16 | A11 B11 C16 | A11 B13 C16 | A11 B14 C16 | A11 B15 C16 | A11 B16 C16 | A11 B17 C16 |
| A11 B4 C17 | A11 B5 C17 | A11 B6 C17 | A11 B7 C17 | A11 B11 C17 | A11 B13 C17 | A11 B14 C17 | A11 B15 C17 | A11 B16 C17 | A11 B17 C17 |
| A11 B4 C18 | A11 B5 C18 | A11 B6 C18 | A11 B7 C18 | A11 B11 C18 | A11 B13 C18 | A11 B14 C18 | A11 B15 C18 | A11 B16 C18 | A11 B17 C18 |
| A11 B4 C19 | A11 B5 C19 | A11 B6 C19 | A11 B7 C19 | A11 B11 C19 | A11 B13 C19 | A11 B14 C19 | A11 B15 C19 | A11 B16 C19 | A11 B17 C19 |
| A11 B4 C20 | A11 B5 C20 | A11 B6 C20 | A11 B7 C20 | A11 B11 C20 | A11 B13 C20 | A11 B14 C20 | A11 B15 C20 | A11 B16 C20 | A11 B17 C20 |
| A11 B4 C21 | A11 B5 C21 | A11 B6 C21 | A11 B7 C21 | A11 B11 C21 | A11 B13 C21 | A11 B14 C21 | A11 B15 C21 | A11 B16 C21 | A11 B17 C21 |
| A11 B4 C22 | A11 B5 C22 | A11 B6 C22 | A11 B7 C22 | A11 B11 C22 | A11 B13 C22 | A11 B14 C22 | A11 B15 C22 | A11 B16 C22 | A11 B17 C22 |
| A11 B4 C23 | A11 B5 C23 | A11 B6 C23 | A11 B7 C23 | A11 B11 C23 | A11 B13 C23 | A11 B14 C23 | A11 B15 C23 | A11 B16 C23 | A11 B17 C23 |
| A11 B4 C24 | A11 B5 C24 | A11 B6 C24 | A11 B7 C24 | A11 B11 C24 | A11 B 13 C24 | A11 B14 C24 | A11 B15 C24 | A11 B16 C24 | A11 B17 C24 |
| A11 B4 C25 | A11 B5 C25 | A11 B6 C25 | A11 B7 C25 | A11 B11 C25 | A11 B13 C25 | A11 B14 C25 | A11 B15 C25 | A11 B16 C25 | A11 B17 C25 |
| A11 B4 C26 | A11 B5 C26 | A11 B6 C26 | A11 B7 C26 | A11 B11 C26 | A11 B13 C26 | A11 B14 C26 | A11 B15 C26 | A11 B16 C26 | A11 B17 C26 |
| A11 B4 C27 | A11 B5 C27 | A11 B6 C27 | A11 B7 C27 | A11 B11 C27 | A11 B13 C27 | A11 B14 C27 | A11 B15 C27 | A11 B16 C27 | A11 B17 C27 |
| A11 B4 C28 | A11 B5 C28 | A11 B6 C28 | A11 B7 C28 | A11 B11 C28 | A11 B13 C28 | A11 B14 C28 | A11 B15 C28 | A11 B16 C28 | A11 B 17 C28 |
| A11 B4 C29 | A11 B5 C29 | A11 B6 C29 | A11 B7 C29 | A11 B11 C29 | A11 B13 C29 | A11 B14 C29 | A11 B15 C29 | A11 B 16 C29 | A11 B17 C29 |
| A11 B4 C30 | A11 B5 C30 | A11 B6 C30 | A11 B7 C30 | A11 B11 C30 | A11 B13 C30 | A11 B14 C30 | A11 B15 C30 | A11 B16 C30 | A11 B17 C30 |
| A11 B4 C31 | A11 B5 C31 | A11 B6 C31 | A11 B7 C31 | A11 B11 C31 | A11 B13 C31 | A11 B14 C31 | A11 B15 C31 | A11 B16 C31 | A11 B17 C31 |
| A11 B4 C32 | A11 B5 C32 | A11 B6 C32 | A11 B7 C32 | A11 B11 C32 | A11 B13 C32 | A11 B14 C32 | A11 B15 C32 | A11 B16 C12 | A11 B17 C32 |
| A11 B4 C33 | A11 B5 C33 | A11 B6 C33 | A11 B7 C33 | A11 B11 C33 | A11 B13 C33 | A11 B14 C33 | A11 B15 C33 | A11 B16 C33 | A11 B17 C33 |
| A11 B4 C34 | A11 B5 C34 | A11 B6 C34 | A11 B7 C34 | A11 B11 C34 | A11 B13 C34 | A11 B14 C34 | A11 B15 C34 | A11 B16 C34 | A11 B17 C34 |
| A11 B4 C35 | A11 B5 C35 | A11 B6 C35 | A11 B7 C35 | A11 B11 C35 | A11 B13 C35 | A11 B14 C35 | A11 B15 C35 | A11 B16 C35 | 111 B17 C35 |
| A11 B4 C36 | A11 B5 C36 | A11 B6 C36 | A11 B7 C36 | A11 B11 C36 | A11 B13 C36 | A11 B14 C36 | A11 B15 C36 | A11 B16 C36 | A11 B17 C36 |
| A11 B4 C37 | A11 B5 C37 | A11 B6 C37 | A11 B7 C37 | A11 B11 C37 | A11 B13 C37 | A11 B14 C37 | A11 B15 C37 | A11 B16 C37 | A11 B17 C37 |
| A11 B4 C38 | A11 B5 C38 | A11 B6 C38 | A11 B7 C38 | A11 B11 C38 | A1 B13 C38 | A11 B14 C38 | A11 B15 C38 | A11 B16 C38 | A11 B17 C38 |
| A11 B4 C39 | A11 B5 C39 | A11 B6 C39 | A11 B7 C39 | A11 B11 C39 | A11 B13 C39 | A11 B14 C39 | A11 B15 C39 | A11 B16 C39 | A11 B17 C39 |
| A11 B4 C40 | A11 B5 C40 | A11 B6 C40 | A11 B7 C40 | A11 B11 C40 | A11 B13 C40 | A11 B14 C40 | A11 B15 C40 | A11 B16 C40 | A11 B17 C40 |
| A11 B4 C41 | A11 B5 C41 | A11 B6 C41 | A11 B7 C41 | A11 B11 C41 | A11 B13 C41 | A11 B14 C41 | A11 B15 C41 | A11 B16 C41 | A11 B17 C41 |
| A13 B4 C1 | A13 B5 C1 | A13 B6 C1 | A13 B7 C1 | A13 B11 C1 | A13 B13 C1 | A13 B14 C1 | A13 B15 C1 | A13 B16 C1 | A13 B17 C1 |
| A13 B4 C2 | A13 B5 C2 | A13 B6 C2 | A13 B7 C2 | A13 B11 C2 | A13 B13 C2 | A13 B14 C2 | A13 B15 C2 | A13 B16 C2 | A13 B17 C2 |
| A13 B4 C3 | A13 B5 C3 | A13 B6 C3 | A13 B7 C3 | A13 B11 C3 | A13 B13 C3 | A13 B14 C3 | A13 B15 C3 | A13 B16 C3 | A13 B17 C3 |
| A13 B4 C4 | A13 B5 C4 | A13 B6 C4 | A13 B7 C4 | A13 B11 C4 | A13 B13 C4 | A13 B14 C4 | A13 B15 C4 | A13 B16 C4 | A13 B17 C4 |
| A13 B4 C5 | A13 B5 C5 | A13 B6 C5 | A13 B7 C5 | A13 B11 C5 | A13 B13 C5 | A13 B14 C5 | A13 B15 C5 | A13 B16 C5 | A13 B17 C5 |
| A13 B4 C6 | A13 B5 C6 | A13 B6 C6 | A13 B7 C6 | A13 B11 C6 | A13 B13 C6 | A13 B14 C6 | A13 B15 C6 | A13 B16 C6 | A13 B17 C6 |
| A13 B4 C7 | A13 B5 C7 | A13 B6 C7 | A13 B7 C7 | A13 B11 C7 | A13 B13 C7 | A13 B14 C7 | A13 B15 C7 | A13 B16 C7 | A13 B17 C7 |
| A13 B4 C8 | A13 B5 C8 | A13 B6 C8 | A13 B7 C8 | A13 B11 C8 | A13 B13 C8 | A13 B14 C8 | A13 B15 C8 | A13 B16 C8 | A13 B17 C8 |
| A13 B4 C9 | A13 B5 C9 | A13 B6 C9 | A13 B7 C9 | A13 B11 C9 | A13 B13 C9 | A13 B14 C9 | A13 B15 C9 | A13 B16 C9 | A13 B17 C9 |
| A13 B4 C10 | A13 B5 C10 | A13 B6 C10 | A13 B7 C10 | A13 B11 C10 | A13 B13 C10 | A13 B14 C10 | A13 B15 C10 | A13 B16 C10 | A13 B17 C10 |
| A13 B4 C11 | A13 B5 C11 | A13 B6 C11 | A13 B7 C11 | A13 B11 C11 | A13 B13 C11 | A13 B14 C11 | A13 B15 C11 | A13 B16 C11 | A13 B17 C11 |
| A13 B4 C12 | A13 B5 C12 | A13 B6 C12 | A13 B7 C12 | A13 B11 C12 | A13 B13 C12 | A13 B14 C12 | A13 B15 C12 | A13 B16 C12 | A13 B17 C12 |
| A13 B4 C13 | A13 B5 C13 | A13 B6 C13 | A13 B7 C13 | A13 B11 C13 | A13 B13 C13 | A13 B14 C13 | A13 B15 C13 | A13 B16 C13 | A13 B17 C13 |
| A13 B4 C14 | A13 B5 C14 | A13 B6 C14 | A13 B7 C14 | A13 B11 C14 | A13 B13 C14 | A13 B14 C14 | A13 B15 C14 | A13 B16 C14 | A13 B17 C14 |
| A13 B4 C15 | A13 B5 C15 | A13 B6 C15 | A13 B7 C15 | A13 B11 C15 | A13 B13 C15 | A13 B14 C15 | A13 B15 C15 | A13 B16 C15 | A13 B17 C15 |
| A13 B4 C16 | A13 B5 C16 | A13 B6 C16 | A13 B7 C16 | A13 B11 C16 | A13 B13 C16 | A13 B14 C16 | A13 B15 C16 | A13 B16 C16 | A13 B17 C16 |
| A13 B4 C17 | A13 B5 C17 | A13 B6 C17 | A13 B7 C17 | A13 B11 C17 | A13 B13 C17 | A13 B14 C17 | A13 B15 C17 | A13 B16 C17 | A13 B17 C17 |
| A13 B4 C18 | A13 B5 C18 | A13 B6 C18 | A13 B7 C18 | A13 B11 C18 | A13 B13 C18 | A13 B14 C18 | A13 B15 C18 | A13 B16 C18 | A13 B17 C18 |
| A13 B4 C19 | A13 B5 C19 | A13 B6 C19 | A13 B7 C19 | A13 B11 C19 | A13 B13 C19 | A13 B14 C19 | A13 B15 C19 | A13 B16 C19 | A13 B17 C19 |
| A13 B4 C20 | A13 B5 C20 | A13 B6 C20 | A13 B7 C20 | A13 B11 C20 | A13 B13 C20 | A13 B14 C20 | A13 B15 C20 | A13 B16 C20 | A13 B17 C20 |
| A13 B4 C21 | A13 B5 C21 | A13 B6 C21 | A13 B7 C21 | A13 B11 C21 | A13 B13 C21 | A13 B14 C21 | A13 B15 C21 | A13 B16 C21 | A13 B17 C21 |
| A13 B4 C22 | A13 B5 C22 | A13 B6 C22 | A13 B7 C22 | A13 B11 C22 | A13 B13 C22 | A13 B14 C22 | A13 B15 C22 | A13 B16 C22 | A13 B17 C22 |
| A13 B4 C23 | A13 B5 C23 | A13 B6 C23 | A13 B7 C23 | A13 B11 C23 | A13 B13 C23 | A13 B14 C23 | A13 B15 C23 | A13 B16 C23 | A13 B17 C23 |
| A13 B4 C24 | A13 B5 C24 | A13 B6 C24 | A13 B7 C24 | A13 B11 C24 | A13 B13 C24 | A13 B14 C24 | A13 B15 C24 | A13 B16 C24 | A13 B17 C24 |
| A13 B4 C25 | A13 B5 C25 | A13 B6 C25 | A13 B7 C25 | A13 B11 C25 | A13 B13 C25 | A13 B14 C25 | A13 B15 C25 | A13 B16 C25 | A13 B17 C25 |
| A13 B4 C26 | A13 B5 C26 | A13 B6 C26 | A13 B7 C26 | A13 B11 C26 | A13 B13 C26 | A13 B14 C26 | A13 B15 C26 | A13 B16 C26 | A13 B17 C26 |
| A13 B4 C27 | A13 B5 C27 | A13 B6 C27 | A13 B7 C27 | A13 B11 C27 | A13 B13 C27 | A13 B14 C27 | A13 B15 C27 | A13 B16 C27 | A13 B17 C27 |
| A13 B4 C28 | A13 B5 C28 | A13 B6 C28 | A13 B7 C28 | A13 B11 C28 | A13 B13 C28 | A13 B14 C28 | A13 B15 C28 | A13 B16 C28 | A13 B17 C28 |
| A13 B4 C29 | A13 B5 C29 | A13 B6 C29 | A13 B7 C29 | A13 B11 C29 | A13 B13 C29 | A13 B14 C29 | A13 B15 C29 | A13 B16 C29 | A13 B17 C29 |
| A13 B4 C30 | A13 B5 C30 | A13 B6 C30 | A13 B7 C30 | A13 B11 C30 | A13 B13 C30 | A13 B14 C30 | A13 B15 C30 | A13 B16 C30 | A13 B17 C30 |
| A13 B4 C31 | A13 B5 C31 | A13 B6 C31 | A13 B7 C31 | A13 B11 C31 | A13 B13 C31 | A13 B14 C31 | A13 B15 C31 | A13 B16 C31 | A13 B17 C31 |
| A13 B4 C32 | A13 B5 C32 | A13 B6 C32 | A13 B7 C32 | A13 B11 C32 | A13 B13 C32 | A13 B14 C32 | A13 B15 C32 | A13 B16 C32 | A13 B17 C32 |
| A13 B4 C33 | A13 B5 C33 | A13 B6 C33 | A13 B7 C33 | A13 B11 C33 | A13 B13 C33 | A13 B14 C33 | A13 B15 C33 | A13 B16 C33 | A13 B17 C33 |
| A13 B4 C34 | A13 B5 C34 | A13 B6 C34 | A13 B7 C34 | A13 B11 C34 | A13 B13 C34 | A13 B14 C34 | A13 B15 C34 | A13 B16 C34 | A13 B17 C34 |
| A13 B4 C35 | A13 B5 C35 | A13 B6 C35 | A13 B7 C35 | A13 B11 C35 | A13 B13 C35 | A13 B14 C35 | A13 B15 C35 | A13 B16 C35 | A13 B17 C35 |
| A13 B4 C36 | A13 B5 C36 | A13 B6 C36 | A13 B7 C36 | A13 B11 C36 | A13 B13 C36 | A13 B14 C36 | A13 B15 C36 | A13 B16 C36 | A13 B17 C36 |
| A13 B4 C37 | A13 B5 C37 | A13 B6 C37 | A13 B7 C37 | A13 B11 C37 | A13 B13 C37 | A13 B14 C37 | A13 B15 C37 | A13 B16 C37 | A13 B17 C37 |
| A13 B4 C38 | A13 B5 C38 | A13 B6 C38 | A13 B7 C38 | A13 B11 C38 | A13 B13 C38 | A13 B14 C38 | A13 B15 C38 | A13 B16 C38 | A13 BI7 C38 |
| A13 B4 C39 | A13 B5 C39 | A13 B6 C39 | A13 B7 C39 | A13 B11 C39 | A13 B13 C39 | A13 B14 C39 | A13 B15 C39 | A13 B16 C39 | A13 B17 C39 |
| A13 B4 C40 | A13 B5 C40 | A13 B6 C40 | A13 B7 C40 | A13 B11 C40 | A13 B13 C40 | A13 B14 C40 | A13 B15 C40 | A13 B16 C40 | A13 B17 C40 |
| A13 B4 C41 | A13 B5 C41 | A13 B6 C41 | A13 B7 C41 | A13 B11 C41 | A13 B13 C41 | A13 B14 C41 | A13 B15 C41 | A13 B16 C41 | A13 B17 C41 |
| | | | | | | | | | |
| A15 B4 C1 | A15 B5 C1 | A15 86 C1 | A15 B7 C1 | A15 B11 C1 | A15 B13 C1 | A15 B14 C1 | A15 B15 C1 | A15 B16 C1 | A15 B17 C1 |
| A15 B4 C2 | A15 B5 C2 | A15 B6 C2 | A15 B7 C2 | A15 B11 C2 | A15 B13 C2 | A15 B14 C2 | A15 B15 C2 | A15 B16 C2 | A15 B17 C2 |
| A15 B4 C3 | A15 B5 C3 | A15 B6 C3 | A15 B7 C3 | A15 B11 C3 | A15 B13 C3 | A15 B14 C3 | A15 B15 C3 | A15 B16 C3 | A15 B17 C3 |
| A15 B4 C4 | A15 B5 C4 | A15 B6 C4 | A15 B7 C4 | A15 B11 C4 | A15 B13 C4 | A15 B14 C4 | A15 B15 C4 | A15 B16 C4 | A15 B17 C4 |
| A15 B4 C5 | A15 B5 C5 | A15 B6 C5 | A15 B7 C5 | A15 B11 C5 | A15 B13 C5 | A15 B14 C5 | A15 B15 C5 | A15 B16 C5 | A15 B17 C5 |
| A15 B4 C6 | A15 B5 C6 | A15 B6 C6 | A15 B7 C6 | A15 B11 C6 | A15 B13 C6 | A15 B14 C6 | A15 B15 C6 | A15 B16 C6 | A15 B17 C6 |
| A15 B4 C7 | A15 B5 C7 | A15 B6 C7 | A15 B7 C7 | A15 B11 C7 | A15 B13 C7 | A15 B14 C7 | A15 B15 C7 | A15 B16 C7 | A15 B17 C7 |
| A15 B4 C8 | A15 B5 C8 | A15 B6 C8 | A15 B7 C8 | A15 B11 C8 | A15 B13 C8 | A15 B14 C8 | A15 B15 C8 | A15 B16 C13 | A15 B17 C8 |
| A15 B4 C9 | A15 B5 C9 | A15 B6 C9 | A15 B7 C9 | A15 B11 C9 | A15 B13 C9 | A15 B14 C9 | A15 B15 C9 | A15 B16 C9 | A15 B17 C9 |
| A15 B4 C10 | A15 B5 C10 | A15 B6 C10 | A15 B7 C10 | A15 B11 C10 | A15 B13 C10 | A15 B14 C10 | A15 B15 C10 | A15 B16 C10 | A15 B17 C10 |
| A15 B4 C11 | A15 B5 C11 | A15 B6 C11 | A15 B7 C11 | A15 B11 C11 | A15 B13 C11 | A15 B14 C11 | A15 B15 C11 | A15 B16 C11 | A15 B17 C11 |
| A15 B4 C12 | A15 B5 C12 | A15 B6 C12 | A15 B7 C12 | A15 B11 C12 | A15 B13 C12 | A15 B14 C12 | A15 B15 C12 | A15 B16 C12 | A15 B17 C12 |
| A15 B4 C13 | A15 B5 C13 | A15 B6 C13 | A15 B7 C13 | A15 B11 C13 | A15 B13 C13 | A15 B14 C13 | A15 B15 C13 | A15 B16 C13 | A15 B17 C13 |
| A15 B4 C14 | A15 B5 C14 | A15 B6 C14 | A15 B7 C14 | A15 B11 C14 | A15 B13 C14 | A15 B14 C14 | A15 B15 C14 | A15 B16 C14 | A15 B17 C14 |
| A15 B4 C15 | A15 B5 C15 | A15 B6 C15 | A15 B7 C15 | A15 B11 C15 | A15 B13 C15 | A15 B14 C15 | A15 B15 C15 | A15 B16 C15 | A15 B17 C15 |
| A15 B4 C16 | A15 B5 C16 | A15 B6 C16 | A15 B7 C16 | A15 B11 C16 | A15 B13 C16 | A15 B14 C16 | A15 B15 C16 | A15 B16 C16 | A15 B17 C16 |
| A15 B4 C17 | A15 B5 C17 | A15 B6 C17 | A15 B7 C17 | A15 B11 C17 | A15 B13 C17 | A15 B14 C17 | A15 B15 C17 | A15 B16 C17 | A15 B17 C17 |
| A15 B4 C18 | A15 B5 C18 | A15 B6 C18 | A15 B7 C18 | A15 B11 C18 | A15 B13 C18 | A15 B14 C18 | A15 B15 C18 | A15 B16 C18 | A15 B17 C18 |
| A15 B4 C19 | A15 B5 C19 | A15 B6 C19 | A15 B7 C19 | A15 B11 C19 | A15 B13 C19 | A15 B14 C19 | A15 B15 C19 | A15 B16 C19 | A15 B17 C19 |
| A15 B4 C20 | A15 B5 C20 | A15 B6 C20 | A15 B7 C20 | A15 B11 C20 | A15 B13 C20 | A15 B14 C20 | A15 B15 C20 | A15 B16 C20 | A15 B17 C20 |
| A15 B4 C21 | A15 B5 C21 | A15 B6 C21 | A15 B7 C21 | A15 B11 C21 | A15 B13 C21 | A15 B14 C21 | A15 B15 C21 | A15 B16 C21 | A15 B17 C21 |
| A15 B4 C22 | A15 B5 C22 | A15 B6 C22 | A15 B7 C22 | A15 B11 C22 | A15 B13 C22 | A15 B14 C22 | A15 B15 C22 | A15 B16 C22 | A15 B17 C22 |
| A15 B4 C23 | A15 B5 C23 | A15 B6 C23 | A15 B7 C23 | A15 B11 C23 | A15 B13 C23 | A15 B14 C23 | A15 B15 C23 | A15 B16 C23 | A15 B17 C23 |
| A15 84 C24 | A15 B5 C24 | A15 B6 C24 | A15 B7 C24 | A15 B11 C24 | A15 B13 C24 | A15 B14 C24 | A15 B15 C24 | A15 B16 C24 | A15 B17 C24 |
| A15 B4 C25 | A15 B5 C25 | A15 B6 C25 | A15 B7 C25 | A15 B11 C25 | A15 B13 C25 | A15 B14 C25 | A15 B15 C25 | A15 B16 C25 | A15 B17 C25 |
| A15 B4 C26 | A15 B5 C26 | A15 B6 C26 | A15 B7 C26 | A15 B11 C26 | A15 B13 C26 | A15 B14 C26 | A15 B15 C26 | A15 B16 C26 | A15 B17 C26 |
| A15 B4 C27 | A15 B5 C27 | A15 B6 C27 | A15 B7 C27 | A15 B11 C27 | A15 B13 C27 | A15 B14 C27 | A15 B15 C27 | A15 B16 C27 | A15 B17 C27 |
| A15 B4 C28 | A15 B5 C28 | A15 B6 C28 | A15 B7 C28 | A15 B11 C28 | A15 B13 C28 | A15 B14 C28 | A15 B15 C28 | A15 B16 C28 | A15 B17 C28 |
| A15 B4 C29 | A15 B5 C29 | A15 B6 C29 | A15 B7 C29 | A15 B11 C29 | A15 B13 C29 | A15 B14 C29 | A15 B15 C29 | A15 B16 C29 | A15 B17 C29 |
| A15 B4 C30 | A15 B5 C30 | A15 B6 C30 | A15 B7 C30 | A15 B11 C30 | A15 B13 C30 | A15 B14 C30 | A15 B15 C30 | A15 B16 C30 | A15 B17 C30 |
| A15 B4 C31 | A15 B5 C31 | A15 B6 C31 | A15 B7 C31 | A15 B11 C31 | A15 B13 C31 | A15 B14 C31 | A15 B15 C31 | A15 B16 C31 | A15 B17 C31 |
| A15 B4 C32 | A15 B5 C32 | A15 B6 C32 | A15 B7 C32 | A15 B11 C32 | A15 B13 C32 | A15 B14 C32 | A15 B15 C32 | A15 B16 C32 | A15 B17 C32 |
| A15 B4 C33 | A15 B5 C33 | A15 B6 C33 | A15 B7 C33 | A15 B11 C33 | A15 B13 C33 | A15 B14 C33 | A15 B15 C33 | A15 B16 C33 | A15 B17 C33 |
| A15 B4 C34 | A15 B5 C34 | A15 B6 C34 | A15 B7 C34 | A15 B11 C34 | A15 B13 C34 | A15 B14 C34 | A15 B15 C34 | A15 B16 C34 | A15 B17 C34 |
| A15 B4 C35 | A15 B5 C35 | A15 B6 C35 | A15 B7 C35 | A15 B11 C35 | A15 B13 C35 | A15 B14 C35 | A15 B15 C35 | A15 B16 C35 | A15 B17 C35 |
| A15 B4 C36 | A15 B5 C36 | A15 B6 C36 | A15 B7 C36 | A15 B11 C36 | A15 B13 C36 | A15 B14 C36 | A15 B15 C36 | A15 B16 C36 | A15 B17 C36 |
| A15 B4 C37 | A15 B5 C37 | A15 B6 C37 | A15 B7 C37 | A15 B11 C37 | A15 B13 C37 | A15 B14 C37 | A15 B15 C37 | A15 B16 C37 | A15 B17 C37 |
| A15 B4 C38 | A15 B5 C38 | A15 B6 C38 | A15 B7 C38 | A15 B11 C38 | A15 B13 C38 | A15 B14 C38 | A15 B15 C38 | A15 B16 C18 | A15 B17 C38 |
| A15 B4 C39 | A15 B5 C39 | A15 B6 C39 | A15 B7 C39 | A15 B11 C39 | A15 B13 C39 | A15 B14 C39 | A15 B15 C39 | A15 B16 C39 | A15 B17 C39 |
| A15 B4 C40 | A15 B5 C40 | A15 B6 C40 | A15 B7 C40 | A15 B11 C40 | A15 B13 C40 | A15 B14 C40 | A15 B15 C40 | A15 B16 C40 | A15 B17 C40 |
| A15 B4 C41 | A15 85 C41 | A15 B6 C41 | A15 B7 C41 | A15 B11 C41 | A15 B13 C41 | A15 B14 C41 | A15 B15 C41 | A15 B16 C41 | A15 B17 C41 |
| | | | | | | | | | |
| A17 B4 C1 | A17 85 C1 | A17 B6 C1 | A17 B7 C1 | A17 B11 C1 | A17 B13 C1 | A17 B14 C1 | A17 B15 C1 | A17 B16 C1 | A17 B17 C1 |
| A17 B4 C2 | A17 B5 C2 | A17 B6 C2 | A17 B7 C2 | A17 B11 C2 | A17 B13 C2 | A17 B14 C2 | A17 B15 C2 | A17 B16 C2 | A17 B17 C2 |
| A17 B4 C3 | A17 B5 C3 | A17 B6 C3 | A17 B7 C3 | A17 B11 C3 | A17 B13 C3 | A17 B14 C3 | A17 B15 C3 | A17 B16 C3 | A17 B17 C3 |
| A17 B4 C4 | A17 B5 C4 | A17 B6 C4 | A17 B7 C4 | A17 B11 C4 | A17 B13 C4 | A17 B14 C4 | A17 B15 C4 | A17 B16 C4 | A17 B17 C4 |
| A17 B4 C5 | A17 B5 C5 | A17 B6 C5 | A17 B7 C5 | A17 B11 C5 | A17 B13 C5 | A17 B14 C5 | A17 B15 C5 | A17 B16 C5 | A17 B17 C5 |
| A17 B4 C6 | A17 B5 C6 | A17 B6 C6 | A17 B7 C6 | A17 B11 C6 | A17 B13 C6 | A17 B14 C6 | A17 B15 C6 | A17 B16 C6 | A17 B17 C6 |
| A17 B4 C7 | A17 B5 C7 | A17 B6 C7 | A17 B7 C7 | A17 B11 C7 | A17 B13 C7 | A17 B14 C7 | A17 B15 C7 | A17 B16 C7 | A17 B17 C7 |
| A17 B4 C8 | A17 B5 C8 | A17 B6 C8 | A17 B7 C8 | A17 B11 C8 | A17 B13 C8 | A17 B14 C8 | A17 B15 C8 | A17 B16 C8 | A17 B17 C8 |
| A17 B4 C9 | A17 B5 C9 | A17 B6 C9 | A17 B7 C9 | A17 B11 C9 | A17 B13 C9 | A17 B14 C9 | A17 B15 C9 | A17 B16 C9 | A17 B17 C9 |
| A17 B4 C10 | A17 B5 C10 | A17 B6 C10 | A17 B7 C10 | A17 B11 C10 | A17 B13 C10 | A17 B14 C10 | A17 B15 C10 | A17 B16 C10 | A17 B17 C10 |
| A17 B4 C11 | A17 B5 C11 | A17 86 C11 | A17 B7 C11 | A17 B11 C11 | A17 B13 C11 | A17 B14 C11 | A17 B15 C11 | A17 B16 C11 | A17 B17 C11 |
| A17 B4 C12 | A17 B5 C12 | A17 B6 C12 | A17 B7 C12 | A17 B11 C12 | A17 B13 C12 | A17 B14 C12 | A17 B15 C12 | A17 B16 C12 | A17 B17 C12 |
| A17 B4 C13 | A17 85 C13 | A17 B6 C13 | A17 B7 C13 | A17 B11 C13 | A17 B13 C13 | A17 B14 C13 | A17 B15 C13 | A17 B16 C13 | A17 B17 C13 |
| A17 B4 C14 | A17 B5 C14 | A17 B6 C14 | A17 B7 C14 | A17 B11 C14 | A17 B13 C14 | A17 B14 C14 | A17 B15 C14 | A17 B16 C14 | A17 B17 C14 |
| A17 B4 C15 | A17 B5 C15 | A17 B6 C15 | A17 B7 C15 | A17 B11 C15 | A17 B13 C15 | A17 B14 C15 | A17 B15 C15 | A17 B16 C15 | A17 B17 C15 |
| A17 B4 C16 | A17 B5 C16 | A17 B6 C16 | A17 B7 C16 | A17 B11 C16 | A17 B13 C16 | A17 B14 C16 | A17 B15 C16 | A17 B16 C16 | A17 B17 C16 |
| A17 B4 C17 | A17 B5 C17 | A17 B6 C17 | A17 B7 C17 | A17 B11 C17 | A17 B13 C17 | A17 B14 C17 | A17 B15 C17 | A17 B16 C17 | A17 B17 C17 |
| A17 B4 C18 | A17 B5 C18 | A17 B6 C18 | A17 B7 C18 | A17 B11 C18 | A17 B13 C18 | A17 B14 C18 | A17 B15 C18 | A17 B16 C18 | A17 B17 C18 |
| A17 B4 C19 | A17 B5 C19 | A17 B6 C19 | A17 B7 C19 | A17 B11 C19 | A17 B13 C19 | A17 B14 C19 | A17 B15 C19 | A17 B16 C19 | A17 B17 C19 |
| A17 B4 C20 | A17 B5 C20 | A17 B6 C20 | A17 B7 C20 | A17 B11 C20 | A17 B13 C20 | A17 B14 C20 | A17 B15 C20 | A17 B16 C20 | A17 B17 C20 |
| A17 B4 C21 | A17 B5 C21 | A17 B6 C21 | A17 B7 C21 | A17 B11 C21 | A17 B13 C21 | A17 B14 C21 | A17 B15 C21 | A17 B16 C21 | A17 B17 C21 |
| A17 B4 C22 | A17 B5 C22 | A17 B6 C22 | A17 B7 C22 | A17 B11 C22 | A17 B13 C22 | A17 B14 C22 | A17 B15 C22 | A17 B16 C22 | A17 B17 C22 |
| A17 B4 C23 | A17 B5 C23 | A17 B6 C23 | A17 B7 C23 | A17 B11 C23 | A17 B13 C23 | A17 B14 C23 | A17 B15 C23 | A17 B16 C23 | A17 B17 C23 |
| A17 B4 C24 | A17 B5 C24 | A17 B6 C24 | A17 B7 C24 | A17 B11 C24 | A17 B13 C24 | A17 B14 C24 | A17 B15 C24 | A17 B16 C24 | A17 B17 C24 |
| A17 B4 C25 | A17 B5 C25 | A17 B6 C25 | A17 B7 C25 | A17 B11 C25 | A17 B13 C25 | A17 B14 C25 | A17 B15 C25 | A17 B16 C25 | A17 B17 C25 |
| A17 B4 C26 | A17 B5 C26 | A17 B6 C26 | A17 B7 C26 | A17 B11 C26 | A17 B13 C26 | A17 B14 C26 | A17 B15 C26 | A17 B16 C26 | A17 B17 C26 |
| A17 B4 C27 | A17 B5 C27 | A17 B6 C27 | A17 B7 C27 | A17 B11 C27 | A17 B13 C27 | A17 B14 C27 | A17 B15 C27 | A17 B16 C27 | A17 B17 C27 |
| A17 B4 C28 | A17 B5 C28 | A17 B6 C28 | A17 B7 C28 | A17 B11 C28 | A17 B13 C28 | A17 B14 C28 | A17 B15 C28 | A17 B16 C28 | A17 B17 C28 |
| A17 B4 C29 | A17 B5 C29 | A17 B6 C29 | A17 B7 C29 | A17 B11 C29 | A17 B13 C29 | A17 B14 C29 | A17 B15 C29 | A17 B16 C29 | A17 B17 C29 |
| A17 B4 C30 | A17 B5 C30 | A17 B6 C30 | A17 B7 C30 | A17 B11 C30 | A17 B13 C30 | A17 B14 C30 | A17 B15 C30 | A17 B16 C30 | A17 B17 C30 |
| A17 B4 C31 | A17 B5 C31 | A17 B6 C31 | A17 B7 C31 | A17 B11 C31 | A17 B13 C31 | A17 B14 C31 | A17 B15 C31 | A17 B16 C31 | A17 B17 C31 |
| A17 B4 C32 | A17 B5 C32 | A17 B6 C32 | A17 B7 C32 | A17 B11 C32 | A17 B13 C32 | A17 B14 C32 | A17 B15 C32 | A17 B16 C32 | A17 B17 C32 |
| A17 B4 C33 | A17 B5 C33 | A17 B6 C33 | A17 B7 C33 | A17 B11 C33 | A17 B13 C33 | A17 B14 C33 | A17 B15 C33 | A17 B16 C33 | A17 B17 C33 |
| A17 B4 C34 | A17 B5 C34 | A17 B6 C34 | A17 B7 C34 | A17 B11 C34 | A17 B13 C34 | A17 B14 C34 | A17 B15 C34 | A17 B16 C34 | A17 B17 C34 |
| A17 B4 C35 | A17 B5 C35 | A17 B6 C35 | A17 B7 C35 | A17 B11 C35 | A17 B13 C35 | A17 B14 C35 | A17 B15 C35 | A17 B16 C35 | A17 B17 C35 |
| A17 B4 C36 | A17 B5 C36 | A17 B6 C36 | A17 B7 C36 | A17 B11 C36 | A17 B13 C36 | A17 B14 C36 | A17 B15 C36 | A17 B16 C36 | A17 B17 C36 |
| A17 B4 C37 | A17 B5 C37 | A17 B6 C37 | A17 B7 C37 | A17 B11 C37 | A17 B13 C37 | A17 B14 C37 | A17 B15 C37 | A17 B16 C37 | A17 B17 C37 |
| A17 B4 C38 | A17 B5 C38 | A17 B6 C38 | A17 B7 C38 | A17 B11 C38 | A17 B13 C38 | A17 B14 C38 | A17 B15 C38 | A17 B16 C38 | A17 B17 C38 |
| A17 B4 C39 | A17 B5 C39 | A17 B6 C39 | A17 B7 C39 | A17 B11 C39 | A17 B13 C39 | A17 B14 C39 | A17 B15 C39 | A17 B16 C39 | A17 B17 C39 |
| A17 B4 C40 | A17 B5 C40 | A17 B6 C40 | A17 B7 C40 | A17 B11 C40 | A17 B13 C40 | A17 B14 C40 | A17 B15 C40 | A17 B16 C40 | A17 B17 C40 |
| A17 B4 C41 | A17 B5 C41 | A17 B6 C41 | A17 B7 C41 | A17 B11 C41 | A17 B13 C41 | A17 B14 C41 | A17 B15 C41 | A17 B16 C41 | A17 B17 C41 |
| | | | | | | | | | |
| A26 B4 C1 | A26 B5 C1 | A26 B6 C1 | A26 B7 C1 | A26 B11 C1 | A26 B13 C1 | A26 B14 C1 | A26 B15 C1 | A26 B16 C1 | A26 B17 C1 |
| A26 B4 C2 | A26 B5 C2 | A26 B6 C2 | A26 B7 C2 | A26 B11 C2 | A26 B13 C2 | A26 B14 C2 | A26 B15 C2 | A26 B16 C2 | A26 B17 C2 |
| A26 B4 C3 | A26 B5 C3 | A26 B6 C3 | A26 B7 C3 | A26 B11 C3 | A26 B13 C3 | A26 B14 C3 | A26 B15 C3 | A26 B16 C3 | A26 B17 C3 |
| A26 B4 C4 | A26 B5 C4 | A26 B6 C4 | A26 B7 C4 | A26 B11 C4 | A26 B13 C4 | A26 B14 C4 | A26 B15 C4 | A26 B16 C4 | A26 B17 C4 |
| A26 B4 C5 | A26 B5 C5 | A26 B6 C5 | A26 B7 C5 | A26 B11 C5 | A26 B13 C5 | A26 B14 C5 | A26 B15 C5 | A26 B16 C5 | A26 B17 C5 |
| A26 B4 C6 | A26 B5 C6 | A26 B6 C6 | A26 B7 C6 | A26 B11 C6 | A26 B13 C6 | A26 B14 C6 | A26 B15 C6 | A26 B16 C6 | A26 B17 C6 |
| A26 B4 C7 | A26 B5 C7 | A26 B6 C7 | A26 B7 C7 | A26 B11 C7 | A26 B13 C7 | A26 B14 C7 | A26 B15 C7 | A26 B16 C7 | A26 B17 C7 |
| A26 B4 C8 | A26 B5 C8 | A26 B6 C8 | A26 B7 C8 | A26 B11 C8 | A26 B13 C8 | A26 B14 C8 | A26 B15 C8 | A26 B16 C8 | A26 B17 C8 |
| A26 B4 C9 | A26 B5 C9 | A26 B6 C9 | A26 B7 C9 | A26 B11 C9 | A26 B13 C9 | A26 B14 C9 | A26 B15 C9 | A26 B16 C9 | A26 B17 C9 |
| A26 B4 C10 | A26 B5 C10 | A26 B6 C10 | A26 B7 C10 | A26 B11 C10 | A26 B13 C10 | A26 B14 C10 | A26 B15 C10 | A26 B16 C10 | A26 B17 C10 |
| A26 B4 C11 | A26 B5 C11 | A26 B6 C11 | A26 B7 C11 | A26 B11 C11 | A26 B13 C11 | A26 B14 C11 | A26 B15 C11 | A26 B16 C11 | A26 B17 C11 |
| A26 B4 C12 | A26 B5 C12 | A26 B6 C12 | A26 B7 C12 | A26 B11 C12 | A26 B13 C12 | A26 B14 C12 | A26 B15 C12 | A26 B16 C12 | A26 B17 C12 |
| A26 B4 C33 | A26 B5 C13 | A26 B6 C13 | A26 B7 C13 | A26 B11 C13 | A26 B13 C13 | A26 B14 C13 | A26 B15 C13 | A26 B16 C13 | A26 B17 C13 |
| A26 B4 C14 | A26 B5 C14 | A26 B6 C14 | A26 B7 C14 | A26 B11 C14 | A26 B13 C14 | A26 B14 C14 | A26 B15 C14 | A26 B16 C14 | A26 B17 C14 |
| A26 B4 C15 | A26 B5 C15 | A26 B6 C15 | A26 B7 C15 | A26 B11 C15 | A26 B13 C15 | A26 B14 C15 | A26 B15 C15 | A26 B16 C15 | A26 B17 C15 |
| A26 B4 C16 | A26 B5 C16 | A26 B6 C16 | A26 B7 C16 | A26 B11 C16 | A26 B13 C16 | A26 B14 C16 | A26 B15 C16 | A26 B16 C16 | A26 B17 C16 |
| A26 B4 C17 | A26 B5 C17 | A26 B6 C17 | A26 B7 C17 | A26 B11 C17 | A26 B13 C17 | A26 B14 C17 | A26 B15 C17 | A26 B16 C17 | A26 B17 C17 |
| A26 B4 C18 | A26 B5 C18 | A26 B6 C18 | A26 B7 C18 | A26 B11 C18 | A26 B13 C18 | A26 B14 C18 | A26 B15 C18 | A26 B16 C18 | A26 B17 C18 |
| A26 B4 C19 | A26 B5 C19 | A26 B6 C19 | A26 B7 C19 | A26 B11 C19 | A26 B13 C19 | A26 B14 C19 | A26 B15 C19 | A26 B16 C19 | A26 B17 C19 |
| A26 B4 C20 | A26 B5 C20 | A26 B6 C20 | A26 B7 C20 | A26 B11 C20 | A26 B13 C20 | A26 B14 C20 | A26 B15 C20 | A26 B16 C20 | A26 B17 C20 |
| A26 B4 C21 | A26 B5 C21 | A26 B6 C21 | A26 B7 C21 | A26 B11 C21 | A26 B13 C21 | A26 B14 C21 | A26 B15 C21 | A26 B16 C21 | A26 B17 C21 |
| A26 B4 C22 | A26 B5 C22 | A26 B6 C22 | A26 B7 C22 | A26 B11 C22 | A26 B13 C22 | A26 B14 C22 | A26 B15 C22 | A26 B16 C22 | A26 B17 C22 |
| A26 B4 C23 | A26 B5 C23 | A26 B6 C23 | A26 B7 C23 | A26 B11 C23 | A26 B13 C23 | A26 B14 C23 | A26 B15 C23 | A26 B16 C23 | A26 B17 C23 |
| A26 B4 C24 | A26 B5 C24 | A26 B6 C24 | A26 B7 C24 | A26 B11 C24 | A26 B13 C24 | A26 B14 C24 | A26 B15 C24 | A26 B16 C24 | A26 B17 C24 |
| A26 B4 C25 | A26 B5 C25 | A26 B6 C25 | A26 B7 C25 | A26 B11 C25 | A26 B13 C25 | A26 B14 C25 | A26 B15 C25 | A26 B16 C25 | A26 B17 C25 |
| A26 B4 C26 | A26 B5 C26 | A26 B6 C26 | A26 B7 C26 | A26 B11 C26 | A26 B13 C26 | A26 B14 C26 | A26 B15 C26 | A26 B16 C26 | A26 B17 C26 |
| A26 B4 C27 | A26 B5 C27 | A26 B6 C27 | A26 B7 C27 | A26 B11 C27 | A26 B13 C27 | A26 B14 C27 | A26 B15 C27 | A26 B16 C27 | A26 B17 C27 |
| A26 B4 C28 | A26 B5 C28 | A26 B6 C28 | A26 B7 C28 | A26 B11 C28 | A26 B13 C28 | A26 B14 C28 | A26 B15 C28 | A26 B16 C28 | A26 B17 C28 |
| A26 B4 C29 | A26 B5 C29 | A26 B6 C29 | A26 B7 C29 | A26 B11 C29 | A26 B13 C29 | A26 B14 C29 | A26 B15 C29 | A26 B16 C29 | A26 B17 C29 |
| A26 B4 C30 | A26 B5 C30 | A26 B6 C30 | A26 B7 C30 | A26 B11 C30 | A26 B13 C30 | A26 B14 C30 | A26 B15 C30 | A26 B16 C30 | A26 B17 C30 |
| A26 B4 C31 | A26 B5 C31 | A26 B6 C31 | A26 B7 C31 | A26 B11 C31 | A26 B13 C31 | A26 B14 C31 | A26 B15 C31 | A26 B16 C31 | A26 B17 C31 |
| A26 B4 C32 | A26 B5 C32 | A26 B6 C32 | A26 B7 C32 | A26 B11 C32 | A26 B13 C32 | A26 B14 C32 | A26 B15 C32 | A26 B16 C32 | A26 B17 C32 |
| A26 B4 C33 | A26 B5 C33 | A26 B6 C33 | A26 B7 C33 | A26 B11 C33 | A26 B13 C33 | A26 B14 C33 | A26 B15 C33 | A26 B16 C33 | A26 B17 C33 |
| A26 B4 C34 | A26 B5 C34 | A26 B6 C34 | A26 B7 C34 | A26 B11 C34 | A26 B13 C34 | A26 B14 C34 | A26 B15 C34 | A26 B16 C34 | A26 B17 C34 |
| A26 B4 C35 | A26 B5 C35 | A26 B6 C35 | A26 B7 C35 | A26 B11 C35 | A26 B13 C35 | A26 B14 C35 | A26 B15 C35 | A26 B16 C35 | A26 B17 C35 |
| A26 B4 C36 | A26 B5 C36 | A26 B6 C36 | A26 B7 C36 | A26 B11 C36 | A26 B13 C36 | A26 B14 C36 | A26 B15 C36 | A26 B16 C36 | A26 B17 C36 |
| A26 B4 C37 | A26 B5 C37 | A26 B6 C37 | A26 B7 C37 | A26 B11 C37 | A26 B13 C37 | A26 B14 C37 | A26 B15 C37 | A26 B16 C37 | A26 B17 C37 |
| A26 B4 C38 | A26 B5 C38 | A26 B6 C38 | A26 B7 C38 | A26 B11 C38 | A26 B13 C38 | A26 B14 C38 | A26 B15 C38 | A26 B16 C38 | A26 B17 C38 |
| A26 B4 C39 | A26 B5 C39 | A26 B6 C39 | A26 B7 C39 | A26 B11 C39 | A26 B13 C39 | A26 B14 C39 | A26 B15 C39 | A26 B16 C39 | A26 B17 C39 |
| A26 B4 C40 | A26 B5 C40 | A26 B6 C40 | A26 B7 C40 | A26 B11 C40 | A26 B13 C40 | A26 B14 C40 | A26 B15 C40 | A26 B16 C40 | A26 B17 C40 |
| A26 B4 C41 | A26 B5 C41 | A26 B6 C41 | A26 B7 C41 | A26 B11 C41 | A26 B13 C41 | A26 B14 C41 | A26 B15 C41 | A26 B16 C41 | A26 B17 C41 |
| | | | | | | | | | |
| A27 B4 C1 | A27 B5 C1 | A27 B6 C1 | A27 B7 C1 | A27 B11 C1 | A27 B13 C1 | A27 B14 C1 | A27 B15 C1 | A27 B16 C1 | A27 B17 C1 |
| A27 B4 C2 | A27 B5 C2 | A27 B6 C2 | A27 B7 C2 | A27 B11 C2 | A27 B13 C2 | A27 B14 C2 | A27 B15 C2 | A27 B16 C2 | A27 B17 C2 |
| A27 B4 C3 | A27 B5 C3 | A27 B6 C3 | A27 B7 C3 | A27 B11 C3 | A27 B13 C3 | A27 B14 C3 | A27 B15 C3 | A27 B16 C3 | A27 B17 C3 |
| A27 B4 C4 | A27 B5 C4 | A27 B6 C4 | A27 B7 C4 | A27 B11 C4 | A27 B13 C4 | A27 B14 C4 | A27 B15 C4 | A27 B16 C4 | A27 B17 C4 |
| A27 B4 C5 | A27 B5 C5 | A27 B6 C5 | A27 B7 C5 | A27 B1 C5 | A27 B13 C5 | A27 B14 C5 | A27 B15 C5 | A27 B16 C5 | A27 B17 C5 |
| A27 B4 C6 | A27 B5 C6 | A27 B6 C6 | A27 B7 C6 | A27 B11 C6 | A27 B13 C6 | A27 B14 C6 | A27 B15 C6 | A27 B16 C6 | A27 B17 C6 |
| A27 B4 C7 | A27 B5 C7 | A27 B6 C7 | A27 B7 C7 | A27 B11 C7 | A27 B13 C7 | A27 B14 C7 | A27 B15 C7 | A27 B16 C7 | A27 B17 C7 |
| A27 B4 C8 | A27 B5 C8 | A27 B6 C8 | A27 B7 C8 | A27 B11 C8 | A27 B13 C8 | A27 B14 C8 | A27 B15 C8 | A27 B16 C8 | A27 B17 C8 |
| A27 B4 C9 | A27 B5 C9 | A27 B6 C9 | A27 B7 C9 | A27 B11 C9 | A27 B13 C9 | A27 B14 C9 | A27 B15 C9 | A27 B16 C9 | A27 B17 C9 |
| A27 B4 C10 | A27 B5 C10 | A27 B6 C10 | A27 B7 C10 | A27 B11 C10 | A27 B13 C10 | A27 B14 C10 | A27 B15 C10 | A27 B16 C10 | A27 B17 C10 |
| A27 B4 C11 | A27 B5 C11 | A27 B6 C11 | A27 B7 C11 | A27 B11 C11 | A27 B13 C11 | A27 B14 C11 | A27 B15 C11 | A27 B16 C11 | A27 B17 C11 |
| A27 B4 C12 | A27 B5 C12 | A27 B6 C12 | A27 B7 C12 | A27 B11 C12 | A27 B13 C12 | A27 B14 C12 | A27 B15 C12 | A27 B16 C12 | A27 B17 C12 |
| A27 B4 C13 | A27 B5 C13 | A27 B6 C13 | A27 B7 C13 | A27 B11 C13 | A27 B13 C13 | A27 B14 C13 | A27 B15 C13 | A27 B16 C13 | A27 B17 C13 |
| A27 B4 C14 | A27 B5 C14 | A27 B6 C14 | A27 B7 C14 | A27 B11 C14 | A27 B13 C14 | A27 B14 C14 | A27 B15 C14 | A27 B16 C14 | A27 B17 C14 |
| A27 B4 C15 | A27 B5 C15 | A27 B6 C15 | A27 B7 C15 | A27 B11 C15 | A27 B13 C15 | A27 B14 C15 | A27 B15 C15 | A27 B16 C15 | A27 B17 C15 |
| A27 B4 C16 | A27 B5 C16 | A27 B6 C16 | A27 B7 C16 | A27 B11 C16 | A27 B13 C16 | A27 B14 C16 | A27 B15 C16 | A27 B16 C16 | A27 B17 C16 |
| A27 B4 C17 | A27 B5 C17 | A27 B6 C17 | A27 B7 C17 | A27 B11 C17 | A27 B13 C17 | A27 B14 C17 | A27 B15 C17 | A27 B16 C17 | A27 B17 C17 |
| A27 B4 C18 | A27 B5 C18 | A27 B6 C18 | A27 B7 C18 | A27 B11 C18 | A27 B13 C18 | A27 B14 C18 | A27 B15 C18 | A27 B16 C18 | A27 B17 C18 |
| A27 B4 C19 | A27 B5 C19 | A27 B6 C19 | A27 B7 C19 | A27 B11 C19 | A27 B13 C19 | A27 B14 C19 | A27 B15 C19 | A27 B16 C19 | A27 B17 C19 |
| A27 B4 C20 | A27 B5 C20 | A27 B6 C20 | A27 B7 C20 | A27 B11 C20 | A27 B13 C20 | A27 B14 C20 | A27 B15 C20 | A27 B16 C20 | A27 B17 C20 |
| A27 B4 C21 | A27 B5 C21 | A27 B6 C21 | A27 B7 C21 | A27 B11 C21 | A27 B13 C21 | A27 B14 C21 | A27 B15 C21 | A27 B16 C21 | A27 B17 C21 |
| A27 B4 C22 | A27 B5 C22 | A27 B6 C22 | A27 B7 C22 | A27 B11 C22 | A27 B13 C22 | A27 B14 C22 | A27 B15 C22 | A27 B16 C22 | A27 B17 C22 |
| A27 B4 C23 | A27 B5 C23 | A27 B6 C23 | A27 B7 C23 | A27 B11 C23 | A27 B13 C23 | A27 B14 C23 | A27 B15 C23 | A27 B16 C23 | A27 B17 C23 |
| A27 B4 C24 | A27 B5 C24 | A27 B6 C24 | A27 B7 C24 | A27 B11 C24 | A27 B13 C24 | A27 B14 C24 | A27 B15 C24 | A27 B16 C24 | A27 B17 C24 |
| A27 B4 C25 | A27 B5 C25 | A27 B6 C25 | A27 B7 C25 | A27 B11 C25 | A27 B13 C25 | A27 B14 C25 | A27 B15 C25 | A27 B16 C25 | A27 B17 C25 |
| A27 B4 C26 | A27 B5 C26 | A27 B6 C26 | A27 B7 C26 | A27 B11 C26 | A27 B13 C26 | A27 B14 C26 | A27 B15 C26 | A27 B16 C26 | A27 B17 C26 |
| A27 B4 C27 | A27 B5 C27 | A27 B6 C27 | A27 B7 C27 | A27 B11 C27 | A27 B13 C27 | A27 B14 C27 | A27 B15 C27 | A27 B16 C27 | A27 B17 C27 |
| A27 B4 C28 | A27 B5 C28 | A27 B6 C28 | A27 B7 C28 | A27 B11 C28 | A27 B13 C28 | A27 B14 C28 | A27 B15 C28 | A27 B16 C28 | A27 B17 C28 |
| A27 B4 C29 | A27 B5 C29 | A27 B6 C29 | A27 B7 C29 | A27 B11 C29 | A27 B13 C29 | A27 B14 C29 | A27 B15 C29 | A27 B16 C29 | A27 B17 C29 |
| A27 B4 C30 | A27 B5 C30 | A27 B6 C30 | A27 B7 C30 | A27 B11 C30 | A27 B13 C30 | A27 B14 C30 | A27 B15 C30 | A27 B16 C30 | A27 B17 C30 |
| A27 B4 C31 | A27 B5 C31 | A27 B6 C31 | A27 B7 C31 | A27 B11 C31 | A27 B13 C31 | A27 B14 C31 | A27 B15 C31 | A27 B16 C31 | A27 B17 C31 |
| A27 B4 C32 | A27 B5 C32 | A27 B6 C32 | A27 B7 C32 | A27 B11 C32 | A27 B13 C32 | A27 B14 C32 | A27 B15 C32 | A27 B16 C32 | A27 B17 C32 |
| A27 B4 C33 | A27 B5 C33 | A27 B6 C33 | A27 B7 C33 | A27 B11 C33 | A27 B13 C33 | A27 B14 C33 | A27 B15 C33 | A27 B16 C33 | A27 B17 C33 |
| A27 B4 C34 | A27 B5 C34 | A27 B6 C34 | A27 B7 C34 | A27 B11 C34 | A27 B13 C34 | A27 B14 C34 | A27 B15 C34 | A27 B16 C34 | A27 B17 C34 |
| A27 B4 C35 | A27 B5 C35 | A27 B6 C35 | A27 B7 C35 | A27 B11 C35 | A27 B13 C35 | A27 B14 C35 | A27 B15 C35 | A27 B16 C35 | A27 B17 C35 |
| A27 B4 C36 | A27 B5 C36 | A27 B6 C36 | A27 B7 C36 | A27 B11 C36 | A27 B13 C36 | A27 B14 C36 | A27 B15 C36 | A27 B16 C36 | A27 B17 C36 |
| A27 B4 C37 | A27 B5 C37 | A27 B6 C37 | A27 B7 C37 | A27 B11 C37 | A27 B13 C37 | A27 B14 C37 | A27 B15 C37 | A27 B16 C37 | A27 B17 C37 |
| A27 B4 C38 | A27 B5 C38 | A27 B6 C38 | A27 B7 C38 | A27 B11 C38 | A27 B13 C38 | A27 B14 C38 | A27 B15 C38 | A27 B16 C33 | A27 B17 C38 |
| A27 B4 C39 | A27 B5 C39 | A27 B6 C39 | A27 B7 C39 | A27 B11 C39 | A27 B13 C39 | A27 B14 C39 | A27 B15 C39 | A27 B16 C39 | A27 B17 C39 |
| A27 B4 C40 | A27 B5 C40 | A27 B6 C40 | A27 B7 C40 | A27 B11 C40 | A27 B13 C40 | A27 B14 C40 | A27 B15 C40 | A27B16C40 | A27B17C40 |
| A27 B4 C41 | A27 B5 C41 | A27 B6 C41 | A27 B7 C41 | A27 B11 C41 | A27 B13 C41 | A27 B14 C41 | A27 B15 C41 | A27 B16 C41 | A27 B17 C41 |
| | | | | | | | | | |
| A29 B4 C1 | A29 B5 C1 | A29 B6 C1 | A29 B7 C1 | A29 B11 C1 | A29 B13 C1 | A29 B14 C1 | A29 B15 C1 | A29 B16 C1 | A29 B17 C1 |
| A29 B4 C2 | A29 B5 C2 | A29 B6 C2 | A29 B7 C2 | A29 B11 C2 | A29 B13 C2 | A29 B14 C2 | A29 B15 C2 | A29 B16 C2 | A29 B17 C2 |
| A29 B4 C3 | A29 B5 C3 | A29 B6 C3 | A29 B7 C3 | A29 B11 C3 | A29 B13 C3 | A29 B14 C3 | A29 B15 C3 | A29 B16 C3 | A29 B17 C3 |
| A29 B4 C4 | A29 B5 C4 | A29 B6 C4 | A29 B7 C4 | A29 B11 C4 | A29 B13 C4 | A29 B14 C4 | A29 B15 C4 | A29 B16 C4 | A29 B17 C4 |
| A29 B4 C5 | A29 B5 C5 | A29 B6 C5 | A29 B7 C5 | A29 B11 C5 | A29 B13 C5 | A29 B14 C5 | A29 B15 C5 | A29 B16 C5 | A29 B17 C5 |
| A29 B4 C6 | A29 B5 C6 | A29 B6 C6 | A29 B7 C6 | A29 B11 C6 | A29 B13 C6 | A29 B14 C6 | A29 B15 C6 | A29 B16 C6 | A29 B17 C6 |
| A29 B4 C7 | A29 B5 C7 | A29 B6 C7 | A29 B7 C7 | A29 B11 C7 | A29 B13 C7 | A29 B14 C7 | A29 B15 C7 | A29 B16 C7 | A29 B17 C7 |
| A29 B4 C8 | A29 B5 C8 | A29 B6 C8 | A29 B7 C8 | A29 B11 C8 | A29 B13 C8 | A29 B14 C8 | A29 B15 C8 | A29 B16 C8 | A29 B17 C8 |
| A29 B4 C9 | A29 B5 C9 | A29 B6 C9 | A29 B7 C9 | A29 B11 C9 | A29 B13 C9 | A29 B14 C9 | A29 B15 C9 | A29 B16 C9 | A29 B17 C9 |
| A29 B4 C10 | A29 B5 C10 | A29 B6 C10 | A29 B7 C10 | A29 B11 C10 | A29 B13 C10 | A29 B14 C10 | A29 B15 C10 | A29 B16 C10 | A29 B17 C10 |
| A29 B4 C11 | A29 B5 C11 | A29 B6 C11 | A29 B7 C11 | A29 B11 C11 | A29 B13 C11 | A29 B14 C11 | A29 B15 C11 | A29 B16 C11 | A29 B17 C11 |
| A29 B4 C12 | A29 B5 C12 | A29 B6 C12 | A29 B7 C12 | A29 B11 C12 | A29 B13 C12 | A29 B14 C12 | A29 B15 C12 | A29 B16 C12 | A29 B17 C12 |
| A29 B4 C13 | A29 B5 C13 | A29 B6 C13 | A29 B7 C13 | A29 B11 C13 | A29 B13 C13 | A29 B14 C13 | A29 B15 C13 | A29 B16 C13 | A29 B17 C13 |
| A29 B4 C14 | A29 B5 C14 | A29 B6 C14 | A29 B7 C14 | A29 B11 C14 | A29 B13 C14 | A29 B14 C14 | A29 B15 C14 | A29 B16 C14 | A29 B17 C14 |
| A29 B4 C15 | A29 B5 C15 | A29 B6 C15 | A29 B7 C15 | A29 B11 C15 | A29 B13 C15 | A29 B14 C15 | A29 B15 C15 | A29 B16 C15 | A29 B17 C15 |
| A29 B4 C16 | A29 B5 C16 | A29 B6 C16 | A29 B7 C16 | A29 B11 C16 | A29 B13 C16 | A29 B14 C16 | A29 B15 C16 | A29 B16 C16 | A29 B17 C16 |
| A29 B4 C17 | A29 B5 C17 | A29 B6 C17 | A29 B7 C17 | A29 B11 C17 | A29 B13 C17 | A29 B14 C17 | A29 B15 C17 | A29 B16 C17 | A29 B17 C17 |
| A29 B4 C18 | A29 B5 C18 | A29 B6 C18 | A29 B7 C18 | A29 B11 C18 | A29 B13 C18 | A29 B14 C18 | A29 B1S C18 | A29 B16 C18 | A29 B17 C18 |
| A29 B4 C19 | A29 B5 C19 | A29 B6 C19 | A29 B7 C19 | A29 B11 C19 | A29 B13 C19 | A29 B14 C19 | A29 B15 C19 | A29 B16 C19 | A29 B17 C19 |
| A29 B4 C20 | A29 B5 C20 | A29 B6 C20 | A29 B7 C20 | A29 B11 C20 | A29 B13 C20 | A29 B14 C20 | A29 B15 C20 | A29 B16 C20 | A29 B17 C20 |
| A29 B4 C21 | A29 B5 C21 | A29 B6 C21 | A29 B7 C21 | A29 B11 C21 | A29 B13 C21 | A29 B14 C21 | A29 B15 C21 | A29 B16 C21 | A29 B17 C21 |
| A29 B4 C22 | A29 B5 C22 | A29 B6 C22 | A29 B7 C22 | A29 B11 C22 | A29 B13 C22 | A29 B14 C22 | A29 B15 C22 | A29 B16 C22 | A29 B17 C22 |
| A29 B4 C23 | A29 B5 C23 | A29 B6 C23 | A29 B7 C23 | A29 B11 C23 | A29 B13 C23 | A29 B14 C23 | A29 B15 C23 | A29 B16 C23 | A29 B17 C23 |
| A29 B4 C24 | A29 B5 C24 | A29 B6 C24 | A29 B7 C24 | A29 B11 C24 | A29 B13 C24 | A29 B14 C24 | A29 B15 C24 | A29 B16 C24 | A29 B17 C24 |
| A29 B4 C25 | A29 B5 C2S | A29 B6 C25 | A29 B7 C25 | A29 B11 C25 | A29 B13 C25 | A29 B14 C25 | A29 B15 C25 | A29 B16 C25 | A29 B17 C25 |
| A29 B4 C26 | A29 B5 C26 | A29 B6 C26 | A29 B7 C26 | A29 B11 C26 | A29 B13 C26 | A29 B14 C26 | A29 B15 C26 | A29 B16 C26 | A29 B17 C26 |
| A29 B4 C27 | A29 B5 C27 | A29 B6 C27 | A29 B7 C27 | A29 B11 C27 | A29 B13 C27 | A29 B14 C27 | A29 B15 C27 | A29 B16 C27 | A29 B17 C27 |
| A29 B4 C28 | A29 B5 C28 | A29 B6 C28 | A29 B7 C28 | A29 B11 C28 | A29 B13 C28 | A29 B14 C28 | A29 B15 C28 | A29 B16 C28 | A29 B17 C28 |
| A29 B4 C29 | A29 B5 C29 | A29 B6 C29 | A29 B7 C29 | A29 B11 C29 | A29 B13 C29 | A29 B14 C29 | A29 B15 C29 | A29 B16 C29 | A29 B17 C29 |
| A29 B4 C30 | A29 B5 C30 | A29 B6 C30 | A29 B7 C30 | A29 B11 C30 | A29 B13 C30 | A29 B14 C30 | A29 B15 C30 | A29 B16 C30 | A29 B17 C30 |
| A29 B4 C31 | A29 B5 C31 | A29 B6 C31 | A29 B7 C31 | A29 B11 C31 | A29 B13 C31 | A29 B14 C31 | A29 B15 C31 | A29 B16 C31 | A29 B17 C31 |
| A29 B4 C32 | A29 B5 C32 | A29 B6 C32 | A29 B7 C32 | A29 B11 C32 | A29 B13 C32 | A29 B14 C32 | A29 B15 C32 | A29 B16 C32 | A29 B17 C32 |
| A29 B4 C33 | A29 B5 C33 | A29 B6 C33 | A29 B7 C33 | A29 B11 C33 | A29 B13 C33 | A29 B14 C33 | A29 B15 C33 | A29 B16 C33 | A29 B17 C33 |
| A29 B4 C34 | A29 B5 C34 | A29 B6 C34 | A29 B7 C34 | A29 B11 C34 | A29 B13 C34 | A29 B14 C34 | A29 B15 C34 | A29 B16 C34 | A29 B17 C34 |
| A29 B4 C35 | A29 B5 C35 | A29 B6 C35 | A29 B7 C35 | A29 B11 C35 | A29 B13 C35 | A29 B14 C35 | A29 B15 C35 | A29 B16 C35 | A29 B17 C35 |
| A29 B4 C36 | A29 B5 C36 | A29 B6 C36 | A29 B7 C36 | A29 B11 C36 | A29 B13 C36 | A29 B14 C36 | A29 B15 C36 | A29 B16 C36 | A29 B17 C36 |
| A29 B4 C37 | A29 B5 C37 | A29 B6 C37 | A29 B7 C37 | A29 B11 C37 | A29 B13 C37 | A29 B14 C37 | A29 B15 C37 | A29 B16 C37 | A29 B17 C37 |
| A29 B4 C38 | A29 B5 C38 | A29 B6 C38 | A29 B7 C38 | A29 B11 C38 | A29 B13 C38 | A29 B14 C38 | A29 B15 C38 | A29 B16 C38 | A29 B17 C38 |
| A29 B4 C39 | A29 B5 C39 | A29 B6 C39 | A29 B7 C39 | A29 B11 C39 | A29 B13 C39 | A29 B14 C39 | A29 B15 C39 | A29 B16 C39 | A29 B17 C39 |
| A29 B4 C40 | A29 B5 C40 | A29 B6 C40 | A29 B7 C40 | A29 B11 C40 | A29 B13 C40 | A29 B14 C40 | A29 B15 C40 | A29 B16 C40 | A29 B17 C40 |
| A29 B4 C41 | A29 B5 C41 | A29 B6 C41 | A29 B7 C41 | A29 B11 C41 | A29 B13 C41 | A29 B14 C41 | A29 B15 C41 | A29 B16 C11 | A29 B17 C41 |
| | | | | | | | | | |
| A30 B4 C1 | A30 B5 C1 | A30 B6 C1 | A30 B7 C1 | A30 B11 C1 | A30 B13 C1 | A30 B14 C1 | A30 B15 C1 | A30 B16 C1 | A30 B17 C1 |
| A30 B4 C2 | A30 B5 C2 | A30 B6 C2 | A30 B7 C2 | A30 B11 C2 | A30 B13 C2 | A30 B14 C2 | A30 B15 C2 | A30 B16 C2 | A30 B17 C2 |
| A30 B4 C3 | A30 B5 C3 | A30 B6 C3 | A30 B7 C3 | A30 B11 C3 | A30 B13 C3 | A30 B14 C3 | A30 B15 C3 | A30 B16 C3 | A30 B17 C3 |
| A30 B4 C4 | A30 B5 C4 | A30 B6 C4 | A30 B7 C4 | A30 B11 C4 | A30 B13 C4 | A30 B14 C4 | A30 B15 C4 | A30 B16 01 | A30 B17 C4 |
| A30 B4 C5 | A30 B5 C5 | A30 B6 C5 | A30 B7 C5 | A30 B11 C5 | A30 B13 C5 | A30 B14 C5 | A30 B15 C5 | A30 B16 C5 | A30 B17 C5 |
| A30 B4 C6 | A30 B5 C6 | A30 B6 C6 | A30 B7 C6 | A30 B11 C6 | A30 B13 C6 | A30 B14 C6 | A30 B15 C6 | A30 B16 C6 | A30 B17 C6 |
| A30 B4 C7 | A30 B5 C7 | A30 B6 C7 | A30 B7 C7 | A30 B11 C7 | A30 B13 C7 | A30 B14 C7 | A30 B15 C7 | A30 B16 C7 | A30 B17 C7 |
| A30 B4 C8 | A30 B5 C8 | A30 B6 C8 | A30 B7 C8 | A30 B11 C8 | A30 B13 C8 | A30 B14 C8 | A30 B15 C8 | A30 B16 C13 | A30 B17 C8 |
| A30 B4 C9 | A30 B5 C9 | A30 B6 C9 | A30 B7 C9 | A30 B11 C9 | A30 B13 C9 | A30 B14 C9 | A30 B15 C9 | A30 B16 C9 | A30 B17 C9 |
| A30 B4 C10 | A30 B5 C10 | A30 B6 C10 | A30 B7 C10 | A30 B11 C10 | A30 B13 C10 | A30 B14 C10 | A30 B15 C10 | A30 B16 C10 | A30 B17 C10 |
| A30 B4 C11 | A30 B5 C11 | A30 B6 C11 | A30 B7 C11 | A30 B11 C11 | A30 B13 C11 | A30 B14 C11 | A30 B15 C11 | A30 B16 C11 | A30 B17 C11 |
| A30 B4 C12 | A30 B5 C12 | A30 B6 C12 | A30 B7 C12 | A30 B11 C12 | A30 B13 C12 | A30 B14 C12 | A30 B15 C12 | A30 B16 C12 | A30 B17 C12 |
| A30 B4 C13 | A30 B5 C13 | A30 B6 C13 | A30 B7 C13 | A30 B11 C13 | A30 B13 C13 | A30 B14 C13 | A30 B15 C13 | A30 B16 C13 | A30 B17 C13 |
| A30 B4 C14 | A30 B5 C14 | A30 B6 C14 | A30 B7 C14 | A30 B11 C14 | A30 B13 C14 | A30 B14 C14 | A30 B15 C14 | A30 B16 C14 | A30 B17 C14 |
| A30 B4 C15 | A30 B5 C15 | A30 B6 C15 | A30 B7 C15 | A30 B11 C15 | A30 B13 C15 | A30 B14 C15 | A30 B15 C15 | A30 B16 C15 | A30 B17 C15 |
| A30 B4 C16 | A30 B5 C16 | A30 B6 C16 | A30 B7 C16 | A30 B11 C16 | A30 B13 C16 | A30 B14 C16 | A30 B15 C16 | A30 B16 C16 | A30 B17 C16 |
| A30 B4 C17 | A30 B5 C17 | A30 B6 C17 | A30 B7 C17 | A30 B11 C17 | A30 B13 C17 | A30 B14 C17 | A30 B15 C17 | A30 B16 C17 | A30 B17 C17 |
| A30 B4 C18 | A30 B5 C18 | A30 B6 C18 | A30 B7 C18 | A30 B11 C18 | A30 B13 C18 | A30 B14 C18 | A30 B15 C18 | A30 B16 C18 | A30 B17 C18 |
| A30 B4 C19 | A30 B5 C19 | A30 B6 C19 | A30 B7 C19 | A30 B11 C19 | A30 B13 C19 | A30 B14 C19 | A30 B15 C19 | A30 B16 C19 | A30 B17 C19 |
| A30 B4 C20 | A30 B5 C20 | A30 B6 C20 | A30 B7 C20 | A30 B11 C20 | A30 B13 C20 | A30 B14 C20 | A30 B15 C20 | A30 B16 C20 | A30 B17 C20 |
| A30 B4 C21 | A30 B5 C21 | A30 B6 C21 | A30 B7 C21 | A30 B11 C21 | A30 B13 C21 | A30 B14 C21 | A30 B15 C21 | A30 B16 C21 | A30 B17 C21 |
| A30 B4 C22 | A30 B5 C22 | A30 B6 C22 | A30 B7 C22 | A30 B11 C22 | A30 B13 C22 | A30 B14 C22 | A30 B15 C22 | A30 B16 C22 | A30 B17 C22 |
| A30 B4 C23 | A30 B5 C23 | A30 B6 C23 | A30 B7 C23 | A30 B11 C23 | A30 B13 C23 | A30 B14 C23 | A30 B15 C23 | A30 B16 C23 | A30 B17 C23 |
| A30 B4 C24 | A30 B5 C24 | A30 B6 C24 | A30 B7 C24 | A30 B11 C24 | A30 B13 C24 | A30 B14 C24 | A30 B15 C24 | A30 B16 C24 | A30 B17 C24 |
| A30 B4 C25 | A30 B5 C25 | A30 B6 C25 | A30 B7 C25 | A30 B11 C25 | A30 B13 C25 | A30 B14 C25 | A30 B15 C25 | A30 B16 C25 | A30 B17 C25 |
| A30 B4 C26 | A30 B5 C26 | A30 B6 C26 | A30 B7 C26 | A30 B11 C26 | A30 B13 C26 | A30 B14 C26 | A30 B15 C26 | A30 B16 C26 | A30 B17 C26 |
| A30 B4 C27 | A30 B5 C27 | A30 B6 C27 | A30 B7 C27 | A30 B11 C27 | A30 B13 C27 | A30 B14 C27 | A30 B15 C27 | A30 B16 C27 | A30 B17 C27 |
| A30 B4 C28 | A30 B5 C28 | A30 B6 C28 | A30 B7 C28 | A30 811 C28 | A30 B13 C28 | A30 B14 C28 | A30 B15 C28 | A30 B16 C28 | A30 B17 C28 |
| A30 B4 C29 | A30 B5 C29 | A30 B6 C29 | A30 B7 C29 | A30 B11 C29 | A30 B13 C29 | A30 B14 C29 | A30 B15 C29 | A30 B16 C29 | A30 B17 C29 |
| A30 B4 C30 | A30 B5 C30 | A30 B6 C30 | A30 B7 C30 | A30 B11 C30 | A30 B13 C30 | A30 B14 C30 | A30 B15 C30 | A30 B16 C30 | A30 B17 C30 |
| A30 B4 C31 | A30 B5 C31 | A30 B6 C31 | A30 B7 C31 | A30 B11 C31 | A30 B13 C31 | A30 B14 C31 | A30 B15 C31 | A30 B16 C31 | A30 B17 C31 |
| A30 B4 C32 | A30 B5 C32 | A30 B6 C32 | A30 B7 C32 | A30 B11 C32 | A30 B13 C32 | A30 B14 C32 | A30 B15 C32 | A30 B16 C32 | A30 B17 C32 |
| A30 B4 C33 | A30 B5 C33 | A30 B6 C33 | A30 B7 C33 | A30 B11 C33 | A30 B13 C33 | A30 B14 C33 | A30 B15 C33 | A30 B16 C33 | A30 B17 C33 |
| A30 B4 C34 | A30 B5 C34 | A30 B6 C34 | A30 B7 C34 | A30 B11 C34 | A30 B13 C34 | A30 B14 C34 | A30 B15 C34 | A30 B16 C34 | A30 B17 C34 |
| A30 B4 C35 | A30 B5 C35 | A30 B6 C35 | A30 B7 C35 | A30 B11 C35 | A30 B13 C35 | A30 B14 C35 | A30 B15 C35 | A30 B16 C35 | A30 B17 C35 |
| A30 B4 C36 | A30 B5 C36 | A30 B6 C36 | A30 B7 C36 | A30 B11 C36 | A30 B13 C36 | A30 B14 C36 | A30 B15 C36 | A30 B16 C36 | A30 B17 C36 |
| A30 B4 C37 | A30 B5 C37 | A30 B6 C37 | A30 B7 C37 | A30 B11 C37 | A30 B13 C37 | A30 B14 C37 | A30 B15 C37 | A30 B16 C37 | A30 B17 C37 |
| A30 B4 C38 | A30 B5 C38 | A30 B6 C38 | A30 B7 C38 | A30 B11 C38 | A30 B13 C38 | A30 B14 C38 | A30 B15 C38 | A30 B16 C38 | A30 B17 C38 |
| A30 B4 C39 | A30 B5 C39 | A30 B6 C39 | A30 B7 C39 | A30 B11 C39 | A30 B13 C39 | A30 B14 C39 | A30 B15 C39 | A30 B16 C39 | A30 B17 C39 |
| A30 B4 C40 | A30 B5 C40 | A30 B6 C40 | A30 B7 C40 | A30 B11 C40 | A30 B13 C40 | A30 B14 C40 | A30 B15 C40 | A30 B16 C40 | A30 B17 C40 |
| A30 B4 C41 | A30 B5 C41 | A30 B6 C41 | A30 B7 C41 | A30 B11 C41 | A30 B13 C41 | A30 B14 C41 | A30 B15 C41 | A30 B16 C41 | A30 B17 C41 |
| | | | | | | | | | |
| A32 B4 C1 | A32 B5 C1 | A32 B6 C1 | A32 B7 C1 | A32 B11 C1 | A32 B13 C1 | A32 B14 C1 | A32 B15 C1 | A32 B16 C1 | A32 B17 C1 |
| A32 B4 C2 | A32 B5 C2 | A32 B6 C2 | A32 B7 C2 | A32 B11 C2 | A32 B13 C2 | A32 B14 C2 | A32 B15 C2 | A32 B16 C2 | A32 B17 C2 |
| A32 B4 C3 | A32 B5 C3 | A32 B6 C3 | A32 B7 C3 | A32 B11 C3 | A32 B13 C3 | A32 B14 C3 | A32 B15 C3 | A32 B16 C3 | A32 B17 C3 |
| A32 B4 C4 | A32 B5 C4 | A32 B6 C4 | A32 B7 C4 | A32 B11 C4 | A32 B13 C4 | A32 B14 C4 | A32 B15 C4 | A32 B16 C4 | A32 B17 C4 |
| A32 B4 C5 | A32 B5 C5 | A32 B6 C5 | A32 B7 C5 | A32 B11 C5 | A32 B13 C5 | A32 B14 C5 | A32 B15 C5 | A32 B16 C5 | A32 B17 C5 |
| A32 B4 C6 | A32 B5 C6 | A32 B6 C6 | A32 B7 C6 | A32 B11 C6 | A32 B13 C6 | A32 B14 C6 | A32 B15 C6 | A32 B16 C6 | A32 B17 C6 |
| A32 B4 C7 | A32 B5 C7 | A32 B6 C7 | A32 B7 C7 | A32 B11 C7 | A32 B13 C7 | A32 B14 C7 | A32 B15 C7 | A32 B16 C7 | A32 B17 C7 |
| A32 B4 C8 | A32 B5 C8 | A32 B6 C8 | A32 B7 C8 | A32 B11 C8 | A32 B13 C8 | A32 B14 C8 | A32 B15 C8 | A32 B16 C8 | A32 B17 C8 |
| A32 B4 C9 | A32 B5 C9 | A32 B6 C9 | A32 B7 C9 | A32 B11 C9 | A32 B13 C9 | A32 B14 C9 | A32 B15 C9 | A32 B16 C9 | A32 B17 C9 |
| A32 B4 C10 | A32 B5 C10 | A32 B6 C10 | A32 B7 C10 | A32 B11 C10 | A32 B13 C10 | A32 B14 C10 | A32 B15 C10 | A32 B16 C10 | A32 B17 CIO |
| A32 B4 C11 | A32 B5 C11 | A32 B6 C11 | A32 B7 C11 | A32 B11 C11 | A32 B13 C11 | A32 B14 C11 | A32 B15 C11 | A32 B16 C11 | A32 B17 C11 |
| A32 B4 C12 | A32 B5 C12 | A32 B6 C12 | A32 B7 C12 | A32 B11 C12 | A32 B13 C12 | A32 B14 C12 | A32 B15 C12 | A32 B16 C12 | A32 B17 C12 |
| A32 B4 C13 | A32 B5 C13 | A32 B6 C13 | A32 B7 C13 | A32 B11 C13 | A32 B13 C13 | A32 B14 C13 | A32 B15 C13 | A32 B16 C13 | A32 B17 C13 |
| A32 B4 C14 | A32 B5 C14 | A32 B6 C14 | A32 B7 C14 | A32 B11 C14 | A32 B13 C14 | A32 B14 C14 | A32 B15 C14 | A32 B16 C14 | A32 B17 C14 |
| A32 B4 C15 | A32 B5 C15 | A32 B6 C15 | A32 B7 C15 | A32 B11 C15 | A32 B13 C15 | A32 B14 C15 | A32 B15 C15 | A32 B16 C15 | A32 B17 C15 |
| A32 B4 C16 | A32 B5 C6 | A32 B6 C6 | A32 B7 C16 | A32 B11 C16 | A32 B13 C16 | A32 B14 C16 | A32 B15 C16 | A32 B16 C16 | A32 B17 C16 |
| A32 B4 C17 | A32 B5 C17 | A32 B6 C17 | A32 B7 C17 | A32 B11 C17 | A32 B13 C17 | A32 B14 C17 | A32 B15 C17 | A32 B16 C17 | A32 B17 C17 |
| A32 B4 C18 | A32 B5 C18 | A32 B6 C18 | A32 B7 C18 | A32 B11 C18 | A32 B13 C18 | A32 B14 C18 | A32 B15 C18 | A32 B16 C18 | A32 B17 C18 |
| A32 B4 C19 | A32 B5 C19 | A32 B6 C19 | A32 B7 C19 | A32 B11 C19 | A32 B13 C19 | A32 B14 C19 | A32 B15 C19 | A32 B16 C19 | A32 B17 C19 |
| A32 B4 C20 | A32 B5 C20 | A32 B6 C20 | A32 B7 C20 | A32 B11 C20 | A32 B13 C20 | A32 B14 C20 | A32 B15 C20 | A32 B16 C20 | A32 B17 C20 |
| A32 B4 C21 | A32 B5 C21 | A32 B6 C21 | A32 B7 C21 | A32 B11 C21 | A32 B13 C21 | A32 B14 C21 | A32 B15 C21 | A32 B16 C21 | A32 B17 C21 |
| A32 B4 C22 | A32 B5 C22 | A32 B6 C22 | A32 B7 C22 | A32 B11 C22 | A32 B13 C22 | A32 B14 C22 | A32 B15 C22 | A32 B16 C22 | A32 B17 C22 |
| A32 B4 C23 | A32 B5 C23 | A32 B6 C23 | A32 B7 C23 | A32 B11 C23 | A32 B13 C23 | A32 B14 C23 | A32 B15 C23 | A32 B16 C23 | A32 B17 C23 |
| A32 B4 C24 | A32 B5 C24 | A32 B6 C24 | A32 B7 C24 | A32 B11 C24 | A32 B13 C24 | A32 B14 C24 | A32 B15 C24 | A32 B16 C24 | A32 B17 C24 |
| A32 B4 C25 | A32 B4 C25 | A32 B6 C25 | A32 B7 C25 | A32 B11 C25 | A32 B13 C25 | A32 B14 C25 | A32 B15 C25 | A32 B16 C25 | A32 B17 C25 |
| A32 B4 C26 | A32 B5 C26 | A32 B6 C26 | A32 B7 C26 | A32 B11 C26 | A32 B13 C26 | A32 B14 C26 | A32 B15 C26 | A32 B16 C26 | A32 B17 C26 |
| A32 B4 C27 | A32 B5 C27 | A32 B6 C27 | A32 B7 C27 | A32 B11 C27 | A32 B13 C27 | A32 B14 C27 | A32 B15 C27 | A32 B16 C27 | A32 B17 C27 |
| A32 B4 C28 | A32 B5 C28 | A32 B6 C28 | A32 B7 C28 | A32 B11 C28 | A32 B13 C28 | A32 B14 C28 | A32 B15 C28 | A32 B16 C28 | A32 B17 C28 |
| A32 B4 C29 | A32 B5 C29 | A32 B6 C29 | A32 B7 C29 | A32 B11 C29 | A32 B13 C29 | A32 B14 C29 | A32 B15 C29 | A32 B16 C29 | A32 B17 C29 |
| A32 B4 C30 | A32 B5 C30 | A32 B6 C30 | A32 B7 C30 | A32 B11 C30 | A32 B13 C30 | A32 B14 C30 | A32 B15 C30 | A32 B16 C30 | A32 B17 C30 |
| A32 B4 C31 | A32 B5 C31 | A32 B6 C31 | A32 B7 C31 | A32 B11 C31 | A32 B13 C31 | A32 B14 C31 | A32 B15 C31 | A32 B16 C31 | A32 B17 C31 |
| A32 B4 C32 | A32 B5 C32 | A32 B6 C32 | A32 B7 C32 | A32 B11 C32 | A32 B13 C32 | A32 B14 C32 | A32 B15 C32 | A32 B16 C32 | A32 B17 C32 |
| A32 B4 C33 | A32 B5 C33 | A32 B6 C33 | A32 B7 C33 | A32 B11 C33 | A32 B13 C33 | A32 B14 C33 | A32B15C33 | A32 B16 C33 | A32 B17 C33 |
| A32 B4 C34 | A32 B5 C34 | A32 B6 C34 | A32 B7 C34 | A32 B11 C34 | A32 B13 C34 | A32 B14 C34 | A32 B15 C34 | A32 B16 C34 | A32 B17 C34 |
| A32 B4 C35 | A32 B5 C35 | A32 B6 C35 | A32 B7 C35 | A32 B11 C35 | A32 B13 C35 | A32 B14 C35 | A32 B15 C35 | A32 B16 C35 | A32 B17 C35 |
| A32 B4 C36 | A32 B5 C36 | A32 B6 C36 | A32 B7 C36 | A32 B11 C36 | A32 B13 C36 | A32 B14 C36 | A32 B15 C36 | A32 B16 C36 | A32 B17 C36 |
| A32 B4 C37 | A32 B5 C37 | A32 B6 C37 | A32 B7 C37 | A32 B11 C37 | A32 B13 C37 | A32 B14 C37 | A32 B15 C37 | A32 B16 C37 | A32 B17 C37 |
| A32 B4 C38 | A32B5 C38 | A32 B6 C38 | A32 B7 C38 | A32 B11 C38 | A32 B13 C38 | A32 B14 C38 | A32 B15 C38 | A32 B16 C38 | A32 B17 C38 |
| A32 B4 C39 | A32 B5 C39 | A32 B6 C39 | A32 B7 C39 | A32 B11 C39 | A32 B13 C39 | A32 B14 C39 | A32 B15 C39 | A32 B16 C39 | A32 B17 C39 |
| A32 B4 C40 | A32 B5 C40 | A32 B6 C40 | A32 B7 C40 | A32 B11 C40 | A32 B13 C40 | A32 B14 C40 | A32 B15 C40 | A32 B16 C40 | A32 B17 C40 |
| A32 B4 C41 | A32 B5 C41 | A32 B6 C41 | A32 B7 C41 | A32 B11 C41 | A32 B13 C41 | A32 B14 C41 | A32 B15 C41 | A32 B16 C41 | A32 B17 C41 |

Specific compounds that are preferred for Cathepsin S inhibitory activity:

| | | | | | | | | | |
|---|---|---|---|---|---|---|---|---|---|
| A4 B6 C1 | A4 B7 C1 | A4 B8 C1 | A4 B9 C1 | A4 B10 C1 | A4 B12 C1 | A4 B18 C1 | A4 B22 C1 | A4 B23 C1 | A4 B25 C1 |
| A4 B6 C2 | A4 B7 C2 | A4 B8 C2 | A4 B9 C2 | A4 B10 C2 | A4 B12 C2 | A4 B18 C2 | A4 B22 C2 | A4 B23 C2 | A4 B25 C2 |
| A4 B6 C3 | A4 B7 C3 | A4 B8 C3 | A4 B9 C3 | A4 B10 C3 | A4 B12 C3 | A4 B18 C3 | A4 B22 C3 | A4 B23 C3 | A4 B25 C3 |
| A4 B6 C4 | A4 B7 C4 | A4 B8 C4 | A4 B9 C4 | A4 B10 C4 | A4 B12 C4 | A4 B18 C4 | A4 B22 C4 | A4 B23 C4 | A4 B25 C4 |
| A4 B6 C5 | A4 B7 C5 | A4 B8 C5 | A4 B9 C5 | A4 B10 C5 | A4 B12 C5 | A4 B18 C5 | A4 B22 C5 | A4 B23 C5 | A4 B25 C5 |
| A4 B6 C6 | A4 B7 C6 | A4 B8 C6 | A4 B9 C6 | A4 B10 C6 | A4 B12 C6 | A4 B18 C6 | A4 B22 C6 | A4 B23 C6 | A4 B25 C6 |
| A4 B6 C7 | A4 B7 C7 | A4 B8 C7 | A4 B9 C7 | A4 B10 C7 | A4 B12 C7 | A4 B18 C7 | A4 B22 C7 | A4 B23 C7 | A4 B25 C7 |
| A4 B6 C8 | A4 B7 C8 | A4 B8 C8 | A4 B9 C8 | A4 B10 C8 | A4 B12 C8 | A4 BI8 C8 | A4 B22 C8 | A4 B23 C8 | A4 B25 C8 |
| A4 B6 C9 | A4 B7 C9 | A4 B8 C9 | A4 B9 C9 | A4 B10 C9 | A4 B12 C9 | A4 B18 C9 | A4 B22 C9 | A4 B23 C9 | A4 B25 C9 |
| A4 B6 C10 | A4 B7 C10 | A4 B8 C10 | A4 B9 C10 | A4 B10 C10 | A4 B12 C10 | A4 B18 C10 | A4 B22 C10 | A4 B23 C10 | A4 B25 C10 |
| A4 B6 C11 | A4 B7 C11 | A4 B8 C11 | A4 B9 C11 | A4 B10 C11 | A4 B12 C11 | A4 B18 C11 | A4 B22 C11 | A4 B23 C11 | A4 B25 C11 |
| A4 B6 C12 | A4 B7 C12 | A4 B8 C12 | A4 B9 C12 | A4 B10 C12 | A4 B12 C12 | A4 B18 C12 | A4 B22 C12 | A4 B23 C12 | A4 B25 C12 |
| A4 B6 C13 | A4 B7 C13 | A4 B8 C13 | A4 B9 C13 | A4 B10 C13 | A4 B12 C13 | A4 B18 C13 | A4 B22 C13 | A4 B23 C13 | A4 B25 C13 |
| A4 B6 C14 | A4 B7 C14 | A4 B8 C14 | A4 B9 C14 | A4 B10 C14 | A4 B12 C14 | A4 B18 C14 | A4 B22 C14 | A4 B23 C14 | A4 B25 C14 |
| A4 B6 C15 | A4 B7 C15 | A4 B8 C15 | A4 B9 C15 | A4 B10 C15 | A4 B12 C15 | A4 B18 C15 | A4 B22 C15 | A4 B23 C15 | A4 B25 C15 |
| A4 B6 C16 | A4 B7 C16 | A4 B8 C16 | A4 B9 C16 | A4 B10 C16 | A4 B12 C16 | A4 B18 C16 | A4 B22 C16 | A4 B23 C16 | A4 B25 C16 |
| A4 B6 C17 | A4 B7 C17 | A4 B8 C17 | A4 B9 C17 | A4 B10 C17 | A4 B12 C17 | A4 B18 C17 | A4 B22 C17 | A4 B23 C17 | A4 B25 C17 |
| A4 B6 C18 | A4 B7 C18 | A4 B8 C18 | A4 B9 C18 | A4 B10 C18 | A4 B12 C18 | A4 B18 C18 | A4 B22 C18 | A4 B23 C18 | A4 B25 C18 |
| A4 B6 C19 | A4 B7 C19 | A4 B8 C19 | A4 B9 C19 | A4 B10 C19 | A4 B12 C19 | A4 B18 C19 | A4 B22 C19 | A4 B23 C19 | A4 B25 C19 |
| A4 B6 C20 | A4 B7 C20 | A4 B8 C20 | A4 B9 C20 | A4 B10 C20 | A4 B12 C20 | A4 B18 C20 | A4 B22 C20 | A4 B23 C20 | A4 B25 C20 |
| A4 B6 C21 | A4 B7 C21 | A4 B8 C21 | A4 B9 C21 | A4 B10 C21 | A4 B12 C21 | A4 B18 C21 | A4 B22 C21 | A4 B23 C21 | A4 B25 C21 |
| A4 B6 C22 | A4 B7 C22 | A4 B8 C22 | A4 B9 C22 | A4 B10 C22 | A4 B12 C22 | A4 B18 C22 | A4 B22 C22 | A4 B23 C22 | A4 B25 C22 |
| A4 B6 C23 | A4 B7 C23 | A4 B8 C23 | A4 B9 C23 | A4 BIO C23 | A4 B12 C23 | A4 B18 C23 | A4 B22 C23 | A4 B23 C23 | A4 B25 C23 |
| A4 B6 C24 | A4 B7 C24 | A4 B8 C24 | A4 B9 C24 | A4 BIO C24 | A4 B12 C24 | A4 B18 C24 | A4 B22 C24 | A4 B23 C14 | A4 B25 C24 |
| A4 B6 C25 | A4 B7 C25 | A4 B8 C25 | A4 B9 C25 | A4 B10 C25 | A4 B12 C25 | A4 B18 C25 | A4 B22 C25 | A4 B23 C25 | A4 B25 C25 |
| A4 B6 C26 | A4 B7 C26 | A4 B8 C26 | A4 B9 C26 | A4 B10 C26 | A4 B12 C26 | A4 B18 C26 | A4 B22 C26 | A4 B23 C26 | A4 B25 C26 |
| A4 B6 C27 | A4 B7 C27 | A4 B8 C27 | A4 B9 C27 | A4 B10 C27 | A4 B12 C27 | A4 B18 C27 | A4 B22 C27 | A4 B23 C27 | A4 B25 C27 |
| A4 B6 C28 | A4 B7 C28 | A4 B8 C28 | A4 B9 C28 | A4 B10 C28 | A4 B12 C28 | A4 B18 C28 | A4 B22 C28 | A4 B23 C28 | A4 B25 C28 |
| A4 B6 C29 | A4 B7 C29 | A4 B8 C29 | A4 B9 C29 | A4 B10 C29 | A4 B12 C29 | A4 B18 C29 | A4 B22 C29 | A4 B23 C29 | A4 B25 C29 |
| A4 B6 C30 | A4 B7 C30 | A4 B8 C30 | A4 B9 C30 | A4 B10 C30 | A4 B12 C30 | A4 B18 C30 | A4 B22 C30 | A4 B23 C30 | A4 825 C30 |
| A4 B6 C31 | A4 B7 C31 | A4 B8 C31 | A4 B9 C31 | A4 B10 C31 | A4 B12 C31 | A4 B18 C31 | A4 B22 C31 | A4 B23 C31 | A4 B25 C31 |
| A4 B6 C32 | A4 B7 C32 | A4 B8 C32 | A4 B9 C32 | A4 B10 C32 | A4 B12 C32 | A4 B18 C32 | A4 B22 C32 | A4 B23 C32 | A4 B25 C32 |
| A4 B6 C33 | A4 B7 C33 | A4 B8 C33 | A4 B9 C33 | A4 B10 C33 | A4 B12 C33 | A4 B18 C33 | A4 B22 C33 | A4 B23 C33 | A4 B25 C33 |
| A4 B6 C34 | A4 B7 C34 | A4 B8 C34 | A4 B9 C34 | A4 B10 C34 | A4 B12 C34 | A4 B18 C34 | A4 B22 C34 | A4 B23 C34 | A4 B25 C34 |
| A4 B6 C35 | A4 B7 C35 | A4 B8C35 | A4 B9 C35 | A4 B10 C35 | A4 B12 C35 | A4 B18 C35 | A4 B22 C35 | A4 B23 C35 | A4 B25 C35 |
| A4 B6 C36 | A4 B7 C36 | A4 B8 C36 | A4 B9 C36 | A4 B10 C36 | A4 B12 C36 | A4 B18 C36 | A4 B22 C36 | A4 B23 C36 | A4 B25 C36 |
| A4 B6 C37 | A4 B7 C37 | A4 B8 C37 | A4 B9 C37 | A4 B10 C37 | A4 B12 C37 | A4 B18 C37 | A4 B22 C37 | A4 B23 C17 | A4 B25 C37 |
| A4 B6 C38 | A4 B7 C38 | A4 B8 C38 | A4 B9 C38 | A4 B10 C38 | A4 B12 C38 | A4 B18 C38 | A4 B22 C38 | A4 B23 C38 | A4 B25 C38 |
| A4 B6 C39 | A4 B7 C39 | A4 B8 C39 | A4 B9 C39 | A4 B10 C39 | A4 B12 C39 | A4 B18 C39 | A4 B22 C39 | A4 B23 C39 | A4 B25 C39 |
| A4 B6 C40 | A4 B7 C40 | A4 B8 C40 | A4 B9 C40 | A4 B10 C40 | A4 B12 C40 | A4 B18 C40 | A4 B22 C40 | A4 B23 C40 | A4 B25 C40 |
| A4 B6 C41 | A4 B7 C41 | A4 B8 C41 | A4 B9 C41 | A4 B10 C41 | A4 B12 C41 | A4 B18 C41 | A4 B22 C41 | A4 B23 C41 | A4 B25 C41 |
| | | | | | | | | | |
| A5 B6 C1 | A5 B7 C1 | A5 B8 C1 | A5 B9 C1 | A5 B10 C1 | A5 B12 C1 | A5 B18 C1 | A5 B22 C1 | A5 B23 C1 | A5 B25 C1 |
| A5 B6 C2 | A5 B7 C2 | A5 B8 C2 | A5 B9 C2 | A5 B10 C2 | A5 B12 C2 | A5 B18 C2 | A5 B22 C2 | A5 B23 C2 | A5 B25 C2 |
| A5 B6 C3 | A5 B7 C3 | A5 B8 C3 | A5 B9 C3 | A5 B10 C3 | A5 B12 C3 | A5 B18 C3 | A5 B22 C3 | A5 B23 C3 | A5 B25 C3 |
| A5 B6 C4 | A5 B7 C4 | A5 B8 C4 | A5 B9 C4 | A5 B10 C4 | A5 B12 C4 | A5 B18 C4 | A5 B22 C4 | A5 B23 C4 | A5 B25 C4 |
| A5 B6 C5 | A5 B7 C5 | A5 B8 C5 | A5 B9 C5 | A5 B10 C5 | A5 B12 C5 | A5 B18 C5 | A5 B22 C5 | A5 B23 C5 | A5 B25 C5 |
| A5 B6 C6 | A5 B7 C6 | A5 B8 C6 | A5 B9 C6 | A5 B10 C6 | A5 B12 C6 | A5 B18 C6 | A5 B22 C6 | A5 B23 C6 | A5 B25 C6 |
| A5 B6 C7 | A5 B7 C7 | A5 B8 C7 | A5 B9 C7 | A5 B10 C7 | A5 B12 C7 | A5 B18 C7 | A5 B22 C7 | A5 B23 C7 | A5 B25 C7 |
| A5 B6 C8 | A5 B7 C8 | A5 B8 C8 | A5 B9 C8 | A5 B10 C8 | A5 B12 C8 | A5 B18 C8 | A5 B22 C8 | A5 B23 C8 | A5 B25 C8 |
| A5 B6 C9 | A5 B7 C9 | A5 B8 C9 | A5 B9 C9 | A5 B10 C9 | A5 B12 C9 | A5 B18 C9 | A5 B22 C9 | A5 B23 C9 | A5 B25 C9 |
| A5 B6 C10 | A5 B7 C10 | A5 B8 C10 | A5 B9 C10 | A5 B10 C10 | A5 B12 C10 | A5 B18 C10 | A5 B22 C10 | A5 B23 C10 | A5 B25 C10 |
| A5 B6 C11 | A5 B7 C11 | A5 B8 C11 | A8 B9 C11 | A5 B10 C11 | A5 B12 C11 | A5 B18 C11 | A5 B22 C11 | A5 B23 C11 | A5 B25 C11 |
| A5 B6 C12 | A5 B7 C12 | A5 B8 C12 | A5 B9 C12 | A5 B10 C12 | A5 B12 C12 | A5 B18 C12 | A5 B22 C12 | A5 B23 C12 | A5 B25 C12 |
| A5 B6 C13 | A5 B7 C13 | A5 B8 C13 | A5 B9 C13 | A5 B10 C13 | A5 B12 C13 | A5 B18 C13 | A5 B22 C13 | A5 B23 C13 | A5 B25 C13 |
| A5 B6 C14 | A5 B7 C14 | A5 B8 C14 | A5 B9 C14 | A5 B10 C14 | A5 B12 C14 | A5 B18 C14 | A5 B22 C14 | A5 B23 C14 | A5 B25 C14 |
| A5 B6 C15 | A5 B7 C15 | A5 B8 C15 | A5 B9 C15 | A5 B10 C15 | A5 B12 C15 | A5 B18 C15 | A5 B22 C15 | A5 B23 C15 | A5 B25 C15 |
| A5 B6 C16 | A5 B7 C16 | A5 B8 C16 | A5 B9 C16 | A5 B10 C16 | A5 B12 C16 | A5 B18 C16 | A5 B22 C16 | A5 B23 C16 | A5 B25 C16 |
| A5 B6 C17 | A5 B7 C17 | A5 B8 C17 | A5 B9 C17 | A5 B10 C17 | A5 B12 C17 | A5 B18 C17 | A5 B22 C17 | A5 B23 C17 | A5 B25 C17 |
| A5 B6 C18 | A5 B7 C18 | A5 B8 C18 | A5 B9 C18 | A5 B10 C18 | A5 B12 C18 | A5 B18 C18 | A5 B22 C18 | A5 B23 C18 | A5 B25 C18 |
| A5 B6 C19 | A5 B7 C19 | A5 B8 C19 | A5 B9 C19 | A5 B10 C19 | A5 B12 C19 | A5 B18 C19 | A5 B22 C19 | A5 B23 C19 | A5 B25 C19 |
| A5 B6 C20 | A5 B7 C20 | A5 B8 C20 | A5 B9 C20 | A5 B10 C20 | A5 B12 C20 | A5 B18 C20 | A5 B22 C20 | A5 B23 C20 | A5 B25 C20 |
| A5 B6 C21 | A5 B7 C21 | A5 B8 C21 | A5 B9 C21 | A5 B10 C21 | A5 B12 C21 | A5 B18 C21 | A5 B22 C21 | A5 B23 C21 | A5 B25 C21 |
| A5 B6 C22 | A5 B7 C22 | A5 B8 C22 | A5 B9 C22 | A5 B10 C22 | A5 B12 C22 | A5 B18 C22 | A5 B22 C22 | A5 B23 C22 | A5 B25 C22 |
| A5 B6 C23 | A5 B7 C23 | A5 B8 C23 | A5 B9 C23 | A5 B10 C23 | A5 B12 C23 | A5 B18 C23 | A5 B22 C23 | A5 B23 C23 | A55 B2S C23 |
| A5 B6 C24 | A5 B7 C24 | A5 B8 C24 | A5 B9 C24 | A5 B10 C24 | A5 B12 C24 | A5 B18 C24 | A5 B22 C24 | A5 B23 C24 | A5 B25 C24 |
| A5 B6 C25 | A5 B7 C25 | A5 B8 C25 | A5 B9 C25 | A5 B10 C25 | A5 B12 C25 | A5 B18 C25 | A5 B22 C25 | A5 B23 C25 | A5 B25 C25 |
| A5 B6 C26 | A5 B7 C26 | A5 B8 C26 | A5 B9 C26 | A5 B10 C26 | A5 B12 C26 | A5 B18 C26 | A5 B22 C26 | A5 B23 C25 | A5 B25 C26 |
| A5 B6 C27 | A5 B7 C27 | A5 B8 C27 | A5 B9 C27 | A5 B10 C27 | A5 B12 C27 | A5 B18 C27 | A5 B22 C27 | A5 B23 C27 | A5 B25 C27 |
| A5 B6 C28 | A5 B7 C28 | A5 B8 C28 | A5 B9 C28 | A5 B10 C28 | A5 B12 C28 | A5 B18 C28 | A5 B22 C28 | A5 B23 C28 | A5 B25 C28 |
| A5 B6 C29 | A5 B7 C29 | A5 B8 C29 | A5 B9 C29 | A5 B10 C29 | A5 B12 C29 | A5 B18 C29 | A5 B22 C29 | A5 B23 C29 | A5 B25 C29 |
| A5 B6 C30 | A5 B7 C30 | A5 B8 C30 | A5 B9 C30 | A5 B10 C30 | A5 B12 C30 | A5 B18 C30 | A5 B22 C30 | A5 B23 C30 | A5 B25 C30 |
| A5 B6 C31 | A5 B7 C31 | A5 B8 C31 | A5 B9 C31 | A5 B10 C31 | A5 B12 C31 | A5 B18 C31 | A5 B22 C31 | A5 B23 C31 | A5 B25 C31 |
| A5 B6 C32 | A5 B7 C32 | A5 B8 C32 | A5 B9 C32 | A5 B10 C32 | A5 B12 C32 | A5 B18 C32 | A5 B22 C32 | A5 B23 C32 | A5 B25 C32 |
| A5 B6 C33 | A5 B7 C33 | A5 B8 C33 | A5 B9 C33 | A5 B10 C33 | A5 B12 C33 | A5 B18 C33 | A5 B22 C33 | A5 B23 C33 | A5 B25 C33 |
| A5 B6 C34 | A5 B7 C34 | A5 B8 C34 | A5 B9 C34 | A5 B10 C34 | A5 B12 C34 | A5 B18 C34 | A5 B22 C34 | A5 B23 C34 | A5 B25 C34 |
| A5 B6 C35 | A5 B7 C35 | A5 B8 C35 | A5 B9 C35 | A5 B10 C35 | A5 B12 C35 | A5 B18 C35 | A5 B22 C35 | A5 B23 C35 | A5 B25 C35 |
| A5 B6 C36 | A5 B7 C36 | A5 B8 C36 | A5 B9 C36 | A5 B10 C36 | A5 B12 C36 | A5 B18 C36 | A5 B22 C36 | A5 B23 C36 | A5 B25 C36 |
| A5 B6 C37 | A5 B7 C37 | A5 B8 C37 | A5 B9 C37 | A5 B10 C37 | A5 B12 C37 | A5 B18 C37 | A5 B22 C37 | A5 B23 C37 | A5 B25 C37 |
| A5 B6 C38 | A5 B7 C38 | A5 B8 C38 | A5 B9 C38 | A5 B10 C38 | A5 B12 C38 | A5 B18 C38 | A5 B22 C38 | A5 B23 C38 | A5 B25 C38 |
| A5 B6 C39 | A5 B7 C39 | A5 B8 C39 | A5 B9 C39 | A5 B10 C39 | A5 B12 C39 | A5 B18 C39 | A5 B22 C39 | A5 B23 C39 | A5 B25 C39 |
| A5 B6 C40 | A5 B7 C40 | A5 B8 C40 | A5 B9 C40 | A5 B10 C40 | A5 B12 C40 | A5 B18 C40 | A5 B22 C40 | A5 B23 C40 | A5 B25 C40 |
| A5 B6 C41 | A5 B7 C41 | A5 B8 C41 | A5 B9 C41 | A5 B10 C41 | A5 B12 C41 | A5 B18 C41 | A5 B22 C41 | A5 B23 C41 | A5 B25 C41 |
| | | | | | | | | | |
| A6 B6 C1 | A6 B7 C1 | A6 B8 C1 | A6 B9 C1 | A6 B10 C1 | A6 B12 C1 | A6 B18 C1 | A6 B22 C1 | A6 B23 C1 | A6 B25 C1 |
| A6 B6 C2 | A6 B7 C2 | A6 B8 C2 | A6 B9 C2 | A6 B10 C2 | A6 B12 C2 | A6 B18 C2 | A6 B22 C2 | A6 B23 C2 | A6 B25 C2 |
| A6 B6 C3 | A6 B7 C3 | A6 B8 C3 | A6 B9 C3 | A6 B10 C3 | A6 B12 C3 | A6 B18 C3 | A6 B22 C3 | A6 B23 C3 | A6 B25 C3 |
| A6 B6 C4 | A6 B7 C4 | A6 B8 C4 | A6 B9 C4 | A6 B10 C4 | A6 B12 C4 | A6 B18 C4 | A6 B22 C4 | A6 B23 C4 | A6 B25 C4 |
| A6 B6 C5 | A6 B7 C5 | A6 B8 C5 | A6 B9 C5 | A6 B10 C5 | A6 B12 C5 | A6 B18 C5 | A6 B22 C5 | A6 B23 C5 | A6 B25 C5 |
| A6 B6 C6 | A6 B7 C6 | A6 B8 C6 | A6 B9 C6 | A6 B10 C6 | A6 B12 C6 | A6 B18 C6 | A6 B22 C6 | A6 B23 C6 | A6 B25 C6 |
| A6 B6 C7 | A6 B7 C7 | A6 B8 C7 | A6 B9 C7 | A6 B10 C7 | A6 B12 C7 | A6 B18 C7 | A6 B22 C7 | A6 B23 C7 | A6 B25 C7 |
| A6 B6 C8 | A6 B7 C8 | A6 B8 C8 | A6 B9 C8 | A6 B10 C8 | A6 B12 C8 | A6 B18 C8 | A6 B22 C8 | A6 B23 C8 | A6 B25 C8 |
| A6 B6 C9 | A6 B7 C9 | A6 B8 C9 | A6 B9 C9 | A6 B10 C9 | A6 B12 C9 | A6 B18 C9 | A6 B22 C9 | A6 B23 C9 | A6 B25 C9 |
| A6 B6 C10 | A6 B7 C10 | A6 B8 C10 | A6 B9 C10 | A6 B10 C10 | A6 B12 C10 | A6 B18 C10 | A6 B22 C10 | A6 B23 C10 | A6 B25 C10 |
| A6 B6 C11 | A6 B7 C11 | A6 B8 C11 | A6 B9 C11 | A6 B10 C11 | A6 B12 C11 | A6 B18 C11 | A6 B22 C11 | A6 B23 C11 | A6 B25 C11 |
| A6 B6 C12 | A6 B7 C12 | A6 B8 C12 | A6 B9 C12 | A6 B10 C12 | A6 B12 C12 | A6 B18 C12 | A6 B22 C12 | A6 B23 C12 | A6 B25 C12 |
| A6 B6 C13 | A6 B7 C13 | A6 B8 C13 | A6 B9 C13 | A6 B10 C13 | A6 B12 C13 | A6 B18 C13 | A6 B22 C13 | A6 B23 C13 | A6 B25 C13 |
| A6 B6 C14 | A6 B7 C14 | A6 B8 C14 | A6 B9 C14 | A6 B10 C14 | A6 B12 C14 | A6 B18 C14 | A6 B22 C14 | A6 B23 C14 | A6 B25 C14 |
| A6 B6 C15 | A6 B7 C15 | A6 B8 C15 | A6 B9 C15 | A6 B10 C15 | A6 B12 C15 | A6 B18 C15 | A6 B22 C15 | A6 B23 C15 | A6 B25 C15 |
| A6 B6 C16 | A6 B7 C16 | A6 B8 C16 | A6 B9 C16 | A6 B10 C16 | A6 B12 C16 | A6 B18 C16 | A6 B22 C16 | A6 B23 C16 | A6 B25 C16 |
| A6 B6 C17 | A6 B7 C17 | A6 B8 C17 | A6 B9 C17 | A6 B10 C17 | A6 B12 C17 | A6 B18 C17 | A6 B22 C17 | A6 B23 C17 | A6 B25 C17 |
| A6 B6 C18 | A6 B7 C18 | A6 B8 C18 | A6 B9 C18 | A6 B10 C18 | A6 B12 C18 | A6 B18 C18 | A6 B22 C18 | A6 B23 C18 | A6 B25 C18 |
| A6 B6 C19 | A6 B7 C19 | A6 B8 C19 | A6 B9 C19 | A6 B10 C19 | A6 B12 C19 | A6 B18 C19 | A6 B22 C19 | A6 B23 C19 | A6 B25 C19 |
| A6 B6 C20 | A6 B7 C20 | A6 B8 C20 | A6 B9 C20 | A6 B10 C20 | A6 B12 C20 | A6 B18 C20 | A6 B22 C20 | A6 B23 C20 | A6 B25 C20 |
| A6 B6 C21 | A6 B7 C21 | A6 B8 C21 | A6 B9 C21 | A6 B10 C21 | A6 B12 C21 | A6 B18 C21 | A6 B22 C21 | A6 B23 C21 | A6 B25 C21 |
| A6 B6 C22 | A6 B7 C22 | A6 B8 C22 | A6 B9 C22 | A6 B10 C22 | A6 B12 C22 | A6 B18 C22 | A6 B22 C22 | A6 B23 C22 | A6 B25 C22 |
| A6 B6 C23 | A6 B7 C23 | A6 B8 C23 | A6 B9 C23 | A6 B10 C23 | A6 B12 C23 | A6 B18 C23 | A6 B22 C23 | A6 B23 C23 | A6 B25 C23 |
| A6 B6 C24 | A6 B7 C24 | A6 B8 C24 | A6 B9 C24 | A6 B10 C24 | A6 B12 C24 | A6 B18 C24 | A6 B22 C24 | A6 B23 C24 | A6 B25 C24 |
| A6 B6 C25 | A6 B7 C25 | A6 B8 C25 | A6 B9 C25 | A6 B10 C25 | A6 B12 C25 | A6 B18 C25 | A6 B22 C25 | A6 B23 C25 | A6 B25 C25 |
| A6 B6 C26 | A6 B7 C26 | A6 B8 C26 | A6 B9 C26 | A6 B10 C26 | A6 B12 C26 | A6 B18 C26 | A6 B22 C26 | A6 B23 C26 | A6 B25 C26 |
| A6 B6 C27 | A6 B7 C27 | A6 B8 C27 | A6 B9 C27 | A6 B10 C27 | A6 B12 C27 | A6 B18 C27 | A6 B22 C27 | A6 B23 C27 | A6 B25 C27 |
| A6 B6 C28 | A6 B7 C28 | A6 B8 C28 | A6 B9 C28 | A6 B10 C28 | A6 B12 C28 | A6 B18 C28 | A6 B22 C28 | A6 B23 C28 | A6 B25 C28 |
| A6 B6 C29 | A6 B7 C29 | A6 B8 C29 | A6 B9 C29 | A6 B10 C29 | A6 B12 C29 | A6 B18 C29 | A6 B22 C29 | A6 B23 C29 | A6 B25 C29 |
| A6 B6 C30 | A6 B7 C30 | A6 B8 C30 | A6 B9 C30 | A6 B10 C30 | A6 B12 C30 | A6 B18 C30 | A6 B22 C30 | A6 B23 C30 | A6 B25 C30 |
| A6 B6 C31 | A6 B7 C31 | A6 B8 C31 | A6 B9 C31 | A6 B10 C31 | A6 B12 C31 | A6 B18 C31 | A6 B22 C31 | A6 B23 C31 | A6 B25 C31 |
| A6 B6 C32 | A6 B7 C32 | A6 B8 C32 | A6 B9 C32 | A6 B10 C32 | A6 B12 C32 | A6 B18 C32 | A6 B22 C32 | A6 B23 C32 | A6 B25 C32 |
| A6 B6 C33 | A6 B7 C33 | A6 B8 C33 | A6 B9 C33 | A6 B10 C33 | A6 B12 C33 | A6 B18 C33 | A6 B22 C33 | A6 B23 C33 | A6 B25 C33 |
| A6 B6 C34 | A6 B7 C34 | A6 B8 C34 | A6 B9 C34 | A6 B10 C34 | A6 B12 C34 | A6 B18 C34 | A6 B22 C34 | A6 B23 C34 | A6 B25 C34 |
| A6 B6 C35 | A6 B7 C35 | A6 B8 C35 | A6 B9 C35 | A6 B10 C35 | A6 B12 C35 | A6 B18 C35 | A6 B22 C35 | A6 B23 C35 | A6 B25 C35 |
| A6 B6 C36 | A6 B7 C36 | A6 B8 C36 | A6 B9 C36 | A6 B10 C36 | A6 B12 C36 | A6 B18 C36 | A6 B22 C36 | A6 B23 C36 | A6 B25 C36 |
| A6 B6 C37 | A6 B7 C37 | A6 B8 C37 | A6 B9 C37 | A6 B10 C37 | A6 B12 C37 | A6 B18 C37 | A6 B22 C37 | A6 B23 C37 | A6 B25 C37 |
| A6 B6 C38 | A6 B7 C38 | A6 B8 C38 | A6 B9 C38 | A6 B10 C38 | A6 B12 C38 | A6 B18 C38 | A6 B22 C38 | A6 B23 C38 | A6 B25 C38 |
| A6 B6 C39 | A6 B7 C39 | A6 B8 C39 | A6 B9 C39 | A6 B10 C39 | A6 B12 C39 | A6 B18 C39 | A6 B22 C39 | A6 B23 C39 | A6 B25 C39 |
| A6 B6 C40 | A6 B7 C40 | A6 B8 C40 | A6 B9 C40 | A6 BIO C40 | A6 B12 C40 | A6 B18 C40 | A6 B22 C40 | A6 B23 C40 | A6 B25 C40 |
| A6 B6 C41 | A6 B7 C41 | A6 B8 C41 | A6 B9 C41 | A6 BIO C41 | A6 B12 C41 | A6 B18 C41 | A6 B22 C41 | A6 B23 C41 | A6 B25 C41 |
| | | | | | | | | | |
| A8 B6 C1 | A8 B7 C1 | A8 B8 C1 | A8 B9 C1 | A8 B10 C1 | A8 B12 C1 | A8 B18 C1 | A8 B22 C1 | A8 B23 C1 | A8 B25 C1 |
| A8 B6 C2 | A8 B7 C2 | A8 B8 C2 | A8 B9 C2 | A8 B10 C2 | A8 B12 C2 | A8 B18 C2 | A8 B22 C2 | A8 B23 C2 | A8 B25 C2 |
| A8 B6 C3 | A8 B7 C3 | A8 B8 C3 | A8 B9 C3 | A8 B10 C3 | A8 B12 C3 | A8 B18 C3 | A8 B22 C3 | A8 B23 C3 | A8 B25 C3 |
| A8 B6 C4 | A8 B7 C4 | A8 B8 C4 | A8 B9 C4 | A8 B10 C4 | A8 B12 C4 | A8 B18 C4 | A8 B22 C4 | A8 B23 C4 | A8 B25 C4 |
| A8 B6 C5 | A8 B7 C5 | A8 B8 C5 | A8 B9 C5 | A8 B10 C5 | A8 B12 C5 | A8 B18 C5 | A8 B22 C5 | A8 B23 C5 | A8 B25 C5 |
| A8 B6 C6 | A8 B7 C6 | A8 B8 C6 | A8 B9 C6 | A8 B10 C6 | A8 B12 C6 | A8 B18 C6 | A8 B22 C6 | A8 B23 C6 | A8 B25 C6 |
| A8 B6 C7 | A8 B7 C7 | A8 B8 C7 | A8 B9 C7 | A8 B10 C7 | A8 B12 C7 | A8 B18 C7 | A8 B22 C7 | A8 B23 C7 | A8 B25 C7 |
| A8 B6 C8 | A8 B7 C8 | A8 B8 C8 | A8 B9 C8 | A8 B10 C8 | A8 B12 C8 | A8 B18 C8 | A8 B22 C8 | A8 B23 C8 | A8 B25 C8 |
| A8 B6 C9 | A8 B7 C9 | A8 B8 C9 | A8 B9 C9 | A8 B10 C9 | A8 B12 C9 | A8 B18 C9 | A8 B22 C9 | A8 B23 C9 | A8 B25 C9 |
| A8 B6 C10 | A8 B7 C10 | A8 B8 C10 | A8 B9 C10 | A8 B10 C10 | A8 B12 C10 | A8 B18 C10 | A8 B22 C10 | A8 B23 C10 | A8 B25 C10 |
| A8 B6 C11 | A8 B7 C11 | A8 B8 C11 | A8 B9 C11 | A8 B10 C11 | A8 B12 C11 | A8 B18 C11 | A8 B22 C11 | A8 B23 C11 | A8 B25 C11 |
| A8 B6 C12 | A8 B7 C12 | A8 B8 C12 | A8 B9 C12 | A8 B10 C12 | A8 B12 C12 | A8 B18 C12 | A8 B22 C12 | A8 B23 C12 | A8 B25 C12 |
| A8 B6 C13 | A8 B7 C13 | A8 B8 C13 | A8 B9 C13 | A8 B10 C13 | A8 B12 C13 | A8 B18 C13 | A8 B22 C13 | A8 B23 C13 | A8 B25 C13 |
| A8 B6 C14 | A8 B7 C14 | A8 B8 C14 | A8 B9 C14 | A8 B10 C14 | A8 B12 C14 | A8 B18 C14 | A8 B22 C14 | A8 B23 C14 | A8 B25 C14 |
| A8 B6 C15 | A8 B7 C15 | A8 B8 C15 | A8 B9 C15 | A8 B10 C15 | A8 B12 C15 | A8 B18 C15 | A8 B22 C15 | A8 B23 C15 | A8 B25 C15 |
| A8 B6 C16 | A8 B7 C16 | A8 B8 C16 | A8 B9 C16 | A8 B10 C16 | A8 B12 C16 | A8 B18 C16 | A8 B22 C16 | A8 B23 C16 | A8 B25 C16 |
| A8 B6 C17 | A8 B7 C17 | A8 B8 C17 | A8 B9 C17 | A8 B10 C17 | A8 B12 C17 | A8 B18 C17 | A8 B22 C17 | A8 B23 C17 | A8 B25 C17 |
| A8 B6 C18 | A8 B7 C18 | A8 B8 C18 | A8 B9 C18 | A8 B10 C18 | A8 B12 C18 | A8 B18 C18 | A8 B22 C18 | A8 B23 C18 | A8 B25 C18 |
| A8 B6 C19 | A8 B7 C19 | A8 B8 C19 | A8 B9 C19 | A8 B10 C19 | A8 B12 C19 | A8 B18 C19 | A8 B22 C19 | A8 B23 C19 | A8 B25 C19 |
| A8 B6 C20 | A8 B7 C20 | A8 B8 C20 | A8 B9 C20 | A8 B10 C20 | A8 B12 C20 | A8 B18 C20 | A8 B22 C20 | A8 B23 C20 | A8 B25 C20 |
| A8 B6 C21 | A8 B7 C21 | A8 B8 C21 | A8 B9 C21 | A8 B10 C21 | A8 B12 C21 | A8 B18 C21 | A8 B22 C21 | A8 B23 C21 | A8 B25 C21 |
| A8 B6 C22 | A8 B7 C22 | A8 B8 C22 | A8 B9 C22 | A8 B10 C22 | A8 B12 C22 | A8 B18 C22 | A8 B22 C22 | A8 B23 C22 | A8 B25 C22 |
| A8 B6 C23 | A8 B7 C23 | A8 B8 C23 | A8 B9 C23 | A8 B10 C23 | A8 B12 C23 | A8 B18 C23 | A8 B22 C23 | A8 B23 C23 | A8 B25 C23 |
| A8 B6 C24 | A8 B7 C24 | A8 B8 C24 | A8 B9 C24 | A8 B10 C24 | A8 B12 C24 | A8 B18 C24 | A8 B22 C24 | A8 B23 C24 | A8 B25 C24 |
| A8 B6 C25 | A8 B7 C25 | A8 B8 C25 | A8 B9 C25 | A8 B10 C25 | A8 B12 C25 | A8 B18 C25 | A8 B22 C25 | A8 B23 C25 | A8 B25 C25 |
| A8 B6 C26 | A8 B7 C26 | A8 B8 C26 | A8 B9 C26 | A8 B10 C26 | A8 B12 C26 | A8 B18 C26 | A8 B22 C26 | A8 B23 C26 | A8 B25 C26 |
| A8 B6 C27 | A8 B7 C27 | A8 B8 C27 | A8 B9 C27 | A8 B10 C27 | A8 B12 C27 | A8 B18 C27 | A8 B22 C27 | A8 B23 C27 | A8 B25 C27 |
| A8 B6 C28 | A8 B7 C28 | A8 B8 C28 | A8 B9 C28 | A8 B10 C28 | A8 B12 C28 | A8 B18 C28 | A8 B22 C28 | A8 B23 C28 | A8 B25 C28 |
| A8 B6 C29 | A8 B7 C29 | A8 B8 C29 | A8 B9 C29 | A8 B10 C29 | A8 B12 C29 | A8 B18 C29 | A8 B22 C29 | A8 B23 C29 | A8 B25 C29 |
| A8 B6 C30 | A8 B7 C30 | A8 B8 C30 | A8 B9 C30 | A8 B10 C30 | A8 B12 C30 | A8 B18 C30 | A8 B22 C30 | A8 B23 C30 | A8 B25 C30 |
| A8 B6 C31 | A8 B7 C31 | A8 B8 C31 | A8 B9 C31 | A8 B10 C31 | A8 B12 C31 | A8 B18 C31 | A8 B22 C31 | A8 B23 C31 | A8 B25 C31 |
| A8 B6 C32 | A8 B7 C32 | A8 B8 C32 | A8 B9 C32 | A8 B10 C32 | A8 B12 C32 | A8 B18 C32 | A8 B22 C32 | A8 B23 C32 | A8 B25 C32 |
| A8 B6 C33 | A8 B7 C33 | A8 B8 C33 | A8 B9 C33 | A8 B10 C33 | A8 B12 C33 | A8 B18 C33 | A8 B22 C33 | A8 B23 C33 | A8 B25 C33 |
| A8 B6 C34 | A8 B7 C34 | A8 B8 C34 | A8 B9 C34 | A8 B10 C34 | A8 B12 C34 | A8 B18 C34 | A8 B22 C34 | A8 B23 C34 | A8 B25 C34 |
| A8 B6 C35 | A8 B7 C35 | A8 B8 C35 | A8 B9 C35 | A8 B10 C35 | A8 B12 C35 | A8 B18 C35 | A8 B22 C35 | A8 B23 C35 | A8 B25 C35 |
| A8 B6 C36 | A8 B7 C36 | A8 B8 C36 | A8 B9 C36 | A8 B10 C36 | A8 B12 C36 | A8 B18 C36 | A8 B22 C36 | A8 B23 C36 | A8 B25 C36 |
| A8 B6 C37 | A8 B7 C37 | A8 B8 C37 | A8 B9 C37 | A8 B10 C37 | A8 B12 C37 | A8 B18 C37 | A8 B22 C37 | A8 B23 C37 | A8 B25 C37 |
| A8 B6 C38 | A8 B7 C38 | A8 B8 C38 | A8 B9 C38 | A8 B10 C38 | A8 B12 C38 | A8 B18 C38 | A8 B22 C38 | A8 B23 C38 | A8 B25 C38 |
| A8 B6 C39 | A8 B7 C39 | A8 B8 C39 | A8 B9 C39 | A8 B10 C39 | A8 B12 C39 | A8 B18 C39 | A8 B22 C39 | A8 B23 C39 | A8 B25 C39 |
| A8 B6 C40 | A8 B7 C40 | A8 B8 C40 | A8 B9 C40 | A8 B10 C40 | A8 B12 C40 | A8 B18 C40 | A8 B22 C40 | A8 B23 C40 | A8 B25 C40 |
| A8 B6 C41 | A8 B7 C41 | A8 B8 C41 | A8 B9 C41 | A8 B10 C41 | A8 B12 C41 | A8 B18 C41 | A8 B22 C41 | A8 B23 C41 | A8 B25 C41 |
| | | | | | | | | | |
| A17 B6 C1 | A17 B7 C1 | A17 B8 C1 | A17 B9 C1 | A17 B10 C1 | A17 B12 C1 | A17 B18 C1 | A17 B22 C1 | A17 B23 C1 | A17 B25 C1 |
| A17 B6 C2 | A17 B7 C2 | A17 B8 C2 | A17 B9 C2 | A17 B10 C2 | A17 B12 C2 | A17 B18 C2 | A17 B22 C2 | A17 B23 C2 | A17 B25 C2 |
| A17 B6 C3 | A17 B7 C3 | A17 B8 C3 | A17 B9 C3 | A17 B10 C3 | A17 B12 C3 | A17 B18 C3 | A17 B22 C3 | A17 B23 C3 | A17 B25 C3 |
| A17 B6 C4 | A17 B7 C4 | A17 B8 C4 | A17 B9 C4 | A17 B10 C4 | A17 B12 C4 | A17 B18 C4 | A17 B22 C4 | A17 B23 C4 | A17 B25 C4 |
| A17 B6 C5 | A17 B7 C5 | A17 B8 C5 | A17 B9 C5 | A17 B10 C5 | A17 B12 C5 | A17 B18 C5 | A17 B22 C5 | A17 B23 C5 | A17 B25 C5 |
| A17 B6 C6 | A17 B7 C6 | A17 B8 C6 | A17 B9 C6 | A17 B10 C6 | A17 B12 C6 | A17 B18 C6 | A17 B22 C6 | A17 B23 C6 | A17 B25 C6 |
| A17 B6 C7 | A17 B7 C7 | A17 B8 C7 | A17 B9 C7 | A17 B10 C7 | A17 B12 C7 | A17 B18 C7 | A17 B22 C7 | A17 B23 C7 | A17 B2S C7 |
| A17 B6 C8 | A17 B7 C8 | A17 B8 C8 | A17 B9 C8 | A17 B10 C8 | A17 B12 C8 | A17 B18 C8 | A17 B22 C8 | A17 B23 C8 | A17 B25 C8 |
| A17 B6 C9 | A17 B7 C9 | A17 B8 C9 | A17 B9 C9 | A17 B10 C9 | A17 B12 C9 | A17 B18 C9 | A17 B22 C9 | A17 B23 C9 | A17 B25 C9 |
| A17 B6 C10 | A17 B7 C10 | A17 B8 C10 | A17 B9 C10 | A17 B10 C10 | A17 B12 C10 | A17 B18 C10 | A17 B22 C10 | A17 B23 C10 | A17 825 C10 |
| A17 B6 C11 | A17 B7 C11 | A17 B8 C11 | A17 B9 C11 | A17 B10 C11 | A17 B12 C11 | A17 B18 C11 | A17 B22 C11 | A17 B23 C11 | A17 B25 C11 |
| A17 B6 C12 | A17 B7 C12 | A17 B8 C12 | A17 B9 C12 | A17 B10 C12 | A17 B12 C12 | A17 B18 C12 | A17 B22 C12 | A17 B23 C12 | A17 B25 C12 |
| A17 B6 C13 | A17 B7 C13 | A17 B8 C13 | A17 B9 C13 | A17 B10 C13 | A17 B12 C13 | A17 B18 C13 | A17 B22 C13 | A17 B23 C13 | A17 B25 C13 |
| A17 B6 C14 | A17 B7 C14 | A17 B8 C14 | A17 B9 C14 | A17 B10 C14 | A17 B12 C14 | A17 B18 C14 | A17 B22 C14 | A17 B23 C14 | A17 B25 C14 |
| A17 B6 C15 | A17 B7 C15 | A17 B8 C15 | A17 B9 C1S | A17 B10 C15 | A17 B12 C1S | A17 B18 C15 | A17 B22 C15 | A17 B23 05 | A17 B25 C15 |
| A17 B6 C16 | A17 B7 C16 | A17 B8 C16 | A17 B9 C16 | A17 B10 C16 | A17 B12 C16 | A17 B18 C16 | A17 B22 C16 | A17 B23 C16 | A17 B25 C16 |
| A17 B6 C17 | A17 B7 C17 | A17 B8 C17 | A17 B9 C17 | A17 B10 C17 | A17 B12 C17 | A17 B18 C17 | A17 B22 C17 | A17 B23 C17 | A17 B25 C17 |
| A17 B6 C18 | A17 B7 C18 | A17 B8 C18 | A17 B9 C18 | A17 B10 C18 | A17 B12 C18 | A17 B18 C18 | A17 B22 C18 | A17 B23 C18 | A17 B25 C18 |
| A17 B6 C19 | A17 B7 C19 | A17 B8 C19 | A17 B9 C19 | A17 B10 C19 | A17 B12 C19 | A17 B18 C19 | A17 B22 C19 | A17 B23 C19 | A17 B25 C19 |
| A17 B6 C20 | A17 B7 C20 | A17 B8 C20 | A17 B9 C20 | A17 B10 C20 | A17 B12 C20 | A17 B18 C20 | A17 B22 C20 | A17 B23 C20 | A17 B25 C20 |
| A17 B6 C21 | A17 B7 C21 | A17 B8 C21 | A17 B9 C21 | A17 B10 C21 | A17 B12 C21 | A17 B18 C21 | A17 B22 C21 | A17 B23 C21 | A17 B25 C21 |
| A17 B6 C22 | A17 B7 C22 | A17 B8 C22 | A17 B9 C22 | A17 B10 C22 | A17 B12 C22 | A17 B18 C22 | A17 B22 C22 | A17 B23 C22 | A17 B25 C22 |
| A17 B6 C23 | A17 B7 C23 | A17 B8 C23 | A17 B9 C23 | A17 B10 C23 | A17 B12 C23 | A17 B18 C23 | A17 B22 C23 | A17 B23 C23 | A17 B25 C23 |
| A1 B6 C24 | A17 B7 C24 | A17 B8 C24 | A17 B9 C24 | A17 B10 C24 | A17 B12 C24 | A17 B18 C24 | A17 B22 C24 | A17 B23 C24 | A17 B25 C24 |
| A17 B6 C25 | A17 B7 C25 | A17 B8 C25 | A17 B9 C25 | A17 B10 C25 | A17 B12 C25 | A17 B18 C25 | A17 B22 C25 | A17 B23 C25 | A17 B25 C25 |
| A17 B6 C26 | A17 B7 C26 | A17 B8 C26 | A17 B9 C26 | A17 B10 C26 | A17 B12 C26 | A17 B18 C26 | A17 B22 C26 | A17 B23 C26 | A17 B25 C26 |
| A17 B6 C27 | A17 B7 C27 | A17 B8 C27 | A17 B9 C27 | A17 B10 C27 | A17 B12 C27 | A17 B18 C27 | A17 B22 C27 | A17 B23 C27 | A17 B25 C27 |
| A17 B6 C28 | A17 B7 C28 | A17 B8 C28 | A17 B9 C28 | A17 B10 C28 | A17 B12 C28 | A17 B18 C28 | A17 B22 C28 | A17 B23 C2B | A17 B25 C28 |
| A17 B6 C29 | A17 B7 C29 | A17 B8 C29 | A17 B9 C29 | A17 B10 C29 | A17 B12 C29 | A17 B18 C29 | A17 B22 C29 | A17 B23 C29 | A17 B25 C29 |
| A17 B6 C30 | A17 B7 C30 | A17 B8 C30 | A17 B9 C30 | A17 B10 C30 | A17 B12 C30 | A17 B18 C30 | A17 B22 C30 | A17 B23 C0D | A17 B25 C30 |
| A17 B6 C31 | A17 B7 C31 | A17 B8 C31 | A17 B9 C31 | A17 B10 C31 | A17 B12 C31 | A17 B18 C31 | A17 B22 C31 | A17 B23 C31 | A17 B25 C31 |
| A17 B6 C32 | A17 87 C32 | A17 B8 C32 | A17 B9 C32 | A17 B10 C32 | A17 B12 C32 | A17 B18 C32 | A17 B22 C32 | A17 B23 C32 | A17 B25 C32 |
| A17 B6 C33 | A17 B7 C33 | A17 B8 C33 | A17 B9 C33 | A17 B10 C33 | A17 B12 C33 | A17 B18 C33 | A17 B22 C33 | A17 B23 C33 | A17 B25 C33 |
| A17 B6 C34 | A17 B7 C34 | A17 B8 C34 | A17 B9 C34 | A17 B10 C34 | A17 B12 C34 | A17 B18 C34 | A17 B22 C34 | A17 B23 C34 | A17 B25 C34 |
| A17 B6 C3S | A17 B7 C3S | A17 B8 C35 | A17 B9 C35 | A17 B10 C35 | A17 B12 C35 | A17 B18 C35 | A17 B22 C35 | A17 B23 C35 | A17 B25 C35 |
| A17 B6 C36 | A17 B7 C36 | A17 B8 C36 | A17 B9 C36 | A17 B10 C36 | A17 B12 C36 | A17 B18 C36 | A17 B22 C36 | A17 B23 C36 | A17 B25 C36 |
| A17 B6 C37 | A17 B7 C37 | A17 B8 C37 | A17 B9 C37 | A17 B10 C37 | A17 B12 C37 | A17 B18 C37 | A17 B22 C37 | A17 B23 C37 | A17 B25 C37 |
| A17 B6 C38 | A17 B7 C38 | A17 B8 C38 | A17 B9 C38 | A17 B10 C38 | A17 B12 C38 | A17 B18 C38 | A17 B22 C38 | A17 B23 C38 | A17 B25 C38 |
| A17 B6 C39 | A17 B7 C39 | A17 B8 C39 | A17 B9 C39 | A17 B10 C39 | A17 B12 C39 | A17 B18 C39 | A17 B22 C39 | A17 B23 C39 | A17 B25 C39 |
| A17 B6 C40 | A17 B7 C40 | A17 B8 C40 | A17 B9 C40 | A17 B10 C40 | A17 B12 C40 | A17 B18 C40 | A17 B22 C40 | A17 B23 C40 | A17 B25 C40 |
| A17 B6 C41 | A17 B7 C41 | A17 B8 C41 | A17 B9 C41 | A17 B10 C41 | A17 B12 C41 | A17 B18 C41 | A17 B22 C41 | A17 B23 C41 | A17 B25 C41 |
| | | | | | | | | | |
| A26 B6 C1 | A26 B7 C1 | A26 B8 C1 | A26 B9 C1 | A26 B10 C1 | A26 B12 C1 | A26 B18 C1 | A26 B22 C1 | A26 B23 C1 | A26 B25 C1 |
| A26 B6 C2 | A26 B7 C2 | A26 B8 C2 | A26 B9 C2 | A26 B10 C2 | A26 B12 C2 | A26 B18 C2 | A26 B22 C2 | A26 B23 C2 | A26 B25 C2 |
| A26 B6 C3 | A26 B7 C3 | A26 B8 C3 | A26 B9 C3 | A26 B10 C3 | A26 B12 C3 | A26 B18 C3 | A26 B22 C3 | A26 B23 C3 | A26 B25 C3 |
| A26 B6 C4 | A26 B7 C4 | A26 B8 C4 | A26 B9 C4 | A26 B10 C4 | A26 B12 C4 | A26 B18 C4 | A26 B22 C4 | A26 B23 C4 | A26 B25 C4 |
| A26 B6 C5 | A26 B7 C5 | A26 B8 C5 | A26 B9 C5 | A26 B10 C5 | A26 B12 C5 | A26 B18 C5 | A26 B22 C5 | A26 B23 C5 | A26 B25 C5 |
| A26 B6 C6 | A26 B7 C6 | A26 B8 C6 | A26 B9 C6 | A26 B10 C6 | A26 B12 C6 | A26 B18 C6 | A26 B22 C6 | A26 B23 C6 | A26 B25 C6 |
| A26 B6 C7 | A26 B7 C7 | A26 B8 C7 | A26 B9 C7 | A26 B10 C7 | A26 B12 C7 | A26 B18 C7 | A26 B22 C7 | A26 B23 C7 | A26 B25 C7 |
| A26 B6 C8 | A26 B7 C8 | A26 B8 C8 | A26 B9 C8 | A26 B10 C8 | A26 B12 C8 | A26 B18 C8 | A26 B22 C8 | A26 B23 C8 | A26 B25 C8 |
| A26 B6 C9 | A26 B7 C9 | A26 B8 C9 | A26 B9 C9 | A26 B10 C9 | A26 B12 C9 | A26 B18 C9 | A26 B22 C9 | A26 B23 C9 | A26 B25 C9 |
| A26 B6 C10 | A26 B7 C10 | A26 B8 C10 | A26 B9 C10 | A26 B10 C10 | A26 B12 C10 | A26 B18 C10 | A26 B22 C10 | A26 B23 C10 | A26 B25 C10 |
| A26 B6 C11 | A26 B7 C11 | A26 B8 C11 | A26 B9 C11 | A26 B10 C11 | A26 B12 C11 | A26 B18 C11 | A26 B22 C11 | A26 B23 C11 | A26 B25 C11 |
| A26 B6 C12 | A26 B7 C12 | A26 B8 C12 | A26 B9 C12 | A26 B10 C12 | A26 B12 C12 | A26 B18 C12 | A26 B22 C12 | A26 B23 C12 | A26 B25 C12 |
| A26 B6 C13 | A26 B7 C13 | A26 B8 C13 | A26 B9 C13 | A26 B10 C13 | A26 B12 C13 | A26 B18 C13 | A26 B22 C13 | A26 B23 C13 | A26 B25 C13 |
| A26 B6 C14 | A26 B7 C14 | A26 B8 C14 | A26 B9 C14 | A26 B10 C14 | A26 B12 C14 | A26 B18 C14 | A26 B22 C14 | A26 B23 C14 | A26 B25 C14 |
| A26 B6 C15 | A26 B7 C15 | A26 B8 C15 | A26 B9 C15 | A26 B10 C15 | A26 B12 C15 | A26 B18 C15 | A26 B22 C15 | A26 B23 C15 | A26 B25 C15 |
| A26 B6 C16 | A26 B7 C16 | A26 B8 C16 | A26 B9 C16 | A26 B10 C16 | A26 B12 C16 | A26 B18 C16 | A26 B22 C16 | A26 B23 C16 | A26 B25 C16 |
| A26 B6 C17 | A26 B7 C17 | A26 B8 C17 | A26 B9 C17 | A26 B10 C17 | A26 B12 C17 | A26 B18 C17 | A26 B22 C17 | A26 B23 C17 | A26 B25 C17 |
| A26 B6 C18 | A26 B7 C18 | A26 B8 C18 | A26 B9 C18 | A26 B10 C18 | A26 B12 C18 | A26 B18 C18 | A26 B22 C18 | A26 B23 C18 | A26 B25 C18 |
| A26 B6 C19 | A26 B7 C19 | A26 B8 C19 | A26 B9 C19 | A26 B10 C19 | A26 B12 C19 | A26 B18 C19 | A26 B22 C19 | A26 B23 C19 | A26 B25 C19 |
| A26 B6 C20 | A26 B7 C20 | A26 B8 C20 | A26 B9 C20 | A26 B10 C20 | A26 B12 C20 | A26 B18 C20 | A26 B22 C20 | A26 B23 C20 | A26 B25 C20 |
| A26 B6 C21 | A26 B7 C21 | A26 B8 C21 | A26 B9 C21 | A26 B10 C21 | A26 B12 C21 | A26 B18 C21 | A26 B22 C21 | A26 B23 C21 | A26 B25 C21 |
| A26 B6 C22 | A26 B7 C22 | A26 B8 C22 | A26 B9 C22 | A26 B10 C22 | A26 B12 C22 | A26 B18 C22 | A26 B22 C22 | A26 B23 C22 | A26 B25 C22 |
| A26 B6 C23 | A26 B7 C23 | A26 B8 C23 | A26 B9 C23 | A26 B10 C23 | A26 B12 C23 | A26 B18 C23 | A26 B22 C23 | A26 B23 C23 | A26 B25 C23 |
| A26 B6 C24 | A26 B7 C24 | A26 B8 C24 | A26 B9 C24 | A26 B10 C24 | A26 B12 C24 | A26 B18 C24 | A26 B22 C24 | A26 B23 C14 | A26 B25 C24 |
| A26 B6 C25 | A26 B7 C25 | A26 B8 C25 | A26 B9 C25 | A26 B10 C25 | A26 B12 C25 | A26 B18 C25 | A26 B22 C25 | A26 B23 C25 | A26 B25 C25 |
| A26 B6 C26 | A26 B7 C26 | A26 B8 C26 | A26 B9 C26 | A26 B10 C26 | A26 B12 C26 | A26 B18 C26 | A26 B22 C26 | A26 B23 C26 | A26 B25 C26 |
| A26 B6 C27 | A26 B7 C27 | A26 B8 C27 | A26 B9 C27 | A26 B10 C27 | A26 B12 C27 | A26 B18 C27 | A26 B22 C27 | A26 B23 C27 | A26 B25 C27 |
| A26 B6 C28 | A26 B7 C28 | A26 B8 C28 | A26 B9 C28 | A26 B10 C28 | A26 B12 C28 | A26 B18 C28 | A26 B22 C28 | A26 B23 C28 | A26 B25 C28 |
| A26 B6 C29 | A26 B7 C29 | A26 B8 C29 | A26 B9 C29 | A26 B10 C29 | A26 B12 C29 | A26 B18 C29 | A26 B22 C29 | A26 B23 C29 | A26 B25 C29 |
| A26 B6 C30 | A26 B7 C30 | A26 B8 C30 | A26 B9 C30 | A26 B10 C30 | A26 B12 C30 | A26 B18 C30 | A26 B22 C30 | A26 B23 C30 | A26 B25 C30 |
| A26 B6 C31 | A26 B7 C31 | A26 B8 C31 | A26 B9 C31 | A26 B10 C31 | A26 B12 C31 | A26 B18 C31 | A26 B22 C31 | A26 B23 C31 | A26 B25 C31 |
| A26 B6 C32 | A26 B7 C32 | A26 B8 C32 | A26 B9 C32 | A26 B10 C32 | A26 B12 C32 | A26 B18 C32 | A26 B22 C32 | A26 B23 C32 | A26 B25 C32 |
| A26 B6 C33 | A26 B7 C33 | A26 B8 C33 | A26 B9 C33 | A26 B10 C33 | A26 B12 C33 | A26 B18 C33 | A26 B22 C33 | A26 B23 C33 | A26 B25 C33 |
| A26 B6 C34 | A26 B7 C34 | A26 B8 C34 | A26 B9 C34 | A26 B10 C34 | A26 B12 C34 | A26 B18 C34 | A26 B22 C34 | A26 B23 C34 | A26 B25 C34 |
| A26 B6 C35 | A26 B7 C35 | A26 B8 C35 | A26 B9 C35 | A26 B10 C35 | A26 B12 C35 | A26 B18 C35 | A26 B22 C35 | A26 B23 C35 | A26 B25 C35 |
| A26 B6 C36 | A26 B7 C36 | A26 B8 C36 | A26 B9 C36 | A26 B10 C36 | A26 B12 C36 | A26 B18 C36 | A26 B22 C36 | A26 B23 C36 | A26 B25 C36 |
| A26 B6 C37 | A26 B7 C37 | A26 B8 C37 | A26 B9 C37 | A26 B10 C37 | A26 B12 C37 | A26 B18 C37 | A26 B22 C37 | A26 B23 C37 | A26 B25 C37 |
| A26 B6 C38 | A26 B7 C38 | A26 B8 C38 | A26 B9 C38 | A26 B10 C38 | A26 B12 C38 | A26 B18 C38 | A26 B22 C38 | A26 B23 C38 | A26 B25 C38 |
| A26 B6 C39 | A26 B7 C39 | A26 B8 C39 | A26 B9 C39 | A26 B10 C39 | A26 B12 C39 | A26 B18 C39 | A26 B22 C39 | A26 B23 C39 | A26 B25 C39 |
| A26 B6 C40 | A26 B7 C40 | A26 B8 C40 | A26 B9 C40 | A26 B10 C40 | A26 B12 C40 | A26 B18 C40 | A26 B22 C40 | A26 B23 C40 | A26 B25 C40 |
| A26 B6 C41 | A26 B7 C41 | A26 B8 C41 | A26 B9 C41 | A26 B10 C41 | A26 B12 C41 | A26 B18 C41 | A26 B22 C41 | A26 B23 C41 | A26 B25 C41 |
| | | | | | | | | | |
| A30 B6 C1 | A30 B7 C1 | A30 B8 C1 | A30 B9 C1 | A30 B10 C1 | A30 B12 C1 | A30 B18 C1 | A30 B22 C1 | A30 B23 C1 | A30 B25 C1 |
| A30 B6 C2 | A30 B7 C2 | A30 B8 C2 | A30 B9 C2 | A30 B10 C2 | A30 B12 C2 | A30 B18 C2 | A30 B22 C2 | A30 B23 C2 | A30 B25 C2 |
| A30 B6 C3 | A30 B7 C3 | A30 B8 C3 | A30 B9 C3 | A30 B10 C3 | A30 B12 C3 | A30 B18 C3 | A30 B22 C3 | A30 B23 C3 | A30 B25 C3 |
| A30 B6 C4 | A30 B7 C4 | A30 B8 C4 | A30 B9 C4 | A30 B10 C4 | A30 B12 C4 | A30 B18 C4 | A30 B22 C4 | A30 B23 C4 | A30 B25 C4 |
| A30 B6 C5 | A30 B7 C5 | A30 B8 C5 | A30 B9 C5 | A30 B10 C5 | A30 B12 C5 | A30 B18 C5 | A30 B22 C5 | A30 B23 C5 | A30 B25 C5 |
| A30 B6 C6 | A30 B7 C6 | A30 B8 C6 | A30 B9 C6 | A30 B10 C6 | A30 B12 C6 | A30 B18 C6 | A30 B22 C6 | A30 B23 C6 | A30 B25 C6 |
| A30 B6 C7 | A30 B7 C7 | A30 B8 C7 | A30 B9 C7 | A30 B10 C7 | A30 B12 C7 | A30 B18 C7 | A30 B22 C7 | A30 B23 C7 | A30 B25 C7 |
| A30 B6 C8 | A30 B7 C8 | A30 B8 C8 | A30 B9 C8 | A30 B10 C8 | A30 B12 C8 | A30 B18 C8 | A30 B22 C8 | A30 B23 C8 | A30 B25 C8 |
| A30 B6 C9 | A30 B7 C9 | A30 B8 C9 | A30 B9 C9 | A30 B10 C9 | A30 B12 C9 | A30 B18 C9 | A30 B22 C9 | A30 B23 C9 | A30 B25 C9 |
| A30 B6 C10 | A30 B7 C10 | A30 B8 C10 | A30 B9 C10 | A30 B10 C10 | A30 B12 C10 | A30 B18 C10 | A30 B22 C10 | A30 B23 C10 | A30 B25 C10 |
| A30 B6 C11 | A30 B7 C11 | A30 B8 C11 | A30 B9 C11 | A30 B10 C11 | A30 B12 C11 | A30 B18 C11 | A30 B22 C11 | A30 B23 C11 | A30 B25 C11 |
| A30 B6 C12 | A30 B7 C12 | A30 B8 C12 | A30 B9 C12 | A30 B10 C12 | A30 B12 C12 | A30 B18 C12 | A30 B22 C12 | A30 B23 C12 | A30 B25 C12 |
| A30 B6 C13 | A30 B7 C13 | A30 B8 C13 | A30 B9 C13 | A30 B10 C13 | A30 B12 C13 | A30 B18 C13 | A30 B22 C13 | A30 B23 C13 | A30 B25 C13 |
| A30 B6 C14 | A30 B7 C14 | A30 B8 C14 | A30 B9 C14 | A30 B10 C14 | A30 B12 C14 | A30 B18 C14 | A30 B22 C14 | A30 B23 C14 | A30 B25 C14 |
| A30 B6 C15 | A30 B7 C15 | A30 B8 C15 | A30 B9 C15 | A30 B10 C15 | A30 B12 C15 | A30 B18 C15 | A30 B22 C15 | A30 B23 C15 | A30 B25 C15 |
| A30 B6 C16 | A30 B7 C16 | A30 B8 C16 | A30 B9 C16 | A30 B10 C16 | A30 B12 C16 | A30 B18 C16 | A30 B22 C16 | A30 B23 C16 | A30 B25 C16 |
| A30 B6 C17 | A30 B7 C17 | A30 B8 C17 | A30 B9 C17 | A30 B10 C17 | A30 B12 C17 | A30 B18 C17 | A30 B22 C17 | A30 B23 C17 | A30 B25 C17 |
| A30 B6 C18 | A30 B7 C18 | A30 B8 C18 | A30 B9 C18 | A30 B10 C18 | A30 B12 C18 | A30 B18 C18 | A30 B22 C18 | A30 B23 C18 | A30 B25 C18 |
| A30 B6 C19 | A30 B7 C19 | A30 B8 C19 | A30 B9 C19 | A30 B10 C19 | A30 B12 C19 | A30 B18 C19 | A30 B22 C19 | A30 B23 C19 | A30 B25 C19 |
| A30 B6 C20 | A30 B7 C20 | A30 B8 C20 | A30 B9 C20 | A30 B10 C20 | A30 B12 C20 | A30 B18 C20 | A30 B22 C20 | A30 B23 C20 | A30 B25 C20 |
| A30 B6 C21 | A30 B7 C21 | A30 B8 C21 | A30 B9 C21 | A30 B10 C21 | A30 B12 C21 | A30 B18 C21 | A30 B22 C21 | A30 B23 C21 | A30 B25 C21 |
| A30 B6 C22 | A30 B7 C22 | A30 B8 C22 | A30 B9 C22 | A30 B10 C22 | A30 B12 C22 | A30 B18 C22 | A30 B22 C22 | A30 B23 C22 | A30 B25 C22 |
| A30 B6 C23 | A30 B7 C23 | A30 B8 C23 | A30 B9 C23 | A30 B10 C23 | A30 B12 C23 | A30 B18 C23 | A30 B22 C23 | A30 B23 C23 | A30 B25 C23 |
| A30 B6 C24 | A30 B7 C24 | A30 B8 C24 | A30 B9 C24 | A30 B10 C24 | A30 B12 C24 | A30 B18 C24 | A30 B22 C24 | A30 B23 C24 | A30 B25 C24 |
| A30 B6 C25 | A30 B7 C25 | A30 B8 C25 | A30 B9 C25 | A30 B10 C25 | A30 B12 C25 | A30 B18 C25 | A30 B22 C25 | A30 B23 C25 | A30 B25 C25 |
| A30 B6 C26 | A30 B7 C26 | A30 B8 C26 | A30 B9 C26 | A30 B10 C26 | A30 B12 C26 | A30 B18 C26 | A30 B22 C26 | A30 B23 C26 | A30 B25 C26 |
| A30 B6 C27 | A30 B7 C27 | A30 B8 C27 | A30 B9 C27 | A30 B10 C27 | A30 B12 C27 | A30 B18 C27 | A30 B22 C27 | A30 B23 C17 | A30 B25 C27 |
| A30 B6 C28 | A30 B7 C28 | A30 B8 C28 | A30 B9 C28 | A30 B10 C28 | A30 B12 C28 | A30 B18 C28 | A30 B22 C28 | A30 B23 C28 | A30 B25 C28 |
| A30 B6 C29 | A30 B7 C29 | A30 B8 C29 | A30 B9 C29 | A30 B10 C29 | A30 B12 C29 | A30 B18 C29 | A30 B22 C29 | A30 B23 C29 | A30 B25 C29 |
| A30 B6 C30 | A30 B7 C30 | A30 B8 C30 | A30 B9 C30 | A30 B10 C30 | A30 B12 C30 | A30 B18 C30 | A30 B22 C30 | A30 B23 C30 | A30 B25 C30 |
| A30 B6 C31 | A30 B7 C31 | A30 B8 C31 | A30 B9 C31 | A30 B10 C31 | A30 B12 C31 | A30 B18 C31 | A30 B22 C31 | A30 B23 C31 | A30 B25 C31 |
| A30 B6 C32 | A30 B7 C32 | A30 B8 C32 | A30 B9 C32 | A30 B10 C32 | A30 B12 C32 | A30 B18 C32 | A30 B22 C32 | A30 B23 C32 | A30 B25 C32 |
| A30 B6 C33 | A30 B7 C33 | A30 B8 C33 | A30 B9 C33 | A30 B10 C33 | A30 B12 C33 | A30 B18 C33 | A30 B22 C33 | A30 B23 C33 | A30 B25 C33 |
| A30 B6 C34 | A30 B7 C34 | A30 B8 C34 | A30 B9 C34 | A30 B10 C34 | A30 B12 C34 | A30 B18 C34 | A30 B22 C34 | A30 B23 C34 | A30 B25 C34 |
| A30 B6 C35 | A30 B7 C35 | A30 B8 C35 | A30 B9 C35 | A30 B10 C35 | A30 B12 C35 | A30 B18 C35 | A30 B22 C35 | A30 B23 C35 | A30 B25 C35 |
| A30 B6 C36 | A30 B7 C36 | A30 B8 C36 | A30 B9 C36 | A30 B10 C36 | A30 B12 C36 | A30 B18 C36 | A30 B22 C36 | A30 B23 C36 | A30 B25 C36 |
| A30 B6 C37 | A30 B7 C37 | A30 B8 C37 | A30 B9 C37 | A30 B10 C37 | A30 B12 C37 | A30 B18 C37 | A30 B22 C37 | A30 B23 C37 | A30 B25 C37 |
| A30 B6 C38 | A30 B7 C38 | A30 B8 C38 | A30 B9 C38 | A30 B10 C38 | A30 B12 C38 | A30 B18 C38 | A30 B22 C38 | A30 B23 C38 | A30 B25 C38 |
| A30 B6 C39 | A30 B7 C39 | A30 B8 C39 | A30 B9 C39 | A30 B10 C39 | A30 B12 C39 | A30 B18 C39 | A30 B22 C39 | A30 B23 C39 | A30 B25 C39 |
| A30 B6 C40 | A30 B7 C40 | A30 B8 C40 | A30 B9 C40 | A30 B10 C40 | A30 B12 C40 | A30 B18 C40 | A30 B22 C40 | A30 B23 C40 | A30 B25 C40 |
| A30 B6 C41 | A30 B7 C41 | A30 B8 C41 | A30 B9 C41 | A30 B10 C41 | A30 B12 C41 | A30 B18 C41 | A30 B22 C41 | A30 B23 C41 | A30 B25 C41 |
| | | | | | | | | | |
| A31 B6 C1 | A31 B7 C1 | A31 B8 C1 | A31 B9 C1 | A31 B10 C1 | A31 B12 C1 | A31 B18 C1 | A31 B22 C1 | A31 B23 C1 | A31 B25 C1 |
| A31 B6 C2 | A31 B7 C2 | A31 B8 C2 | A31 B9 C2 | A31 B10 C2 | A31 B12 C2 | A31 B18 C2 | A31 B22 C2 | A31 B23 C2 | A31 B25 C2 |
| A31 B6 C3 | A31 B7 C3 | A31 B8 C3 | A31 B9 C3 | A31 B10 C3 | A31 B12 C3 | A31 B18 C3 | A31 B22 C3 | A31 B23 C3 | A31 B25 C3 |
| A31 B6 C4 | A31 B7 C4 | A31 B8 C4 | A31 B9 C4 | A31 B10 C4 | A31 B12 C4 | A31 B18 C4 | A31 B22 C4 | A31 B23 C4 | A31 B25 C4 |
| A31 B6 C5 | A31 B7 C5 | A31 B8 C5 | A31 B9 C5 | A31 B10 C5 | A31 B12 C5 | A31 B18 C5 | A31 B22 C5 | A31 B23 C5 | A31 B25 C5 |
| A31 B6 C6 | A31 B7 C6 | A31 B8 C6 | A31 B9 C6 | A31 B10 C6 | A31 B12 C6 | A31 B18 C6 | A31 B22 C6 | A31 B23 C6 | A31 B25 C6 |
| A31 B6 C7 | A31 B7 C7 | A31 B8 C7 | A31 B9 C7 | A31 B10 C7 | A31 B12 C7 | A31 B18 C7 | A31 B22 C7 | A31 B23 C7 | A31 B25 C7 |
| A31 B6 C8 | A31 B7 C8 | A31 B8 C8 | A31 B9 C8 | A31 B10 C8 | A31 B12 C8 | A31 B18 C8 | A31 B22 C8 | A31 B23 C8 | A31 B25 C8 |
| A31 B6 C9 | A31 B7 C9 | A31 B8 C9 | A31 B9 C9 | A31 B10 C9 | A31 B12 C9 | A31 B18 C9 | A31 B22 C9 | A31 B23 C9 | A31 B25 C9 |
| A31 B6 C10 | A31 B7 C10 | A31 B8 C10 | A31 B9 C10 | A31 B10 C10 | A31 B12 C10 | A31 B18 C10 | A31 B22 C10 | A31 B23 C10 | A31 B25 C10 |
| A31 B6 C11 | A31 B7 C11 | A31 B8 C11 | A31 B9 C11 | A31 B10 C11 | A31 B12 C11 | A31 B18 C11 | A31 B22 C11 | A31 B23 C11 | A31 B25 C11 |
| A31 B6 C12 | A31 B7 C12 | A31 B8 C12 | A31 B9 C12 | A31 B10 C12 | A31 B12 C12 | A31 B18 C12 | A31 B22 C12 | A31 B23 C12 | A31 B25 C12 |
| A31 B6 C13 | A31 B7 C13 | A31 B8 C13 | A31 B9 C13 | A31 B10 C13 | A31 B12 C13 | A31 B18 C13 | A31 B22 C13 | A31 B23 C13 | A31 B25 C13 |
| A31 B6 C14 | A31 B7 C14 | A31 B8 C14 | A31 B9 C14 | A31 B10 C14 | A31 B12 C14 | A31 B18 C14 | A31 B22 C14 | A31 B23 C14 | A31 B25 C14 |
| A31 B6 C15 | A31 B7 C15 | A31 B8 C15 | A31 B9 C15 | A31 B10 C15 | A31 B12 C15 | A31 B18 C15 | A31 B22 C15 | A31 B23 C15 | A31 B25 C15 |
| A31 B6 C16 | A31 B7 C16 | A31 B8 C16 | A31 B9 C16 | A31 B10 C16 | A31 B12 C16 | A31 B18 C16 | A31 B22 C16 | A31 B23 C16 | A31 B25 C16 |
| A31 B6 C17 | A31 B7 C17 | A31 B8 C17 | A31 B9 C17 | A31 BIO C17 | A31 B12 C17 | A31 B18 C17 | A31 B22 C17 | A31 B23 C17 7 | A31 B25 C17 |
| A31 B6 C18 | A31 B7 C18 | A31 B8 C18 | A31 B9 C18 | A31 B10 C18 | A31 B12 C18 | A31 B18 C18 | A31 B22 C18 | A31 B23 C18 8 | A31 B25 C18 |
| A31 B6 C19 | A31 B7 C19 | A31 B8 C19 | A31 B9 C19 | A31 B10 C19 | A31 B12 C19 | A31 B18 C19 | A31 B22 C19 | A31 B23 C19 | A31 B25 C19 |
| A31 B6 C20 | A31 B7 C20 | A31 B8 C20 | A31 B9 C20 | A31 B10 C20 | A31 B12 C20 | A31 B18 C20 | A31 B22 C20 | A31 B23 C20 | A31 B25 C20 |
| A31 B6 C21 | A31 B7 C21 | A31 B8 C21 | A31 B9 C21 | A31 B10 C21 | A31 B12 C21 | A31 B18 C21 | A31 B22 C21 | A31 B23 C21 | A31 B25 C21 |
| A31 B6 C22 | A31 B7 C22 | A31 B8 C22 | A31 B9 C22 | A31 B10 C22 | A31 B12 C22 | A31 B18 C22 | A31 B22 C22 | A31 B23 C22 | A31 B25 C22 |
| A31 B6 C23 | A31 B7 C23 | A31 B8 C23 | A31 B9 C23 | A31 B10 C23 | A31 B12 C23 | A31 B18 C23 | A31 B22 C23 | A31 B23 C23 | A31 B25 C23 |
| A31 B6 C24 | A31 B7 C24 | A31 B8 C24 | A31 B9 C24 | A31 B10 C24 | A31 B12 C24 | A31 B18 C24 | A31 B22 C24 | A31 B23 C24 | A31 B25 C24 |
| A31 B6 C25 | A31 B7 C25 | A31 B8 C25 | A31 B9 C25 | A31 B10 C25 | A31 B12 C25 | A31 B18 C25 | A31 B22 C25 | A31 B23 C25 | A31 B25 C25 |
| A31 B6 C26 | A31 B7 C26 | A31 B8 C26 | A31 B9 C26 | A31 B10 C26 | A31 B12 C26 | A31 B18 C26 | A31 B22 C26 | A31 B23 C26 | A31 B25 C26 |
| A31 B6 C27 | A31 B7 C27 | A31 B8 C27 | A31 B9 C27 | A31 B10 C27 | A31 B12 C27 | A31 B18 C27 | A31 B22 C27 | A31 B23 C27 | A31 B25 C27 |
| A31 B6 C28 | A31 B7 C28 | A31 B8 C28 | A31 B9 C28 | A31 B10 C28 | A31 B12 C28 | A31 B18 C28 | A31 B22 C28 | A31 B23 C28 | A31 B25 C28 |
| A31 B6 C29 | A31 B7 C29 | A31 B8 C29 | A31 B9 C29 | A31 B10 C29 | A31 B12 C29 | A31 B18 C29 | A31 B22 C29 | A31 B23 C19 | A31 B25 C29 |
| A31 B6 C30 | A31 B7 C30 | A31 B8 C30 | A31 B9 C30 | A31 B10 C30 | A31 B12 C30 | A31 B18 C30 | A31 B22 C30 | A31 B23 C30 | A31 B25 C30 |
| A31 B6 C31 | A31 B7 C31 | A31 B8 C31 | A31 B9 C31 | A31 B10 C31 | A31 B12 C31 | A31 B18 C31 | A31 B22 C31 | A31 B23 C31 | A31 B25 C31 |
| A31 B6 C32 | A31 B7 C32 | A31 B8 C32 | A31 B9 C32 | A31 B10 C32 | A31 B12 C32 | A31 B18 C32 | A31 B22 C32 | A31 B23 C32 | A31 B25 C32 |
| A31 B6 C33 | A31 B7 C33 | A31 B8 C33 | A31 B9 C33 | A31 B10 C33 | A31 B12 C33 | A31 B18 C33 | A31 B22 C33 | A31 B23 C33 | A31 B25 C33 |
| A31 B6 C34 | A31 B7 C34 | A31 B8 C34 | A31 B9 C34 | A31 B10 C34 | A31 B12 C34 | A31 B18 C34 | A31 B22 C34 | A31 B23 C34 | A31 B25 C34 |
| A31 B6 C35 | A31 B7 C35 | A31 B8 C35 | A31 B9 C35 | A31 B10 C35 | A31 B12 C35 | A31 B18 C35 | A31 B22 C35 | A31 B23 C35 | A31 B25 C35 |
| A31 B6 C36 | A31 B7 C36 | A31 B8 C36 | A31 B9 C36 | A31 B10 C36 | A31 B12 C36 | A31 B18 C36 | A31 B22 C36 | A31 B23 C36 | A31 B25 C36 |
| A31 B6 C37 | A31 B7 C37 | A31 B8 C37 | A31 B9 C37 | A31 B10 C37 | A31 B12 C37 | A31 B18 C37 | A31 B22 C37 | A31 B23 C37 | A31 B25 C37 |
| A31 B6 C38 | A31 B7 C38 | A31 B8 C38 | A31 B9 C38 | A31 B10 C38 | A31 B12 C38 | A31 B18 C38 | A31 B22 C38 | A31 B23 C38 | A31 B25 C38 |
| A31 B6 C39 | A31 B7 C39 | A31 B8 C39 | A31 B9 C39 | A31 B10 C39 | A31 B12 C39 | A31 B18 C39 | A31 B22 C39 | A31 B23 C39 | A31 B25 C39 |
| A31 B6 C40 | A31 B7 C40 | A31 B8 C40 | A31 B9 C40 | A31 B10 C40 | A31 B12 C40 | A31 B18 C40 | A31 B22 C40 | A31 B23 C40 | A31 B25 C40 |
| A31 B6 C41 | A31 B7 C41 | A31 B8 C41 | A31 B9 C41 | A31 B10 C41 | A31 B12 C41 | A31 B18 C41 | A31 B22 C41 | A31 B23 C41 | A31 B25 C41 |

or the pharmaceutically acceptable salts, solvates, or tautomers of any of the above compounds.

Even more preferred embodiments of the compounds of the present invention are directed to the compounds of formula (Ia) below, postulated to preferably possess Cathepsin K activity: 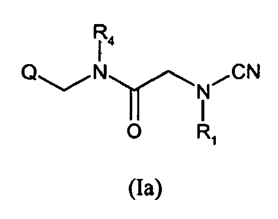 wherein:
R₁ is selected from the following groups: 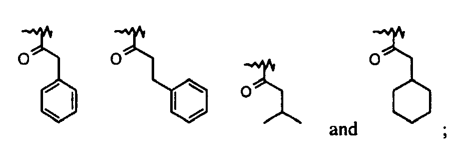
R₄ is selected from the following groups: 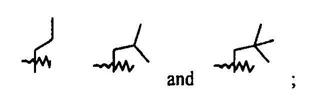
and Q is selected from the following groups: 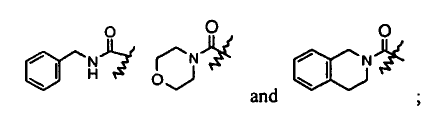 or the pharmaceutically acceptable salts, solvates, or tautomers thereof

The compounds described above can be synthesized by the General schemes and methods described in the experimental section of this document and analogous methods known to those skilled in the art without undue experimentation.

### GENERAL SYNTHETIC METHODS

The invention also provides processes of making the present novel compounds. Compounds of the invention may be prepared by methods described below. In the following schemes, the variables R₁, R₂, R₃, R₄, R₅, R₆ and Q are as defined previously for the compounds of formula (I) except where indicated. Intermediates used in the preparation of the compounds of the invention are either commercially available or readily prepared by methods known to those skilled in the art.

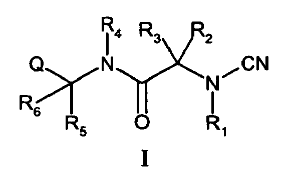

The synthesis of the compounds of formula (I) may be carried out as illustrated in the following Scheme I:

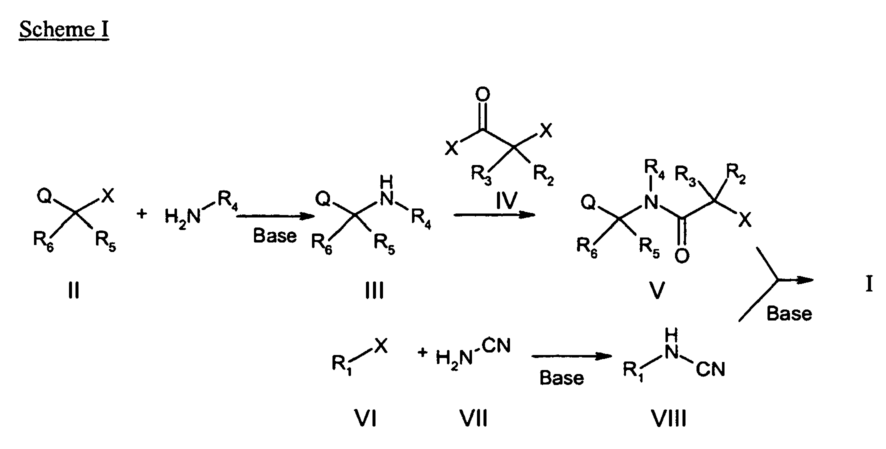

As shown in Scheme I, an amine bearing R₄ is reacted with II, where X is a suitable leaving group such as a halogen, mesylate, triflate or tosylate, in a suitable solvent such as THF. The reaction may be done in the presence of a suitable base such as triethylamine, or alternatively in the presence of excess R₄NH₂. Intermediate III is then reacted with IV, where X is defined as above, in a suitable solvent such as THF, optionally in the presence of a suitable base such as triethylamine, to provide V. Intermediate V is reacted with cyanamide VIII in a suitable solvent such as DMF, in the presence of a suitable base such as potassium t-butoxide to give the desired compound of formula I. Intermediate VIII may be prepared by reacting R₁X, where X is defined as above with cyanamide (VII) in a suitable solvent such as acetone, in the presence of a suitable base, for example aqueous sodium hydroxide. Intermediates II, R₄ NH₂, IV, and VI are either commercially available or easily prepared by methods described in the literature and known to those skilled in the art.

An alternate synthesis of intermediate III is illustrated in the following Scheme II:

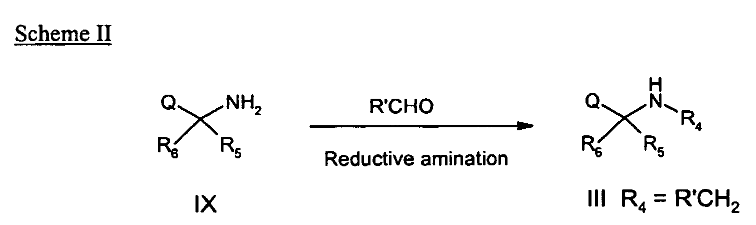

In this method an amine bearing Q, R₅, and R₆ (IX) is reacted with an aldehyde (R'CHO) under reductive amination conditions to provide an intermediate III in which R₄ = R'CH₂. This method is therefore useful to prepare intermediates of formula III as shown in Scheme I wherein the R₄ group is selected from an R₄ group as defined previously having a -CH₂- group bonded to the nitrogen atom in the backbone of formula (III), and R' is that terminal portion of that R₄ group which is bonded to said -CH₂- group which, in turn, is bonded to the nitrogen atom in the backbone of formula (III).

Compounds of formula I may also be prepared as described in the following Scheme III:

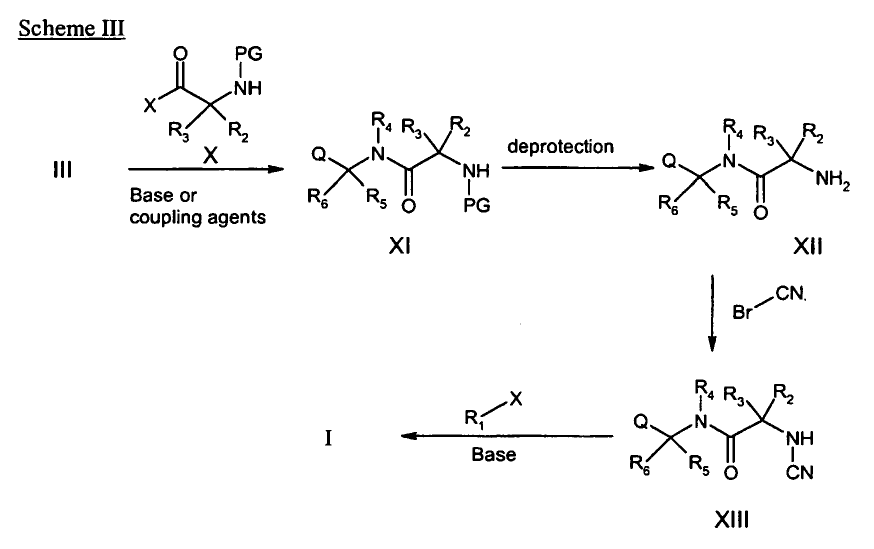

In this method, intermediate III (Scheme I or Scheme II) is reacted with intermediate X, where PG is a suitable protecting group such as a t-butoxycarbonyl (Boc) group, and substituent X is defined as above, in a suitable solvent such as THF and optionally in the presence of a suitable base, such as triethylamine, or a suitable coupling agent, such as EDC to provide XI. Deprotection of XI by standard methods that depend on the group PG used provides XII. Reaction of XII with cyanogen bromide, optionally in the presence of a suitable base such as triethylamine, provides XIII. Reaction of XIII with R₁ bearing a leaving group X defined as above, in the presence of a suitable base such as potassium t-butoxide provides the desired compound of formula I.

In order for this invention to be more fully understood, the following examples are set forth. These examples are for the purpose of illustrating embodiments of this invention, and are not to be construed as limiting the scope of the invention in any way.

The examples which follow are illustrative and, as recognized by one skilled in the art, particular reagents or conditions could be modified as needed for individual compounds without undue experimentation. Starting materials used in the scheme below are either commercially available or easily prepared from commercially available materials by those skilled in the art.

### SYNTHETIC EXAMPLES

### Example 1: N-cyano-N-{[isobutyl-(2-morpholin-4-yl-2-oxo-ethyl)-carbamoyl]-methyl}-2-phenyl-acetamide.

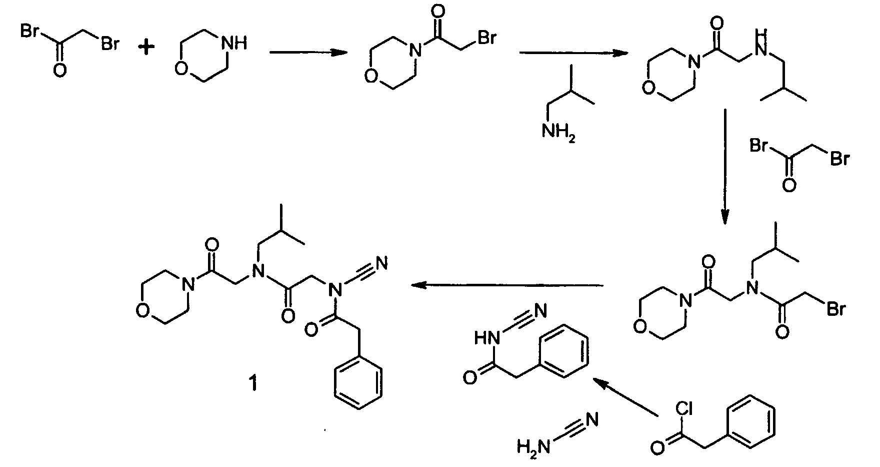

Bromoacetyl bromide (6.0 mL, 68.9 mmol) was dissolved in 30 mL of ether. Morpholine (7.0 mL, 80.3 mmol) was added dropwise to the above solution at -78 °C. The reaction mixture was warmed to room temperature in 30 min. The resulting white solid was removed by filtration and washed with ether. The filtrate was washed with saturated sodium bicarbonate (20 mL x 2). The combined aqueous phase was extracted with ether (20 mL x 4). The combined organic extracts were washed with brine and dried over sodium sulfate. The solvent was removed in vacuo to give *N*-bromoacetyl-morpholine (3.73 g, 45%) as a clear oil.

*N*-Bromoacetyl-morpholine (3.73 g, 18 mmoi) was dissolved in 20 mL of THF. Isobutylamine (5.40 mL, 54.0 mmol) was added. The reaction mixture was stirred at room temperature for 24 h. Solvent and excess reagent were removed in vacuo. The residue was treated with ether. The resulting solid was removed by filtration and washed with ether. The filtrate was concentrated under reduced pressure to give 2-isobutylamino-1-morpholin-4-yl-ethanone (1.43 g, 40%) as a clear oil.

2-Isobutylamino-1-morpholin-4-yl-ethanone (1.43 g, 7.13 mmol) was dissolved in 15 mL of THF. To this solution at -78 °C was added a solution of bromoacetyl bromide (1.24 mL, 14.3 mmol) in 15 mL of THF. The mixture was warmed to room temperature and stirred for 2 h. Solvent was removed in vacuo. The residue was dissolved in 30 mL of methylene chloride, washed with saturated sodium bicarbonate, and brine, then dried over sodium sulfate. The solvent was removed in vacuo and the residue was purified by silica gel flash chromatography eluting with 2% MeOH in methylene chloride to give 2-bromo-N-isobutyl-N-(2-morpholin-4-yl-2-oxo-ethyl)-acetamide (1.37 g, 60%) as a clear oil.

Cyanamide (5.04 g, 120 mmol) was dissolved in 40 mL of 40% sodium hydroxide. To this solution at 0 °C, phenylacetyl chloride (3.97 mL, 30 mmol) in 30 mL of acetone was added over 1 h. After stirring at room temperature for 2 h, the reaction mixture as acidified with concentrated HCl to pH 2. Acetone was removed in vacuo. The resulting suspension was extracted with methylene chloride (30 mL x 4). The combined organic extracts were washed with brine and dried over magnesium sulfate. Solvent was removed in vacuo to give *N*-cyano-2-phenylacetamide (3.96 g, 82%) as a yellow oil.

*N*-Cyano-2-phenylacetamide (160 mg, 1.00 mmol) was dissolved in 5 mL of dry DMF. Potassium t-butoxide (112 mg, 1.00 mmol) was added. This mixture was stirred at room temperature for 30 min. The 2-bromo-*N*-isobutyl-*N*-(2-morpholin-4-yl-2-oxo-ethyl)-acetamide (321 mg, 1.00 mmol) from above was dissolved in 5 mL of dry DMF and added to the reaction mixture. The reaction mixture was stirred at room temperature for 2 h and then heated at 70 °C for 6 h. Solvent was removed in vacuo and residue was purified by HPLC to give the title compound (87 mg, 22%) as a clear oil.

### Example 2: Synthesis of N-({[2-(3,4-dihydro-1H-isoquinolin-2-yl)-2-oxo-ethyl]-isobutyl-carbamoyl}-methyl)-N-cyano-2-phenyl-acetamide

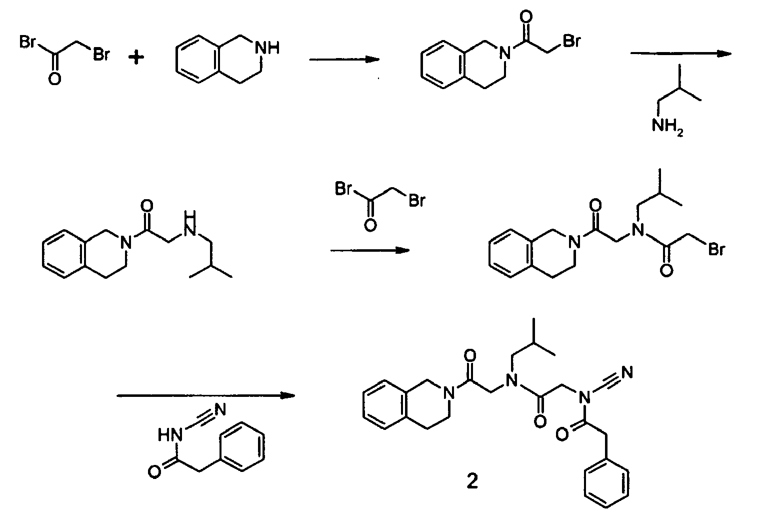

To a stirred solution of bromoacetyl bromide (0.57 mL, 6.4 mmol) in THF (4.0 mL) was added 1,2,3,4-tetrahydroisoquinoline (1.0 g, 7.5 mmol) at-78°C, under argon. Upon complete addition, the cooling bath was removed and the reaction mixture was stirred at ambient temperature for 1 h. After this time, the precipitated solids were removed *via* filtration and washed with EtOAc. The combined filtrates were washed with saturated NaHCO₃ (2X), then once with brine. Concentration of the organic phase gave an orange oil which was used without further purification.

To a stirred solution of the above intermediate in THF (4.0 mL) was added isobutylamine (2.0 mL, 20.1 mmol) at room temperature. After 0.5 h the reaction was poured into saturated NaHCO₃ and EtOAc. The layers were separated and the organic phase was concentrated to give the crude product. Purification by silica gel flash chromatography, eluting with 0-5% MeOH/EtOAc, 30 mL/min, gave 0.45 g (25%) of 1-(3,4-dihydro-1H-isoquinolin-2-yl)-2-isobutylamino-ethanone.

To a stirred solution of 1-(3,4-dihydro- 1H-isoquinolin-2-yl)-2-isobutylamino-ethanone (0.45 g, 1.8 mmol) in THF (4.0 mL), a solution of bromoacetyl bromide (0.32 mL, 3.6mmol) in 4.0 mL THF was added dropwise at -78 °C, under argon. After 3 h (the temperature gradually rose to 10 °C) the reaction was concentrated and the remaining residue was diluted with methylene chloride and saturated NaHCO₃. The layers were separated and the organic phase was washed with brine and then dried (MgSO₄). Filtration and concentration gave the crude product which was purified *via* silica gel flash chromatography (0-5% MeOH/methylene chloride, 30 mL/min.) to give 0.34 g (51%) of 2-bromo-N-[2-(3,4-dihydro-1H-isoquinolin-2-yl)-2-oxo-ethyl]-N-isobutyl-acetamide.

To a stirred solution of N-cyano-2-phenyl-acetamide (0.15 g, 0.9 mmol) (see Example 1) in DMF (5.0 mL), potassium *tert*-butoxide (0.9 mL of a 1.0 M THF solution, 0.9 mmol) was added dropwise at 0 °C, under argon. The resulting mixture was stirred at 0 °C for 20 min, then at ambient temperature for 15 min. After this time, the resulting anion was added dropwise to a stirred solution of the 2-bromo-N-[2-(3,4-dihydro-1H-isoquinolin-2-yl)-2-oxo-ethyl]-N-isobutyl-acetamide (0.34 g, 0.9 mmol) from above in DMF (5.0 mL) at room temperature, under argon. Upon complete addition, the reaction was stirred at room temperature for 1.5 h, then warmed to 40 °C for 6 h. The reaction was cooled to room temperature and stirred for 48 h after which time it was concentrated to dryness. The resulting residue was dissolved in EtOAc and washed with 5% citric acid (aqueous). The layers were separated and the aqueous phase was extracted with EtOAc (2X). The combined organics were dried (MgSO₄), filtered and concentrated to give the crude product which was purified *via* reverse phase HPLC (C18 column, 20-100% CH₃CN/H₂O over 20 min at a flow rate of 20 mL/min, UV detection was at 220 nm). Retention time of the product was 16 min. Product-containing fractions were concentrated to give 49 mg (12%) of the title compound.

### Example 3: Synthesis of N-{[(2,2-dimethyl-propyl)-(2-morpholin-4-yl-2-oxo-ethyl)-carbamoyl]-methyl}-N-cyano-2-phenyl-acetamide

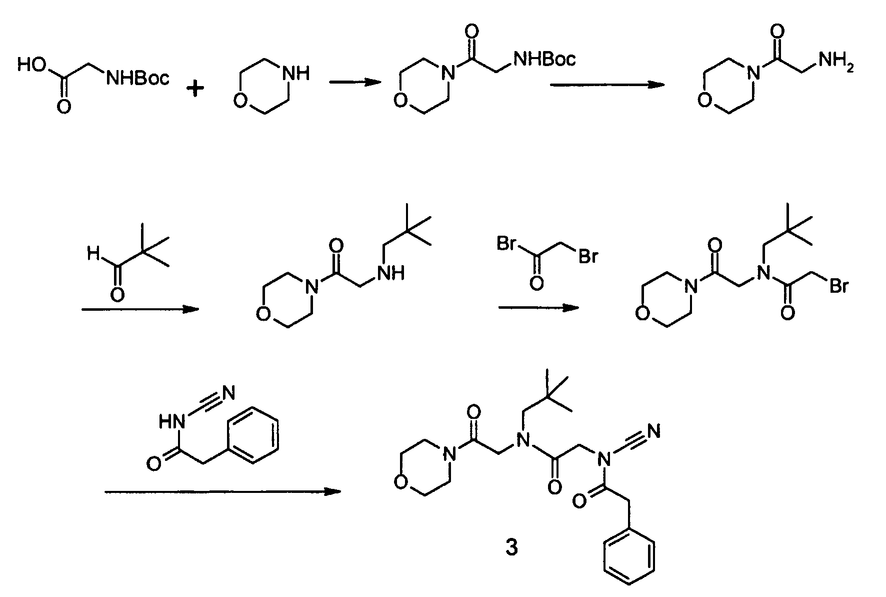

To a stirred solution of N-(tert-butoxycarbonyl)glycine (3.0 g, 17.1 mmol) in DMF (75 mL), N,N-diisopropylethylamine (14.9 mL, 85.5mmol) was added, followed by EDC (3.6 g, 18.8 mmol) and HOBT (2.5 g, 18.8 mmol) at 0 °C, under argon. The resulting mixture was stirred for 0.5 h after which time morpholine (3.0 mL, 34.2 mmol) was introduced in a dropwise manner. Upon complete addition, the reaction was warmed to ambient temperature, stirred overnight and concentrated. The resulting residue was diluted with EtOAc and saturated NaHCO₃ The layers were separated and the aqueous phase was extracted with EtOAc. The combined organics were dried (MgSO₄), filtered and concentrated to give 2.3 g (55%) of (2-morpholin-4-yl-2-oxo-ethyl)-carbamic acid tert-butyl ester which was used without further purification.

To a stirred solution of the above (2-morpholin-4-yl-2-oxo-ethyl)-carbamic acid ten-butyl ester (0.44 g, 1.8 mmol) in 1,4-dioxane (4.0 mL), HCl (4.0 mL of a 4 M solution in 1,4-dioxane) was added at room temperature. After one h, the precipitated product was collected *via* filtration and washed with EtOAc. The solid was dried under high vacuum to give 0.23 g (76%) of 2-amino-1-morpholin-4-yl-ethanone hydrochloride which was used without further purification.

To a stirred solution of the above 2-amino-1-morpholin-4-yl-ethanone hydrochloride (0.23 g, 1.3 mmol) in methylene chloride (4.0 mL) and triethylamine (0.19 mL, 1.4 mmol), 4 angstrom sieves (10 beads, pulverized to a powder) were added, followed by trimethylacetaldehyde (0.35 mL, 3.3mmol) at room temperature, under argon. The resulting mixture was stirred at room temperature overnight after which time sodium triacetoxyborohydride (0.54 g, 2.6mmol) was added. After 6 h, the solids were removed *via* filtration and washed with methylene chloride. The combined filtrates were concentrated and the remaining residue was diluted with EtOAc and water. The aqueous phase was lyophilized to give a white solid that was diluted with EtOAc. The solids were removed *via* filtration and washed with EtOAc. The combined organics were concentrated to give the crude product which was purified *via* silica gel flash chromatography eluting with 100%EtOAc, then 0.5% NH₄OH/10% MeOH/EtOAc, flow rate 30 mL/min. The product-containing fractions were concentrated to give 0.18 g (67%) of 2-(2,2-dimethyl-propylamino)-1-morpholin-4-yl-ethanone.

To a stirred solution of the above 2-(2,2-dimethyl-propylamino)-1-morpholin-4-yl-ethanone (0.18 g, 0.8 mmol) in THF (2.0 mL), a solution of bromoacetyl bromide (0.15 mL, 1.6mmol) in THF (2.0 mL) was added, dropwise at -78 °C, under nitrogen. After 3h the reaction was concentrated and the resulting residue was diluted with methylene chloride and saturated NaHCO₃. The layers were separated and the aqueous phase was extracted with methylene chloride. The combined organic layers were dried (MgSO₄), filtered and concentrated to give the crude product. Purification *via* silica gel flash chromatography, eluting with EtOAc, flow rate 30 mL/min, gave 0.10 g (36%) of 2-bromo-N-(2,2-dimethyl-propyl)-N-(2-morpholin-4-yl-2-oxo-ethyl)-acetamide.

To a stirred solution of N-cyano-2-phenyl-acetamide (35 mg, 0.2 mmol) (see Example 1) in DMF (1.25 mL), potassium *tert*-butoxide (0.22 mL of a 1.0 M THF solution, 0.22 mmol) was added, dropwise at 0 °C, under nitrogen. Upon complete addition, the cooling bath was removed and the reaction was stirred for 0.5 h. After this time, the resulting anion was added dropwise to a stirred solution of the above 2-bromo-N-(2,2-dimethylpropyl)-N-(2-morpholin-4-yl-2-oxo-ethyl)-acetamide (88 mg, 0.24 mmol) in DMF (1.25 mL) at room temperature, under nitrogen. Upon complete addition, the reaction was stirred at room temperature for 0.5 h, then warmed to 45 °C for 15 h. The reaction was cooled to room temperature and concentrated to dryness. The remaining residue was dissolved in EtOAc and washed with 5% citric acid (aqueous). The layers were separated and the aqueous phase was extracted with EtOAc (2X). The combined organics were dried (MgSO₄), filtered and concentrated to give the crude product which was purified *via* reverse phase HPLC (C18 column, 20-100% CH₃CN/H₂O over 20 min at a flow rate of 20 mL/minute, UV detection was at 220 nm). Retention time of the product was 14.7 min. Product-containing fractions were concentrated to give 14 mg (15%) of the title compound.

The following compounds were also made by methods analogous to those described in Examples 1-3:

| Example | Structure | Name |
|---|---|---|
| 4 | 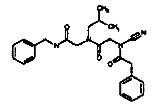 | N-{[(Benzylcarbamoyl-methyl)-isobutyl-carbamoyl]-methyl}-N-cyano-2-phenyl-acetamide |
| 5 | 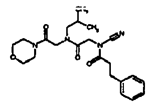 | N-{[Isobutyl-(2-morpholin-4-yl-2-oxo-ethyl)-carbamoyl]-methyl}-N-cyano-3-phenyl-propionamide |
| 6 | 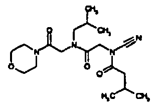 | N-{[Isobutyl-(2-morpholin-4-yl-2-oxo-ethyl)-carbamoyl]-methyl}-3-methyl-N-cyano-butyramide |
| 7 | 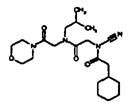 | 2-Cyclohexyl-N-{[isobutyl-(2-morpholin-4-yl-2-oxo-ethyl)-carbamoyl]-methyl}-N-cyano-acetamide |
| 8 | 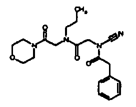 | N-Cyano-N-{[(2-morpholin-4-yl-2-oxo-ethyl)-propyl-carbamoyl]-methyl}-2-phenyl-acetamide |

### METHODS OF THERAPEUTIC USE

The compounds of the invention are useful in inhibiting the activity of cysteine proteases, such as cathepsins S, K, F, L and B. In doing so, these compounds are useful in blocking disease processes mediated by these cysteine proteases. Accordingly, the compounds of the present invention are useful in treating cysteine protease mediated disease states, i.e., those diseases in which cysteine protease activity contributes to the pathology and/or symptomatology of the disease. A variety of such cysteine protease mediated disease states are known in the art and are disclosed, for example, in the references cited in the "Background of the Invention" section above.

Compounds of this invention effectively block degradation of the invariant chain to CLIP by cathepsin S, and thus inhibit antigen presentation and antigen-specific immune responses. Control of antigen specific immune responses is an attractive means for treating autoimmune diseases and other undesirable T-cell mediated immune responses. Thus, there is provided methods of treatment using the compounds of this invention for such conditions. These encompass autoimmune diseases and other diseases involving inappropriate antigen specific immune responses including, but not limited to, rheumatoid arthritis, systemic lupus erythematosus, Crohn's disease, ulcerative colitis, multiple sclerosis, Guillain-Barre syndrome, psoriasis, Grave's disease, myasthenia gravis, scleroderma, endometriosis, glomerulonephritis, atopic dermatitis, insulin-dependent diabetes mellitus and asthma. The compounds of the invention can also be used to treat other disorders associated with extracellular proteolysis such as Alzheimer's disease and atherosclerosis. The compounds of the invention can also be used to treat other disorders associated with inappropriate autoimmune responses, T-cell mediated immune responses, or extracellular proteolysis mediated by cathepsin S, unrelated to those listed above or discussed in the Background of the Invention. Therefore, the invention also provides methods of modulating an autoimmune disease comprising administering to a patient in need of such treatment a pharmaceutically effect amount of a compound according to the invention.

Accordingly, in one embodiment the present invention is directed to preparing a pharmaceutical composition for a method of treating a disease mediated by cathepsin S comprising adminstering to a patient in need of such treatment a therapeutically effective amount of a a compound according to claim 1, or a pharmaceutically acceptable salt, solvate, or tautomer thereof. Examples of diseases mediated by cathepsin S that may be treated include: autoimmune diseases (such as rheumatoid arthritis, systemic lupus erythematosus, Crohn's disease, ulcerative colitis, multiple sclerosis, Guillain-Barre syndrome, psoriasis, Grave's disease, myasthenia gravis, scleroderma, glomerulonephritis, atopic dermatitis and insulin-dependent diabetes mellitus); Alzheimer's disease, atherosclerosis, endometriosis and asthma.

Compounds of the invention also inhibit cathepsin K. In doing so, they may block bone resorption, bone loss and inappropriate degradation of bone collagen and other bone matrix proteases. Thus, there is provided a method for treating diseases characterized by bone resporption, bone loss or excessive cartilage or bone matrix degradation such as osteoporosis, Paget's disease, Gaucher disease, gingivitis, periodontitis, and rheumatoid arthritis, or diseases characterized by increases in bone resporption and demineralization of bone. In view of their Cathepsin K inhibitory activity, the compounds of the invention may also be useful for treating disorders associated with excessive elastin degradation such as lymphangiomyomatosis, vascular inflammation, and cardiovascular diseases such as atherosclerosis. Inhibition of cathepsins F, L, and B are also within the scope of the invention due to similarity of the active sites in cysteine proteases as described above.

Accordingly, in another embodiment the present invention is directed to preparing a pharmaceutical composition for a method of treating a disease mediated by cathepsin K comprising adminstering to a patient in need of such treatment a therapeutically effective amount of a compound according to claim 1, or a pharmaceutically acceptable salt, solvate, or tautomer thereof. Examples of diseases mediated by cathepsin K that may be treated include: a disease characterized by bone resporption, bone loss or excessive cartilage or bone matrix degradation, such as osteoporosis, Paget's disease, Gaucher disease, gingivitis, periodontitis, and rheumatoid arthritis, or diseases characterized by increases in bone resporption and demineralization of bone, such as those associated with many cancers and with bone metastases of breast and prostate tumors, or disorders associated with excessive elastin degradation such as lymphangiomyomatosis, vascular inflammation, and cardiovascular diseases such as atherosclerosis.

In addition, the compounds according to the invention can be used for the treatment of any other specific disease-state or condition not specifically mentioned above which have been treated, are being treated or will in the future be treated with compounds that are inhibitors of cathepsins S, K, F, L or B.

The activity of particular compounds disclosed herein against the various cathepsins, for example, cathepsin S and K, may be determined without undue experimentation by one of ordinary skill in the art in view of the knowledge in the art, the guidance provided throughout this specification and by the screens described in the section below entitled "Assessment of Biological Properties."

In general, it is expected that compounds within formula (I) having less bulky groups at the R₄ position would be more active as inhibitors of cathepsin K, for example, compounds where R₄ = B3 to B6 or B11 from the above Table I. On the other hand, it is expected in general that compounds within formula (I) having bulkier groups at the R₄ position would be more active as inhibitors of cathepsin S, for example, where R₄ = B7 to B10, B12, B18 or B22 to B26 from the above Table I. In particular, Examples 1 to 8 set forth above have been tested and have demonstrated cathepsin S and/or K inhibitory activity in one or more of the screens described below. Specifically, Examples 1 to 8 were tested for and demonstrated cathepsin K inhibitory activity and Examples 1-3 and 5-8 were tested for and demonstrated inhibitory activity against cathepsin S.

For therapeutic use, the compounds of the invention may be administered in any conventional dosage form in any conventional manner to a patient, e.g. a mammal, in need of such treatment. Routes of administration include, but are not limited to, intravenously, intramuscularly, subcutaneously, intrasynovially, by infusion, sublingually, transdermally, orally, topically or by inhalation. The preferred modes of administration are oral and intravenous.

The compounds of this invention may be administered alone or in combination with adjuvants that enhance stability of the inhibitors, facilitate administration of pharmaceutical compositions containing them in certain embodiments, provide increased dissolution or dispersion, increase inhibitory activity, provide adjunct therapy, and the like, including other active ingredients. Advantageously, such combination therapies utilize lower dosages of the conventional therapeutics, thus avoiding possible toxicity and adverse side effects incurred when those agents are used as monotherapies. Compounds of the invention may be physically combined with the conventional therapeutics or other adjuvants into a single pharmaceutical composition. Advantageously, the compounds may then be administered together in a single dosage form. In some embodiments, the pharmaceutical compositions comprising such combinations of compounds contain at least about 15%, but more preferably at least about 20%, of a compound of the invention (w/w) or a combination thereof. Alternatively, the compounds may be administered separately (either serially or in parallel). Separate dosing allows for greater flexibility in the dosing regime.

As mentioned above, pharmaceutical dosage forms of the compounds or this invention include pharmaceutically acceptable carriers and adjuvants known to those of ordinary skill in the art. These carriers and adjuvants include, for example, ion exchangers, alumina, aluminum stearate, lecithin, serum proteins, buffer substances, water, salts or electrolytes and cellulose-based substances. Preferred dosage forms include, tablet, capsule, caplet, liquid, solution, suspension, emulsion, lozenges, syrup, reconstitutable powder, granule, suppository and transdermal patch. Methods for preparing such dosage forms are known (see, for example, H.C. Ansel and N.G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient. In some embodiments, dosage levels range from about 10-1000 mg/dose for a 70 kg patient. Although one dose per day may be sufficient, up to 5 doses per day may be given. For oral doses, up to 2000 mg/day may be required. As the skilled artisan will appreciate, lower or higher doses may be required depending on particular factors. For instance, specific dosage and treatment regimens will depend on factors such as the patient's general health profile, the severity and course of the patient's disorder or disposition thereto, and the judgment of the treating physician. Some degree of routine dose optimization may be required to determine an optimal dosing level and pattern.

### ASSESSMENT OF BIOLOGICAL PROPERTIES

### Expression and Purification of recombinant human Cathepsin S

Expression and Purification of recombinant human Cathepsin S may be done as described in US patent no. 6,313,117.

### Inhibition of Cathepsin S

Human recombinant cathepsin S expressed in Baculovirus is used at a final concentration of 10 nM in buffer. Buffer is 50 mM sodium acetate, pH 6.5, 2.5 mM EDTA, 2.5 mM TCEP. Enzyme is incubated with either compound or DMSO for 10 min at 37°C. Substrate 7-amino-4-methylcoumarin, CBZ-L-valyl-L-valyl-L-arginineamide (custom synthesis by Molecular Probes) is diluted to 20 uM in water (final concentration of 5 uM), added to assay and incubated for additional 10 minutes at 37°C. Compound activity is measured by diminished fluorescence compared to DMSO control when read at 360 nm excitation and 460 nm emission.

Another assay for Cathepsin S inhibitory activity is the cell based assay described in Riese, R.J. et al., Immunity, 1996, 4, 357-366.

Preferred compounds for the inhibition of Cathepsin S are those that will exhibit an IC₅₀ of 10 micromolar or below in the above assays.

### Inhibition of Cathepsin K, F, L and B:

Inhibition of these enzymes by particular compounds of the invention may be determined without undue experimentation by using methods as provided in the references cited hereinbelow:
1. S.K. Lee, S.R. Goldring, and J.A. Lorenzo. (1995) Endocrinology 136: 4572.
2. N. Takahashi et al. (1988) Endocrinology 122: 1373.
3. N. Takahashi et al. (1988) Endocrinology 123:1504.
4. T. Akatsu et al. (1992) J. Bone Miner. Res. 7: 1297.
5. T. Akatsu et al. (1989) J. Bone Miner. Res. 4: 29.
6. T. Shuto et al. (1994) Endocrinology 134:1121.
7. A. Boyde, N.N. Ali, and S.J. Jones. (1984) Br. Dent. J. 156: 216.
8. D.W. Dempster et al. (1987) J. Bone Miner. Res. 2: 443.
9. R.J. Murrills et al. (1989) J. Bone Miner. Res. 4: 259.
10. N.T. Foged et al. (1996) J. Bone Miner. Res. 11: 226.

A Cathepsin K bone resporption cellular assay can be carried out as follows:
*In vitro* culturing of murine osteoclasts can be accomplished by a modification of previous published protocols (1-6). Briefly, tibiae and femur are removed from 6-10 week-old C57BL/6 male mice. Marrow is flushed from the bones and placed into MEM-alpha media (Gibco) supplemented with 10% FCS (Gibco). After washing, 100 mm tissue culture dishes is seeded at 1x10⁶ cells/cm² (at 2x10⁶ cells/mL) and supplemented with 10 nM 1,25-dihydroxyvitamin D3 (Sigma) and cultured at 37°C and 5% CO₂. Cultures are fed every 3 days by removing 80% of the media and replacement with fresh media and vitamin D₃. *In vitro* bone resorption assays can be carried out similar to those previously described (1, 7-9) with modifications: After 7 days in culture, osteoclasts are trypsinized and scraped off 100 mm dishes and split into 96 well plates containing bovine cortical bone slices. After 2 hours at 37°C, non-adherent cells are removed by washing and MEM-alpha media containing 0.7 g sodium bicarbonate/liter is added to the wells and media is supplemented with 100 ng/mL sRANKL (R&D Systems). After 3-4 days, supernatants are removed and analyzed for the presence of C-terminal peptides from type I collagen using a one-step ELISA (Osteometer Biotech) originally described by Foged et al. (10).

Preferred compounds for the inhibition of Cathepsin K are those that will exhibit an IC₅₀ of 50 micromolar or below in the above cellular assay.

A Cathepsin K enzymatic assay is disclosed in the following reference:
Bromme, D., Okamoto, K., Wang, B. B., and Biroc, S. (1996) J. Biol. Chem. 271, 2126-2132.

Preferred compounds for the inhibition of Cathepsin K are those that will exhibit an IC₅₀ of 10 micromolar or below in the above enzymatic assay.

Cathepsins B and L assays are to be found in the following reference:
Methods in Enzymology, Vol.244, Proteolytic Enzymes: Serine and Cysteine Peptidases, Alan J. Barrett, ed.

Cathepsin F assays are to be found in the following references:
Wang, B., Shi, G.P., Yao, P.M., Li, Z., Chapman, H.A., and Bromme, D. (1998) J. Biol. Chem. 273, 32000-32008.
Santamaria, I., Velasco, G., Pendas, A.M., Paz, A., and Lopez-Otin, C (1999) J. Biol. Chem. 274, 13800-13809.

Preferred compounds for the inhibition of Cathepsins B, L and F are those that will exhibit an IC₅₀ of 10 micromolar or below in the above assays.

## Claims

1. A compound of formula (I): 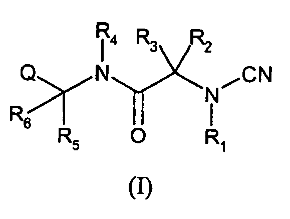 wherein:
R₁ is hydrogen, a C1-10 saturated or unsaturated alkyl group wherein one or more C atoms are optionally replaced by O NH, S(O), S(O)₂ or S and wherein said alkyl group is optionally independently substituted with one or more: oxo groups, -NH₂, C1-10 alkyl, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, tetrahydropyranyl, thiopyranyl, tetrahydrothiopyranyl, furanyl, tetrahydrofuranyl, thienyl, pyrrolyl, pyrazolyl, oxazolyl, isoxazolyl, thiazolyl, imidazolyl pyridinyl, pyrimidinyl, pyrazinyl, indolyl, benzofuranyl, benzothienyl, benzimidazolyl, benzothiazolyl, benzoxazolyl, quinolyl, isoquinolyl, C1-10 alkoxy, aryloxy, C1-10 alkanoyl, aroyl, C1-10 alkoxycarbonyl, aryloxycarbonyl, C1-10 alkanoyloxy, or aroyloxy,
or R₁ is C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, arylsulfonyl, heteroarylsulfonyl, tetrahydronaphthyl, indenyl, indanyl, tetrahydropyranyl, tetrahydrothiopyranyl, tetrahydrofuranyl, pyrrolidinyl, piperidinyl, morpholinyl, thiomorpholinyl, indolinyl, C3-8cycloalkylsulfonylC1-5alkyl, aryisulfonylC1-5alkyl, aryloxyC1-5alkyl, C1-10alkanoyl, aroyl, C1-10alkoxycarbonyl, arylC1-5alkoxycarbonyl or aryloxycarbonyl,
or R₁ is carbamoyl wherein the nitrogen atom may be independently mono or di-substituted by C1-10 alkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
each R₁ may be further optionally independently substituted by one or more Rₐ;
Rₐ is a C1-10 saturated or unsaturated alkyl group wherein one or more C atoms are optionally replaced by O, NH, S(O), S(O)₂ or S and wherein said alkyl group is optionally independently substituted with one or two oxo groups, or one or more: -NH₂, C1-10 alkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₐ is a C1-10alkoxy, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, C3-8 cycloalkyloxy, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl; C1-10alkanoyl, aroyl, C1-10alkanoyloxy, aryloxy, benzyloxy, C1-10 alkoxycarbonyl, arylC1-3alkoxycarbonyl, aryloxycarbonyl or aroyloxy,
or Rₐ is carbamoyl wherein the nitrogen atom may be independently mono or di-substituted by C1-10 alkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or R^{a} is ureido wherein either nitrogen atom may be independently substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₐ is amino wherein the nitrogen atom may be independently mono or di-substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
wherein Rₐ may be further optionally substituted by one or more R_{b};
R_{b} is C3-8 cycloalkyl, tolylsulfonyl, C1-5 alkoxy, aryl, aryloxy, benzyloxy, halogen, hydroxy, oxo, carboxy, cyano, nitro, carboxamide, amidino or guanidino;
or R_{b} is aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or R_{b} is amino wherein the nitrogen atom may be independently mono or di-substituted by C1-10 alkyl,C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or R_{b} is a C1-10 saturated or unsaturated alkyl group wherein one or more C atoms are optionally replaced by O, NH, S(O), S(O)₂ or S and wherein said alkyl group is optionally independently substituted with one or two oxo groups, or one or more: -NH₂, C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
R₂, R₃, R₅, and R₆ independently are hydrogen, or a C1-5 alkyl group;
R₂ and R₃ together with the carbon to which they are attached, and/or R₅ and R₆ together with the carbon to which they are attached, each may independently optionally form a nonaromatic 3-6 membered cycloalkyl;
R₄ is hydrogen, C2-10alkenyl, C3-8 cycloalkyl, arylC1-10alkyl, aryl or a C1-10 alkyl group wherein one or more of the C atoms are optionally replaced by O, NH, -C(=O)-, S, S(O) or S(O)₂; wherein R₄ is optionally substituted by one or more Rₑ; or R₄ is Rₑ;
Rₑ is C1-10 alkyl, C3-8 cycloalkyl, aryl, indanyl, indenyl, bicyclo[2.2.1]heptanyl, bicyclo[2.2.2]octanyl, bicyclo[4.1.0]heptanyl, bicyclo[3.1.0]hexanyl, bicyclo[1.1.1]pentanyl, cubanyl, 1,2,3,4-tetrahydronaphthyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl; C1-10 alkoxy, aryloxy, C1-10 alkanoyl, aroyl, C1-10 alkoxycarbonyl, aryloxycarbonyl, C1-10 alkanoyloxy, or aroyloxy;
or Rₑ is carbamoyl wherein the nitrogen atom may be independently mono or di-substituted by C1-10 alkyl, aryl, indanyl, indenyl, tetrahydronaphthyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₑ is C1-10 alkanoylamino, aroylamino, C1-10 alkylthio wherein the sulfur atom may be oxidized to a sulfoxide or sulfone, arylthio wherein the sulfur atom may be oxidized to a sulfoxide or sulfone, ureido wherein either nitrogen atom may be independently substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₑ is C1-10 alkoxycarbonylamino, aryloxycarbonylamino, C1-10 alkylcarbamoyloxy, arylcarbamoyloxy, C1-10 alkylsulfonylamino, arylsulfonylamino, C1-10 alkylaminosulfonyl, arylaminosulfonyl, amino wherein the nitrogen atom may be independently mono or di-substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, pyrrolidinyl, piperidinyl, morpholinyl, thiomorpholinyl, piperazinyl, indolinyl, pyranyl, thiopyranyl, furanyl, tetrahydropyranyl, tetrahydrothiopyranyl, tetrahydrofuranyl, thienyl, pyrrolyl, pyrazolyl, imidazolyl, oxazolyl, isoxazolyl, thiazolyl, pyridinyl, pyrimidinyl, pyrazinyl, indolyl, benzofuranyl, benzothienyl, benzimidazolyl, benzothiazolyl, benzoxazolyl, quinolyl or isoquinolyl;
or Rₑ is amino, halogen, hydroxy, oxo, carboxy, cyano, amidino or guanidino; Each Rₑ may be further optionally substituted by one or more R_{f};
R_{f} is C1-5 alkyl, C3-6 cycloalkyl, aryl, arylC1-4-alkyl, C1-5 alkoxy, aryloxy, arylC1-5alkoxy, aroyl, halogen, hydroxy, oxo or cyano;
Q is R_{g}, C(O)R_{g}, S(O)R_{g} or S(O)₂R_{g};
wherein R_{g} is C2-10alkenyl, C1-10 alkoxy, aryloxy, C3-8 cycloalkyl, aryl, arylC1-10alkyl, C1-10 alkyl wherein one or more of the C atoms are optionally replaced by O, NH, -C(=O)-, S, S(O) or S(O)₂ indenyl, indanyl, bicyclo[2.2.1]hentanyl, bicyclo[2.2.2]octanyl, bicyclo[4.1.0]heptanyl, bicyclo[3.1.0]hexanyl, bicyclo[1.1.1]pentanyl, cubanyl, tetrahydronaphthyl, C1-10alkylsulfonylC1-10alkyl, C3-8cycloalkylsulfonylC1-10alkyl, arylsulfonylC1-10alkyl, azetidinyl, pyrrolidinyl, piperidinyl, morpholinyl, thiomorpholinyl, piperazinyl, perhydroazepinyl, perhydrodiazepinyl, indolinyl, isoindolinyl, pyranyl, tetrahydropyranyl, thiopyranyl, tetrahydrothiopyranyl, furanyl, tetrahydrofuranyl, thienyl, pyrrolyl, oxazolyl, isoxazolyl, thiazolyl, imidazolyl, pyrazolyl, pyridinyl, pyrimidinyl, pyrazinyl, pyridazinyl, tetrazolyl, triazolyl, indolyl, benzofuranyl, benzothienyl, dihydrobenzofuranyl, dihydrobenzothienyl, indazolyl, isoindazolyl, benzimidazolyl, benzoxazolyl, benzothiazolyl, benzisoxazolyl, benzisothiazolyl, benzotriazolyl, quinolinyl, tetrahydroquinolinyl, isoquinolinyl, tetrahydroisoquinolinyl, quinazolinyl, tetrahydroquinazolinyl, quinoxalinyl, or amino; wherein R_{g} is optionally substituted by one or more Rₕ;
Rₕ is C1-10 alkyl, C3-8 cycloalkyl, aryl, tetrahydronaphthyl, indenyl, indanyl, azetidinyl, pyrrolidinyl, piperidinyl, morpholinyl, thiomorpholinyl, piperazinyl, perhydroazepinyl, perhydrodiazepinyl, indolinyl, isoindolinyl, pyranyl, tetrahydropyranyl, thiopyranyl, tetrahydrothiopyranyl, furanyl, tetrahydrofuranyl, thienyl, pyrrolyl, oxazolyl, isoxazolyl, thiazolyl, imidazolyl, pyrazolyl, pyridinyl, pyrimidinyl, pyrazinyl, pyridazinyl, tetrazolyl, triazolyl, indolyl, benzofuranyl, benzothienyl, dihydrobenzofuranyl, dihydrobenzothienyl, indazolyl, isoindazolyl, benzimidazolyl, benzoxazolyl, benzothiazolyl, benzisoxazolyl, benzisothiazolyl, benzotriazolyl, benzodioxolyl, quinolinyl, tetrahydroquinolinyl, isoquinolinyl, tetrahydroisoquinolinyl, quinazolinyl, tetrahydroquinazolinyl, quinoxalinyl, C1-10 alkoxy, C1-10alkanoyl, C1-10alkanoyloxy, aryloxy, benzyloxy, C1-10 alkoxycarbonyl, aryloxycarbonyl, aroyloxy, bicyclo[2.2.1]heptanyl, bicyclo[2.2.2]octanyl, bicyclo[4.1.0]heptanyl, bicyclo[3.1.0]hexanyl, bicyclo[1.1.1]pentanyl, or cubanyl,
or Rₕ is carbamoyl wherein the nitrogen atom may be independently mono or di-substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, azetidinyl, pyrrolidinyl, piperidinyl, morpholinyl, thiomorpholinyl, piperazinyl, perhydroazepinyl, perhydrodiazepinyl, indolinyl, isoindolinyl, pyranyl, tetrahydropyranyl, thiopyranyl, tetrahydrothiopyranyl, furanyl, tetrahydrofuranyl, thienyl, pyrrolyl, oxazolyl, isoxazolyl, thiazolyl, imidazolyl, pyrazolyl, pyridinyl, pyrimidinyl, pyrazinyl, pyridazinyl, tetrazolyl, triazolyl, indolyl, benzofuranyl, benzothienyl, dihydrobenzofuranyl, dihydrobenzothienyl, indazolyl, isoindazolyl, benzimidazolyl, benzoxazolyl, benzothiazolyl, benzisoxazolyl, benzisothiazolyl, benzotriazolyl, quinolinyl, tetrahydroquinolinyl, isoquinolinyl, tetrahydroisoquinolinyl, quinazolinyl, tetrahydroquinazolinyl, or quinoxalinyl,
or Rₕ is C1-10 alkanoylamino, aroylamino, C1-10 alkylthio, arylthio wherein the sulfur atom may be oxidized to a sulfoxide or sulfone, or Rₕ is ureido wherein either nitrogen atom may be independently substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, isoindolinyl, azetidinyl, pyrrolidinyl, piperidinyl, morpholinyl, thiomorpholinyl, piperazinyl, perhydroazepinyl, perhydrodiazepinyl, indolinyl, pyranyl, tetrahydropyranyl, thiopyranyl, tetrahydrothiopyranyl, furanyl, tetrahydrofuranyl, thienyl, pyrrolyl, oxazolyl, isoxazolyl, thiazolyl, imidazolyl, pyrazolyl, pyridinyl, pyrimidinyl, pyrazinyl, pyridazinyl, tetrazolyl, triazolyl, indolyl, benzofuranyl, benzothienyl, dihydrobenzofuranyl, dihydrobenzothienyl, indazolyl, isoindazolyl, benzimidazolyl, benzoxazolyl, benzothiazolyl, benzisoxazolyl, benzisothiazolyl, benzotriazolyl, quinolinyl, tetrahydroquinolinyl, isoquinolinyl, tetrahydroisoquinolinyl, quinazolinyl, tetrahydroquinazolinyl, or quinoxalinyl;
or Rₕ is C1-10 alkoxycarbonylamino, aryloxycarbonylamino, C1-10 alkylcarbamoyloxy, arylcarbamoyloxy, C1-10 alkylsulfonylamino, arylsulfonylamino, C1-10 alkylaminosulfonyl, arylaminosulfanyl, amino wherein the nitrogen atom may be independently mono or di-substituted by C1-10 alkyl, C1-10alkoxyC1-10alkyl, C1-10alkylaminoC1-10alkyl, C1-10alkylthioC1-10alkyl wherein the sulfur atom may be oxidized to a sulfoxide or sulfone, C3-8 cycloalkyl, aryl, tetrahydronaphthyl, indenyl, indanyl, isoindolinyl, azetidinyl, pyrrolidinyl, piperidinyl, morpholinyl, thiomorpholinyl, piperazinyl, piperazinylcarbonyl, perhydroazepinyl, perhydrodiazepinyl, indolinyl, pyranyl, tetrahydropyranyl, thiopyranyl, tetrahydrothiopyranyl, furanyl, tetrahydrofuranyl, thienyl, pyrrolyl, oxazolyl, isoxazolyl, thiazolyl, imidazolyl, pyrazolyl, pyridinyl, pyrimidinyl, pyrazinyl, pyridazinyl, tetrazolyl, triazolyl, indolyl, benzofuranyl, benzothienyl, dihydrobenzofuranyl, dihydrobenzothienyl, indazolyl, isoindazolyl, benzimidazolyl, benzoxazolyl, benzothiazolyl, benzisoxazolyl, benzisothiazolyl, benzotriazolyl, quinolinyl, tetrahydroquinolinyl, isoquinolinyl, tetrahydroisoquinolinyl, quinazolinyl, tetrahydroquinazolinyl, quinoxalinyl, halogen, hydroxy, oxo, carboxy, cyano, nitro, amidino or guanidino, Rₕ may be further optionally substituted by one or more Rᵢ;
Rᵢ is C1-10 alkyl, C3-8 cycloalkyl, aryl, arylC1-10alkyl, heterocyclyl, heterocyclylC1-10alkyl, C1-10 alkoxy, C1-10 alkoxycarbonyl, aryloxy, benzyloxy, halogen, hydroxy, oxo, carboxy, cyano, nitro, amidino or guanidino;
or a pharmaceutically acceptable salt, solvate, or tautomer thereof;
with the proviso that when R₁ is hydrogen or C1-10alkyl, and R₂, R₃, R₅ and R₆ are each independently hydrogen or C1-Salkyl, and R₄ is hydrogen or C1-10alkyl, then Q is not C1-10alkyl or C1-10alkoxycarbonyl.

2. A compound according to claim 1, wherein:
R₁ is hydrogen, a C1-6 saturated or unsaturated alkyl group wherein one or two C atoms are optionally replaced by O, NH, S, S(O) or S(O)₂, and wherein said alkyl group is optionally independently substituted with one to three: oxo groups, C1-4 alkyl, C3-6 cycloalkyl, aryl, pyrrolidinyl, piperidinyl, piperazinyl, morpholinyl, indolinyl, pyranyl, thiopyranyl, tetrahydropyranyl, tetrahydrothiopyranyl, pyridinyl or indolyl;
or R₁ is C1-6alkylthioC1-6alkyl wherein the sulfur atom may be oxidized to a sulfoxide or sulfone, C3-6 cycloalkyl, phenyl, naphthyl, phenylsulfonyl, pyridinylsulfonyl, phenyloxyC1-6alkyl, C1-6alkanoyl, or piperidinyl;
each R₁ may be further optionally independently substituted by one or more Rₐ;
Rₐ is a C1-6 saturated or unsaturated alkyl group wherein one or two C atoms are optionally replaced by O or S;
or Rₐ is a C1-6alkoxy, C3-6 cycloalkyl, phenyl, naphthyl, pyrrolidinyl, piperidinyl, morpholinyl, thiomorpholinyl, tetrahydropyranyl, piperazinyl, indolyl, or pyridinyl;
wherein Rₐ may be further optionally substituted by one or more R_{b};
R_{b} is C3-6 cycloalkyl, C1-5 alkoxy, halogen, hydroxy, oxo, carboxy, cyano, nitro or carboxamide;
or R_{b} is phenyl, benzyl or naphthyl;
or R_{b} is amino wherein the nitrogen atom may be independently mono or di-substituted by C1-6 alkyl, phenyl or naphthyl;
or R_{b} is a C1-6 saturated or unsaturated alkyl group wherein one or two C atoms are optionally replaced by O or S, and wherein said alkyl group is optionally independently substituted with one or two oxo group;
R₂, R₃, R₅, and R₆ independently are hydrogen or a C1-5 alkyl group;
R₂ and R₃ together with the carbon to which they are attached, and/or R₅ and R₆ together with the carbon to which they are attached, each may independently optionally form a nonaromatic 3-6 membered cycloalkyl;
R₄ is hydrogen, C3-6 cycloalkyl, phenylC1-3alkyl, naphthylC1-3alkyl, phenyl, naphthyl, pyridyl or a C1-6 alkyl group wherein one or two of the C atoms are optionally replaced by O, S or S(O)₂; wherein R₄ is optionally substituted by one or more Rₑ;
Rₑ is C1-5 alkyl, C3-6 cycloalkyl, phenyl, naphthyl, indanyl or indolyl,
or Rₑ is halogen, hydroxy, oxo, carboxy, cyano, amidino or guanidino;
Each Rₑ may be further optionally substituted by one or more R_{f};
R_{f} is C1-5 alkyl, C3-6 cycloalkyl, C1-5 alkoxy, halogen, hydroxy, oxo or cyano;
Q is R_{g}, C(O)R_{g} or S(O)₂R_{g};
wherein R_{g} is C1-5 alkyl wherein one or more C atoms are optionally replaced by O or NH, C1-5 alkoxy, phenyl, benzyl, naphthyl, pyrrolidinyl, piperidinyl, morpholinyl, thiomorpholinyl, piperazinyl, furanyl, thienyl, oxazolyl, thiazolyl, pyridinyl, benzofuranyl, benzothienyl, quinolinyl, isoquinolinyl, isoindolinyl, tetrahydroisoquinolyl, pyrazinyl, quinoxalinyl or amino; wherein R_{g} is optionally substituted by one or more Rₕ;
Rₕ is C1-5 alkyl, C3-6 cycloalkyl, phenyl, naphthyl, indenyl, indanyl, morpholinyl, thiomorpholinyl, pyridinyl, isoindolinyl, isoquinolinyl, tetrahydroisoquinolinyl or benzodioxolyl, or C1-5alkoxy,
or Rₕ is C1-5 alkylthio,
or Rₕ is amino wherein the nitrogen atom may be independently mono or di-substituted by alkyl, phenyl, naphthyl, piperidinyl, morpholinyl, thiomorpholinyl, piperazinyl or piperazinylcarbonyl, halogen, hydroxy, oxo, carboxy, cyano, nitro, amidino or guanidino,
Rₕ may be further optionally substituted by one or more Rᵢ;
Rᵢ is C1-5 alkyl, C3-6 cycloalkyl, aryl, arylC1-5alkyl, morpholinyl, thiomorpholinyl, C1-5 alkoxy, C1-5 alkoxycarbonyl, halogen, hydroxy, oxo, carboxy, cyano, nitro, amidino or guanidino;
wherein one or more of the amino nitrogens in the amidino or guanidino groups in the compound of formula I may be optionally substituted with C₁₋₃alkyl, phenylC₀₋₃alkyl or C₁₋₃alkoxy;
or a pharmaceutically acceptable salt, solvate, or tautomer thereof.

3. A compound according to claim 1, wherein:
R₁ is hydrogen, a C1-6 saturated alkyl group wherein one C atom is optionally replaced by O, S, S(O), S(O)₂ or NH and wherein said alkyl group is optionally independently substituted with one or two: oxo groups, phenyl, naphthyl, cyclohexyl, pyrrolidinyl, piperidinyl, piperazinyl, morpholinyl, indolinyl, tetrahyropyranyl, pyridinyl or indolyl;
or R₁ is C1-3alkylthioC1-3alkyl wherein the sulfur atom may be oxidized to a sulfoxide, cyclohexyl, phenyl, phenylsulfonyl, pyridinylsulfonyl, phenyloxyC1-4alkyl, C1-6alkanoyl or piperidinyl;
each R₁ may be further optionally independently substituted by one or two Rₐ;
Rₐ is a C1-6 saturated alkyl group;
or Rₐ is a C1-6alkoxy, cyclohexyl, phenyl, naphthyl, pyrrolidinyl, piperidinyl, indolyl, piperazinyl, pyridinyl or tetrahydropyranyl;
wherein Rₐ may be further optionally substituted by one or two R_{b};
R_{b} is C3-6 cycloalkyl, phenyl, benzyl, C1-5 alkoxy, halogen, hydroxy, oxo, carboxy, cyano, nitro or carboxamide;
or R_{b} is a C1-6 saturated or unsaturated alkyl group, or is a C1-4alkoxycarbonyl group;
R₂, R₃, R₅, and R₆ independently are hydrogen or a C1-5 alkyl group;
R₂ and R₃ together with the carbon to which they are attached, and/or R₅ and R₆ together with the carbon to which they are attached, each may independently optionally form a cyclopropyl, cyclobutyl, cyclopentyl or cyclohexyl group;
R₄ is hydrogen, cyclopropyl, cyclobutyl, cyclopentyl, cyclohexyl, phenylC1-3alkyl, naphthylC1-3alkyl, phenyl, naphthyl, pyridinyl or a C1-6 alkyl group where a C atom is optionally replaced by S(O)₂; wherein R₄ is optionally substituted by one or more Rₑ;
Rₑ is C1-3 alkyl, cyclopropyl, cyclobutyl, cyclopentyl, cyclohexyl, phenyl, naphthyl, indanyl or indolyl,
or Rₑ is hydroxy, halogen, oxo, carboxy or cyano;
Q is R_{g}, C(O)R_{g} or S(O)₂ R_{g};
wherein R_{g} is C1-5alkyl wherein one or more C atoms are optionally replaced by O or NH, phenyl, naphthyl, pyrrolidinyl, piperidinyl, morpholinyl, thiomorpholinyl piperazinyl, furanyl, thienyl, thiazolyl, benzofuranyl, benzothienyl, quinolyl, isoquinolyl, isoindolinyl, tetrahydroisoquinolyl, pyrazinyl or quinoxalinyl, or amino; wherein R_{g} is optionally substituted by one or more Rₕ;
Rₕ is C1-5 alkyl, C1-5alkoxy, halogen, phenyl, naphthyl, indenyl, indanyl, morpholinyl, thiomorpholinyl, pyridinyl, isoquinolinyl, isoindolinyl, tetrahydroisoquinolyl benzodioxolyl, piperidinylamino or piperazinylcarbonylamino;
Rₕ may be further optionally substituted by one or more Rᵢ;
Rᵢ is C1-3 alkyl, C3-6 cycloalkyl, phenyl, naphthyl, benzyl, morpholinyl, C1-5 alkoxy, C1-5 alkoxycarbonyl, halogen, hydroxy, oxo, carboxy, cyano or nitro;
or a pharmaceutically acceptable salt, solvate, or tautomer thereof.

4. A compound according to claim 1, wherein:
R₁ is hydrogen, C1-6alkyl, C1-6alkanoyl, phenylsulfonyl or pyridylsulfonyl each optionally substituted by cyclohexyl, phenyl, pyrrolidinyl, piperidinyl, indolyl, piperazinyl, pyridinyl, tetrahydropyranyl or naphthyl, or R₁ is benzyloxylC1-3alkyl or benzyloxyC1-3alkanoyl;
R₂, R₃, R₅, and R₆ independently are hydrogen or C1-3 alkyl;
R₂ and R₃ together with the carbon to which they are attached, and/or R₅ and R₆ together with the carbon to which they are attached, each may independently optionally form a cyclopropyl, cyclobutyl, cyclopentyl or cyclohexyl group;
R₄ is hydrogen, a C1-6 alkyl group, phenylC1-6alkyl, cyclopropylC1-6alkyl, cyclohexylC1-6alkyl or pyridinyl;
Q is C(O)R_{g};
wherein R_{g} is piperidinyl, morpholinyl, thiomorpholinyl, piperazinyl, isoindolinyl, benzofuranyl, tetrahydroisoquinolyl, phenylC1-5alkylamino or naphthylC1-5alkylamino, each optionally substituted by C1-5alkyl, morpholinyl or morpholinylC1-5alkoxy;
or a pharmaceutically acceptable salt, solvate, or tautomer thereof.

5. A compound according to claim 1, wherein:
R₁ is C1-6alkanoyl optionally substituted by cyclohexyl or phenyl;
R₂, R₃, R₅, and R₆ are hydrogen;
R₄ is a C1-6 saturated alkyl group;
Q is C(O)R_{g};
wherein R_{g} is morpholinyl, tetrahydroisoquinolyl or phenylC1-3alkylamino;
or a pharmaceutically acceptable salt, solvate, or tautomer thereof.

6. A compound according to claim 1 having the following formula (Ia): 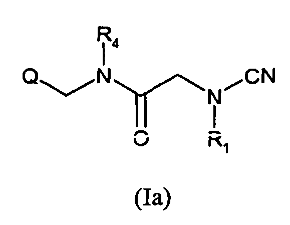 wherein Q is independently selected from groups A1 to A34; R₄ is independently selected from groups B1 to B26; and R₁ is independently selected from groups C1 to C41; wherein groups A1 to A34, B1 to B26 and C1 to C41 are as defined in the following Table:
| **A** | **Q** | **B** | **R₄** | **C** | **R₁** |
|---|---|---|---|---|---|
| A1 | 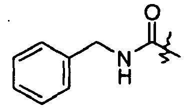 | B1 | -H | C1 | -H |
| A2 | 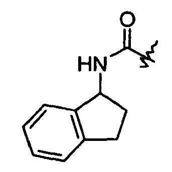 | B2 | -CH₃ | C2 | 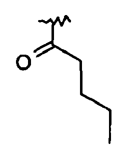 |
| A3 | 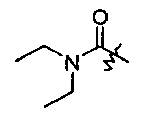 | B3 | 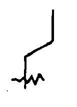 | C3 | 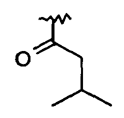 |
| A4 | 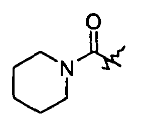 | B4 | 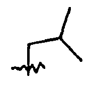 | C4 | 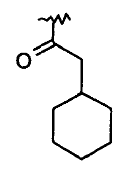 |
| A5 | 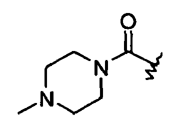 | B5 | 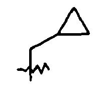 | C5 | 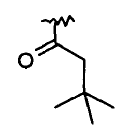 |
| A6 | 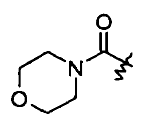 | B6 | 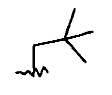 | C6 | 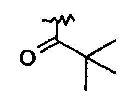 |
| A7 | 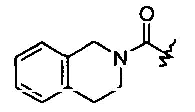 | B7 | 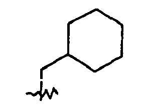 | C7 | 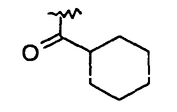 |
| A8 | 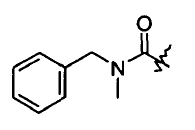 | B8 | 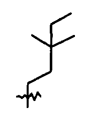 | C8 | 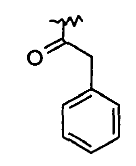 |
| A9 | 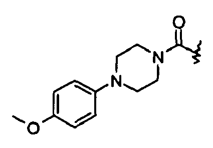 | B9 | 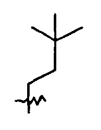 | C9 | 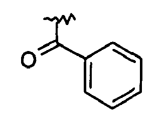 |
| A10 | 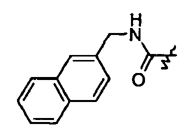 | B10 | 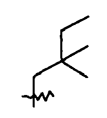 | C10 | 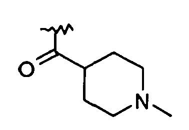 |
| A11 | 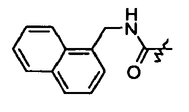 | B11 | 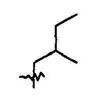 | C11 | 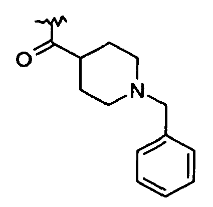 |
| A12 | 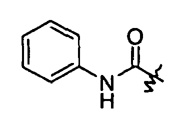 | B12 | 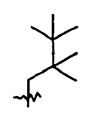 | C12 | 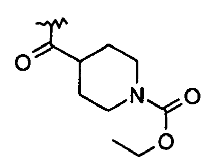 |
| A13 | 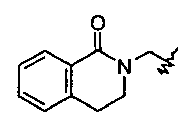 | B13 | 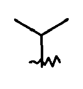 | C13 | 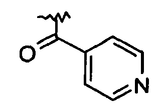 |
| A14 | 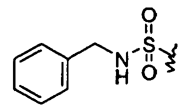 | B14 | 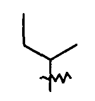 | C14 | 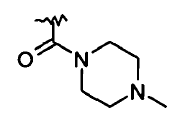 |
| A15 | 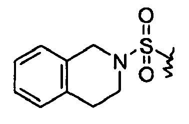 | B15 | 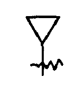 | C15 | 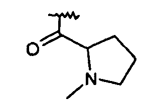 |
| A16 | 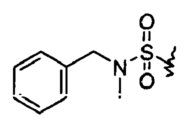 | B16 | 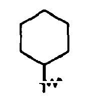 | C16 | 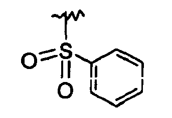 |
| A17 | 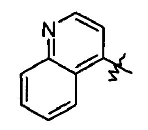 | B17 | 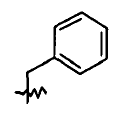 | C17 | 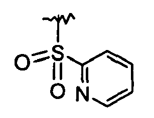 |
| A18 | 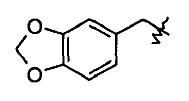 | B18 | 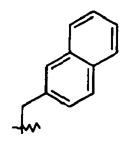 | C18 | -CH₃ |
| A19 | 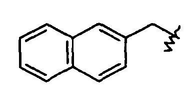 | B19 | 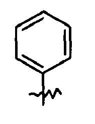 | C19 | 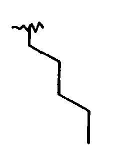 |
| A20 | 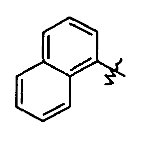 | B20 | 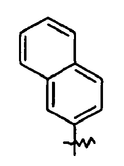 | C20 | 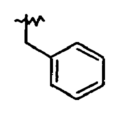 |
| A21 | 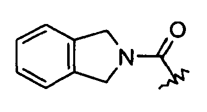 | B21 | 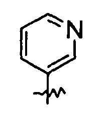 | C21 | 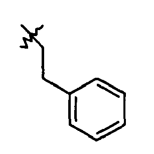 |
| A22 | 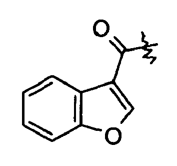 | B22 | 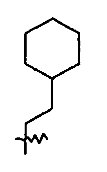 | C22 | 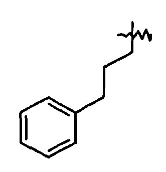 |
| A23 | 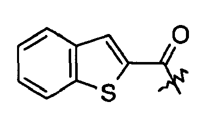 | B23 | 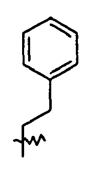 | C23 | 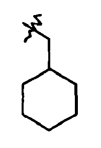 |
| A24 | 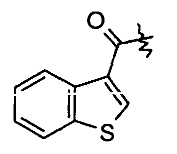 | B24 | 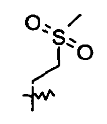 | C24 | 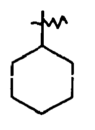 |
| A25 | 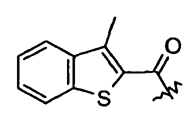 | B25 | 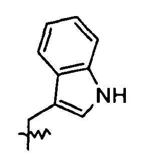 | C25 | 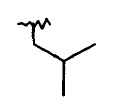 |
| A26 | 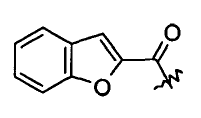 | B26 | 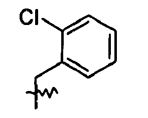 | C26 | 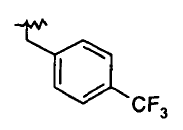 |
| A27 | 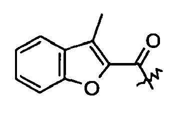 | | | C27 | 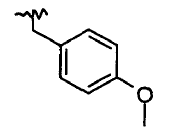 |
| A28 | 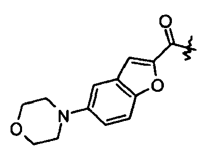 | | | C28 | 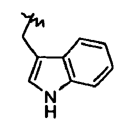 |
| A29 | 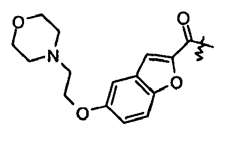 | | | C29 | 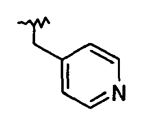 |
| A30 | 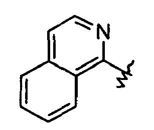 | | | C30 | 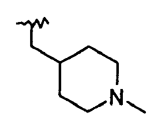 |
| A31 | 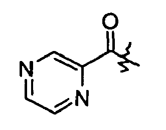 | | | C31 | 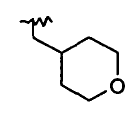 |
| A32 | 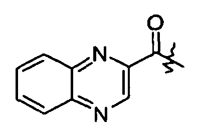 | | | C32 | 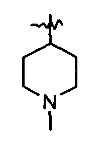 |
| A33 | 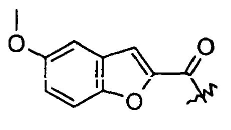 | | | C33 | 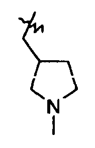 |
| A34 | 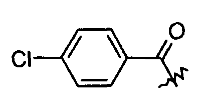 | | | C34 | 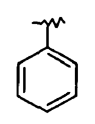 |
| | | | | C35 | 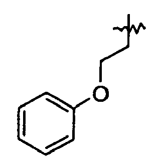 |
| | | | | C36 | 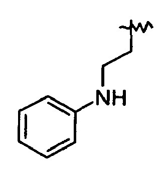 |
| | | | | C37 | 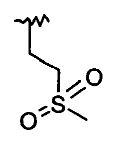 |
| | | | | C38 | 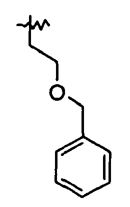 |
| | | | | C39 | 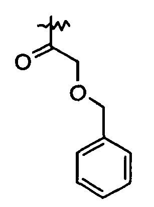 |
| | | | | C40 | 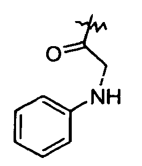 |
| | | | | C41 | 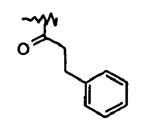 |
or a pharmaceutically acceptable salt, solvate, or tautomer thereof.

7. A compound according to claim 6, wherein:
R₁ is selected from the following groups: 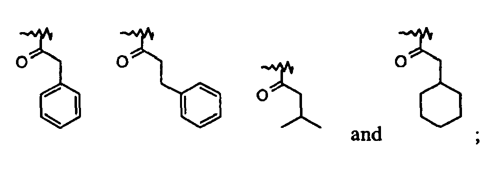
R₄ is selected from the following groups: 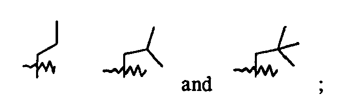
and Q is selected from the following groups: 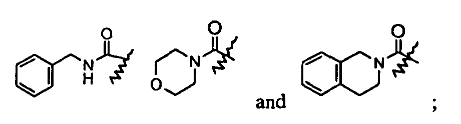 or a pharmaceutically acceptable salt, solvate, or tautomer thereof.

8. A compound according to claim 1 selected from the following compounds:

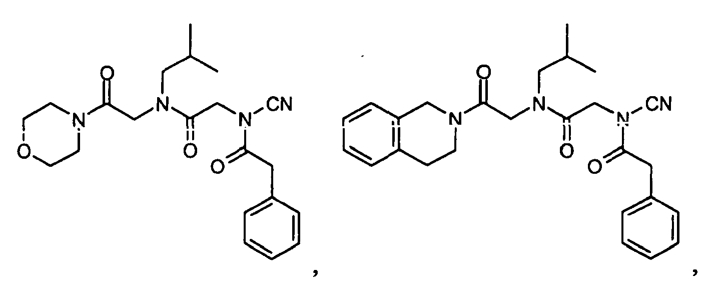

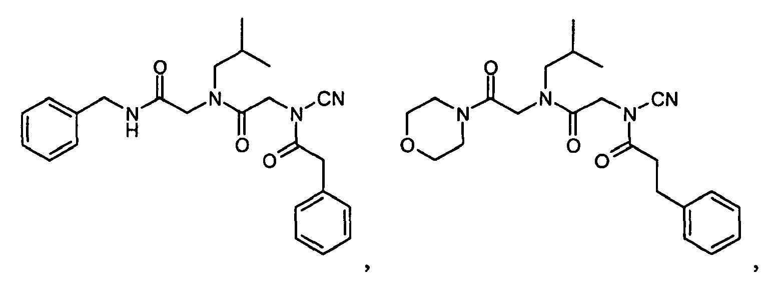

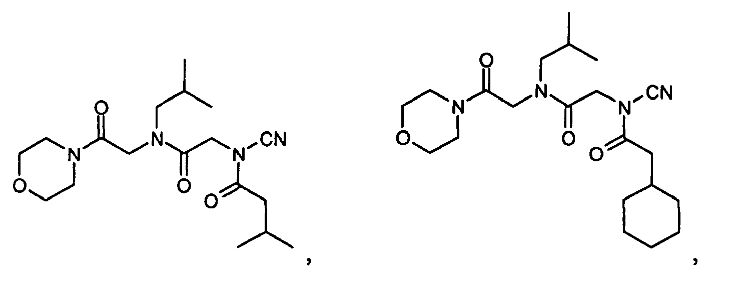

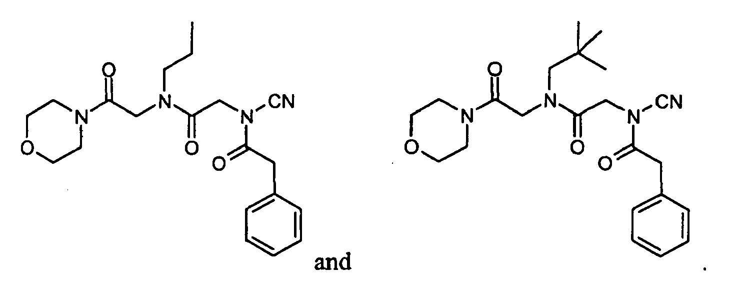

9. A process for preparing a compound of formula (I) according to claim 1, said process comprising reacting a compound of formula V with a cyanamide of formula VIII in the presence of a suitable base to obtain a compound of formula (I): 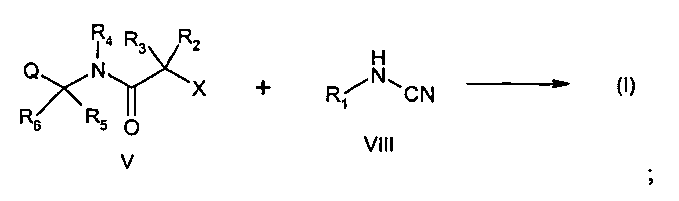 wherein R₁, R₂, R₃, R₄, R₅, R₄ and Q are as defined in claim 1, and X is a leaving group.

10. A process according to claim 9, wherein the compound of formula (V) is prepared by a process comprising:
reacting the compound of formula (III) with a compound of formula (IV) where X is a leaving group to obtain a compound of formula (V): 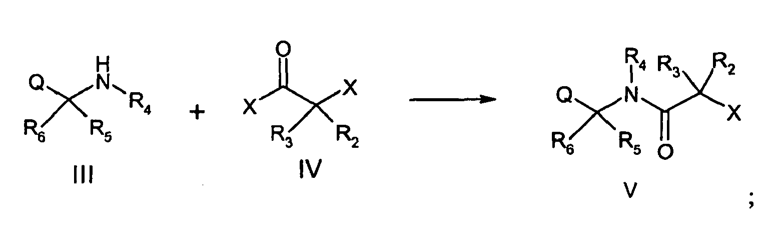
wherein R₂, R₃, R₄, R₅, R₆ and Q are as defined in claim 9.

11. A process according to claim 10, wherein the compound of formula III is prepared by a process comprising:
(a) reacting a compound of formula II where X is a leaving group with a compound of the formula H₂N-R₄ to obtain a compound of formula III: 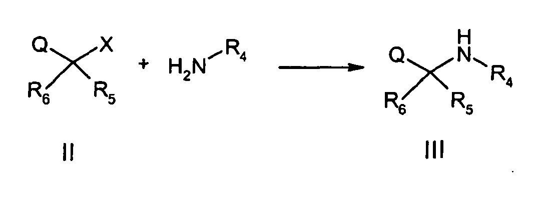 wherein R₄, R₅, R₆ and Q are as defined in claim 10;
or
(b) reacting a compound of the formula IX with an aldehyde of the formula R'CHO under reductive amination conditions to provide a compound of the formula III: 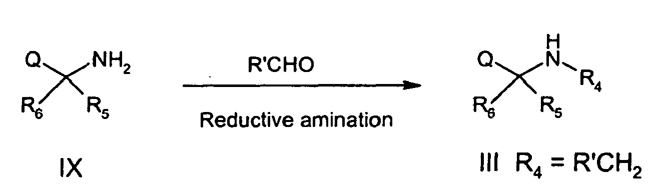
wherein R₅, R₆ and Q are as defined in claim 10, and R₄ is selected from an R₄ group as defined in claim 10 having a -CH₂- group bonded to the nitrogen atom in the backbone of formula (III), and R' is that terminal portion of said R₄ group which is bonded to said -CH₂- group.

12. A process for preparing a compound of formula (I) according to claim 1, said process comprising reacting a compound of formula (XIII) with a compound R₁X where X is a leaving group in the presence of a suitable base to obtain a compound of formula (I): 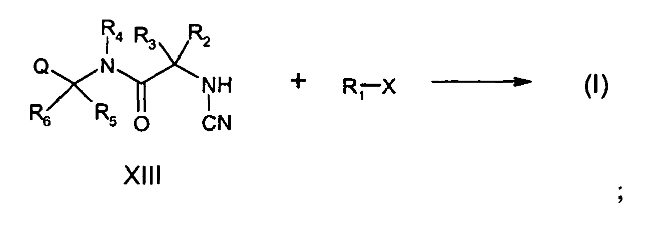 wherein R₁, R₂, R₃, R₄, R₅, R₆ and Q are as defined in claim 1.

13. A process according to claim 12, wherein the compound of formula (XIII) is prepared by a process comprising:
(a) reacting a compound of formula (III) with a compound of formula (X) wherein PG is a protecting group and X is a leaving group to obtain a compound of formula (XI): 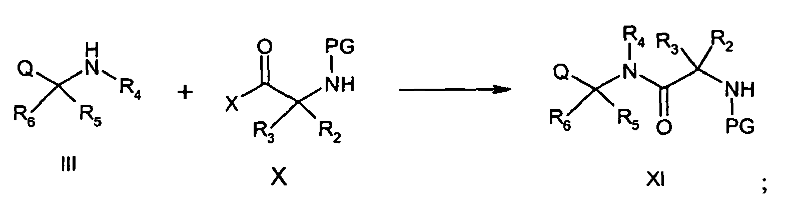
(b) deprotecting a compound of formula (XI) to obtain a compound of formula (XII): 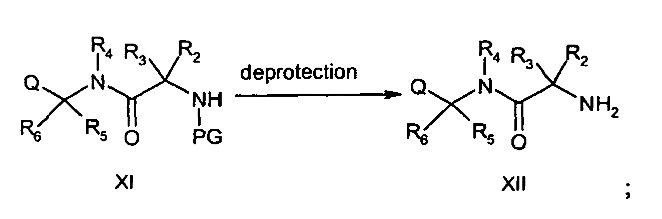
(c) reacting a compound formula (XII) with cyanogen bromide to obtain a compound of formula (XIII): 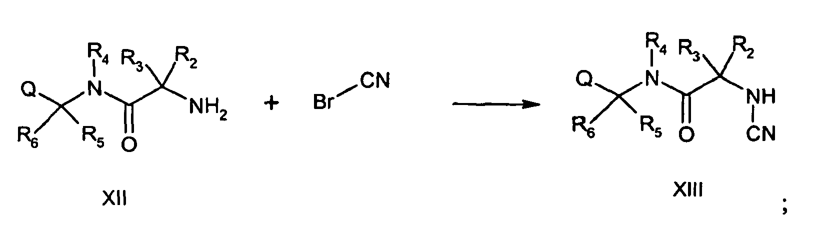
wherein R₂, R₃, R₄, R₅, R₆ and Q are as defined in claim 12.

14. A process according to claim 13, wherein the compound of formula III is prepared by a process comprising:
(a) reacting a compound of formula II where X is a leaving group with a compound of the formula H₂N-R₄ to obtain a compound of formula III: 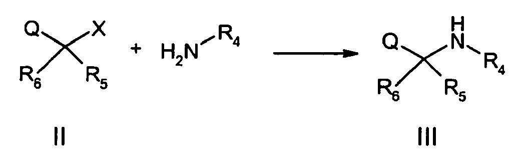 wherein R₄, R₅, R₆ and Q are as defined in claim 13;
or
(b) reacting a compound of the formula IX with an aldehyde of the formula R'CHO under reductive amination conditions to provide a compound of the formula III: 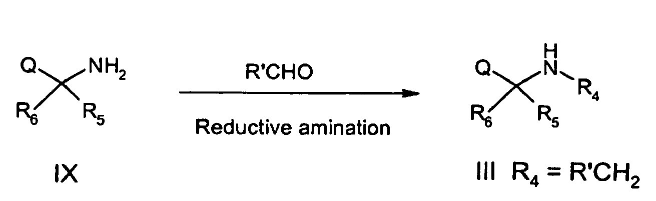
wherein R₅, R₆ and Q are as defined in claim 13, and R₄ is selected from an R₄ group as defined in claim 13 having a -CH₂- group bonded to the nitrogen atom in the backbone of formula (III), and R' is that terminal portion of said R₄ group which is bonded to said CH₂- group.

15. A pharmaceutical composition comprising a therapeutically effective amount of a compound according to one of claims 1 to 8, or a pharmaceutically acceptable salt, solvate, or tautomer thereof.

16. A compound according to one of claims 1 to 8 for use as a medicament.

17. Use of a compound according to one of claims 1 to 8 for preparing a pharmaceutical composition for treating a disease mediated by cathepsin S.

18. Use of a compound according to one of claims 1 to 8 for preparing a pharmaceutical composition for treating a disease mediated by cathepsin K.

## Patentansprüche

1. Verbindung der Formel (I): 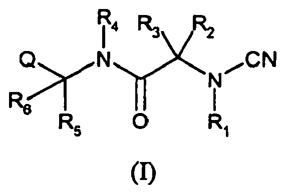 worin:
R₁ ist: Wasserstoff, eine gesättigte oder ungesättigte C₁₋₁₀-Alkylgruppe, worin ein oder mehrere C-Atome gegebenenfalls ersetzt sind durch O, NH, S(O), S(O)₂ oder S, und worin die Alkylgruppe gegebenenfalls unabhängig substituiert ist mit ein oder mehreren von: Oxogruppen, -NH₂, C₁₋₁₀-Alkyl, C₃₋₈-Cycloalkyl, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Tetrahydropyranyl, Thiopyranyl, Tetrahydrothiopyranyl, Furanyl, Tetrahydrofuranyl, Thienyl, Pyrrolyl, Pyrazolyl, Oxazolyl, Isoxazolyl, Thiazolyl, Imidazolylpyridinyl, Pyrimidinyl, Pyrazinyl, Indolyl, Benzofuranyl, Benzothienyl, Benzimidazolyl, Benzothiazolyl, Benzoxazolyl, Chinolyl, Isochinolyl, C₁₋₁₀-Alkoxy, Aryloxy, C₁₋₁₀-Alkanoyl, Aroyl, C₁₋₁₀-Alkoxycarbonyl, Aryloxycarbonyl, C₁₋₁₀-Alkanoyloxy oder Aroyloxy,
oder R₁ ist: C₁₋₁₀-Alkoxy-C₁₋₁₀-alkyl, C₁₋₁₀-Alkylamino-C₁₋₁₀-alkyl, C₁₋₁₀-Alkylthio-C₁₋₁₀-alkyl, worin das Schwefelatom zu einem Sulfoxid oder Sulfon oxidiert sein kann, C₃₋₈-Cycloalkyl, Aryl, Arylsulfonyl, Heteroarylsulfonyl, Tetrahydronaphthyl, Indenyl, Indanyl, Tetrahydropyranyl, Tetrahydrothiopyranyl, Tetrahydrofuranyl, Pyrrolidinyl, Piperidinyl, Morpholinyl, Thiomorpholinyl, Indolinyl, C₃₋₈-Cycloalkylsulfonyl-C₁₋₅-alkyl, Arylsulfonyl-C₁₋₅-alkyl, Aryloxy-C₁₋₅-alkyl, C₁₋₁₀-Alkanoyl, Aroyl, C₁₋₁₀-Alkoxycarbonyl, Aryl-C₁₋₅-Alkoxycarbonyl oder Aryloxycarbonyl,
oder R₁ ist Carbamoyl, worin das Stickstoffatom unabhängig mono- oder disubstituiert sein kann mit C₁₋₁₀-Alkyl, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
jedes R₁ kann weiterhin gegebenenfalls unabhängig substituiert sein mit einem oder mehreren Rₐ;
Rₐ ist eine gesättigte oder ungesättigte C₁₋₁₀-Alkylgruppe, worin ein oder mehrere C-Atome gegebenenfalls mit O, NH, S(O), S(O)₂ oder S ersetzt sind, und worin die Alkylgruppe gegebenenfalls unabhängig mit ein oder zwei Oxogruppen substituiert ist, oder ein oder mehrere von: -NH₂, C₁₋₁₀-Alkyl, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
oder Rₐ ist ein C₁₋₁₀-Alkoxy, C₁₋₁₀-Alkoxy-C₁₋₁₀-alkyl, C₁₋₁₀-Alkylamino-C₁₋₁₀-alkyl, C₁₋₁₀-Alkylthio-C₁₋₁₀-alkyl, worin das Schwefelatom zu einem Sulfoxid oder Sulfon oxidiert sein kann, C₃₋₈-Cycloalkyl, C₃₋₈-Cycloalkyloxy, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl; C₁₋₁₀-Alkanoyl, Aroyl, C₁₋₁₀-Alkanoyloxy, Aryloxy, Benzyloxy, C₁₋₁₀-Alkoxycarbonyl, Aryl-C₁₋₃-alkoxycarbonyl, Aryloxycarbonyl oder Aroyloxy;
oder Rₐ ist Carbamoyl, worin das Stickstoffatom unabhängig mono- oder disubstituiert sein kann mit C₁₋₁₀-Alkyl, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
oder Rₐ ist Ureido, worin jedes Stickstoffatom unabhängig substituiert sein kann mit C₁₋₁₀-Alkyl, C₁₋₁₀-Alkoxy-C₁₋₁₀-alkyl, C₁₋₁₀-Alkylamino-C₁₋₁₀-alkyl, C₁₋₁₀-Alkylthio-C₁₋₁₀-alkyl, worin das Schwefelatom zu einem Sulfoxid oder Sulfon oxidiert sein kann, C₃₋₈-Cycloalkyl, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
worin Rₐ Amino ist, worin das Stickstoffatom unabhängig mono- oder disubstituiert sein kann mit C₁₋₁₀-Alkyl, C₁₋₁₀-Alkoxy-C₁₋₁₀-alkyl, C₁₋₁₀-Alkylamino-C₁₋₁₀-alkyl, C₁₋₁₀-Alkylthio-C₁₋₁₀-alkyl, worin das Schwefelatom zu einem Sulfoxid oder Sulfon oxidiert sein kann, C₃₋₈-Cycloalkyl, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
worin Rₐ weiterhin gegebenenfalls mit einem oder mehreren R_{b} substituiert sein kann;
R_{b} ist C₃₋₈-Cycloalkyl, tolylsulfonyl, C₁₋₅-Alkoxy, Aryl, Aryloxy, Benzyloxy, Halogen, Hydroxy, Oxo, Carboxy, Cyano, Nitro, Carboxamid, Amidino oder Guanidino;
oder R_{b} ist Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
oder R_{b} ist Amino, worin das Stickstoffatom unabhängig mono- oder disubstituiert ist mit C₁₋₁₀-Alkyl, C₁₋₁₀-Alkoxy-C₁₋₁₀-alkyl, C₁₋₁₀-Alkylamino-C₁₋₁₀-alkyl, C₁₋₁₀-Alkylthio-C₁₋₁₀-alkyl, worin das Schwefelatom zu einem Sulfoxid oder Sulfon oxidiert sein kann, C₃₋₈-Cycloalkyl, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
oder R_{b} ist eine gesättigte oder ungesättigte C₁₋₁₀-Alkylgruppe, worin ein oder mehrere C-Atome gegebenenfalls ersetzt sind durch O, NH, S(O), S(O)₂ oder S, und worin die Alkylgruppe gegebenenfalls unabhängig substituiert ist mit einer oder zwei Oxogruppen oder ein oder mehreren von: -NH₂, C₁₋₁₀-Alkyl, C₁₋₁₀-Alkoxy-C₁₋₁₀-alkyl, C₁₋₁₀-Alkylamino-C₁₋₁₀-alkyl, C₁₋₁₀-Alkylthio-C₁₋₁₀-alkyl, worin das Schwefelatom zu einem Sulfoxid oder Sulfon oxidiert sein kann, C₃₋₈-Cycloalkyl, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
R₂, R₃, R₅ und R₆ sind unabhängig Wasserstoff oder eine C₁₋₅-Alkylgruppe;
R₂ und R₃ können zusammen mit dem Kohlenstoff, an den sie gebunden sind, und/oder R₅ und R₆ können zusammen mit dem Kohlenstoff, an den sie gebunden sind, jeweils unabhängig gegebenenfalls ein nicht-aromatisches 3- bis 6-gliedriges Cycloalkyl bilden;
R₄ ist Wasserstoff, C₂₋₁₀-Alkenyl, C₃₋₈-Cycloalkyl, Aryl-C₁₋₁₀-alkyl, Aryl oder eine C₁₋₁₀-Alkylgruppe, worin ein oder mehrere der C-Atome gegebenenfalls mit O, NH, C(=O)-, S, S(O) oder S(O)₂ ersetzt sind; worin R₄ gegebenenfalls mit einem oder mehreren Rₑ substituiert ist; oder R₄ ist Rₑ;
Rₑ ist: C₁₋₁₀-Alkyl, C₃₋₈-Cycloalkyl, Aryl, Indanyl, Indenyl, Bicyclo[2.2.1]-heptanyl, Bicyclo[2.2.2]octanyl, Bicyclo[4.1.0]heptanyl, Bicyclo[3.1.0]hexanyl, Bicyclo[1.1.1]pentanyl, Cubanyl, 1,2,3,4-Tetrahydronaphthyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl; C₁₋₁₀-Alkoxy, Aryloxy, C₁₋₁₀-Alkanoyl, Aroyl, C₁₋₁₀-Alkoxycarbonyl, Aryloxycarbonyl, C₁₋₁₀-Alkanoyloxy oder Aroyloxy;
oder Rₑ ist Carbamoyl, worin das Stickstoffatom unabhängig mono- oder disubstituiert ist mit C₁₋₁₀-Alkyl, Aryl, Indanyl, Indenyl, Tetrahydronaphthyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
oder Rₑ ist C₁₋₁₀-Alkanoylamino, Aroylamino, C₁₋₁₀-Alkylthio, worin das Schwefelatom zu einem Sulfoxid oder Sulfon oxidiert sein kann, Arylthio, worin das Schwefelatom zu einem Sulfoxid oder Sulfon oxidiert sein kann, Ureido, worin jedes Stickstoffatom unabhängig substituiert sein kann mit C₁₋₁₀-Alkyl, C₁₋₁₀-Alkoxy-C₁₋₁₀-alkyl, C₁₋₁₀- Alkylamino-C₁₋₁₀-alkyl, C₁₋₁₀- Alkylthio-C₁₋₁₀-alkyl, worin das Schwefelatom zu einem Sulfoxid oder Sulfon oxidiert sein kann, C₃₋₈-Cycloalkyl, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
oder Rₑ ist C₁₋₁₀-Alkoxycarbonylamino, Aryloxycarbonylamino, C₁₋₁₀-Alkylcarbamoyloxy, Arylcarbamoyloxy, C₁₋₁₀-Alkylsulfonylamino, Arylsulfonylamino, C₁₋₁₀-Alkylaminosulfonyl, Arylaminosulfonyl, Amino, worin das Stickstoffatom unabhängig mono- oder disubstituiert sein kann mit C₁₋₁₀-Alkyl, C₁₋₁₀-Alkoxy-C₁₋₁₀-alkyl, C₁₋₁₀-Alkylamino-C₁₋₁₀-alkyl, C₁₋₁₀-Alkylthio-C₁₋₁₀-alkyl, worin das Schwefelatom zu einem Sulfoxid oder Sulfon oxidiert sein kann, C₃₋₈-Cycloalkyl, Aryl, Tetrahydronaphthyl, Indenyl, Indanyl, Pyrrolidinyl, Piperidinyl, Morpholinyl, Thiomorpholinyl, Piperazinyl, Indolinyl, Pyranyl, Thiopyranyl, Furanyl, Tetrahydropyranyl, Tetrahydrothiopyranyl, Tetrahydrofuranyl, Thienyl, Pyrrolyl, Pyrazolyl, Imidazolyl, Oxazolyl, Isoxazolyl, Thiazolyl, Pyridinyl, Pyrimidinyl, Pyrazinyl, Indolyl, Benzofuranyl, Benzothienyl, Benzimidazolyl, Benzothiazolyl, Benzoxazolyl, Chinolyl oder Isochinolyl;
oder Rₑ ist Amino, Halogen, Hydroxy, Oxo, Carboxy, Cyano, Amidino oder Guanidino;
jedes Rₑ kann weiterhin gegebenenfalls mit einem oder mehreren R_{f} substituiert sein;
R_{f} ist C₁₋₅-Alkyl, C₃₋₆-Cycloalkyl, Aryl, Aryl-C₁₋₄-alkyl, C₁₋₅-Alkoxy, Aryloxy, Aryl-C₁₋₅-alkoxy, Aroyl, Halogen, Hydroxy, Oxo oder Cyano;
Q ist R_{g}, C(O)R_{g}, S(O)R_{g} oder S(O)₂R_{g};
worin R_{g} ist: C₂₋₁₀-Alkenyl, C₁₋₁₀-Alkoxy, Aryloxy, C₃₋₈-Cycloalkyl, Aryl, Aryl-C₁₋₁₀-alkyl, C₁₋₁₀-Alkyl, worin ein oder mehr der C-Atome gegebenenenfalls mit O, NH, -C(=O)-, S-, S(O) oder S(O)₂ ersetzt sind; Indanyl, Indenyl, Bicyclo[2.2.1]-heptanyl, Bicyclo[2.2.2]octanyl, Bicyclo[4.1.0]heptanyl, Bicyclo[3.1.0]hexanyl, Bicyclo[1.1.1]pentanyl, Cubanyl, Tetrahydronaphthyl, C₁₋₁₀- Alkylsulfonyl-C₁₋₁₀-alkyl, C₃₋₈-Cycloalkylsulfonyl-C₁₋₁₀-alkyl, Arylsulfonyl-C₁₋₁₀-alkyl, Azetidinyl, Pyrrolidinyl, Piperidinyl, Morpholinyl, Thiomorpholinyl, Piperazinyl, Perhydroazepinyl, Perhydrodiazepinyl, Indolinyl, Isoindolinyl, Pyranyl, Tetrahydropyranyl, Thiopyranyl, Tetrahydrothiopyranyl, Furanyl, Tetrahydrofuranyl, Thienyl, Pyrrolyl, Oxazolyl, Isoxazolyl, Thiazolyl, Imidazolyl, Pyrazolyl, Pyridinyl, Pyrimidinyl, Pyrazinyl, Pyridazinyl, Tetrazolyl, Triazolyl, Indolyl, Benzofuranyl, Benzothienyl, Dihydrobenzofuranyl, Dihydrobenzothienyl, Indazolyl, Isoindazolyl, Benzimidazolyl, Benzoxazolyl, Benzothiazolyl, Benzisoxazolyl, Benzisothiazolyl, Benzotriazolyl, Chinolyl, Tetrahydrochinolinyl, Isochinolinyl, Tetrahydroisochinolinyl, Chinazolinyl, Tetrahydrochinazolinyl, Chinoxalinyl oder Amino;
worin R_{g} gegebenenfalls substituiert ist mit einem oder mehreren Rₕ;
Rₕ ist C₁₋₁₀-Alkyl, C₃₋₈-Cycloalkyl, Aryl, Tetrahydronaphthyl, Indenyl, Indanyl, Azetidinyl, Pyrrolidinyl, Piperidinyl, Morpholinyl, Thiomorpholinyl, Piperazinyl, Perhydroazepinyl, Perhydrodiazepinyl, Indolinyl, Isoindolinyl, Pyranyl, Tetrahydropyranyl, Thiopyranyl, Tetrahydrothiopyranyl, Furanyl, Tetrahydrofuranyl, Thienyl, Pyrrolyl, Oxazolyl, Isoxazolyl, Thiazolyl, Imidazolyl, Pyrazolyl, Pyridinyl, Pyrimidinyl, Pyrazinyl, Pyridazinyl, Tetrazolyl, Triazolyl, Indolyl, Benzofuranyl, Benzothienyl, Dihydrobenzofuranyl, Dihydrobenzothienyl, Indazolyl, Isoindazolyl, Benzimidazolyl, Benzoxazolyl, Benzothiazolyl, Benzisoxazolyl, Benzisothiazolyl, Benzotriazolyl, Benzodioxolyl, Chinolinyl, Tetrahydrochinolinyl, Isochinolinyl, Tetrahydroisochinolinyl, Chinazolinyl, Tetrahydrochinazolinyl, Chinoxalinyl, C₁₋₁₀-Alkoxy, C₁₋₁₀-Alkanoyl, C₁₋₁₀-Alkanoyloxy, Aryloxy, Benzyloxy, C₁₋₁₀-Alkoxycarbonyl, Aryloxycarbonyl, Aroyloxy, Bicyclo-[2.2.1]heptanyl, Bicyclo[2.2.2]octanyl, Bicyclo[4.1.0]heptanyl, Bicyclo[3.1.0]-hexanyl, Bicyclo[1.1.1]pentanyl oder Cubanyl;
oder Rₕ ist Carbamoyl, worin das Stickstoffatom unabhängig mono- oder disubstituiert sein kann mit C₁₋₁₀-Alkyl, C₁₋₁₀-Alkoxy-C₁₋₁₀-alkyl, C₁₋₁₀-Alkylamino-C₁₋₁₀-alkyl, C₁₋₁₀-Alkylthio-C₁₋₁₀-alkyl, worin das Schwefelatom zu einem Sulfoxid oder Sulfon oxidiert sein kann, C₃₋₈-Cycloalkyl, Aryl, Tetrahydronaphthyl, Indenyl, Indanyl, Azetidinyl, Pyrrolidinyl, Piperidinyl, Morpholinyl, Thiomorpholinyl, Piperazinyl, Perhydroazepinyl, Perhydrodiazepinyl, Indolinyl, Isoindolinyl, Pyranyl, Tetrahydropyranyl, Thiopyranyl, Tetrahydrothiopyranyl, Furanyl, Tetrahydrofuranyl, Thienyl, Pyrrolyl, Oxazolyl, Isoxazolyl, Thiazolyl, Imidazolyl, Pyrazolyl, Pyridinyl, Pyrimidinyl, Pyrazinyl, Pyridazinyl, Tetrazolyl, Triazolyl, Indolyl, Benzofuranyl, Benzothienyl, Dihydrobenzofuranyl, Dihydrobenzothienyl, Indazolyl, Isoindazolyl, Benzimidazolyl, Benzoxazolyl, Benzothiazolyl, Benzisoxazolyl, Benzisothiazolyl, Benzotriazolyl, Chinolinyl, Tetrahydrochinolinyl, Isochinolinyl, Tetrahydroisochinolinyl, Chinazolinyl, Tetrahydrochinazolinyl oder Chinoxalinyl;
oder Rₕ ist C₁₋₁₀-Alkanoylamino, Aroylamino, C₁₋₁₀-Alkylthio, Arylthio, worin das Schwefelatom zu Sulfoxid oder Sulfon oxidiert sein kann; oder Rₕ ist Ureido, worin jedes Stickstoffatom unabhängig substituiert sein kann mit C₁₋₁₀-Alkyl, C₁₋₁₀-Alkoxy-C₁₋₁₀-alkyl, C₁₋₁₀-Alkylamino-C₁₋₁₀-alkyl, C₁₋₁₀-Alkylthio-C₁₋₁₀-alkyl, worin das Schwefelatom zu einem Sulfoxid oder Sulfon oxidiert sein kann, C₃₋₈-Cycloalkyl, Aryl, Tetrahydronaphthyl, Indenyl, Indanyl, Isoindolinyl, Azetidinyl, Pyrrolidinyl, Piperidinyl, Morpholinyl, Thiomorpholinyl, Piperazinyl, Perhydroazepinyl, Perhydrodiazepinyl, Indolinyl, Pyranyl, Tetrahydropyranyl, Thiopyranyl, Tetrahydrothiopyranyl, Furanyl, Tetrahydrofuranyl, Thienyl, Pyrrolyl, Oxazolyl, Isoxazolyl, Thiazolyl, Imidazolyl, Pyrazolyl, Pyridinyl, Pyrimidinyl, Pyrazinyl, Pyridazinyl, Tetrazolyl, Triazolyl, Indolyl, Benzofuranyl, Benzothienyl, Dihydrobenzofuranyl, Dihydrobenzothienyl, Indazolyl, Isoindazolyl, Benzimidazolyl, Benzoxazolyl, Benzothiazolyl, Benzisoxazolyl, Benzisothiazolyl, Benzotriazolyl, Chinolinyl, Tetrahydrochinolinyl, Isochinolinyl, Tetrahydroisochinolinyl, Chinazolinyl, Tetrahydrochinazolinyl oder Chinoxalinyl;
oder Rₕ ist C₁₋₁₀-Alkoxycarbonylamino, Aryloxycarbonylamino, C₁₋₁₀-Alkylcarbamoyloxy, Arylcarbamoyloxy, C₁₋₁₀-Alkylsulfonylamino, Arylsulfonylamino, C₁₋₁₀-Alkylaminosulfonyl, Arylaminosulfonyl, Amino, worin das Stickstoffatom unabhängig mono- oder disubstituiert sein kann mit C₁₋₁₀-Alkyl, C₁₋₁₀-Alkoxy-C₁₋₁₀-alkyl, C₁₋₁₀-Alkylamino-C₁₋₁₀-alkyl, C₁₋₁₀-Alkylthio-C₁₋₁₀-alkyl, worin das Schwefelatom zu Sulfoxid oder Sulfon oxidiert sein kann, C₃₋₈-Cycloalkyl, Aryl, Tetrahydronaphthyl, Indenyl, Indanyl, Isoindolinyl, Azetidinyl, Pyrrolidinyl, Piperidinyl, Morpholinyl, Thiomorpholinyl, Piperazinyl, Piperazinylcarbonyl, Perhydroazepinyl, Perhydrodiazepinyl, Indolinyl, Pyranyl, Tetrahydropyranyl, Thiopyranyl, Tetrahydrothiopyranyl, Furanyl, Tetrahydrofuranyl, Thienyl, Pyrrolyl, Oxazolyl, Isoxazolyl, Thiazolyl, Imidazolyl, Pyrazolyl, Pyridinyl, Pyrimidinyl, Pyrazinyl, Pyridazinyl, Tetrazolyl, Triazolyl, Indolyl, Benzofuranyl, Benzothienyl, Dihydrobenzofuranyl, Dihydrobenzothienyl, Indazolyl, Isoindazolyl, Benzimidazolyl, Benzoxazolyl, Benzothiazolyl, Benzisoxazolyl, Benzisothiazolyl, Benzotriazolyl, Chinolinyl, Tetrahydrochinolinyl, Isochinolinyl, Tetrahydroisochinolinyl, Chinazolinyl, Tetrahydrochinazolinyl, Chinoxalinyl, Halogen, Hydroxy, Oxo, Carboxy, Cyano, Nitro, Amidino oder Guanidino;
Rₕ kann weiterhin gegebenenfalls substituiert sein mit einem oder mehreren Rᵢ;
Rᵢ ist C₁₋₁₀-Alkyl, C₃₋₈-Cycloalkyl, Aryl, Aryl-C₁₋₁₀-alkyl, Heterocyclyl, Heterocyclyl-C₁₋₁₀-alkyl, C₁₋₁₀-Alkoxy, C₁₋₁₀-alkoxycarbonyl, Aryloxy, Benzyloxy, Halogen, Hydroxy, Oxo, Carboxy, Cyano, Nitro, Amidino oder Guanidino;
oder ein pharmazeutisch akzeptables Salz, Solvat oder Tautomer hiervon;
mit der Maßgabe, dass wenn R₁ Wasserstoff oder C₁₋₁₀-Alkyl ist, und R₂, R₃, R₅ und R₆ jeweils unabhängig Wasserstoff oder C₁₋₅-Alkyl sind, und R₄ Wasserstoff oder C₁₋₁₀-Alkyl ist, dann ist Q kein C₁₋₁₀-Alkyl oder C₁₋₁₀-Alkoxycarbonyl.

2. Verbindung nach Anspruch 1, worin:
R₁ ist: Wasserstoff, eine gesättigte oder ungestättigte C₁₋₆-Alkylgruppe, worin ein oder zwei C-Atome gegebenenfalls ersetzt sind mit O, NH, S, S(O) oder S(O)₂, und worin die Alkylgruppe gegebenenfalls unabhängig substituiert ist mit ein bis drei von: Oxogruppen, C₁₋₄-Alkyl, C₃₋₆-Cycloalkyl, Aryl, Pyrrolidinyl, Piperidinyl, Piperazinyl, Morpholinyl, Indolinyl, Pyranyl, Thiopyranyl, Tetrahydropyranyl, Tetrahydrothiopyranyl, Pyridinyl oder Indolyl;
oder R₁ ist C₁₋₆-Alkylthio-C₁₋₆-alkyl, worin das Schwefelatom zu einem Sulfoxid oder Sulfon oxidiert sein kann, C₃₋₆-Cycloalkyl, Phenyl, Naphthyl, Phenylsulfonyl, Pyridinylsulfonyl, Phenyloxy-C₁₋₆-alkyl, C₁₋₆-Alkanoyl oder Piperidinyl;
jedes R₁ kann ferner gegebenenfalls unabhängig mit einem oder mehreren Rₐ substituiert sein;
Rₐ ist eine gesättigte oder ungesättigte C₁₋₆-Alkylgruppe, worin ein oder zwei C-Atome gegebenenfalls mit O oder S ersetzt sind;
oder Rₐ ist ein C₁₋₆-Alkoxy, C₃₋₆-Cycloalkyl, Phenyl, Naphthyl, Pyrrolidinyl, Piperidinyl, Morpholinyl, Thiomorpholinyl, Tetrahydropyranyl, Piperazinyl, Indolyl oder Pyridinyl;
worin Rₐ weiterhin gegebenenfalls mit einem oder mehreren R_{b} substituiert sein kann;
R_{b} ist C₃₋₆-Cycloalkyl, C₁₋₅-Alkoxy, Halogen, Hydroxy, Oxo, Carboxy, Cyano, Nitro oder Carboxamid;
oder R_{b} ist Phenyl, Benzyl oder Naphthyl;
oder R_{b} ist Amino, worin das Stickstoffatom unabhängig mono- oder disubstituiert sein kann mit C₁₋₆-Alkyl, Phenyl oder Naphthyl;
oder R_{b} ist eine gesättigte oder ungesättigte C₁₋₆-Alkylgruppe, worin ein oder zwei C-Atome gegebenenfalls durch O oder S ersetzt sind, und worin die Alkylgruppe gegebenenfalls unabhängig mit einer oder zwei Oxogruppen substituiert ist;
R₂, R₃, R₅ und R₆ sind unabhängig Wasserstoff oder eine C₁₋₅-Alkylgruppe;
R₂ und R₃ können zusammen mit dem Kohlenstoff, an den sie gebunden sind, und/oder R₅ und R₆ können zusammen mit dem Kohlenstoff, an den sie gebunden sind, jeweils unabhängig gegebenenfalls ein nicht-aromatisches 3- bis 6-gliedriges Cycloalkyl bilden;
R₄ ist Wasserstoff, C₃₋₆-Cycloalkyl, Phenyl-C₁₋₃-alkyl, Naphthyl-C₁₋₃-alkyl, Phenyl, Naphthyl, Pyridyl oder eine C₁₋₆-Alkylgruppe, worin ein oder zwei C-Atome gegebenenfalls durch O, S oder S(O)₂ ersetzt sind; worin R₄ gegebenenfalls mit einem oder mehreren Rₑ substituiert ist;
Rₑ ist C₁₋₅-Alkyl, C₃₋₆-Cycloalkyl, Phenyl, Naphthyl, Indanyl oder Indolyl;
oder Rₑ ist Halogen, Hydroxy, Oxo, Carboxy, Cyano, Amidino oder Guanidino;
jedes Rₑ kann weiterhin gegebenenfalls mit ein oder mehreren R_{f} substituiert sein;
R_{f} ist C₁₋₅-Alkyl, C₃₋₆-Cycloalkyl, C₁₋₅-Alkoxy, Halogen, Hydroxy, Oxo oder Cyano;
Q ist R_{g}, C(O)R_{g} oder S(O)₂R_{g};
worin R_{g} C₁₋₅-Alkyl ist, worin ein oder mehrere C-Atome gegebenenfalls ersetzt sind durch O oder NH, C₁₋₅-Alkoxy, Phenyl, Benzyl, Naphthyl, Pyrrolidinyl, Piperidinyl, Morpholinyl, thiomorpholinyl, Piperazinyl, Furanyl, Thienyl, Oxazolyl, Thiazolyl, Pyridinyl, Benzofuranyl, Benzothienyl, Chinolinyl, Isochinolinyl, Isoindolinyl, Tetrahydroisochinolyl, Pyrazinyl, Chinoxalinyl oder Amino; worin R_{g} gegebenenfalls mit einem oder mehreren Rₕ substituiert ist;
Rₕ ist C₁₋₅-Alkyl, C₃₋₆-Cyloalkyl, Phenyl, Naphthyl, Indenyl, Indanyl, Morpholinyl, Thiomorpholinyl, Pyridinyl, Isoindolinyl, Isochinolinyl, Tetrahydroisochinolinyl oder Benzodioxolyl oder C₁₋₅-Alkoxy;
oder Rₕ ist C₁₋₅-Alkylthio;
oder Rₕ ist Amino, worin das Stickstoffatom unabhängig mono- oder disubstituiert sein kann mit Alkyl, Phenyl, Naphthyl, Piperidinyl, Morpholinyl, Thiomorpholinyl, Piperazinyl oder Piperazinylcarbonyl, Halogen, Hydroxy, Oxo, Carboxy, Cyano, Nitro, Amidino oder Guanidino;
Rₕ kann weiterhin gegebenenfalls mit einem oder mehreren Rᵢ substituiert sein;
Rᵢ ist C₁₋₅-Alkyl, C₃₋₆-Cycloalkyl, Aryl, Aryl-C₁₋₅-alkyl, Morpholinyl, Thiomorpholinyl, C₁₋₅-Alkoxy, C₁₋₅-Alkoxycarbonyl, Halogen, Hydroxy, Oxo, Carboxy, Cyano, Nitro, Amidino oder Guanidino;
worin ein oder mehrere der Aminostickstoffe in den Amidino- oder Guanidinogruppen in der Verbindung der Formel I gegebenenfalls mit C₁₋₃-Alkyl, Phenyl-C₀₋₃-alkyl oder C₁₋₃-Alkoxy substituiert sein können;
oder ein pharmazeutisch akzeptables Salz, Solvat oder Tautomer hiervon.

3. Verbindung nach Anspruch 1, worin:
R₁ ist: Wasserstoff, eine gesättigte C₁₋₆-Alkylgruppe, worin ein C-Atom gegebenenfalls ersetzt ist durch O, S, S(O), S(O)₂ oder NH, und worin die Alkylgruppe gegebenenfalls unabhängig substituiert ist mit ein oder zwei von: Oxogruppen, Phenyl,
Naphthyl, Cyclohexyl, Pyrrolidinyl, Piperidinyl, Piperazinyl, Morpholinyl, Indolinyl, Tetrahydropyranyl, Pyridinyl oder Indolyl;
oder R₁ ist C₁₋₃-Alkylthio-C₁₋₃-alkyl, worin das Schwefelatom zu einem Sulfoxid oder Sulfon oxidiert sein kann, Cyclohexyl, Phenyl, Phenylsulfonyl, Pyridinylsulfonyl, Phenyloxy-C₁₋₄-alkyl, C₁₋₆-Alkanoyl oder Piperidinyl;
jedes R₁ kann ferner gegebenenfalls unabhängig mit einem oder zwei Rₐ substituiert sein;
Rₐ ist eine gesättigte C₁₋₆-Alkylgruppe;
oder Rₐ ist ein C₁₋₆Alkoxy, Cyclohexyl, Phenyl, Naphthyl, Pyrrolidinyl, Piperidinyl, Indolyl, Piperazinyl, Pyridinyl oder Tetrahydropyranyl;
worin Rₐ weiterhin gegebenenfalls mit einem oder zwei R_{b} substituiert sein kann;
R_{b} ist C₃₋₆-Cycloalkyl, Phenyl, Benzyl, C₁₋₅-Alkoxy, Halogen, Hydroxy, Oxo, Carboxy, Cyano, Nitro oder Carboxamid;
oder R_{b} ist eine gesättigte oder ungesättigte C₁₋₆-Alkylgruppe oder eine C₁₋₄-Alkoxycarbonylgruppe;
R₂, R₃, R₅ und R₆ sind unabhängig Wasserstoff oder eine C₁₋₅-Alkylgruppe;
R₂ und R₃ können zusammen mit dem Kohlenstoff, an den sie gebunden sind, und/oder R₅ und R₆ können zusammen mit dem Kohlenstoff, an den sie gebunden sind, jeweils unabhängig gegebenenfalls eine Cyclopropyl, Cyclobutyl, Cyclopentyl oder Cyclohexylgruppe bilden;
R₄ ist Wasserstoff, Cyclopropyl, Cyclobutyl, Cyclopentyl, Cyclohexyl, Phenyl-C₁₋₃-alkyl, Naphthyl-C₁₋₃-alkyl, Phenyl, Naphthyl, Pyridinyl oder eine C₁₋₆-Alkylgruppe, worin ein C-Atom gegebenenfalls mit S(O)₂ ersetzt ist; worin R₄ gegebenenfalls mit einem oder mehreren Rₑ substituiert ist;
Rₑ ist C₁₋₃-Alkyl, Cyclopropyl, Cyclobutyl, Cyclopentyl, Cyclohexyl, Phenyl, Naphthyl, Indanyl oder Indolyl;
oder Rₑ ist Hydroxy, Halogen, Oxo, Carboxy oder Cyano;
Q ist R_{g}, C(O)R_{g} oder S(O)₂R_{g};
worin R_{g} C₁₋₅-Alkyl ist, worin ein oder mehrere C-Atome gegebenenfalls ersetzt sind durch O oder NH, Phenyl, Naphthyl, Pyrrolidinyl, Piperidinyl, Morpholinyl, Thiomorpholinyl, Piperazinyl, Furanyl, Thienyl, Thiazolyl, Benzofuranyl, Benzothienyl, Chinolinyl, Isochinolinyl, Isoindolinyl, Tetrahydroisochinolyl, Pyrazinyl, Chinoxalinyl oder Amino; worin R_{g} gegebenenfalls mit einem oder mehreren Rₕ substituiert ist;
Rₕ ist C₁₋₅-Alkyl, C₁₋₅-Alkoxy, Halogen, Phenyl, Naphthyl, Indenyl, Indanyl, Morpholinyl, Thiomorpholinyl, Pyridinyl, Isoindolinyl, Isochinolinyl, Tetrahydroisochinolyl, Benzodioxolyl, Piperidinylamino oder Piperazinylcarbonylamino;
Rₕ kann weiterhin gegebenenfalls mit einem oder mehreren Rᵢ substituiert sein; R; ist C₁₋₃-Alkyl, C₃₋₆-Cycloalkyl, Phenyl, Naphthyl, Benzyl, Morpholinyl, C₁₋₅-Alkoxy, C₁₋₅-Alkoxycarbonyl, Halogen, Hydroxy, Oxo, Carboxy, Cyano oder Nitro;
oder ein pharmazeutisch akzeptables Salz, Solvat oder Tautomer hiervon.

4. Verbindung nach Anspruch 1, worin:
R₁ ist: Wasserstoff, C₁₋₆-Alkyl, C₁₋₆-Alkanoyl, Phenylsulfonyl oder Pyridylsulfonyl, jedes gegebenenfalls substituiert mit Cyclohexyl, Phenyl, Pyrrolidinyl, Piperidinyl, Indolyl, Piperazinyl, Pyridinyl, Tetrahydropyranyl oder Naphthyl, oder R₁ ist Benzyloxy-C₁₋₃-alkyl oder Benzyloxy-C₁₋₃-alkanoyl;
R₂, R₃, R₅ und R₆ sind unabhängig Wasserstoff oder C₁₋₃-Alkyl;
R₂ und R₃ können zusammen mit dem Kohlenstoff, an den sie gebunden sind, und/oder R₅ und R₆ können zusammen mit dem Kohlenstoff, an den sie gebunden sind, jeweils unabhängig gegebenenfalls eine Cyclopropyl, Cyclobutyl, Cyclopentyl oder Cyclohexylgruppe bilden;
R₄ ist Wasserstoff, eine C₁₋₆-Alkylgruppe, Phenyl-C₁₋₆-alkyl, Cyclopropyl-C₁₋₆-alkyl, Cyclohexyl-C₁₋₆-alkyl oder Pyridinyl;
Q ist C(O)R_{g};
worin R_{g} ist Piperidinyl, Morpholinyl, Thiomorpholinyl, Piperazinyl, Isoindolinyl, Benzofuranyl, Tetrahydroisochinolyl, Phenyl-C₁₋₅-alkylamino oder Naphthyl-C₁₋₅-alkylamino, jedes gegebenenfalls mit C₁₋₅-Alkyl, Morpholinyl oder Morpholinyl-C₁₋₅-alkoxy substituiert;
oder ein pharmazeutisch akzeptables Salz, Solvat oder Tautomer hiervon.

5. Verbindung nach Anspruch 1, worin:
R₁ ist: C₁₋₆-Alkanoyl, gegebenenfalls substituiert mit Cyclohexyl oder Phenyl;
R₂, R₃, R₅ und R₆ sind Wasserstoff;
R₄ ist eine gesättigte C₁₋₆-Alkylgruppe;
Q ist C(O)R_{g};
worin R_{g} ist: Morpholinyl, Tetrahydroisochinolyl oder Phenyl-C₁₋₃-alkylamino;
oder ein pharmazeutisch akzeptables Salz, Solvat oder Tautomer hiervon.

6. Verbindung nach Anspruch 1 mit der nachfolgenden Formel (Ia): 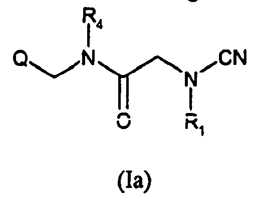 worin Q unabhängig ausgewählt ist aus den Gruppen A1 bis A34; R₄ ist unabhängig ausgewählt aus den Gruppen B1 bis B26; und R₁ ist unabhängig ausgewählt aus den Gruppen C1 bis C41; worin die Gruppen A1 bis A34, B1 bis B26 und C1 bis C41 wie in der nachfolgenden Tabelle definiert sind:
| **A** | **Q** | **B** | **R₄** | **C** | **R₁** |
|---|---|---|---|---|---|
| A1 | 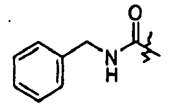 | B1 | -H | C1 | -H |
| A2 | 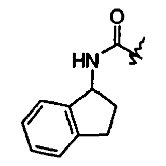 | B2 | -CH₃ | C2 | 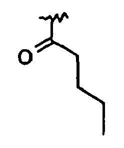 |
| A3 | 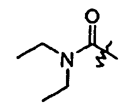 | B3 | 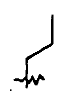 | C3 | 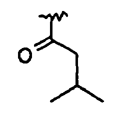 |
| A4 | 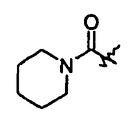 | B4 | 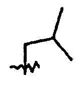 | C4 | 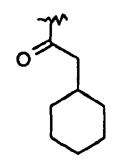 |
| A5 | 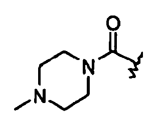 | B5 | 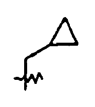 | C5 | 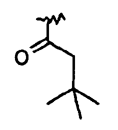 |
| A6 | 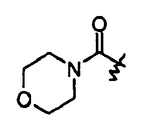 | B6 | 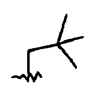 | C6 | 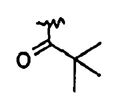 |
| A7 | 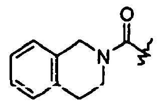 | B7 | 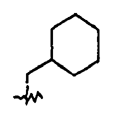 | C7 | 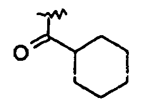 |
| A8 | 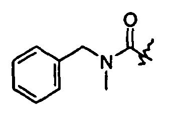 | B8 | 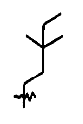 | C8 | 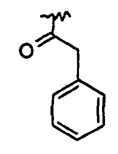 |
| A9 | 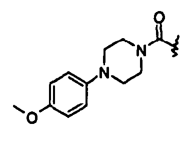 | B9 | 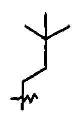 | C9 | 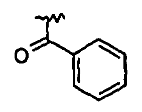 |
| A10 | 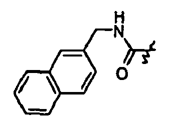 | B10 | 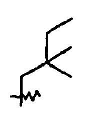 | C10 | 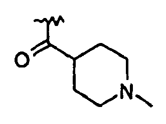 |
| A11 | 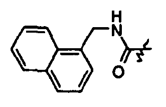 | B11 | 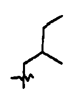 | C11 | 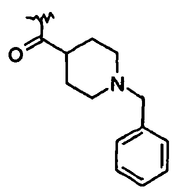 |
| A12 | 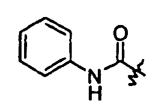 | B12 | 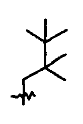 | C12 | 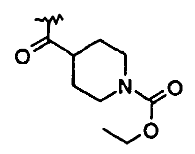 |
| A13 | 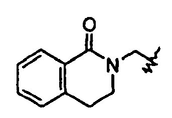 | B13 | 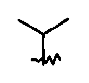 | C13 | 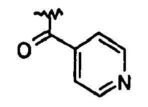 |
| A14 | 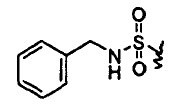 | B14 | 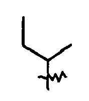 | C14 | 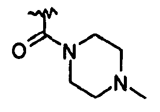 |
| A15 | 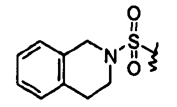 | B15 | 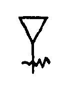 | C15 | 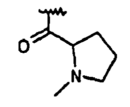 |
| A16 | 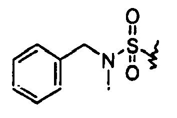 | B16 | 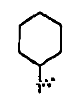 | C16 | 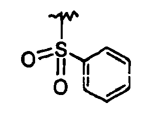 |
| A17 | 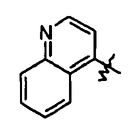 | B17 | 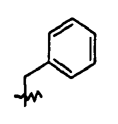 | C17 | 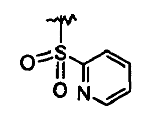 |
| A18 | 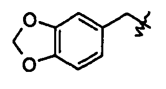 | B18 | 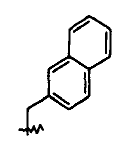 | C18 | -CH₃ |
| A19 | 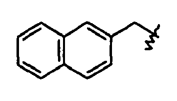 | B19 | 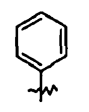 | C19 | 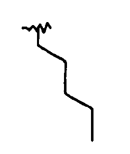 |
| A20 | 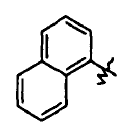 | B20 | 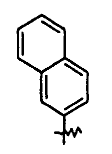 | C20 | 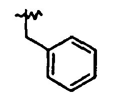 |
| A21 | 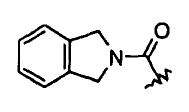 | B21 | 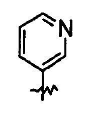 | C21 | 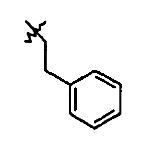 |
| A22 | 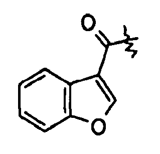 | B22 | 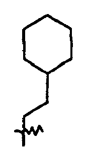 | C22 | 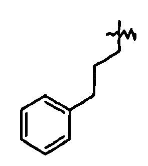 |
| A23 | 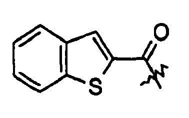 | B23 | 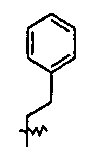 | C23 | 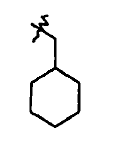 |
| A24 | 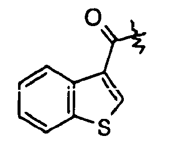 | B24 | 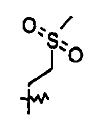 | C24 | 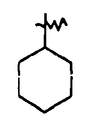 |
| A25 | 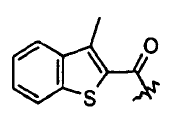 | B25 | 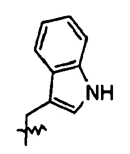 | C25 | 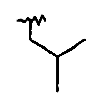 |
| A26 | 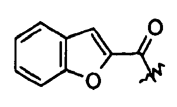 | B26 | 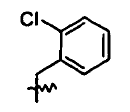 | C26 | 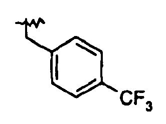 |
| A27 | 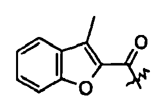 | | | C27 | 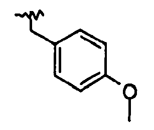 |
| A28 | 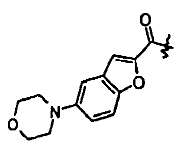 | | | C28 | 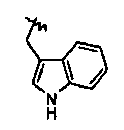 |
| A29 | 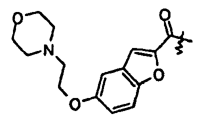 | | | C29 | 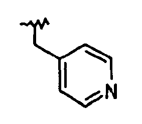 |
| A30 | 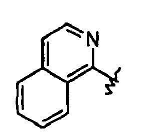 | | | C30 | 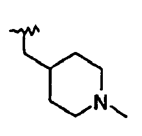 |
| A31 | 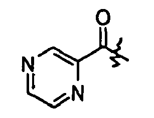 | | | C31 | 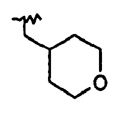 |
| A32 | 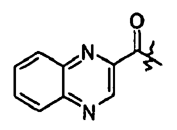 | | | C32 | 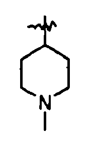 |
| A33 | 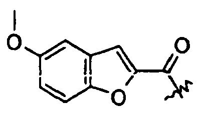 | | | C33 | 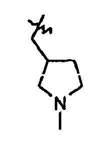 |
| A34 | 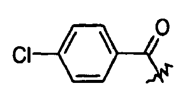 | | | C34 | 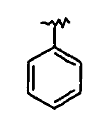 |
| | | | | C35 | 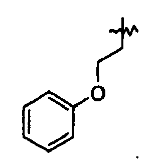 |
| | | | | C36 | 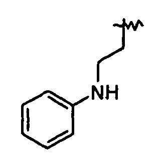 |
| | | | | C37 | 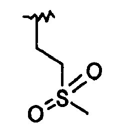 |
| | | | | C38 | 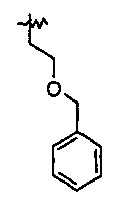 |
| | | | | C39 | 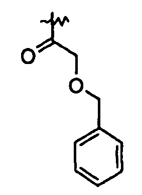 |
| | | | | C40 | 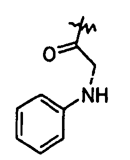 |
| | | | | C41 | 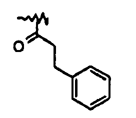 |
oder ein pharmazeutisch akzeptables Salz, Solvat oder Tautomer hiervon.

7. Verbindung nach Anspruch 6, worin:
R₁ ausgewählt ist aus den folgenden Gruppen: 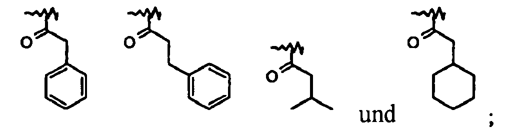
R₄ ausgewählt ist aus den folgenden Gruppen: 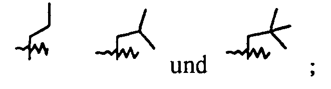
und Q ausgewählt ist aus den nachfolgenden Gruppen: 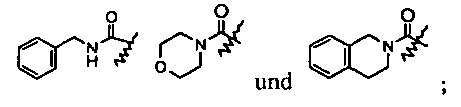 oder ein pharmazeutisch akzeptables Salz, Solvat oder Tautomer hiervon.

8. Verbindung nach Anspruch 1, ausgewählt aus den nachfolgenden Verbindungen:

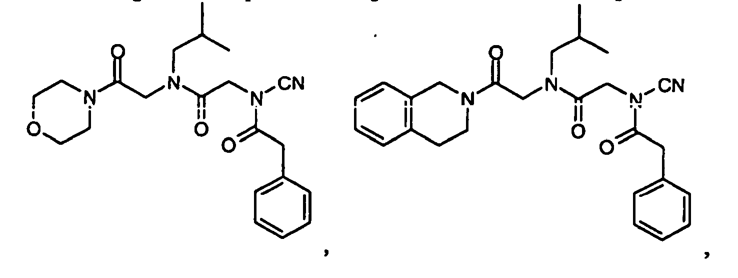

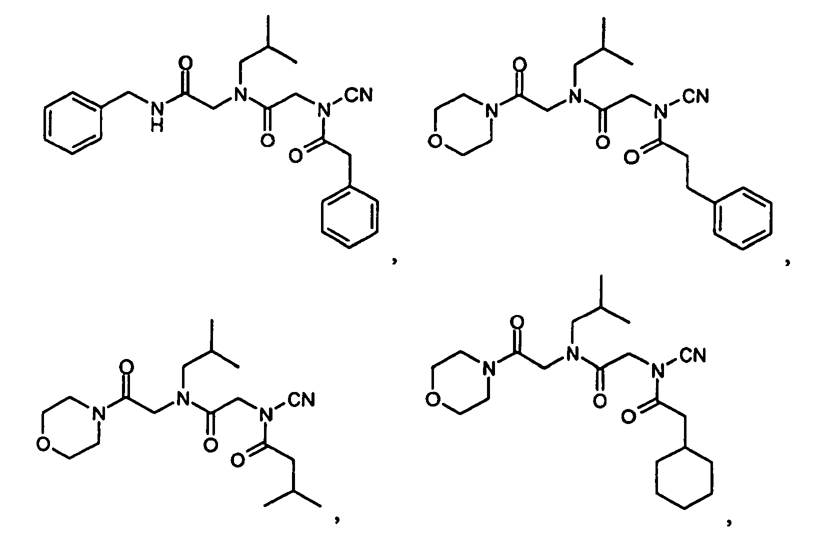

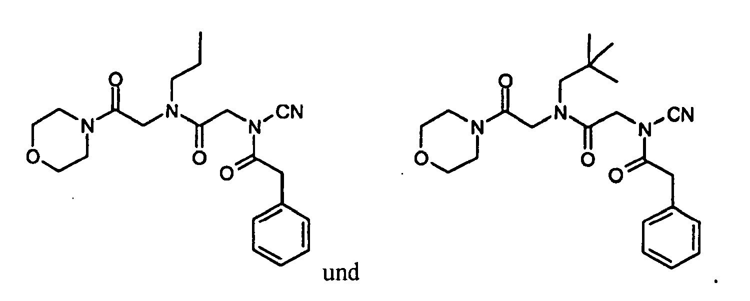

9. Verfahren zur Herstellung einer Verbindung der Formel (I) nach Anspruch 1, wobei das Verfahren umfasst: Umsetzen einer Verbindung der Formel V mit einem Cyanamid der Formel VIII in Gegenwart einer geeigneten Base, um eine Verbindung der Formel (I) zu erhalten: 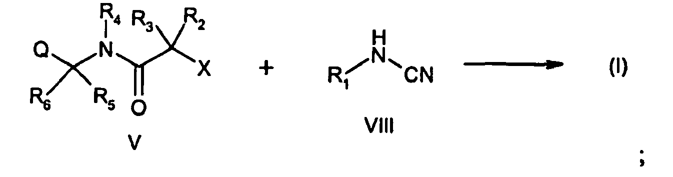 worin R₁, R₂, R₃, R₄, R₅, R₆ und Q wie in Anspruch 1 definiert sind, und X eine Abgangsgruppe darstellt.

10. Verfahren nach Anspruch 9, worin die Verbindung der Formel (V) durch ein Verfahren hergestellt wird, das umfasst:
Umsetzen der Verbindung der Formel (III) mit einer Verbindung der Formel (IV), worin X eine Abgangsgruppe darstellt, um eine Verbindung der Formel (V) zu erhalten: 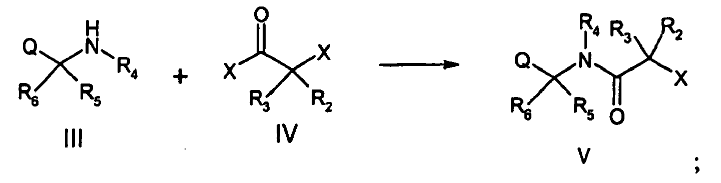
worin R₂, R₃, R₄, R₅, R₆ und Q wie in Anspruch 9 definiert sind.

11. Verfahren nach Anspruch 10, worin die Verbindung der Formel III hergestellt wird durch ein Verfahren, das umfasst:
(a) Umsetzen einer Verbindung der Formel II, worin X eine Abgangsgruppe darstellt, mit einer Verbindung der Formel H₂N-R₄, um eine Verbindung der Formel III zu erhalten: 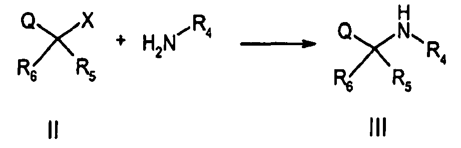 worin R₄, R₅, R₆ und Q wie in Anspruch 10 definiert sind;
oder
(b) Umsetzen einer Verbindung der Formel IX mit einem Aldehyd der Formel R'CHO unter reduktiven Aminierungsbedingungen, um eine Verbindung der Formel III zu liefern: 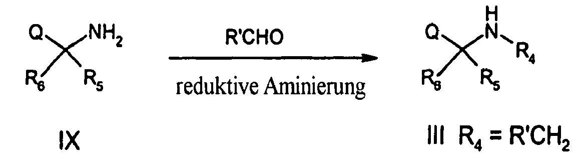
worin R₅, R₆ und Q wie in Anspruch 10 definiert sind, und R₄ ausgewählt ist aus einer R₄-Gruppe, wie in Anspruch 10 definiert, mit einer -CH₂-Gruppe, gebunden an das Stickstoffatom im Grundgerüst der Formel (III), und R' ist der Endabschnitt der R₄-Gruppe, die an die -CH₂-Gruppe gebunden ist.

12. Verfahren zur Herstellung einer Verbindung der Formel (I) nach Anspruch 1, wobei das Verfahren umfasst: Umsetzen einer Verbindung der Formel (XIII) mit einer Verbindung R₁X, worin X eine Abgangsgruppe darstellt, in Gegenwart einer geeigneten Base, um eine Verbindung der Formel (I) zu erhalten: 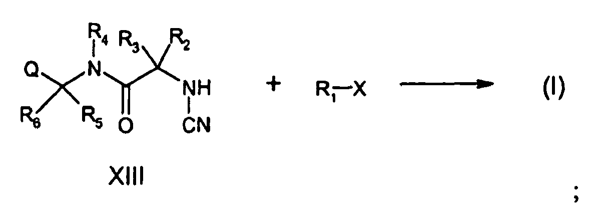 worin R₁, R₂, R₃, R₄, R₅, R₆ und Q wie in Anspruch 1 definiert sind.

13. Verfahren nach Anspruch 12, worin die Verbindung der Formel (XTII) hergestellt wird durch ein Verfahren, das umfasst:
(a) Umsetzen einer Verbindung der Formel (III) mit einer Verbindung der Formel (X), worin PG eine Schutzgruppe darstellt, und X eine Abgangsgruppe darstellt, um eine Verbindung der Formel (XI) zu erhalten: 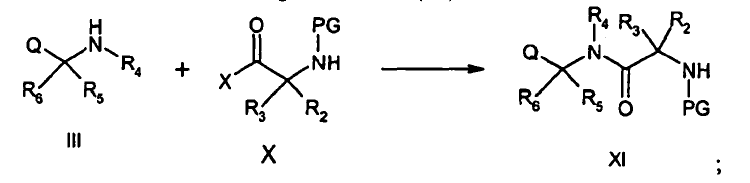
(b) Entschützen einer Verbindung der Formel (XI), um eine Verbindung der Formel (XII) zu erhalten: 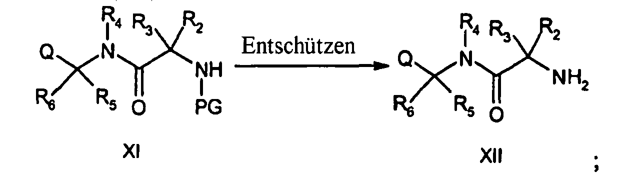
(c) Umsetzen einer Verbindung der Formel (XII) mit Cyanbromid, um eine Verbindung der Formel (XIII) zu erhalten: 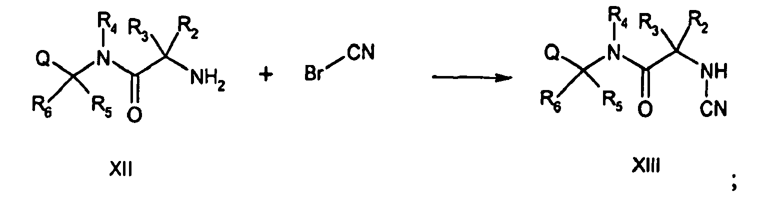
worin R₂, R₃, R₄, R₅, R₆ und Q wie in Anspruch 12 definiert sind.

14. Verfahren nach Anspruch 13, worin die Verbindung der Formel III hergestellt wird durch ein Verfahren, das umfasst:
(a) Umsetzen einer Verbindung der Formel II, worin X eine Abgangsgruppe darstellt, mit einer Verbindung der Formel H₂N-R₄, um eine Verbindung der Formel III zu erhalten: 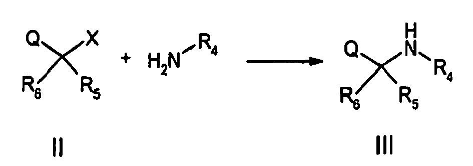 worin R₄, R₅, R₆ und Q wie in Anspruch 13 definiert sind;
oder
(b) Umsetzen einer Verbindung der Formel IX mit einem Aldehyd der Formel R'CHO unter reduktiven Aminierungsbedingungen, um eine Verbindung der Formel III zu liefern: 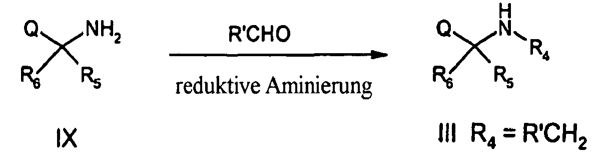
worin R₅, R₆ und Q wie in Anspruch 13 definiert sind, und R₄ ausgewählt ist aus einer R₄-Gruppe, wie in Anspruch 13 definiert, mit einer -CH₂-Gruppe, gebunden an das Stickstoffatom im Grundgerüst der Formel (III), und R' ist der Endabschnitt der R₄-Gruppe, die an die -CH₂-Gruppe gebunden ist.

15. Pharmazeutische Zusammensetzung, umfassend eine therapeutisch wirksame Menge einer Verbindung gemäß einem der Ansprüche 1 bis 8, oder ein pharmazeutisch akzeptables Salz, Solvat oder Tautomer hiervon.

16. Verbindung nach einem der Ansprüche 1 bis 8 zur Verwendung als Arzneimittel.

17. Verwendung einer Verbindung nach einem der Ansprüche 1 bis 8 zur Herstellung einer pharmazeutischen Zusammensetzung zur Behandlung einer Erkrankung, vermittelt durch Cathepsin S.

18. Verwendung einer Verbindung nach einem der Ansprüche 1 bis 8 zur Herstellung einer pharmazeutischen Zusammensetzung zur Behandlung einer Erkrankung, vermittelt durch Cathepsin K.

## Revendications

1. Composé de formule (I) : 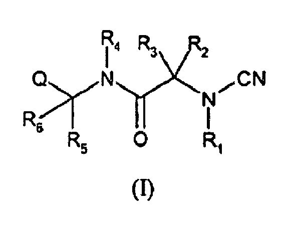 dans laquelle :
R₁ représente un hydrogène, un groupement C1-10 alkyle saturé ou insaturé dans lequel un ou plusieurs atomes de carbone sont éventuellement remplacés par O, NH, S(O), S(O)₂ ou S et dans lequel ledit groupement alkyle est éventuellement indépendamment substitué par un ou plusieurs : groupements oxo, -NH₂, C1-10 alkyle, C3-8 cycloalkyle, aryle, tétrahydronaphtyle, indényle, indanyle, pyrrolidinyle, pipéridinyle, morpholinyle, thiomorpholinyle, pipérazinyle, indolinyle, pyranyle, tétrahydropyranyle, thiopyranyle, tétrahydrothiopyranyle, furanyle, tétrahydrofuranyle, thiényle, pyrrolyle, pyrazolyle, oxazolyle, isoxazolyle, thiazolyle, imidazolyle, pyridinyle, pyrimidinyle, pyrazinyle, indolyle, benzofuranyle, benzothiényle, benzimidazolyle, benzothiazolyle, benzoxazolyle, quinolyle, isoquinolyle, C1-10 alcoxy, aryloxy, C1-10 alcanoyle, aroyle, C1-10 alcoxycarbonyle, aryloxycarbonyle, C1-10 alcanoyloxy ou aroyloxy,
ou R₁ représente un C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, aryle, arylsulfonyle, hétéroarylsulfonyle, tétrahydronaphtyle, indényle, indanyle, tétrahydropyranyle, tétrahydrothiopyranyle, tétrahydrofuranyle, pyrrolidinyle, pipéridinyle, morpholinyle, thiomorpholinyle, indolinyle, C3-8 cycloalkylsulfonyl C1-5 alkyle, arylsulfonyl C1-5 alkyle, aryloxy C1-5 alkyle, C1-10 alcanoyle, aroyle, C1-10 alcoxycarbonyle, aryle C1-5 alcoxycarbonyle ou aryloxycarbonyle,
ou R₁ représente un carbamoyle dans lequel l'atome d'azote peut indépendamment être mono ou di-substitué par un C1-10 alkyle, aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
chaque R₁ peut en outre éventuellement être indépendamment substitué par un ou plusieurs Rₐ ;
Rₐ représente un groupement C1-10 alkyle saturé ou insaturé dans lequel un ou plusieurs atomes de carbone sont éventuellement remplacés par O, NH, S(O), S(O)₂ ou S et dans lequel ledit groupement alkyle est éventuellement indépendamment substitué par un ou plusieurs groupements oxo, ou un ou plusieurs : -NH₂, C1-10 alkyle, aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
ou Rₐ représente un C1-10 alcoxy, C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, C3-8 cycloalkyloxy, aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ; C1-10 alcanoyle, aroyle, C1-10 alcanoyloxy, aryloxy, benzyloxy, C1-10 alcoxycarbonyle, aryle C1-3 alcoxycarbonyle, aryloxycarbonyle ou aroyloxy,
ou Rₐ représente un carbamoyle dans lequel l'atome d'azote peut indépendamment être mono ou di-substitué par un C1-10 alkyle, aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
ou Rₐ représente un uréido dans lequel chaque atome d'azote peut indépendamment être substitué par un C1-10 alkyle, C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
ou Rₐ représente un amino dans lequel l'atome d'azote peut indépendamment être mono ou di-substitué par un C1-10 alkyle, C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
Rₐ pouvant en outre être substitué par un ou plusieurs R_{b} ;
R_{b} représente un C3-8 cycloalkyle, tolylsulfonyle, C1-5 alcoxy, aryle, aryloxy, benzyloxy, halogène, hydroxy, oxo, carboxy, cyano, nitro, carboxamide, amidino ou guanidino ;
ou R_{b} représente aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
ou R_{b} représente un amino dans lequel l'atome d'azote peut indépendamment être mono ou di-substitué par un C1-10 alkyle, C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
ou R_{b} représente un groupement C1-10 alkyle saturé ou insaturé dans lequel un ou plusieurs atomes de carbone sont éventuellement remplacés par O, NH, S(O), S(O)₂ ou S et dans lequel ledit groupement alkyle est éventuellement indépendamment substitué par un ou deux groupements oxo, ou un ou plusieurs : -NH₂, C1-10 alkyle, C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
R₂, R₃, R₅ et R₆ représentent indépendamment un hydrogène ou un groupement C1-5 alkyle ;
R₂ et R₃, avec le carbone auquel ils sont attachés, et/ou R₅ et R₆, avec le carbone auquel ils sont attachés, peuvent chacun éventuellement former indépendamment un cycloalkyle non aromatique ayant 3 à 6 éléments ;
R₄ représente l'hydrogène, un C2-10 alcényle, C3-8 cycloalkyle, aryl C1-10 alkyle, aryle ou un groupement C1-10 alkyle dans lequel un ou plusieurs des atomes de carbone sont éventuellement remplacés par O, NH, C(=O), S, S(O) ou S(O)₂ ; R₄ étant éventuellement substitué par un ou plusieurs Rₑ ; ou R₄ représentant Rₑ ;
Rₑ représente un C1-10 alkyle, C3-8 cycloalkyle, aryle, indanyle, indényle, bicyclo[2.2.1]heptanyle, bicyclo[2.2.2]octanyle, bicyclo[4.1.0]heptanyle, bicyclo[3.1.0]hexanyle, bicyclo[1.1.1]pentanyle, cubanyle, 1,2,3,4-tétrahydronaphtyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle; C1-10 alcoxy, aryloxy, C1-10 alcanoyle, aroyle, C1-10 alcoxycarbonyle, aryloxycarbonyle, C1-10 alcanoyloxy ou aroyloxy ;
ou Rₑ représente un carbamoyle dans lequel l'atome d'azote peut indépendamment être mono ou di-substitué par un C1-10 alkyle, aryle, indanyle, indényle, tétrahydronaphtyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
ou Rₑ représente un C1-10 alcanoylamino, aroylamino, C1-10 alkylthio dans lequel l'atome de soufre peut être oxydé en un sulfoxyde ou une sulfone, arylthio dans lequel l'atome de soufre peut être oxydé en un sulfoxyde ou une sulfone, uréido dans lequel chaque atome d'azote peut indépendamment être substitué par C1-10 alkyle, C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
ou Rₑ représente un C1-10 alcoxycarbonylamino, aryloxycarbonylamino, C1-10 alkylcarbamoyloxy, arylcarbamoyloxy, C1-10 alkylsulfonylamino, arylsulfonylamino, C1-10 alkylaminosulfonyle, arylaminosulfonyle, amino dans lequel l'atome d'azote peut indépendamment être mono- ou di-substitué par C1-10 alkyle, C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, aryle, tétrahydronaphtyle, indényle, indanyle, pyrrolidinyle, pipéridinyle, morpholinyle, thiomorpholinyle, pipérazinyle, indolinyle, pyranyle, thiopyranyle, furanyle, tétrahydropyranyle, tétrahydrothiopyranyle, tétrahydrofuranyle, thiényle, pyrrolyle, pyrazolyle, imidazolyle, oxazolyle, isoxazolyle, thiazolyle, pyridinyle, pyrimidinyle, pyrazinyle, indolyle, benzofuranyle, benzothiényle, benzimidazolyle, benzothiazolyle, benzoxazolyle, quinolyle ou isoquinolyle ;
ou Rₑ représente amino, halogène, hydroxy, oxo, carboxy, cyano, amidino ou guanidino ;
chaque Rₑ peut en outre être également substitué par un ou plusieurs R_{f} ;
R_{f} représente C1-5 alkyle, C3-6 cycloalkyle, aryle, aryle C1-4 alkyle, C1-5 alcoxy, aryloxy, aryle C1-5 alcoxy, aroyle, halogène, hydroxy, oxo ou cyano ;
Q représente R_{g}, C(O)R_{g}, S(O)R_{g} ou S(O)₂R_{g} ;
R_{g} représentant un C2-10 alcényle, C1-10 alcoxy, aryloxy, C3-8 cycloalkyle, aryle, aryle C1-10 alkyle, C1-10 alkyle dans lequel un ou plusieurs des atomes de carbone sont éventuellement remplacés par O, NH, -C(=O)-, S, S(O) ou S(O)₂ ; indényle, indanyle, bicyclo[2.2.1]heptanyle, bicyclo[2.2.2]octanyle, bicyclo[4.1.0]heptanyle, bicyclo[3.1.0]hexanyle, bicyclo[1.1.1]pentanyle, cubanyle, tétrahydronaphtyle, C1-10 alkylsulfonyl C1-10 alkyle, C3-8 cycloalkylsulfonyl C1-10 alkyle, arylsulfonyl C1-10 alkyle, azétidinyle, pyrrolidinyle, pipéridinyle, morpholinyle, thiomorpholinyle, pipérazinyle, perhydroazépinyle, perhydrodiazépinyle, indolinyle, isoindolinyle, pyranyle, tétrahydropyranyle, thiopyranyle, tétrahydrothiopyranyle, furanyle, tétrahydrofuranyle, thiényle, pyrrolyle, oxazolyle, isoxazolyle, thiazolyle, imidazolyle, pyrazolyle, pyridinyle, pyrimidinyle, pyrazinyle, pyridazinyle, tétrazolyle, triazolyle, indolyle, benzofuranyle, benzothiényle, dihydrobenzofuranyle, dihydrobenzothiényle, indazolyle, isoindazolyle, benzimidazolyle, benzoxazolyle, benzothiazolyle, benzisoxazolyle, benzisothiazolyle, benzotriazolyle, quinolinyle, tétrahydroquinolinyle, isoquinolinyle, tétrahydroisoquinolinyle, quinazolinyle, tétrahydroquinazolinyle, quinoxalinyle ou amino ; R_{g} étant éventuellement substitué par un ou plusieurs Rₕ ;
Rₕ représente un C1-10 alkyle, C3-6 cycloalkyle, aryle, tétrahydronaphtyle, indényle, indanyle, azétidinyle, pyrrolidinyle, pipéridinyle, morpholinyle, thiomorpholinyle, pipérazinyle, perhydroazépinyle, perhydrodiazépinyle, indolinyle, isoindolinyle, pyranyle, tétrahydropyranyle, thiopyranyle, tétrahydrothiopyranyle, furanyle, tétrahydrofuranyle, thiényle, pyrrolyle, oxazolyle, isoxazolyle, thiazolyle, imidazolyle, pyrazolyle, pyridinyle, pyrimidinyle, pyrazinyle, pyridazinyle, tétrazolyle, triazolyle, indolyle, benzofuranyle, benzothiényle, dihydrobenzofuranyle, dihydrobenzothiényle, indazolyle, isoindazolyle, benzimidazolyle, benzoxazolyle, benzothiazolyle, benzisoxazolyle, benzisothiazolyle, benzotriazolyle, benzodioxolyle, quinolinyle, tétrahydroquinolinyle, isoquinolinyle, tétrahydroisoquinolinyle, quinazolinyle, tétrahydroquinazolinyle, quinoxalinyle, C1-10 alcoxy, C1-10 alcanoyle, C1-10 alcanoyloxy, aryloxy, benzyloxy, C1-10 alcoxycarbonyle, aryloxycarbonyle, aroyloxy, bicyclo[2.2.1]heptanyle, bicyclo[2.2.2]octanyle, bicyclo[4.1.0]heptanyle, bicyclo[3.1.0]hexanyle, bicyclo[1.1.1]pentanyle ou cubanyle,
ou Rₕ représente un carbamoyle dans lequel l'atome d'azote peut indépendamment être mono ou di-substitué par un C1-10 alkyle, C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, aryle, tétrahydronaphtyle, indényle, indanyle, azétidinyle, pyrrolidinyle, pipéridinyle, morpholinyle, thiomorpholinyle, pipérazinyle, perhydroazépinyle, perhydrodiazépinyle, indolinyle, isoindolinyle, pyranyle, tétrahydropyranyle, thiopyranyle, tétrahydrothiopyranyle, furanyle, tétrahydrofuranyle, thiényle, pyrrolyle, oxazolyle, isoxazolyle, thiazolyle, imidazolyle, pyrazolyle, pyridinyle, pyrimidinyle, pyrazinyle, pyridazinyle, tétrazolyle, triazolyle, indolyle, benzofuranyle, benzothiényle, dihydrobenzofuranyle, dihydrobenzothiényle, indazolyle, isoindazolyle, benzimidazolyle, benzoxazolyle, benzothiazolyle, benzisoxazolyle, benzisothiazolyle, benzotriazolyle, quinolinyle, tétrahydroquinolinyle, isoquinolinyle, tétrahydroisoquinolinyle, quinazolinyle, tétrahydroquinazolinyle ou quinoxalinyle,
ou Rₕ représente un C1-10 alcanoylamino, aroylamino, C1-10 alkylthio, arylthio dans lequel l'atome de soufre peut être oxydé en un sulfoxyde ou une sulfoné, ou Rₕ représente un uréido dans lequel chaque atome d'azote peut indépendamment être substitué par C1-10 alkyle, C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, aryle, tétrahydronaphtyle, indényle, indanyle, isoindolinyle, azétidinyle, pyrrolidinyle, pipéridinyle, morpholinyle, thiomorpholinyle, pipérazinyle, perhydroazépinyle, perhydrodiazépinyle, indolinyle, pyranyle, tétrahydropyranyle, thiopyranyle, tétrahydrothiopyranyle, furanyle, tétrahydrofuranyle, thiényle, pyrrolyle, oxazolyle, isoxazolyle, thiazolyle, imidazolyle, pyrazolyle, pyridinyle, pyrimidinyle, pyrazinyle, pyridazinyle, tétrazolyle, triazolyle, indolyle, benzofuranyle, benzothiényle, dihydrobenzofuranyle, dihydrobenzothiényle, indazolyle, isoindazolyle, benzimidazolyle, benzoxazolyle, benzothiazolyle, benzisoxazolyle, benzisothiazolyle, benzotriazolyle, quinolinyle, tétrahydroquinolinyle, isoquinolinyle, tétrahydroisoquinolinyle, quinazolinyle, tétrahydroquinazolinyle ou quinoxalinyle ;
ou Rₕ représente C1-10 alcoxycarbonylamino, aryloxycarbonylamino, C1-10 alkylcarbamoyloxy, arylcarbamoyloxy, C1-10 alkylsulfonylamino, arylsulfonylamino, C1-10 alkylaminosulfonyle, arylaminosulfonyle, amino dans lequel l'atome d'azote peut indépendamment être mono- ou di-substitué par C1-10 alkyle, C1-10 alcoxy C1-10 alkyle, C1-10 alkylamino C1-10 alkyle, C1-10 alkylthio C1-10 alkyle dans lequel l'atome de soufre peut être oxydé en un sulfoxyde ou une sulfone, C3-8 cycloalkyle, aryle, tétrahydronaphtyle, indényle, indanyle, isoindolinyle, azétidinyle, pyrrolidinyle, pipéridinyle, morpholinyle, thiomorpholinyle, pipérazinyle, pipérazinylcarbonyle, perhydroazépinyle, perhydrodiazépinyle, indolinyle, pyranyle, tétrahydropyranyle, thiopyranyle, tétrahydrothiopyranyle, furanyle, tétrahydrofuranyle, thiényle, pyrrolyle, oxazolyle, isoxazolyle, thiazolyle, imidazolyle, pyrazolyle, pyridinyle, pyrimidinyle, pyrazinyle, pyridazinyle, tétrazolyle, triazolyle, indolyle, benzofuranyle, benzothiényle, dihydrobenzofuranyle, dihydrobenzothiényle, indazolyle, isoindazolyle, benzimidazolyle, benzoxazolyle, benzothiazolyle, benzisoxazolyle, benzisothiazolyle, benzotriazolyle, quinolinyle, tétrahydroquinolinyle, isoquinolinyle, tétrahydroisoquinolinyle, quinazolinyle, tétrahydroquinazolinyle, quinoxalinyle, halogène, hydroxy, oxo, carboxy, cyano, nitro, amidino ou guanidino, Rₕ peut en outre éventuellement être substitué par un ou plusieurs Rᵢ;
Rᵢ représente un C1-10 alkyle, C3-8 cycloalkyle, aryle, aryl C1-10alkyle, hétérocyclyle, hétérocyclyl C1-10 alkyle, C1-10 alcoxy, C1-10 alcoxycarbonyle, aryloxy, benzyloxy, halogène, hydroxy, oxo, carboxy, cyano, nitro, amidino ou guanidino ;
ou un sel, solvate ou tautomère pharmaceutiquement acceptable de celui-ci ;
à condition que lorsque R₁ représente un hydrogène ou C1-10 alkyle et R₂, R₃, R₅ et R₆ représentent chacun indépendamment un hydrogène ou C1-5 alkyle et R₄ représente un hydrogène ou C1-10 alkyle, alors Q ne représente ni C1-10 alkyle, ni C1-10 alcoxycarbonyle.

2. Composé selon la revendication 1, dans lequel ;
R₁ représente un hydrogène, un groupement C1-6 alkyle saturé ou insaturé dans lequel un ou deux atomes de carbone sont éventuellement remplacés par O, NH, S, S(O) ou S(O)₂ et dans lequel ledit groupement alkyle est éventuellement indépendamment substitué par un à trois : groupements oxo, C1-4 alkyle, C3-6 cycloalkyle, aryle, pyrrolidinyle, pipéridinyle, pipérazinyle, morpholinyle, indolinyle, pyranyle, thiopyranyle, tétrahydropyranyle, tétrahydrothiopyranyle, pyridinyle ou indolyle ;
ou R₁ représente C1-6 alkylthio C1-6 alkyle dans lequel l'atome de soufre peut être oxydé en un sulfoxyde ou une sulfone, C3-6 cycloalkyle, phényle, naphtyle, phénylsulfonyle, pyridinylsulfonyle, phényloxy C1-6 alkyle, C1-6 alcanoyle ou pipéridinyle ;
chaque R₁ peut en outre éventuellement indépendamment être substitué par un ou plusieurs Rₐ ;
Rₐ représente un groupement C1-6 alkyle saturé ou insaturé dans lequel un ou deux atomes de carbone sont éventuellement remplacés par O ou S ;
ou Rₐ représente un C1-6 alcoxy, C3-6 cycloalkyle, phényle, naphtyle, pyrrolidinyle, pipéridinyle, morpholinyle, thiomorpholinyle, tétrahydropyranyle, pipérazinyle, indolyle ou pyridinyle ;
Rₐ pouvant en outre être éventuellement substitué par un ou plusieurs R_{b} ;
R_{b} représente un C3-6 cycloalkyle, C1-5 alcoxy, halogène, hydroxy, oxo, carboxy, cyano, nitro ou carboxamide ;
ou R_{b} représente phényle, benzyle ou naphtyle ;
ou R_{b} représente un amino dans lequel l'atome d'azote peut indépendamment être mono- ou di-substitué par un C1-6 alkyle, phényle ou naphtyle ;
ou R_{b} représente un groupement C1-6 alkyle saturé ou insaturé dans lequel un ou deux atomes de carbone sont éventuellement remplacés par O ou S, et ledit groupement alkyle étant éventuellement indépendamment substitué par un ou deux groupements oxo ;
R₂, R₃, R₅ et R₆ représentent indépendamment un hydrogène ou un groupement C1-5 alkyle ;
R₂ et R₃, avec le carbone auquel ils sont attachés, et/ou R₅ et R₆, avec le carbone auquel ils sont attachés, peuvent chacun éventuellement former indépendamment un cycloalkyle non aromatique ayant 3 à 6 éléments ;
R₄ représente un hydrogène, C3-6 cycloalkyle, phényl C1-3 alkyle, naphtyl C1-3 alkyle, phényle, naphtyle, pyridyle ou un groupement C1-6 alkyle dans lequel un ou deux des atomes de carbone sont éventuellement remplacés par O, S ou S(O)₂ ; R₄ étant éventuellement substitué par un ou plusieurs Rₑ ;
Rₑ représente un C1-5 alkyle, C3-6 cycloalkyle, phényle, naphtyle, indanyle ou indolyle,
ou Rₑ représente halogène, hydroxy, oxo, carboxy, cyano, amidino ou guanidino ;
chaque Rₑ peut en outre éventuellement être substitué par un ou plusieurs R_{f} ;
R_{f} représente C1-5 alkyle, C3-6 cycloalkyle, C1-5 alcoxy, halogène, hydroxy, oxo ou cyano ;
Q représente R_{g}, C(O)R_{g} ou S(O)₂R_{g};
R_{g} représentant un C1-5 alkyle dans lequel un ou plusieurs atomes de carbone sont éventuellement remplacés par O ou NH, C1-5 alcoxy, phényle, benzyle, naphtyle, pyrrolidinyle, pipéridinyle, morpholinyle, thiomorpholinyle, pipérazinyle, furanyle, thiényle, oxazolyle, thiazolyle, pyridinyle, benzofuranyle, benzothiényle, quinolinyle, isoquinolinyle, isoindolinyle, tétrahydroisoquinolyle, pyrazinyle, quinoxalinyle ou amino ; R_{g} étant éventuellement substitué par un ou plusieurs Rₕ ;
Rₕ représente un C1-5 alkyle, C3-6 cycloalkyle, phényle, naphtyle, indényle, indanyle, morpholinyle, thiomorpholinyle, pyridinyle, isoindolinyle, isoquinolinyle, tétrahydroisoquinolinyle ou benzodioxolyle ou C1-5 alcoxy,
ou Rₕ représente C1-5 alkylthio,
ou Rₕ représente un amino dans lequel l'atome d'azote peut indépendamment être mono- ou di-substitué par alkyle, phényle, naphtyle, pipéridinyle, morpholinyle, thiomorpholinyle, pipérazinyle ou pipérazinylcarbonyle, halogène, hydroxy, oxo, carboxy, cyano, nitro, amidino ou guanidino,
Rₕ peut en outre éventuellement être substitué par un ou plusieurs Rᵢ ;
Rᵢ représente un C1-5 alkyle, C3-6 cycloalkyle, aryle, aryle C1-5alkyle, morpholinyle, thiomorpholinyle, C1-5 alcoxy, C1-5 alcoxycarbonyle, halogène, hydroxy, oxo, carboxy, cyano, nitro, amidino ou guanidino ;
un ou plusieurs des azotes amino dans les groupements amidino ou guanidino du composé de formule I pouvant éventuellement être substitués par C₁₋₃ alkyle, phényl C₀₋₃ alkyle ou C₁₋₃ alcoxy ;
ou un sel, solvate ou tautomère pharmaceutiquement acceptable de celui-ci.

3. Composé selon la revendication 1, dans lequel ;
R₁ représente l'hydrogène, un groupement C1-6 alkyle saturé dans lequel un atome de carbone est éventuellement remplacé par O, S, S(O), S(O)₂ ou NH et dans lequel ledit groupement alkyle est éventuellement indépendamment substitué par un ou deux : groupements oxo, phényle, naphtyle, cyclohexyle, pyrrolidinyle, pipéridinyle, pipérazinyle, morpholinyle, indolinyle, tétrahydropyranyle, pyridinyle ou indolyle ;
ou R₁ représente un C1-3 alkylthio C1-3 alkyle dans lequel l'atome de soufre peut être oxydé en un sulfoxyde, cyclohexyle, phényle, phénylsulfonyle, pyridinylsulfonyle, phényloxy C1-4 alkyle, C1-6 alcanoyle ou pipéridinyle ;
chaque R₁ peut en outre éventuellement être substitué par un ou deux Rₐ ;
Rₐ représente un groupement C1-6 alkyle saturé ;
ou Rₐ représente un C1-6 alcoxy, cyclohexyle, phényle, naphtyle, pyrrolidinyle, pipéridinyle, indolyle, pipérazinyle, pyridinyle ou tétrahydropyranyle ;
Rₐ pouvant en outre éventuellement être substitué par un ou deux R_{b} ;
R_{b} représente un C3-6 cycloalkyle, phényle, benzyle, C1-5 alcoxy, halogène, hydroxy, oxo, carboxy, cyano, nitro ou carboxamide ;
ou R_{b} représente un groupement C1-6 alkyle saturé ou insaturé, ou un groupement C1-4 alcoxycarbonyle ;
R₂, R₃, R₅ et R₆ représentent indépendamment un hydrogène ou un groupement C1-5 alkyle ;
R₂ et R₃, avec le carbone auquel ils sont attachés, et/ou R₅ et R₆, avec le carbone auquel ils sont attachés, peuvent chacun éventuellement former indépendamment un groupement cyclopropyle, cyclobutyle, cyclopentyle ou cyclohexyle ;
R₄ représente hydrogène, cyclopropyle, cyclobutyle, cyclopentyle, cyclohexyle, phényle C1-3 alkyle, naphtyle C1-3 alkyle, phényle, naphtyle, pyridinyle ou un groupement C1-6 alkyle dans lequel un atome de carbone est éventuellement remplacé par S(O)₂ ; R₄ étant éventuellement substitué par un ou plusieurs Rₑ ;
Rₑ représente C1-3 alkyle, cyclopropyle, cyclobutyle, cyclopentyle, cyclohexyle, phényle, naphtyle, indanyle ou indolyle,
ou Rₑ représente hydroxy, halogène, oxo, carboxy ou cyano ;
Q représente R_{g}, C(O)R_{g} ou S(O)₂R_{g} ;
R_{g} représentant un C1-5 alkyle dans lequel un ou plusieurs atomes de carbone sont éventuellement remplacés par O ou NH, phényle, naphtyle, pyrrolidinyle, pipéridinyle, morpholinyle, thiomorpholinyle, pipérazinyle, furanyle, thiényle, thiazolyle, benzofuranyle, benzothiényle, quinolyle, isoquinolyle, isoindolinyle, tétrahydroisoquinolyle, pyrazinyle ou quinoxalinyle ou amino ; R_{g} étant éventuellement substitué par un ou plusieurs Rₕ ;
Rₕ représente C1-5 alkyle, C1-5 alcoxy, halogène, phényle, naphtyle, indényle, indanyle, morpholinyle, thiomorpholinyle, pyridinyle, isoquinolinyle, isoindolinyle, tétrahydroisoquinolyle, benzodioxolyle, pipéridinylamino ou pipérazinylcarbonylamino ;
Rₕ peut en outre éventuellement être substitué par un ou plusieurs Rᵢ ;
Rᵢ représente C1-3 alkyle, C3-6 cycloalkyle, phényle, naphtyle, benzyle, morpholinyle, C1-5 alcoxy, C1-5 alcoxycarbonyle, halogène, hydroxy, oxo, carboxy, cyano ou nitro ;
ou un sel, solvate ou tautomère pharmaceutiquement acceptable de celui-ci.

4. Composé selon la revendication 1, dans lequel ;
R₁ représente hydrogène, C1-6 alkyle, C1-6 alcanoyle, phénylsulfonyle ou pyridylsulfonyle, chacun éventuellement substitué par cyclohexyle, phényle, pyrrolidinyle, pipéridinyle, indolyle, pipérazinyle, pyridinyle, tétrahydropyranyle ou naphtyle ou R₁ représente benzyloxyl C1-3 alkyle ou benzyloxy C1-3 alcanoyle ;
R₂, R₃, R₅ et R₆ représentent indépendamment un hydrogène ou un groupement C1-3 alkyle ;
R₂ et R₃, avec le carbone auquel ils sont attachés, et/ou R₅ et R₆, avec le carbone auquel ils sont attachés, peuvent chacun éventuellement former indépendamment un groupement cyclopropyle, cyclobutyle, cyclopentyle ou cyclohexyle ;
R₄ représente un hydrogène, un groupement C1-6 alkyle, phényl C1-6 alkyle, cyclopropyl C1-6 alkyle, cyclohexyl C1-6 alkyle ou pyridinyle ;
Q représente C(O)R_{g} ;
R_{g} représente pipéridinyle, morpholinyle, thiomorpholinyle, pipérazinyle, isoindolinyle, benzofuranyle, tétrahydroisoquinolyle, phényl C1-5 alkylamino ou naphtyl C1-5 alkylamino, chacun éventuellement substitué par C1-5 alkyle, morpholinyle ou morpholinyle C1-5 alcoxy ;
ou un sel, solvate ou tautomère pharmaceutiquement acceptable de celui-ci.

5. Composé selon la revendication 1, dans lequel ;
R₁ représente C₁₋₆ alcanoyle éventuellement substitué par cyclohexyle ou phényle ;
R₂, R₃, R₅ et R₆ représentent un hydrogène ;
R₄ représente un groupement C1-6 alkyle saturé ;
Q représente C(O)R_{g};
R_{g} représentant morpholinyle, tétrahydroisoquinolyle ou phényle C1-3 alkylamino ;
ou un sel, solvate ou tautomère pharmaceutiquement acceptable de celui-ci.

6. Composé selon la revendication 1, ayant la formule (Ia) suivante : 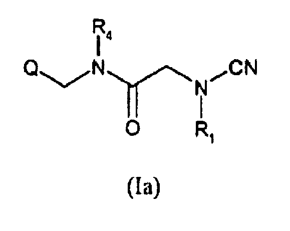 dans laquelle Q est choisi indépendamment parmi les groupements A1 à A34, R₄ est choisi indépendamment parmi les groupements B1 à B26 ; et R₁ est choisi indépendamment parmi les groupements C1 à C41 ; les groupements A1 à A34, B1 à B26 et C1 à C41 étant tels que définis dans le tableau suivant :
| **A** | **Q** | **B** | **R₄** | **C** | **R₁** |
|---|---|---|---|---|---|
| A1 | 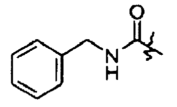 | B1 | -H | C1 | -H |
| A2 | 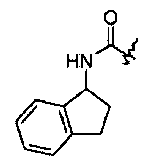 | B2 | -CH₃ | C2 | 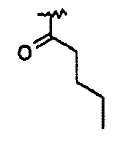 |
| A3 | 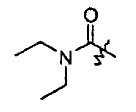 | 83 | 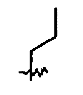 | C3 | 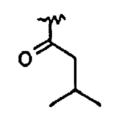 |
| A4 | 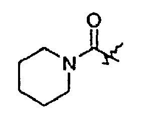 | B4 | 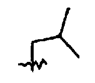 | C4 | 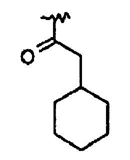 |
| A5 | 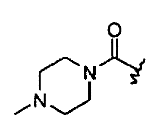 | B5 | 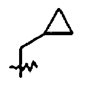 | C5 | 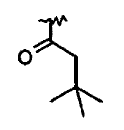 |
| A6 | 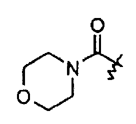 | B6 | 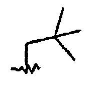 | C6 | 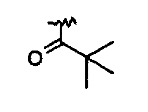 |
| A7 | 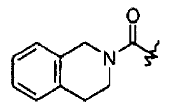 | B7 | 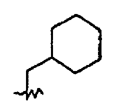 | C7 | 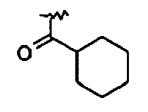 |
| A8 | 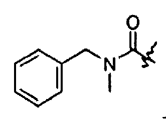 | B8 | 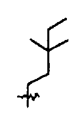 | C8 | 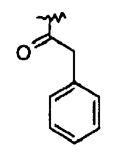 |
| A9 | 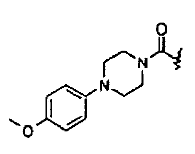 | B9 | 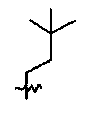 | C9 | 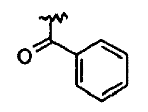 |
| A10 | 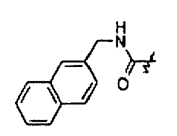 | B10 | 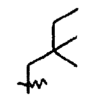 | C10 | 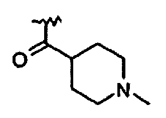 |
| A11 | 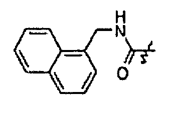 | B11 | 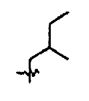 | C11 | 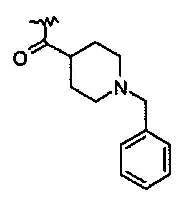 |
| A12 | 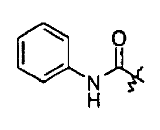 | B12 | 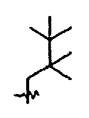 | C12 | 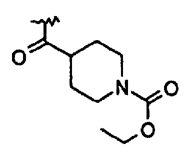 |
| A13 | 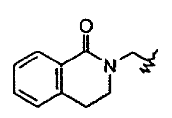 | B13 | 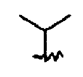 | C13 | 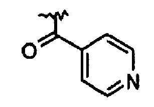 |
| A14 | 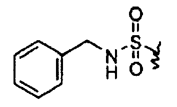 | B14 | 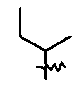 | C14 | 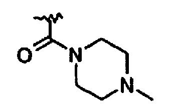 |
| A15 | 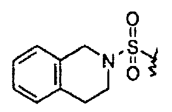 | B15 | 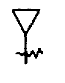 | C15 | 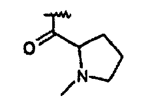 |
| A16 | 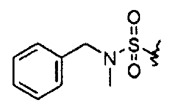 | B16 | 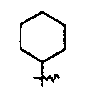 | C16 | 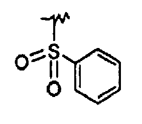 |
| A17 | 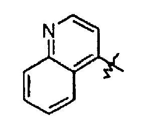 | B17 | 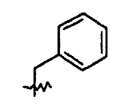 | C17 | 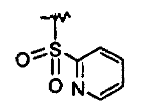 |
| A18 | 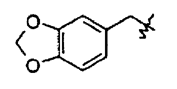 | B18 | 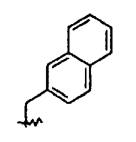 | C18 | -CH₃ |
| A19 | 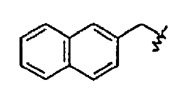 | B19 | 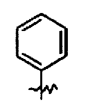 | C19 | 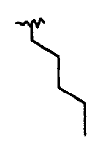 |
| A20 | 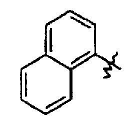 | B20 | 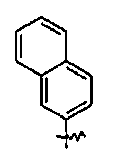 | C20 | 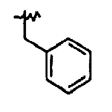 |
| A21 | 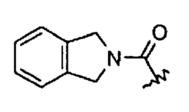 | B21 | 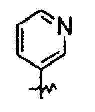 | C21 | 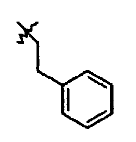 |
| A22 | 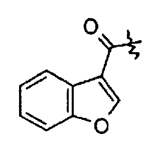 | B22 | 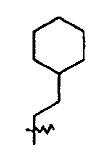 | C22 | 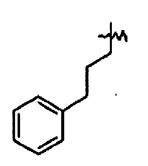 |
| A23 | 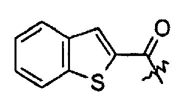 | B23 | 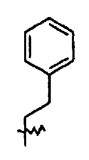 | C23 | 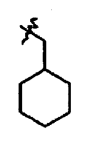 |
| A24 | 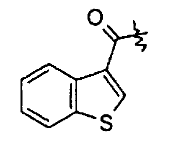 | B24 | 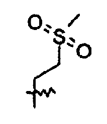 | C24 | 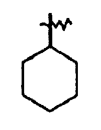 |
| A25 | 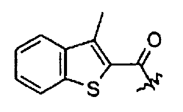 | B25 | 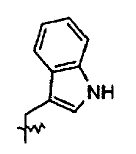 | C25 | 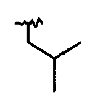 |
| A26 | 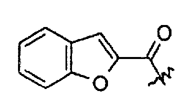 | B26 | 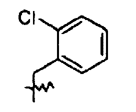 | C26 | 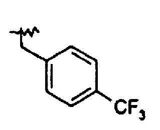 |
| A27 | 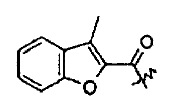 | | | C27 | 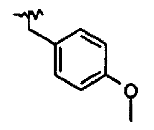 |
| A28 | 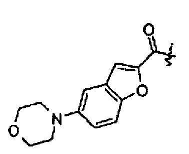 | | | C28 | 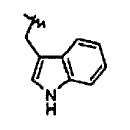 |
| A29 | 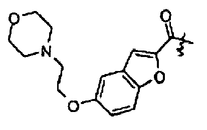 | | | C29 | 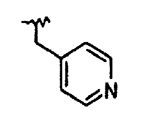 |
| A30 | 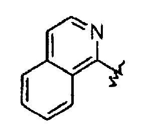 | | | C30 | 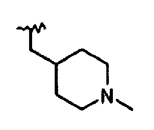 |
| A31 | 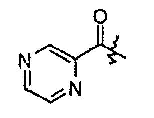 | | | C31 | 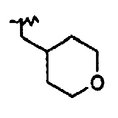 |
| A32 | 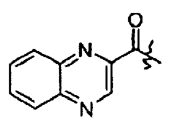 | | | C32 | 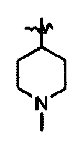 |
| A33 | 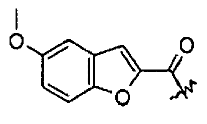 | | | C33 | 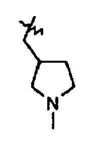 |
| A34 | 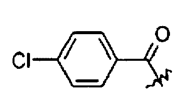 | | | C34 | 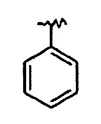 |
| | | | | C35 | 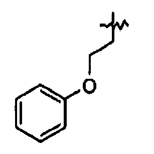 |
| | | | | C36 | 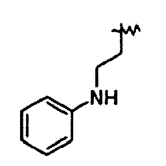 |
| | | | | C37 | 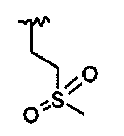 |
| | | | | C38 | 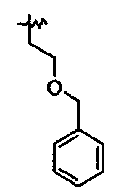 |
| | | | | C39 | 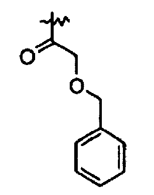 |
| | | | | C40 | 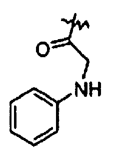 |
| | | | | C41 | 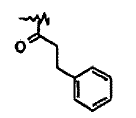 |
ou un sel, solvate ou tautomère pharmaceutiquement acceptable de celui-ci.

7. Composé selon la revendication 6, dans lequel ;
R₁ est choisi parmi les groupements suivants : 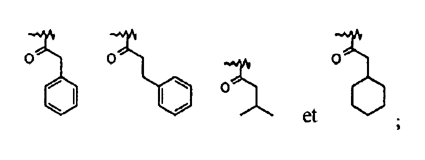 R₄ est choisi parmi les groupements suivants : 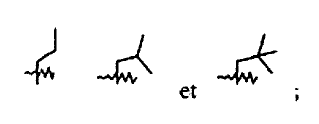 et Q est choisi parmi les groupements suivants : 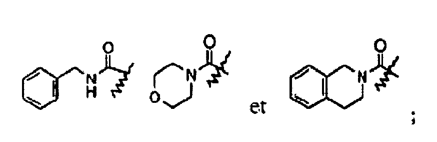 ou un sel, solvate ou tautomère pharmaceutiquement acceptable de celui-ci.

8. Composé selon la revendication 1, choisi parmi les composés suivants :

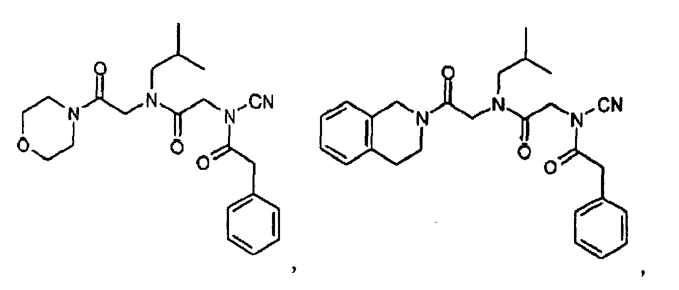

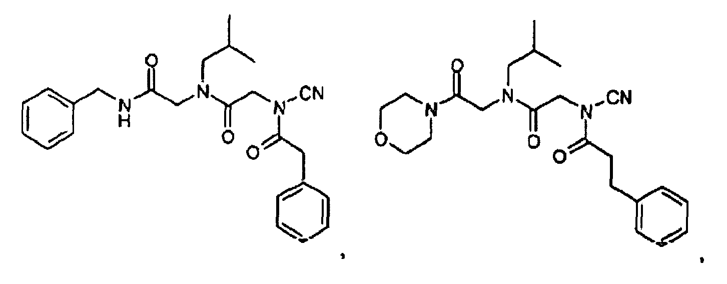

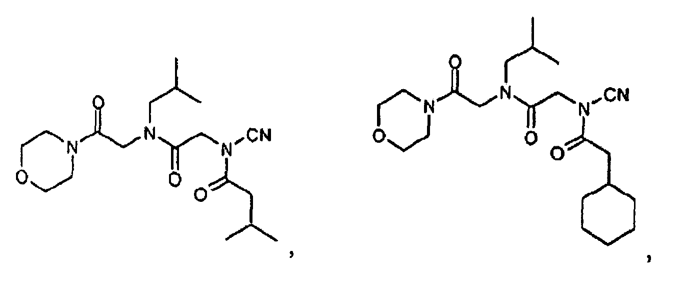

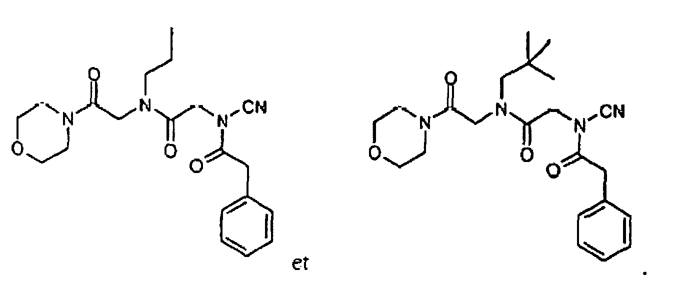

9. Procédé de préparation d'un composé de formule (I) selon la revendication 1, ledit procédé comprenant la mise en réaction d'un composé de formule V avec un cyanamide de formule VIII en présence d'une base appropriée pour obtenir un composé de formule (I) : 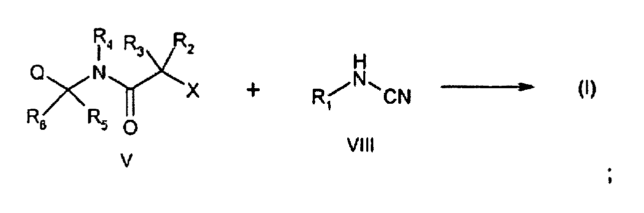 R₁, R₂, R₃, R₄, R₅, R₆ et Q étant tels que définis dans la revendication 1 et X étant un groupe partant.

10. Procédé selon la revendication 9, dans lequel le composé de formule (V) est préparé par un procédé comprenant :
la mise en réaction du composé de formule (III) avec un composé de formule (IV), dans lequel X est un groupe partant, pour obtenir un composé de formule (V) : 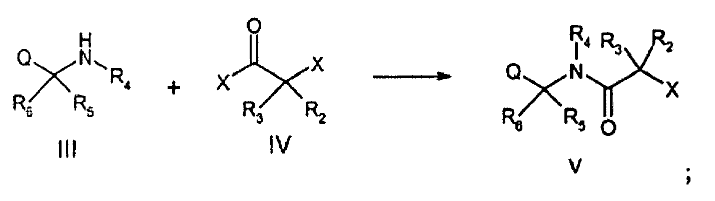 R₂, R₃, R₄, R₅, R₆ et Q étant tels que définis dans la revendication 9.

11. Procédé selon la revendication 10, dans lequel le composé de formule III est préparé par un procédé comprenant :
(a) la mise en réaction d'un composé de formule II, dans lequel X est un groupe partant, avec un composé de formule H₂N-R₄ pour obtenir un composé de formule III : 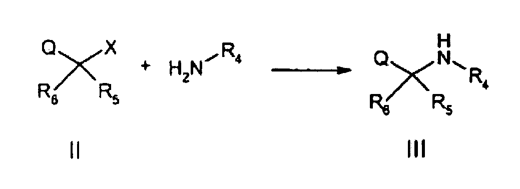 R₄, R₅, R₆ et Q étant tels que définis dans la revendication 10.
ou
b) la mise en réaction d'un composé de formule IX avec un aldéhyde de formule R'CHO dans des conditions d'amination réductrice pour obtenir un composé de formule III : 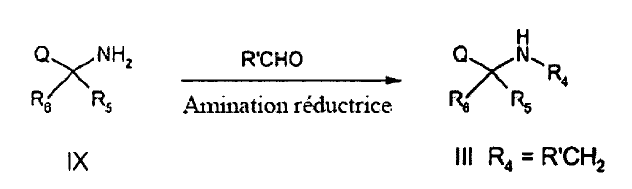 R₅, R₆ et Q étant tels que définis dans la revendication 10, et R₄ étant choisi parmi les groupements R₄ tels que définis dans la revendication 10 ayant un groupement -CH₂- lié à l'atome d'azote dans le squelette de la formule (III), et R' étant la partie terminale dudit groupement R₄ lié audit groupement -CH₂-.

12. Procédé de préparation d'un composé de formule (I) selon la revendication 1, ledit procédé comprenant la mise en réaction d'un composé de formule (XIII) avec un composé R₁X, dans lequel X est un groupe partant, en présence d'une base appropriée pour obtenir un composé de formule (I) : 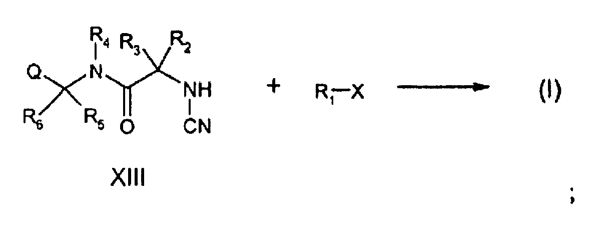 R₁, R₂, R₃, R₄, R₅, R₆ et Q étant tels que définis dans la revendication 1.

13. Procédé selon la revendication 12, dans lequel le composé de formule (XIII) est préparé par un procédé comprenant :
(a) la mise en réaction d'un composé de formule (III) avec un composé de formule (X), dans lequel PG est un groupe protecteur et X est un groupe partant, pour obtenir un composé de formule (XI) : 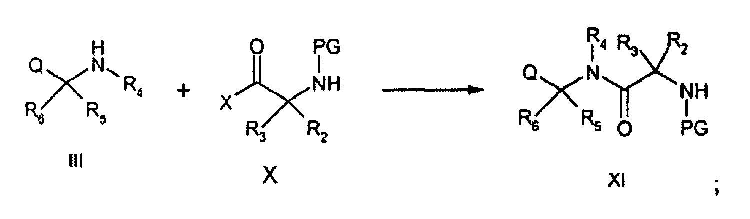
(b) la déprotection d'un composé de formule (XI) pour obtenir un composé de formule (XII) : 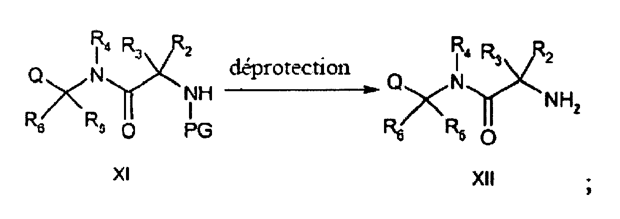
(c) la mise en réaction d'un composé de formule (XII) avec du bromure de cyanogène pour obtenir un composé de formule (XIII) : 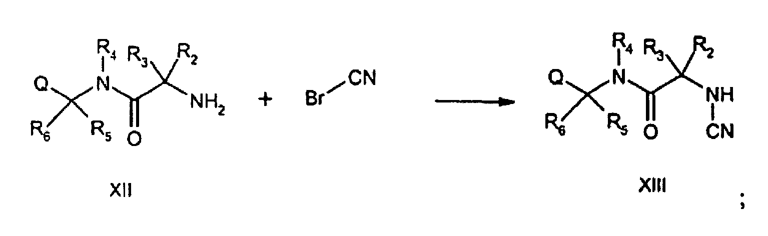 R₂, R₃, R₄, R₅, R₆ et Q étant tels que définis dans la revendication 12.

14. Procédé selon la revendication 13, dans lequel le composé de formule III est préparé par un procédé comprenant :
(a) la mise en réaction d'un composé de formule II, dans lequel X est un groupe partant, avec un composé de formule H₂N-R₄ pour obtenir un composé de formule III : 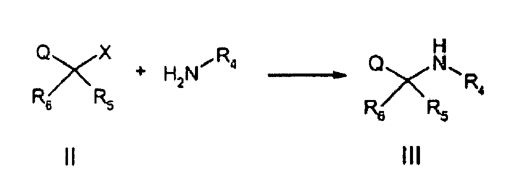 R₄, R₅, R₆ et Q étant tels que définis dans la revendication 13 ;
ou
(b) la mise en réaction d'un composé de formule IX avec un aldéhyde de formule R'CHO dans des conditions d'amination réductrice pour obtenir un composé de formule III ; 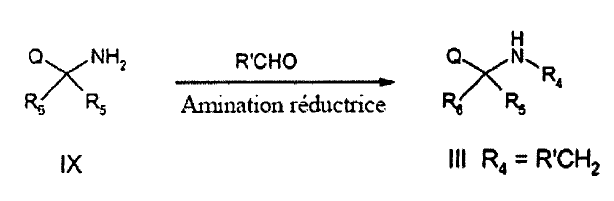
R₅, R₆ et Q étant tels que définis dans la revendication 13, et R₄ étant choisi parmi les groupements R₄ tels que définis dans la revendication 13 ayant un groupement -CH₂- lié à l'atome d'azote dans le squelette de la formule (III), et R' étant la partie terminale dudit groupement R₄ lié audit groupement -CH₂-.

15. Composition pharmaceutique comprenant une quantité thérapeutiquement efficace d'un composé selon l'une quelconque des revendications 1 à 8 ou d'un sel, solvate ou tautomère pharmaceutiquement acceptable de celui-ci.

16. Composé selon l'une quelconque des revendications 1 à 8 destiné à une utilisation en tant que médicament.

17. Utilisation d'un composé selon l'une quelconque des revendications 1 à 8 pour la préparation d'une composition pharmaceutique destinée au traitement d'une maladie induite par la cathépsine S.

18. Utilisation d'un composé selon l'une quelconque des revendications 1 à 8 pour la préparation d'une composition pharmaceutique destinée au traitement d'une maladie induite par la cathépsine K.
